(19) Europäisches Patentamt
European Patent Office
Office européen des brevets

(11) EP 4 752 142 A1

(12) EUROPEAN PATENT APPLICATION
published in accordance with Art. 153(4) EPC

(43) Date of publication:
**03.06.2026 Bulletin 2026/23**

(21) Application number: **24844811.0**

(22) Date of filing: **24.07.2024**

(51) International Patent Classification (IPC):
***C07D 403/04*** (2006.01) ***C07D 403/14*** (2006.01)
***A61K 31/41*** (2006.01) ***A61P 25/00*** (2006.01)

(52) Cooperative Patent Classification (CPC):
**A61K 31/41; A61P 25/00; C07D 403/04; C07D 403/14**

(86) International application number:
**PCT/CN2024/107334**

(87) International publication number:
**WO 2025/021120 (30.01.2025 Gazette 2025/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.07.2023 CN 202310907997**
**26.09.2023 CN 202311247946**
**08.03.2024 CN 202410265433**

(71) Applicant: **Innovstone Therapeutics Limited**
**Shanghai 201203 (CN)**

(72) Inventors:
- **SONG, Yunlong**
  **Shanghai 201203 (CN)**
- **FU, Yiwei**
  **Shanghai 201203 (CN)**
- **MU, Yongzhao**
  **Shanghai 201203 (CN)**
- **LU, Kai**
  **Shanghai 201203 (CN)**
- **WANG, Disha**
  **Shanghai 201203 (CN)**
- **KOU, Hongyan**
  **Shanghai 201203 (CN)**
- **WANG, Feng**
  **Shanghai 201203 (CN)**
- **CHENG, Qianyi**
  **Shanghai 201203 (CN)**

(74) Representative: **Barrow, Nicholas Martin et al**
**Venner Shipley LLP**
**406 Cambridge Science Park**
**Milton Road**
**Cambridge CB4 0WW (GB)**

# (54) 5-HT2A RECEPTOR AGONIST, AND PREPARATION METHOD THEREFOR AND USE THEREOF

(57) Provided are a compound having a new structure as a 5-$HT_{2A}$ receptor agonist, a preparation method therefor, and the use thereof in the treatment of diseases related to the 5-$HT_{2A}$ receptor. Experiments have confirmed that the compounds have a strong affinity on the 5-$HT_{2A}$ receptor, and can be used as a promising compound for treating diseases related to the 5-$HT_{2A}$ receptor.

EP 4 752 142 A1

## Description

## Technical Field

The present invention relates to a class of compounds serving as 5-hydroxytryptamine 2A (5-$HT_{2A}$) receptor agonists, preparation methods therefor, and use thereof in treating diseases associated with the 5-$HT_{2A}$ receptor.

## Background Technology

Depression is a type of mental disorder characterized primarily by significant and persistent low mood due to various causes, and it is also one of the most common mental illnesses associated with suicide. World Health Organization data indicated that in 2015, there were over 320 million people worldwide suffering from depression. The point prevalence rate of depression in China is 3.02%, and currently, there are over 40 million individuals with depression.

5-hydroxytryptamine receptors, also known as serotonin receptors or 5-HT receptors, are a group of G protein-coupled receptor and ligand-gated ion channels found in the central nervous system and the peripheral nervous system. They regulate the transmission of both excitatory and inhibitory neurotransmitters.

The 5-$HT_{2A}$ receptor, belonging to the 5-HT receptor family, is widely expressed throughout the central nervous system (CNS). It is abundantly expressed near most serotonergic terminal regions, including the neocortex (primarily prefrontal, parietal, and somatosensory cortices) and the olfactory tubercle.

The 5-$HT_{2A}$ receptor is known to be primarily coupled to the Gαq signal transduction pathway. Upon agonist stimulation of the receptor, Gαq and β-γ subunits dissociate to initiate downstream effector pathways. Gαq stimulates phospholipase C (PLC) activity, subsequently promoting the release of diacylglycerol (DAG) and inositol trisphosphate (IP3), which in turn stimulates protein kinase C (PKC) activity and $Ca^{2+}$ release.

Besides the classical G protein signaling pathway, the β-arrestin-dependent signaling pathway is also a recognized signal transduction pathway for GPCRs. After GPCR activation, a family of protein kinases called G protein-coupled receptor kinases (GRKs) phosphorylate the intracellular domains following G-protein release. Phosphorylated GPCRs recruit β-arrestin proteins, which mediate the desensitization of the GPCR signaling and the internalization of GPCRs, thereby "switching off" the signaling, leading to negative feedback on G protein-dependent GPCR signaling.

Currently, psilocybin, in phase III clinical trials, exhibits rapid, significant, and sustained antidepressant effects. However, psilocybin has a strong hallucinogenic effect.

Therefore, there is an urgent need to develop novel 5-$HT_{2A}$ receptor agonists with antidepressant effects but without hallucinogenic effects, particularly small molecule 5-$HT_{2A}$ receptor partial agonists.

## Summary of the Invention

The present invention provides compounds possessing 5-$HT_{2A}$ receptor agonistic activity, pharmaceutical compositions containing said compounds for treating central nervous system disorders, and further provides therapeutic uses for central nervous system disorders.

A first aspect of the present invention provides a compound represented by the following formula (I), or a stereoisomer thereof, a pharmaceutically acceptable salt thereof, or a mixture thereof:

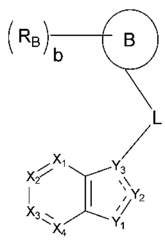

(I)

wherein,

- - - represents a single bond or a double bond, with one and only one being a double bond;
$X_1$ is N, or $CR_{X1}$; $X_2$ is N, or $CR_{X2}$; $X_3$ is N, or $CR_{X3}$; $X_4$ is N, or $CR_{X4}$;
and $X_1$, $X_2$, $X_3$, and $X_4$ are not N at the same time;
$Y_1$ is selected from O, S, $NR_{Y11}$, and $CR_{Y12}$;

$Y_2$ is N, or $CR_{Y22}$;
$Y_3$ is N, or C;
and when $Y_3$ is N, a single bond is present between $Y_3$ and $Y_2$; when $Y_3$ is C, a double bond is present between $Y_3$ and $Y_2$;
$R_{X1}$ is selected from hydrogen, deuterium, halogen, -OH, -CN, -$NH_2$, and the following groups that are optionally substituted: $C_{1}$-$_{4}$alkyl, $C_{1}$-$_{4}$oxaalkyl, and $C_{1}$-$_{4}$thiaalkyl; wherein optionally substituted means being unsubstituted or being substituted by one or more substituents selected from deuterium, halogen, oxo, -CN, -OH, and -$NH_2$;
$R_{X2}$ is selected from hydrogen, deuterium, halogen, -OH, -CN, -$NH_2$, and the following groups that are optionally substituted: $C_{1}$-$_{4}$alkyl, $C_{1}$-$_{4}$oxaalkyl, and $C_{1}$-$_{4}$thiaalkyl; wherein optionally substituted means being unsubstituted or being substituted by one or more substituents selected from deuterium, halogen, oxo, -CN, -OH, and -$NH_2$;
$R_{X3}$ is selected from hydrogen, deuterium, halogen, -OH, -CN, -$NH_2$, and the following groups that are optionally substituted: $C_{1}$-$_{4}$alkyl, $C_{1}$-$_{4}$oxaalkyl, and $C_{1}$-$_{4}$thiaalkyl; wherein optionally substituted means being unsubstituted or being substituted by one or more substituents selected from deuterium, halogen, oxo, -CN, -OH, and -$NH_2$;
$R_{X4}$ is selected from hydrogen, deuterium, halogen, and optionally substituted $C_{1}$-$_{4}$alkyl; wherein optionally substituted means being unsubstituted or being substituted by one or more substituents selected from deuterium, halogen, oxo, -CN, -OH, and -$NH_2$;
$R_{Y11}$ is selected from hydrogen, deuterium, and optionally substituted $C_{1-3}$alkyl, wherein optionally substituted means being unsubstituted or being substituted by one or more substituents selected from deuterium, halogen, oxo, -CN, -OH, and -$NH_2$;
$R_{Y12}$ is selected from hydrogen, deuterium, and optionally substituted $C_{1-3}$alkyl, wherein optionally substituted means being unsubstituted or being substituted by one or more substituents selected from deuterium, halogen, oxo, -CN, -OH, and -$NH_2$;
$R_{Y22}$ is selected from hydrogen, deuterium, halogen, and optionally substituted $C_{1-3}$alkyl, wherein optionally substituted means being unsubstituted or being substituted by one or more substituents selected from deuterium, halogen, oxo, -CN, -OH, and -$NH_2$;
L is -Z-L'-, -L'-Z-, or -L'-Z-L'-;
L' is selected from the following groups that are optionally substituted: -$C_{1}$-$_{4}$alkylene-, -$C_{1}$-$_{4}$oxaalkylene-, -$C_{1}$-$_{4}$thiaalkylene-, and -$C_{1}$-$_{4}$azaalkylene-, wherein optionally substituted means being unsubstituted or being substituted by one or more substituents selected from deuterium, halogen, -CN, -OH, -$NH_2$, $C_{1-3}$alkyl, $C_{1-3}$alkoxy, $C_{1-3}$haloalkyl, and $C_{1-3}$haloalkoxy;
Z is selected from the following groups that are optionally substituted by $R_A$ group(s) in an amount of a: 4-12 membered heterocyclyl, and 4-12 membered heterocycloalkenyl; wherein each $R_A$ is independently selected from deuterium, halogen, -OH, -CN, -$NH_2$, $C_{1-3}$alkyl, $C_{1-3}$alkoxy, $C_{1-3}$haloalkyl, and $C_{1-3}$haloalkoxy;
ring B is $C_{6-14}$aryl, 5-16 membered heterocyclyl, or 5-16 membered heteroaryl;
each $R_B$ is independently selected from deuterium, halogen, -OH, -CN, -$NH_2$, oxo, and the following groups that are optionally substituted: $C_{1}$-$_{4}$alkyl, $C_{2}$-$_{6}$alkenyl, $C_{2}$-$_{6}$alkynyl, $C_{1}$-$_{4}$oxaalkyl, $C_{1}$-$_{4}$thiaalkyl, $C_{3}$-$_{6}$cycloalkyl, $C_{3}$-$_{6}$cycloalkenyl, wherein optionally substituted means being unsubstituted or being substituted by one or more substituents selected from deuterium, halogen, oxo, -CN, -OH, -$NH_2$, $C_{1}$-$_{4}$alkyl, and $C_{1}$-$_{4}$haloalkyl;
a is 0, 1, 2, or 3;
b is selected from 0, 1, 2, 3, and 4;
Unless otherwise specified, the heteroatoms in the above heterocyclyl and heteroaryl are independently selected from O, N and S, with the number of heteroatoms being 1, 2, 3, or 4; provided that Z is neither

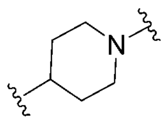 nor 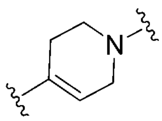 ;

and when $Y_1$ is NH, and $X_4$ is N, L is not

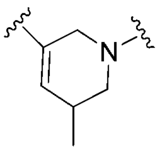 .

In a preferable embodiment of the present invention, $X_1$, $X_2$, $X_3$, $X_4$ are all not N;
In a preferable embodiment of the present invention, $X_1$ is N, $X_2$ is $CR_{X2}$, $X_3$ is $CR_{X3}$, $X_4$ is $CR_{X4}$;

In a preferable embodiment of the present invention, $X_2$ is N, $X_1$ is $CR_{X1}$, $X_3$ is $CR_{X3}$, $X_4$ is $CR_{X4}$;
In a preferable embodiment of the present invention, $X_3$ is N, $X_1$ is $CR_{X1}$, $X_2$ is $CR_{X2}$, $X_4$ is $CR_{X4}$;
In a preferable embodiment of the present invention, $X_4$ is N, $X_1$ is $CR_{X1}$, $X_2$ is $CR_{X2}$, $X_3$ is $CR_{X3}$;
In a preferable embodiment of the present invention, $X_2$ and $X_4$ are N, $X_1$ is $CR_{X1}$, $X_3$ is $CR_{X3}$;
In a preferable embodiment of the present invention, $X_1$ and $X_3$ are N, $X_2$ is $CR_{X2}$, $X_4$ is $CR_{X4}$;
In a preferable embodiment of the present invention, $Y_1$ is $CR_{Y12}$, $Y_2$ is $CR_{Y22}$, $Y_3$ is N;
In a preferable embodiment of the present invention, $Y_1$ is $NR_{Y11}$, $Y_2$ is N or $CR_{Y22}$, $Y_3$ is C;
In a preferable embodiment of the present invention, $Y_1$ is $NR_{Y11}$, $Y_2$ is $CR_{Y22}$, $Y_3$ is C;
In a preferable embodiment of the present invention, $Y_1$ is O, $Y_2$ is N, $Y_3$ is C;
In a preferable embodiment of the present invention, $R_{X1}$ is selected from hydrogen, deuterium, halogen, -OH, -CN, $-NH_2$, and the following groups that are optionally substituted: $C_{1-4}$oxaalkyl, and $C_{1-4}$thiaalkyl; wherein optionally substituted means being unsubstituted or being substituted by one or more substituents selected from deuterium, halogen, oxo, -CN, -OH, and $-NH_2$;
In a preferable embodiment of the present invention, $R_{X1}$ is selected from hydrogen, deuterium, -F, -Cl, -Br, -OH, -CN, $-NH_2$, and the following groups that are optionally substituted: methoxy, ethoxy, and methylthio; wherein optionally substituted means being unsubstituted or being substituted by one or more substituents selected from deuterium, halogen, oxo, -CN, -OH, and $-NH_2$;
In a preferable embodiment of the present invention, $R_{X1}$ is selected from hydrogen, -F, -OH, and - $OCH_3$.
In a preferable embodiment of the present invention, $R_{X2}$ is selected from hydrogen, deuterium, halogen, -OH, -CN, $-NH_2$, and the following groups that are optionally substituted: $C_{1-4}$oxaalkyl, and $C_{1-4}$thiaalkyl; wherein optionally substituted means being unsubstituted or being substituted by one or more substituents selected from deuterium, halogen, oxo, -CN, -OH, and $-NH_2$;
In a preferable embodiment of the present invention, $R_{X2}$ is selected from hydrogen, deuterium, -F, -Cl, -Br, -OH, -CN, $-NH_2$, and the following groups that are optionally substituted: methoxy, ethoxy, and methylthio; wherein optionally substituted means being unsubstituted or being substituted by one or more substituents selected from deuterium, halogen, oxo, -CN, -OH, and $-NH_2$;
In a preferable embodiment of the present invention, $R_{X2}$ is selected from hydrogen, -F, and $-OCH_3$;
In a preferable embodiment of the present invention, $R_{X2}$ is hydrogen.
In a preferable embodiment of the present invention, $R_{X3}$ is selected from hydrogen, deuterium, halogen, -OH, -CN, $-NH_2$, and the following groups that are optionally substituted: $C_{1-4}$oxaalkyl, and $C_{1-4}$thiaalkyl; wherein optionally substituted means being unsubstituted or being substituted by one or more substituents selected from deuterium, halogen, oxo, -CN, -OH, and $-NH_2$;
In a preferable embodiment of the present invention, $R_{X3}$ is selected from hydrogen, deuterium, -F, -Cl, -Br, -OH, -CN, $-NH_2$, and the following groups that are optionally substituted: methoxy, ethoxy, and methylthio; wherein optionally substituted means being unsubstituted or being substituted by one or more substituents selected from deuterium, halogen, oxo, -CN, -OH, and $-NH_2$;
In a preferable embodiment of the present invention, $R_{X3}$ is selected from hydrogen, -F, and $-OCH_3$;
In a preferable embodiment of the present invention, $R_{X3}$ is hydrogen.
In a preferable embodiment of the present invention, $R_{X4}$ is selected from hydrogen, deuterium, halogen, and the following groups that are optionally substituted: methyl, and ethyl; wherein optionally substituted means being unsubstituted or being substituted by one or more substituents selected from deuterium, halogen, oxo, -CN, -OH, and $-NH_2$;
In a preferable embodiment of the present invention, $R_{X4}$ is selected from hydrogen, deuterium, -F, -Cl, and -Br;
In a preferable embodiment of the present invention, $R_{X4}$ is hydrogen.
In a preferable embodiment of the present invention, $R_{Y11}$ is selected from hydrogen, deuterium, and the following groups that are optionally substituted: methyl, and ethyl, wherein optionally substituted means being unsubstituted or being substituted by one or more substituents selected from deuterium, halogen, oxo, -CN, -OH, and $-NH_2$;
In a preferable embodiment of the present invention, $R_{Y11}$ is selected from hydrogen and methyl;
In a preferable embodiment of the present invention, $R_{Y11}$ is hydrogen.
In a preferable embodiment of the present invention, $R_{Y22}$ is selected from hydrogen, deuterium, -F, - Cl, -Br, and the following groups that are optionally substituted: methyl, and ethyl, wherein optionally substituted means being unsubstituted or being substituted by one or more substituents selected from deuterium, halogen, oxo, -CN, -OH, and $-NH_2$;
In a preferable embodiment of the present invention, $R_{Y22}$ is selected from hydrogen, -Br, and methyl;
In a preferable embodiment of the present invention, $R_{Y22}$ is hydrogen.

In a preferable embodiment of the present invention, L' is selected from the following groups that are optionally substituted: $-C_{1-4}$alkylene-, and $-C_{1-4}$oxaalkylene-; wherein optionally substituted means being unsubstituted or being

substituted by one or more substituents selected from deuterium, halogen, -CN, -OH, $-NH_2$, $C_{1-3}$alkyl, $C_{1-3}$alkoxy, and $C_{1-3}$haloalkyl;

In a preferable embodiment of the present invention, L' is selected from the following groups that are optionally substituted: $-C_{1-3}$alkylene-, $-C_{1-3}$oxaalkylene-; wherein optionally substituted means being unsubstituted or being substituted by one or more substituents selected from deuterium, halogen, $C_{1-3}$alkyl, $C_{1-3}$alkoxy, and $C_{1-3}$haloalkyl;

In a preferable embodiment of the present invention, L' is selected from the following groups that are optionally substituted: $-C_{1-3}$alkylene-, $-C_{1-3}$oxaalkylene-; wherein optionally substituted means being unsubstituted or being substituted by one or more substituents selected from deuterium, F, Cl, methyl, methoxy, and $-CF_3$;

In a preferable embodiment of the present invention, L' is

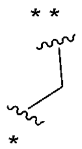, 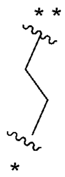, 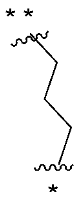, 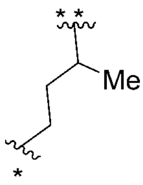,

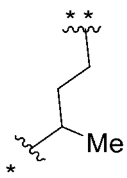, 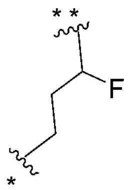, 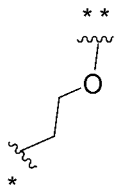;

** represents the attachment point to ring B; * represents the attachment point to ring Z;

In a preferable embodiment of the present invention, Z is selected from the following groups that are optionally substituted by $R_A$ group(s) in an amount of a: 4-10 membered heterocyclyl, and 4-10 membered heterocycloalkenyl; wherein $R_A$ is selected from deuterium, halogen, -CN, $-NH_2$, $C_{1-3}$alkyl, $C_{1-3}$alkoxy, halo$C_{1-3}$alkyl, and halo$C_{1-3}$alkoxy;

In a preferable embodiment of the present invention, Z is selected from the following groups that are optionally substituted by $R_A$ group(s) in an amount of a: 4-10 membered heterocyclyl, and 4-10 membered heterocycloalkenyl; wherein $R_A$ is selected from deuterium, halogen, $C_{1-3}$alkyl, $C_{1-3}$alkoxy, halo$C_{1-3}$alkyl, and halo$C_{1-3}$alkoxy;

In a preferable embodiment of the present invention, Z is selected from the following groups that are optionally substituted by $R_A$ group(s) in an amount of a: 4-8 membered heterocyclyl, 4-8 membered heterocycloalkenyl; wherein $R_A$ is selected from deuterium, F, Cl, methyl, ethyl, methoxy, $-CH_2F$, $-CHF_2$, and $-CF_3$;

In a preferable embodiment of the present invention, Z is selected from

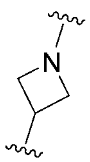, 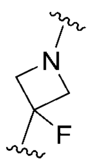, 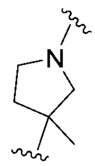,

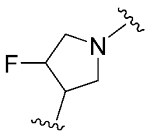, 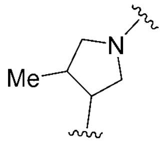, 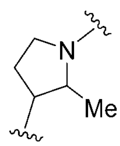, 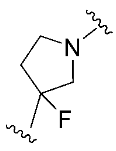, 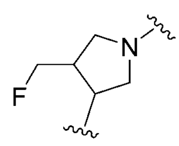, 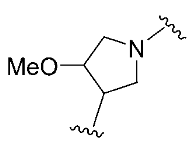, 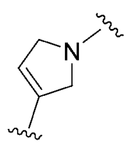,

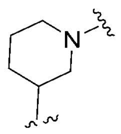, 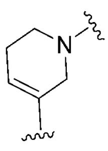, 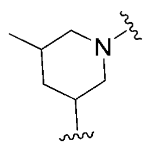, 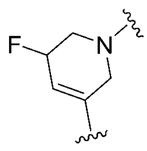, 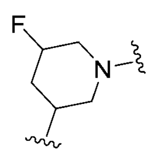, 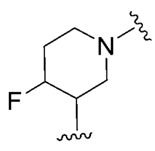, 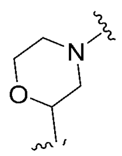,

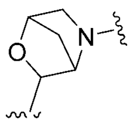 , 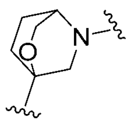 , 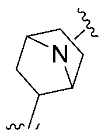 , 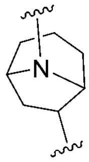 , 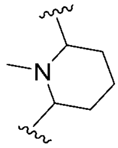 , 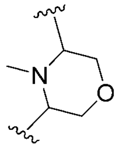 , 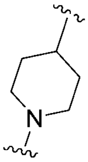 , 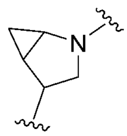 ,

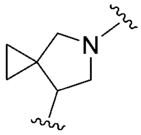 , 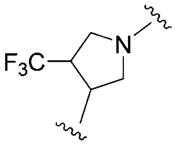 , 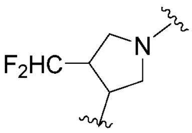 , 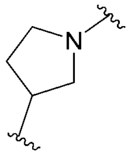 , 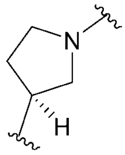 , 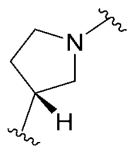 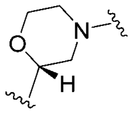 , and

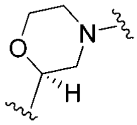 ;

In a preferable embodiment of the present invention, Z is selected from the following groups that are optionally substituted by $R_A$ group(s) in an amount of a:

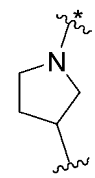 , 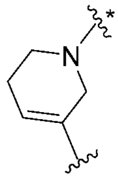 ;

* represents the attachment point to L';
In a preferable embodiment of the present invention, L is selected from

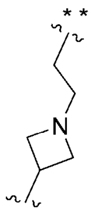 , 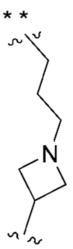 , 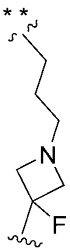 , 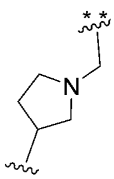 , 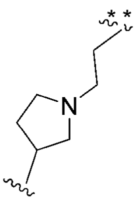 , 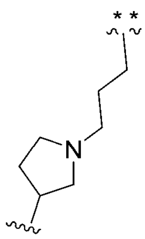 , 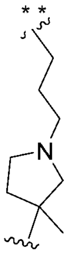 , 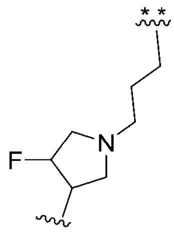 ,

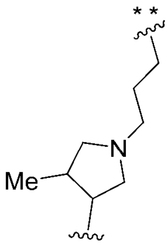 , 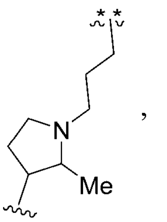 , 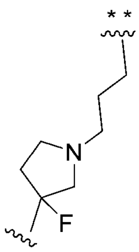 , 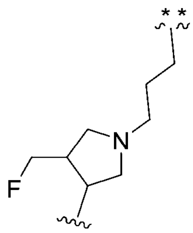 , 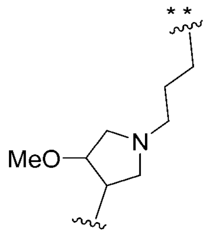 , 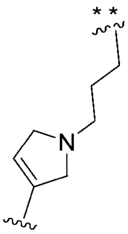 ,

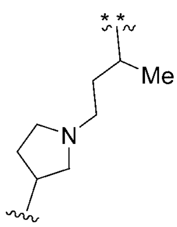 , 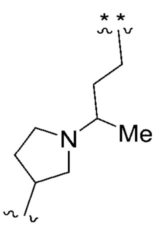 , 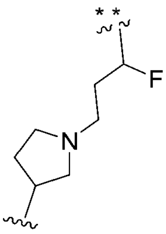 , 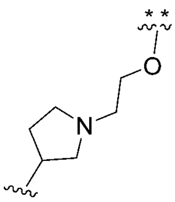 , 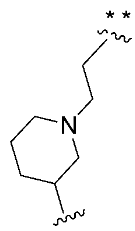 , 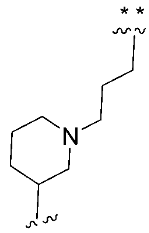 ,

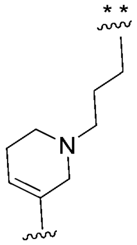, 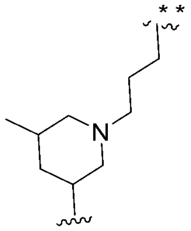, 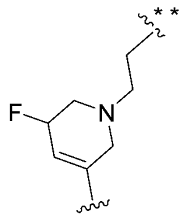, 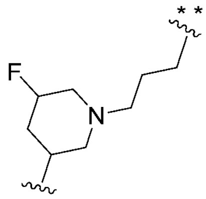, 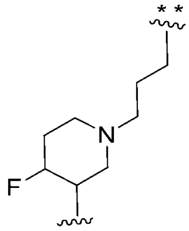, 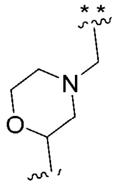,

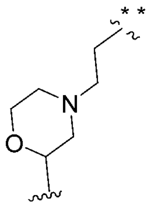, 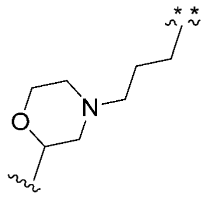, 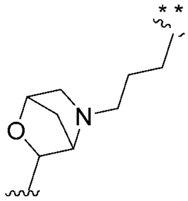, 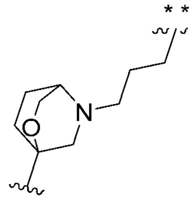, 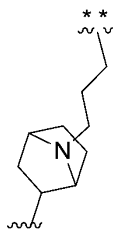, 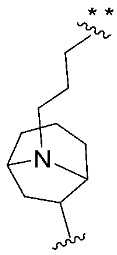,

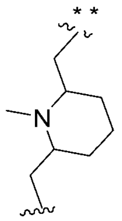, 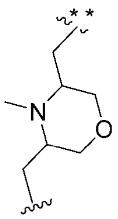, 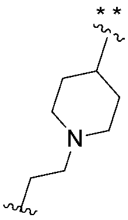, 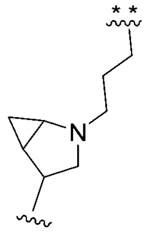, 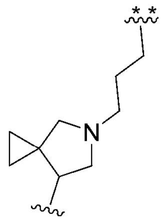, 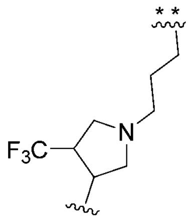,

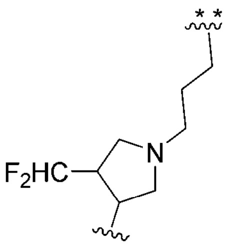, 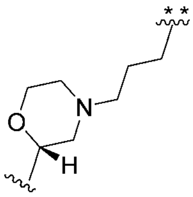, 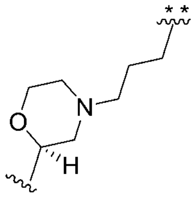, 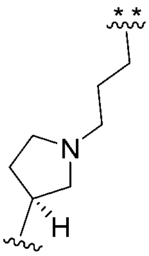, and 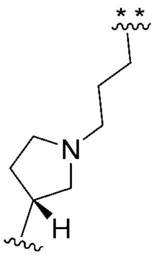;

** represents the attachment point to ring B.

In a preferable embodiment of the present invention, ring B is phenyl, 5-12 membered heterocyclyl, 5-12 membered heteroaryl;

In a preferable embodiment of the present invention, ring B is phenyl, 5-6 membered monocyclic heteroaryl, 8-12 membered bicyclic heterocyclyl, or 8-12 membered bicyclic heteroaryl;

In a preferable embodiment of the present invention, ring B is phenyl, 5-6 membered monocyclic heteroaryl, 8-10 membered bicyclic heterocyclyl, or 8-10 membered bicyclic heteroaryl;

In a preferable embodiment of the present invention, ring B is phenyl, or 5-membered monocyclic heteroaryl;

In a preferable embodiment of the present invention, ring B is

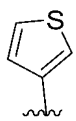, 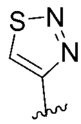, 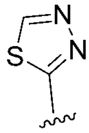, 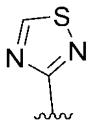, 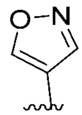,

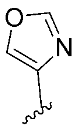, 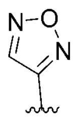, 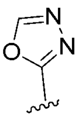, 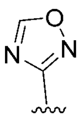, 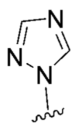, 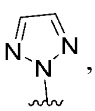, 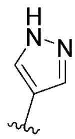, 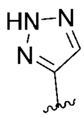, 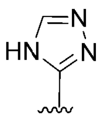, 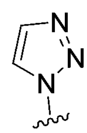, 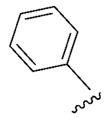,

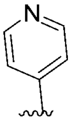, 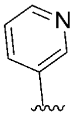, 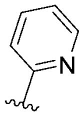, 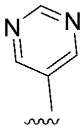, 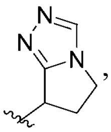, 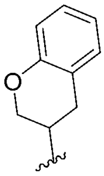, 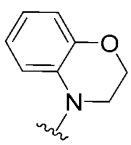, 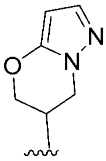, 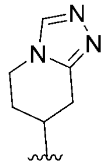, 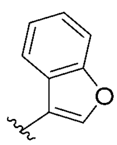,

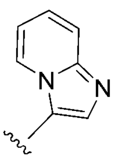, 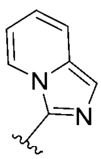, 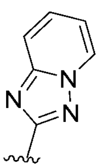, 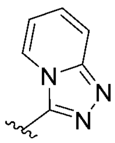, 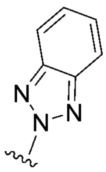, 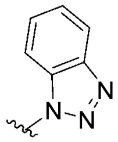, 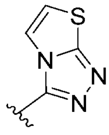, 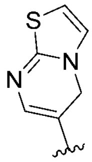, 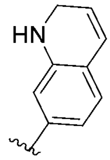, or

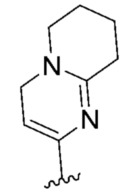;

In a preferable embodiment of the present invention, ring B is

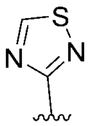, 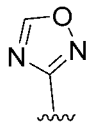, 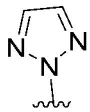, 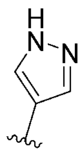,

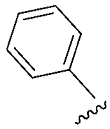, 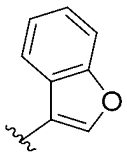, or 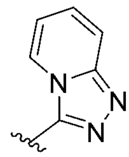;

In a preferable embodiment of the present invention, ring B is

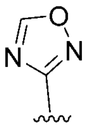, or 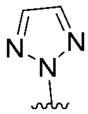;

In a preferable embodiment of the present invention, each $R_B$ is independently selected from deuterium, halogen, -OH, -CN, -$NH_2$, oxo, and the following groups that are optionally substituted: $C_1$-$_4$alkyl, $C_1$-$_4$oxaalkyl, $C_1$-$_4$thiaalkyl, and $C_3$-$_6$cycloalkyl, wherein optionally substituted means being unsubstituted or being substituted by one or more substituents selected from deuterium, halogen, oxo, -CN, -OH, - $NH_2$, $C_1$-$_4$alkyl, and $C_1$-$_4$haloalkyl;

In a preferable embodiment of the present invention, each $R_B$ is independently selected from deuterium, halogen, -CN, -$NH_2$, oxo, and the following groups that are optionally substituted: $C_{1-3}$alkyl, $C_{1-3}$oxaalkyl, and $C_3$-$_6$cycloalkyl, wherein optionally substituted means being unsubstituted or being substituted by one or more substituents selected from deuterium, halogen, oxo, -CN, -OH, -$NH_2$, $C_{1-3}$alkyl, $C_{1-3}$haloalkyl;

In a preferable embodiment of the present invention, each $R_B$ is independently selected from deuterium, F, Cl, Br, -CN, -$NH_2$, oxo, and the following groups that are optionally substituted: methyl, ethyl, propyl, isopropyl, methoxy, ethoxy, cyclopropyl, cyclobutyl, cyclopentyl, and cyclohexyl, wherein optionally substituted means being unsubstituted or being substituted by one or more substituents selected from deuterium, halogen, oxo, -CN, -OH, -$NH_2$, $C_{1-3}$alkyl, and $C_{1-3}$haloalkyl;

In a preferable embodiment of the present invention, each $R_B$ is independently selected from F, CN, oxo, methyl, cyclopropyl, cyclobutyl, methoxy, trifluoromethyl,

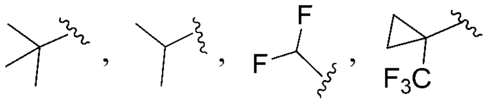

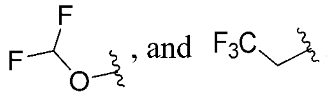

In a preferable embodiment of the present invention, b is 0, 1, 2, or 3;

In a preferable embodiment of the present invention, b is 0, 1, or 2;

In a preferable embodiment of the present invention, a is 0, 1, or 2;

In a preferable embodiment of the present invention, a is 0 or 1;

In a preferable embodiment of the present invention, a is 0;

In a preferable embodiment of the present invention,

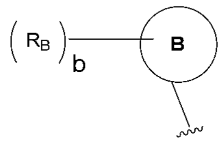

is selected from

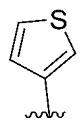 ,

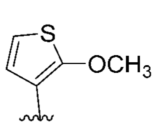 , 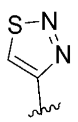 , 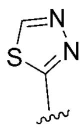 , 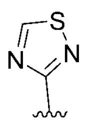 , 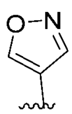 , 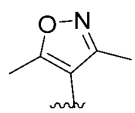 , 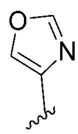 , 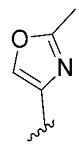 , 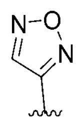 , 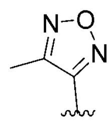 ,

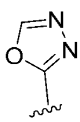 , 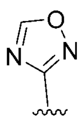 , 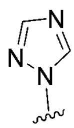 , 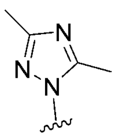 , 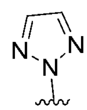 , 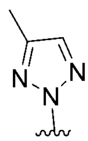 , 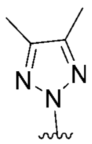 , 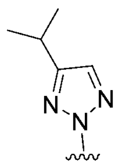 , 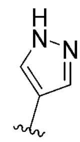 , 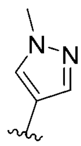 , 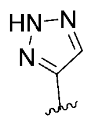 ,

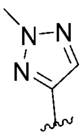 , 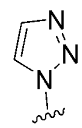 , 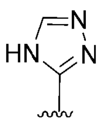 , 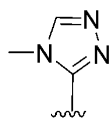 , 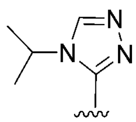 , 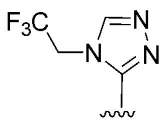 , 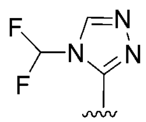 , 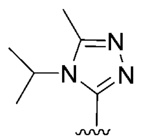 ,

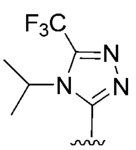 , 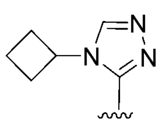 , 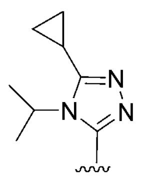 , 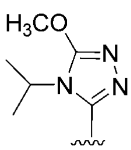 , 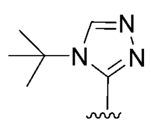 , 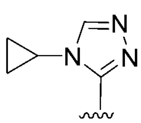 , 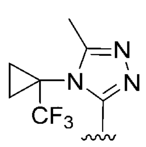 ,

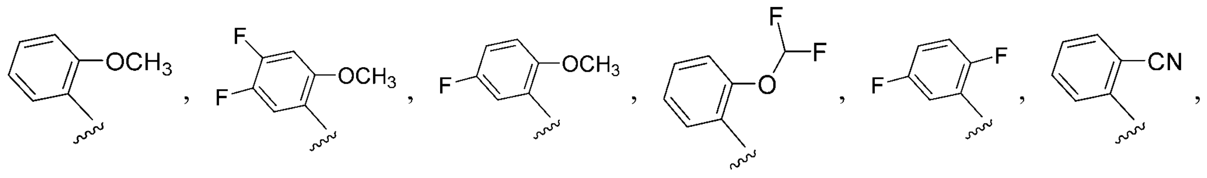
, , , , , ,

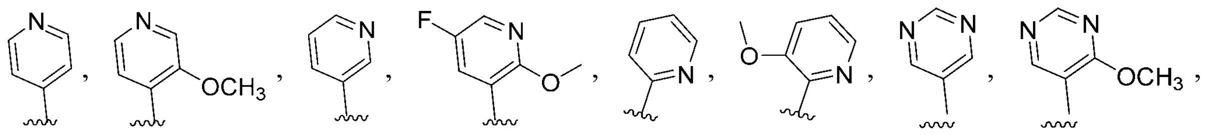
, , , , , , , ,

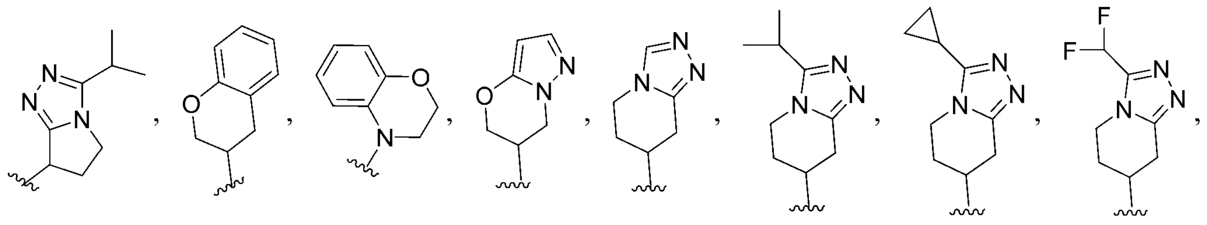
, , , , , , , ,

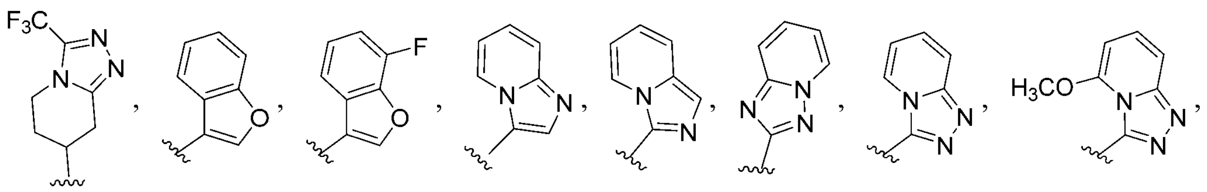
, , , , , , , ,

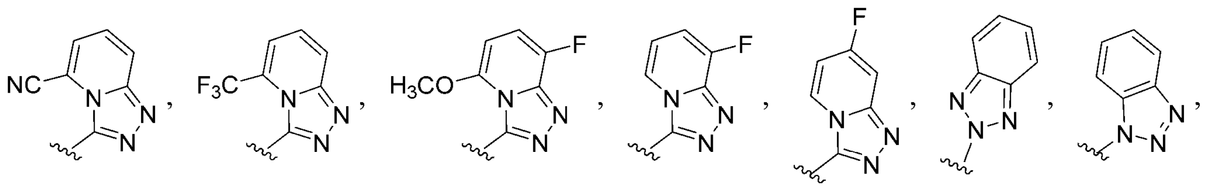
, , , , , , , ,

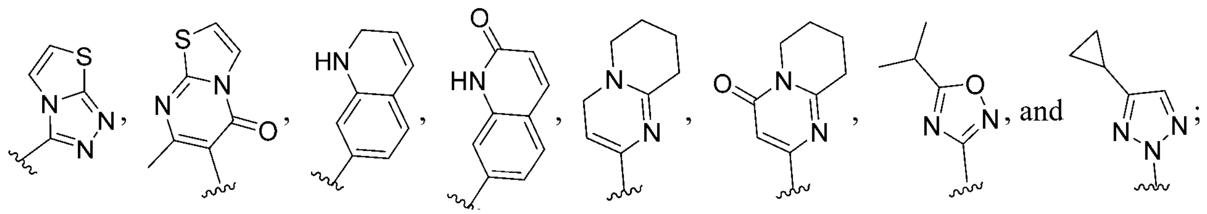
, , , , , , and ;

The present invention also provides a compound represented by the following formula (II), or a stereoisomer thereof, a pharmaceutically acceptable salt thereof, or a mixture thereof:

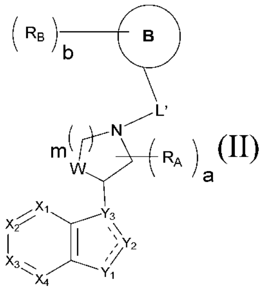
(II)

wherein, $X_1$, $X_2$, $X_3$, $X_4$, $Y_1$, $Y_2$, $Y_3$, L', ring B, $R_B$, $R_A$, a, and b are as defined for the compound of formula (I);

W is $-CH_2-$, -O-, -S-, -N-;
each $R_A$ is independently selected from deuterium, halogen, -OH, oxo, -CN, $-NH_2$, $C_{1-3}$alkyl, $C_{1-3}$alkoxy, halo$C_{1-3}$alkyl, and halo$C_{1-3}$alkoxy;
m is 0, 1, 2, or 3.
In a preferable embodiment of the present invention, W is $-CH_2-$, -O-, or -S-;
In a preferable embodiment of the present invention, W is $-CH_2-$, or -O-;
In a preferable embodiment of the present invention, W is $-CH_2-$.
In a preferable embodiment of the present invention, each $R_A$ is independently selected from deuterium, halogen, -OH, -CN, $-NH_2$, $C_{1-3}$alkyl, and $C_{1-3}$oxaalkyl;
In a preferable embodiment of the present invention, each $R_A$ is independently selected from deuterium, halogen, and $C_{1-3}$alkyl;
In a preferable embodiment of the present invention, each $R_A$ is independently selected from F, and methyl.
In a preferable embodiment of the present invention, ring B is

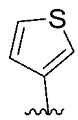, 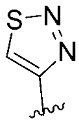, 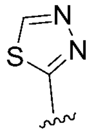, 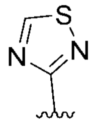, 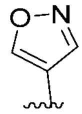,

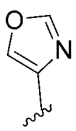, 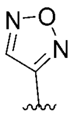, 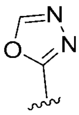, 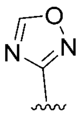, 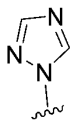, 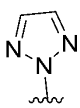, 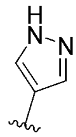, 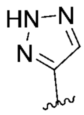, 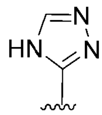, 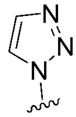, 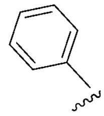,

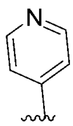, 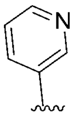, 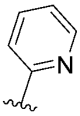, 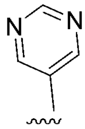, 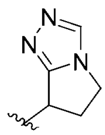, 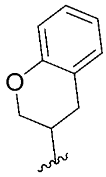, 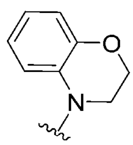, 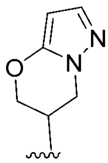, 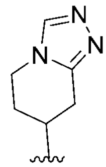, 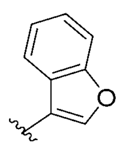,

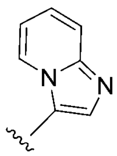, 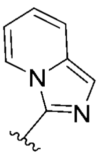, 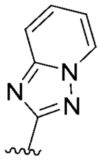, 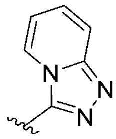, 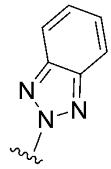, 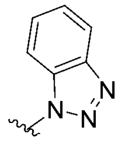, 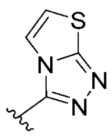, 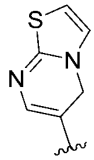, 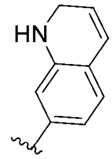, or

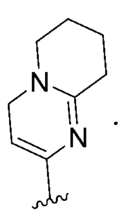.

In a preferable embodiment of the present invention, m is 0;
In a preferable embodiment of the present invention, m is 3;
In a preferable embodiment of the present invention, m is 2;
In a preferable embodiment of the present invention, m is 1.
In a preferable embodiment of the present invention, a is 0, 1, or 2;
In a preferable embodiment of the present invention, a is 0;
In a preferable embodiment of the present invention, a is 1.
The present invention also provides a compound represented by any one of the following formulae (III-1) to (III-8), or a stereoisomer thereof, a pharmaceutically acceptable salt thereof, or a mixture thereof:

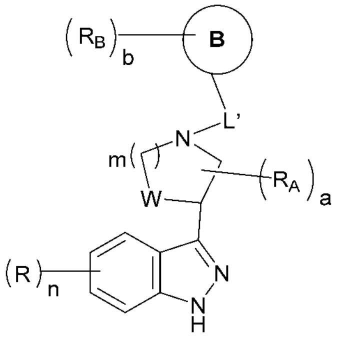

(III-1)

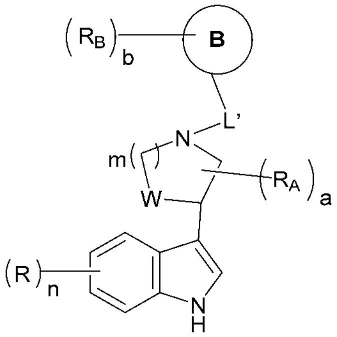

(III-2)

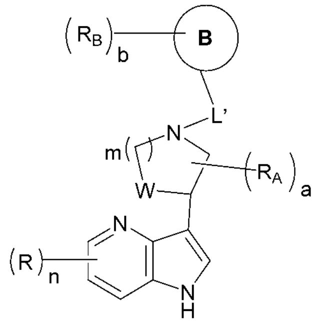

(III-3)

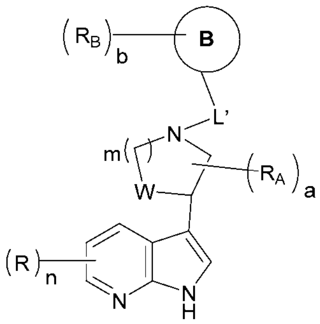

(III-4)

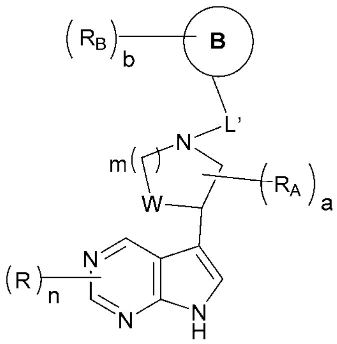

(III-5)

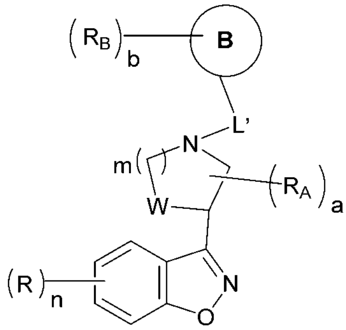

(III-6)

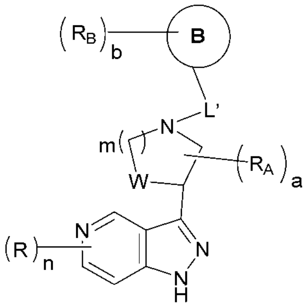

(III-7)

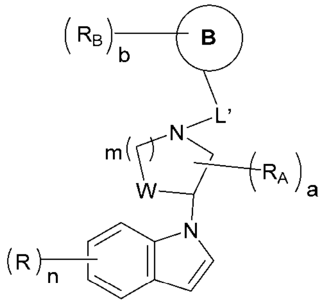

(III-8)

wherein, L', ring B, $R_B$, b, W, $R_A$, a, and m are as defined for the compound of formula (I) or (II);

R is selected from deuterium, halogen, -OH, -CN, $-NH_2$, and the following groups that are optionally substituted: $C_{1-4}$alkyl, $C_{1-4}$oxaalkyl, and $C_{1-4}$thiaalkyl; wherein optionally substituted means being unsubstituted or being substituted by one or more substituents selected from deuterium, halogen, oxo, - CN, -OH, and $-NH_2$;

n is 0, 1, 2, or 3.

In a preferable embodiment of the present invention, W is $-CH_2-$, m is 1;

In a preferable embodiment of the present invention, R is selected from deuterium, -OH, F, Cl, Br, methyl, and methoxy;

In a preferable embodiment of the present invention, R is F;

In a preferable embodiment of the present invention, R is -OH;

In a preferable embodiment of the present invention, n is 0, 1, or 2;

In a preferable embodiment of the present invention, n is 0;

In a preferable embodiment of the present invention, n is 1;

In a preferable embodiment of the present invention, in formula (III-2),

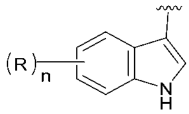 is 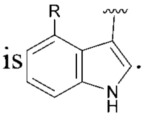.

The present invention also provides a compound represented by any one of the following formulae (A1) to (A16), or a stereoisomer thereof, a pharmaceutically acceptable salt thereof, or a mixture thereof:

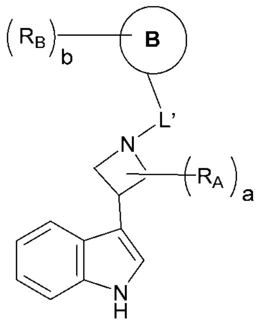
(A1)

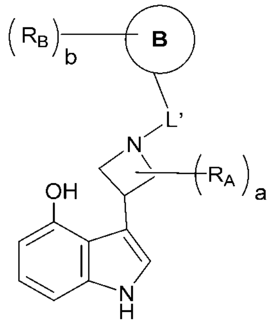
(A2)

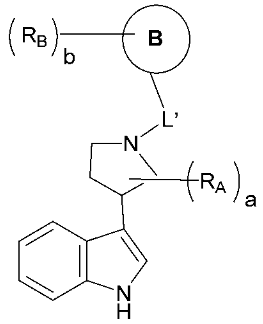
(A3)

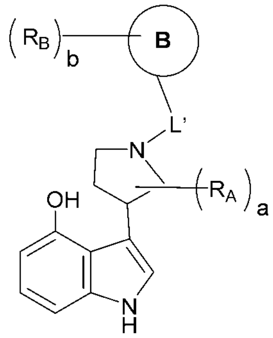
(A4)

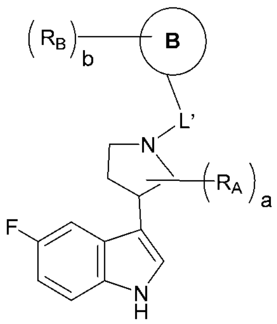
(A5)

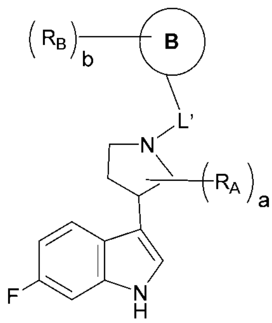
(A6)

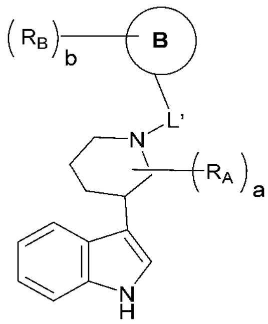
(A7)

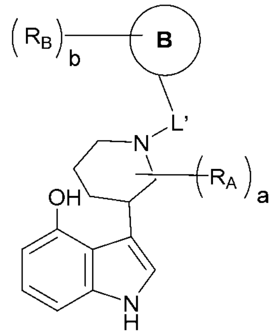
(A8)

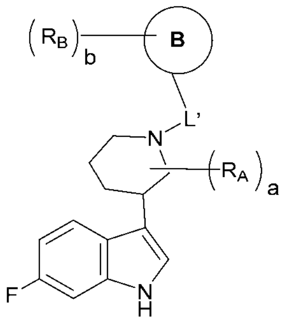
(A9)

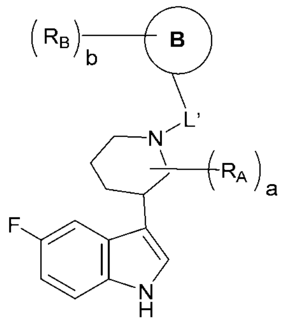
(A10)

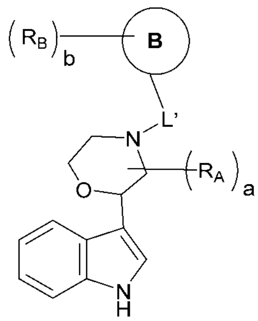
(A11)

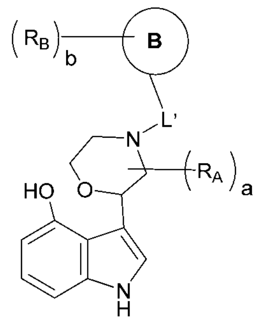
(A12)

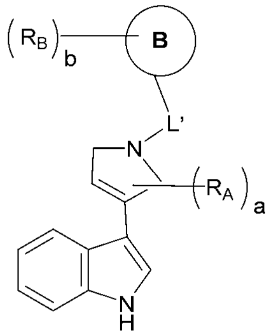
(A13)

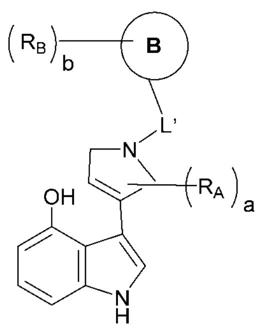
(A14)

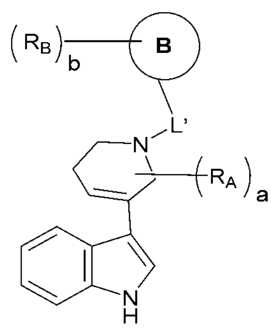
(A15)

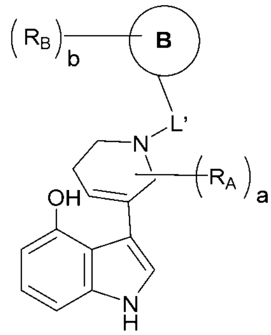
(A16)

wherein, L', ring B, $R_B$, b, $R_A$, and a are as defined for the compounds of formulae (I), (II), (III-1) to (III-8);
In a preferable embodiment of the present invention, ring B is phenyl, or 5-membered monocyclic heteroaryl;
In a preferable embodiment of the present invention, ring B is

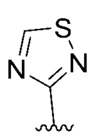,
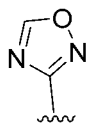,
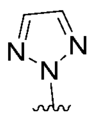,
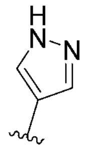,

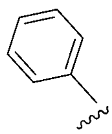,
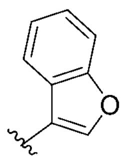, or
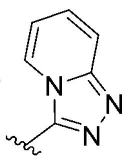;

In a preferable embodiment of the present invention, L' is

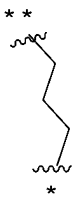;

** represents the attachment point to ring B; * represents the attachment point to ring Z.

The present invention also provides a compound represented by the following formula (a), or a stereoisomer thereof, a pharmaceutically acceptable salt thereof, or a mixture thereof:

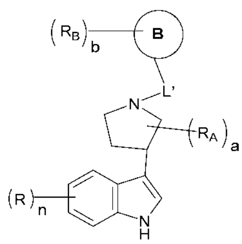
(a)

wherein, R, L', ring B, $R_A$, $R_B$, n, a, and b are as defined for the compounds of formulae (I), (II), and (III-2);
In a preferable embodiment of the present invention, R is -OH or methoxy;
In a preferable embodiment of the present invention, L' is an optionally substituted $-C_{3-4}$alkylene-, wherein optionally substituted means being unsubstituted or being substituted by one or more substituents selected from deuterium, halogen, -CN, -OH, $-NH_2$, $C_{1-3}$alkyl, $C_{1-3}$alkoxy, $C_{1-3}$haloalkyl, and $C_{1-3}$haloalkoxy;

In a preferable embodiment of the present invention, L' is an optionally substituted -propylene-;

In a preferable embodiment of the present invention, ring B is $C_{6-10}$aryl, or 5-10 membered heteroaryl;

In a preferable embodiment of the present invention, ring B is phenyl,

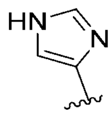, 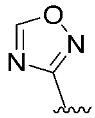, 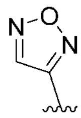,

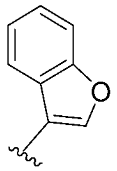, 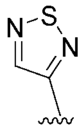, or 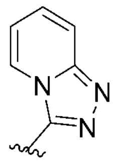;

or ring B is phenyl,

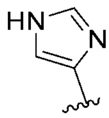, 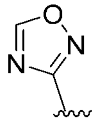, 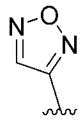, 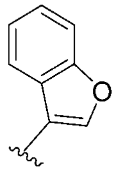, 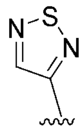,

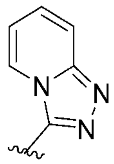, or 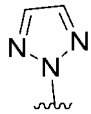, e.g. 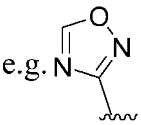 or 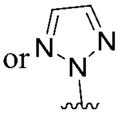;

In a preferable embodiment of the present invention, each $R_A$ is independently selected from deuterium, halogen, -OH, -CN, -$NH_2$, $C_{1-3}$alkyl, and $C_{1-3}$alkoxy;

In a preferable embodiment of the present invention, each $R_A$ is independently selected from deuterium, F, Cl, -OH, CN, -$NH_2$, methyl, ethyl, methoxy, and ethoxy;

In a preferable embodiment of the present invention, each $R_B$ is independently selected from deuterium, and the following groups that are optionally substituted: $C_1$-$_4$alkyl, $C_1$-$_4$oxaalkyl, $C_1$-$_4$thiaalkyl, and $C_3$-$_6$cycloalkyl, wherein optionally substituted means being unsubstituted or being substituted by one or more substituents selected from deuterium, -OH, -$NH_2$, and $C_1$-$_4$alkyl;

In a preferable embodiment of the present invention, each $R_B$ is independently selected from methyl, and methoxy;

In a preferable embodiment of the present invention, n is 0;

In a preferable embodiment of the present invention, n is 1;

In a preferable embodiment of the present invention, a is 0 or 1;

In a preferable embodiment of the present invention, a is 0;

In a preferable embodiment of the present invention, b is 0, 1, or 2;

In a preferable embodiment of the present invention, b is 0;

In a preferable embodiment of the present invention, b is 1.

The present invention also provides a compound represented by the following formula (b), or a stereoisomer thereof, a pharmaceutically acceptable salt thereof, or a mixture thereof:

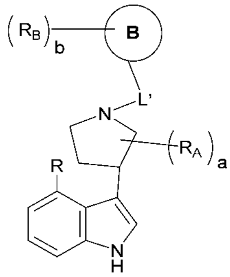 (b)

wherein, R, L', ring B, $R_A$, $R_B$, a, and b are as defined for the compound of formula (a).

The present invention also provides a compound represented by the following formula (c), or a stereoisomer thereof, a pharmaceutically acceptable salt thereof, or a mixture thereof:

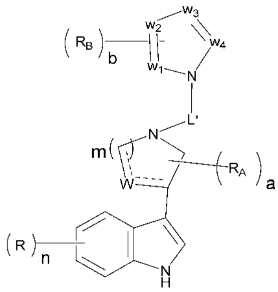 (c)

wherein, R, L', $R_A$, $R_B$, m, n, a, and b are as defined for the compounds of formulae (I), (II), (III-2); $w_1$, $w_2$, $w_3$, and $w_4$ are each independently selected from CH and N;

W is -CH, $-CH_2-$, -O-, or -S-;

two - - - s represent a single bond or a double bond, but the two are not a double bond at the same time; when any - - - is a double bond, W is -CH-;

In a preferable embodiment of the present invention, R is selected from deuterium, F, Cl, -OH, -CN, - $NH_2$, and the following groups that are optionally substituted: $C_{1-3}$alkyl, $C_{1-3}$oxaalkyl, and $C_{1-3}$thiaalkyl; wherein optionally substituted means being unsubstituted or being substituted by one or more substituents selected from deuterium, halogen, oxo, -CN, -OH, and $-NH_2$;

In a preferable embodiment of the present invention, R is F or -OH;

In a preferable embodiment of the present invention, L' is an optionally substituted -propylene-, wherein optionally substituted means being unsubstituted or being substituted by one or more substituents selected from deuterium, halogen, -CN, -OH, $-NH_2$, $C_{1-3}$alkyl, and $C_{1-3}$alkoxy;

In a preferable embodiment of the present invention, L' is -propylene-;

In a preferable embodiment of the present invention, each $R_B$ is independently selected from deuterium, and the following groups that are optionally substituted: $C_{1-3}$alkyl, $C_{2-4}$alkenyl, $C_{2-4}$alkynyl, $C_{1-3}$oxaalkyl, $C_{1-3}$thiaalkyl, $C_{3-6}$cycloalkyl, and $C_{3-6}$cycloalkenyl, wherein optionally substituted means being unsubstituted or being substituted by one or more substituents selected from deuterium, halogen, oxo, -CN, -OH, $-NH_2$, $C_{1-4}$alkyl, and $C_{1-4}$haloalkyl;

**In** a preferable embodiment of the present invention, each $R_B$ is independently selected from methyl, isopropyl, and cyclopropyl;

**In** a preferable embodiment of the present invention, $w_1$, $w_2$, $w_3$, and $w_4$ are N;

**In** a preferable embodiment of the present invention, $w_1$ and $w_4$ are N, $w_2$ and $w_3$ are CH;

**In** a preferable embodiment of the present invention, $w_1$ and $w_3$ are N, $w_2$ and $w_4$ are CH;

**In** a preferable embodiment of the present invention, n is 0, 1, or 2;

**In** a preferable embodiment of the present invention, n is 0;

**In** a preferable embodiment of the present invention, n is 1;

**In** a preferable embodiment of the present invention, a is 0 or 1;

**In** a preferable embodiment of the present invention, a is 1;

**In** a preferable embodiment of the present invention, b is 0 or 1;

**In** a preferable embodiment of the present invention, b is 1;

**In** a preferable embodiment of the present invention, two - - - s are both single bonds, W is $-CH_2-$;

**In** a preferable embodiment of the present invention, m is 1;

**In** a preferable embodiment of the present invention, m is 2.

The present invention also provides a compound represented by the following formula (d), or a stereoisomer thereof, a pharmaceutically acceptable salt thereof, or a mixture thereof:

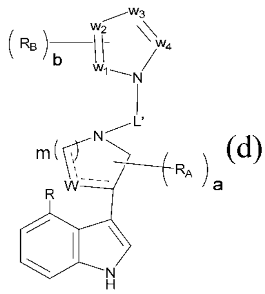

(d)

wherein, R, L', ring B, $R_A$, $R_B$, a, and b are as defined for the compound of formula (c).

The present invention also provides a compound represented by the following formula (e), or a stereoisomer thereof, a pharmaceutically acceptable salt thereof, or a mixture thereof:

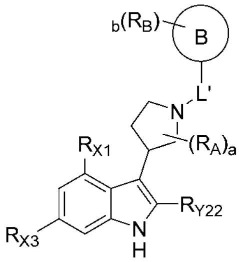

(e)

wherein, $R_{X1}$, $R_{X3}$, $R_{Y22}$, L', ring B, $R_A$, $R_B$, a, and b are as defined for the compounds of formulae (I), (II), (III-2);

In a preferable embodiment of the present invention, $R_{X1}$ is selected from hydrogen, -F, -OH, and - $OCH_3$;

In a preferable embodiment of the present invention, $R_{X1}$ is -OH;

In a preferable embodiment of the present invention, $R_{X3}$ is selected from hydrogen, -F, and -$OCH_3$;

In a preferable embodiment of the present invention, $R_{Y22}$ is selected from hydrogen, -Br, and methyl;

In a preferable embodiment of the present invention, L' is

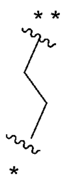

, or

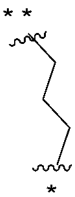

;

In a preferable embodiment of the present invention, ring B is $C_{6-10}$aryl, 5-membered nitrogen-containing heteroaryl or 9-membered heteroaryl;

In a preferable embodiment of the present invention, ring B is phenyl, 5-membered nitrogen-containing heteroaryl or 9-membered heteroaryl; the 5-membered nitrogen-containing heteroaryl at least contains one nitrogen atom, and optionally may further contain one oxygen or sulfur atom; the 9-membered heteroaryl contains 1, 2 or 3 heteroatoms, and the heteroatom is N, O or S;

In a preferable embodiment of the present invention, ring B is phenyl, 5-membered nitrogen-containing heteroaryl or 9-membered heteroaryl; the 5-membered nitrogen-containing heteroaryl contains 1, 2 or 3 nitrogen atoms, and optionally may further contain one oxygen or sulfur atom; the 9-membered heteroaryl contains 1, 2 or 3 heteroatoms, and the heteroatom is N, O or S;

In a preferable embodiment of the present invention, ring B is phenyl, 5-membered nitrogen-containing heteroaryl or 9-membered heteroaryl; the 5-membered nitrogen-containing heteroaryl contains 1, 2 or 3 nitrogen atoms, and optionally may further contain one oxygen or sulfur atom; the 9-membered heteroaryl contains 1, 2 or 3 heteroatoms, and the heteroatom is N, O or S; and the attachment point of ring B to L' is a C atom or an N atom;

In a preferable embodiment of the present invention, ring B is phenyl,

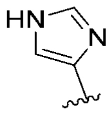, 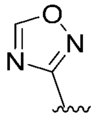, 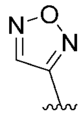,

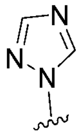, 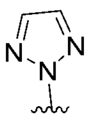, 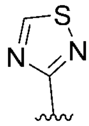, 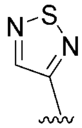, 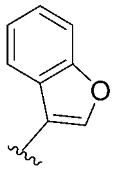, or 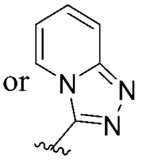;

In a preferable embodiment of the present invention, each $R_B$ is independently selected from methyl, methoxy, and cyclopropyl;

In a preferable embodiment of the present invention, each $R_A$ is methyl;

In a preferable embodiment of the present invention, a is 0 or 1;

In a preferable embodiment of the present invention, a is 0;

In a preferable embodiment of the present invention, b is 0 or 1;

In a preferable embodiment of the present invention, b is 0;

In a preferable embodiment of the present invention, b is 1.

Any non-conflicting combination of the above preferred embodiments is possible.

The present invention also provides compounds 1-189, or stereoisomers thereof, pharmaceutically acceptable salts thereof, or mixtures thereof. Examples for preparing compounds 1-189 are provided below. The structural formulas of compounds 1-189 are listed in the accompanying claims.

An object of the present invention also includes providing methods for preparing the aforementioned compounds, or stereoisomers thereof, pharmaceutically acceptable salts thereof, or mixtures thereof. The following methods are non-exclusively included:

General Scheme I:

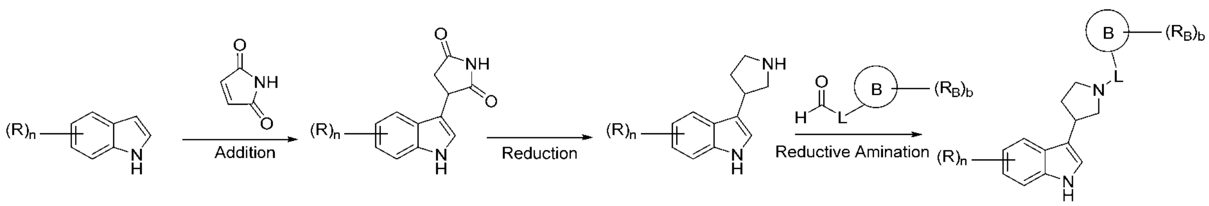


General Scheme II:

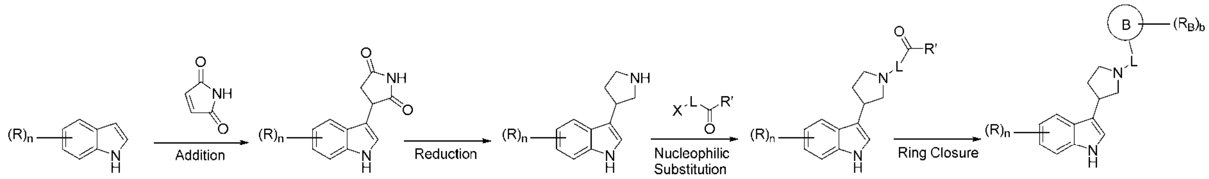


General Scheme III:

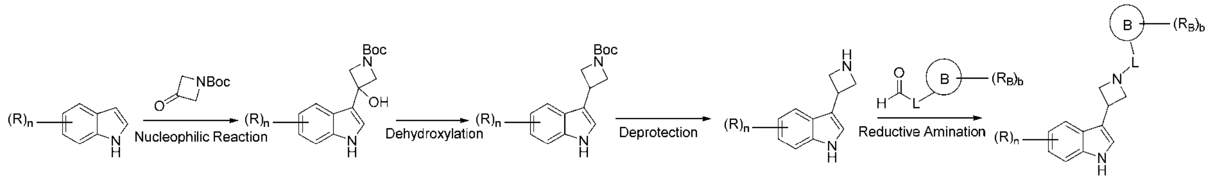

General Scheme IV:

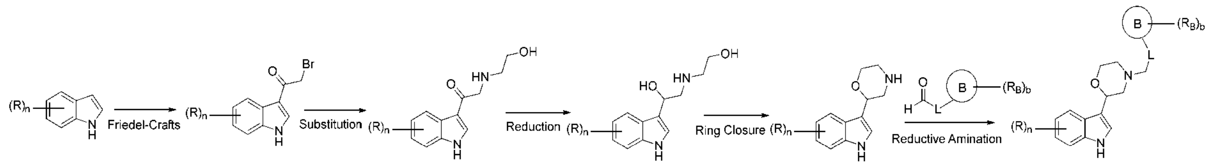


wherein, in the above preparation methods, R' is a protection group; X is H or halogen; R, $R_B$, ring B, L, n, and b are as previously defined.

The present invention also provides a pharmaceutical composition, which contains a compound represented by any one of formulae (I), (II), (III-1) to (III-8), (A1) to (A16), and (a) to (e), or a stereoisomer thereof, a pharmaceutically acceptable salt thereof, or a mixture thereof, and which optionally further contains a pharmaceutically acceptable carrier. In the pharmaceutical composition of the present invention, based on the weight of said pharmaceutical composition, said compound, or a stereoisomer thereof, a pharmaceutically acceptable salt thereof, or a mixture thereof may comprise 0.1-99.9, 1-99, 10-90, 20-80, 30-70, 40-60, 45-55, 1-5, 5-8, 8-10, 10-20, or 20-30 wt%, and said pharmaceutically acceptable carrier may correspondingly comprise 99.9-0.1, 99-1, 90-10, 80-20, 70-30, 60-40, 55-45, 99-95, 95-92, 92-90, 90-80, or 80-70 wt%.

An object of the present invention also includes providing use of a compound represented by any one of formulae (I), (II), (III-1) to (III-8), (A1) to (A16), and (a) to (e), or a stereoisomer thereof, a pharmaceutically acceptable salt thereof, or a mixture thereof, or the above pharmaceutical composition in manufacture of a medicament as 5-$HT_{2A}$ receptor agonist, in particular 5-$HT_{2A}$ receptor partial agonist, preferably, said medicament is a medicament for treating central nervous system disorder, preferably, said medicament is a medicament for treating depression.

An object of the present invention also includes providing use of a compound represented by any one of formulae (I), (II), (III-1) to (III-8), (A1) to (A16), and (a) to (e), or a stereoisomer thereof, a pharmaceutically acceptable salt thereof, or a mixture thereof, or the above pharmaceutical composition in manufacture of a medicament for treating central nervous system disorder, said central nervous system disorder is preferably depression.

An object of the present invention also includes providing a compound represented by any one of formulae (I), (II), (III-1) to (III-8), (A1) to (A16), and (a) to (e), or a stereoisomer thereof, a pharmaceutically acceptable salt thereof, or a mixture thereof, or the above pharmaceutical composition, for use in treating central nervous system disorder, preferably depression.

The present invention also provides a method of treating central nervous system disorder, preferably depression, which comprises administrating a therapeutically effective amount of a compound represented by any one of formulae (I), (II), (III-1) to (III-8), (A1) to (A16), and (a) to (e), or a stereoisomer thereof, a pharmaceutically acceptable salt thereof, or a mixture thereof, or the above pharmaceutical composition, to a subject in need thereof.

Definition

Unless otherwise specified, the term "alkyl" refers to a monovalent saturated aliphatic hydrocarbon group, including a linear or branched group containing 1-20 carbon atoms, preferably containing 1-10 carbon atoms (namely $C_{1-10}$alkyl), further preferably containing 1-8 carbon atoms ($C_{1-8}$alkyl), more preferably containing 1-6 carbon atoms (namely $C_{1-6}$alkyl), more preferably containing 1-4 carbon atoms (namely $C_{1-4}$alkyl), more preferably containing 1-3 carbon atoms (namely $C_{1-3}$alkyl). For example, "$C_{1-6}$ alkyl" means that the group is an alkyl group, and the number of carbon atoms in the carbon chain is between 1 and 6 (specifically 1, 2, 3, 4, 5 or 6). Its example includes but is not limited to methyl, ethyl, n-propyl, isopropyl, n-butyl, iso-butyl, tert-butyl, sec-butyl, n-pentyl, neo-pentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, n-octyl and the like.

"Haloalkyl" means "alkyl" substituted by halogen, e.g. C1-6haloalkyl or haloC1-6alkyl means $C_{1-6}$alkyl substituted by halogen.

Unless otherwise specified, the term "halogen" or "halo" refers to F, Cl, Br, I. The term "haloalkyl" refers to an alkyl group as defined above, in which one, two or more hydrogen atoms or all hydrogen atoms are substituted by halogen. Representative examples of haloalkyl include $CCl_3$, $CF_3$, $CHCl_2$, $CH_2Cl$, $CH_2Br$, $CH_2I$, $CH_2CF_3$, $CF_2CF_3$, and the like.

Unless otherwise specified, the term "alkenyl" refers to a linear or branched unsaturated aliphatic hydrocarbon group consisting of carbon atoms and hydrogen atoms and having at least one double bond. The alkenyl can contain 2-20 carbon atoms, preferably contain 2-10 carbon atoms (namely $C_{2-10}$alkenyl), further preferably contain 2-8 carbon atoms ($C_{2-8}$alkenyl), more preferably contain 2-6 carbon atom (namely $C_{2-6}$alkenyl), 2-5 carbon atom (namely $C_{2-5}$alkenyl), 2-4 carbon atoms (namely $C_{2-4}$alkenyl), 2-3 carbon atoms (namely $C_{2-3}$alkenyl), 2 carbon atoms (namely $C_2$alkenyl). For example "$C_{2-6}$alkenyl" means that the group is alkenyl, and the number of carbon atoms in the carbon chain is between 2 and 6 (specifically 2, 3, 4, 5 or 6). The non-limiting examples of alkenyl include but are not limited to ethenyl, 1-propenyl, 2-propenyl, 1-butenyl, isobutenyl, 1,3-butadienyl and the like.

Unless otherwise specified, the term "alkynyl" refers to a linear or branched unsaturated aliphatic hydrocarbon group consisting of carbon atoms and hydrogen atoms and having at least one triple bond. The alkynyl can contain 2-20 carbon atoms, preferably contain 2-10 carbon atoms (namely $C_{2-10}$alkynyl), further preferably contain 2-8 carbon atoms (namely $C_{2-8}$alkynyl), more preferably contain 2-6 carbon atoms (namely $C_2$-$_6$alkynyl), 2-5 carbon atoms (namely $C_{2-5}$alkynyl), 2-4 carbon atoms (namely $C_2$-$_4$alkynyl), 2-3 carbon atoms (namely $C_{2-3}$alkynyl), 2 carbon atoms (namely $C_2$alkynyl). For example "$C_2$-$_6$alkynyl" means that the group is alkynyl, and the number of carbon atoms in the carbon chain is between 2 and 6 (specifically 2, 3, 4, 5 or 6). The non-limiting examples of alkynyl include but are not limited to ethynyl, 1-propynyl, 2-propynyl, 1-butynyl and the like.

Unless otherwise specified, the term "cycloalkyl" refers to a monocyclic saturated aliphatic hydrocarbyl having a specified number of carbon atoms, preferably containing 3-12 carbon atoms (namely $C_{3-12}$cycloalkyl), more preferably 3-10 carbon atoms ($C_3$-$_{10}$cycloalkyl), further preferably 3-6 carbon atoms ($C_3$-$_6$cycloalkyl), 4-6 carbon atoms (C4-6cycloalkyl), or 5-6 carbon atoms ($C_{5-6}$cycloalkyl). Examples include, but are not limited to cyclopropyl, cyclobutyl, cyclopentyl, cyclohexyl, methylcyclopropyl, 2-ethyl-cyclopentyl, dimethylcyclobutyl and the like.

Unless otherwise specified, the term "oxaalkyl" refers to an alkyl residue wherein one or more carbons (and their associated hydrogens) are replaced by oxygen, such as "alkoxy", "alkoxyalkyl". Examples include methoxy, ethoxy, propoxy, methoxypropyl, etc. The term oxaalkyl is understood in the art [see Naming and Indexing of Chemical Substances for Chemical Abstracts, published by the American Chemical Society, 196, but not limited to 127(a)], i.e., it refers to a compound's moiety wherein oxygen is bonded to its adjacent atoms via a single bond (forming an ether linkage); it does not refer to the double-bonded oxygen found in carbonyl groups.

Unless otherwise specified, the terms "thiaalkyl" and "azaalkyl" refer to the above "oxaalkyl" wherein the oxygen is replaced by sulfur or NH.

Unless otherwise specified, the term "heterocyclyl" refers to a saturated or partially unsaturated monocyclic, bicyclic or polycyclic hydrocarbon substituent, which is in a non-aromatic structure and contains 3-20 ring atoms, of which 1, 2, 3 or more ring atoms are selected from N, O and S, and the remaining ring atoms are C. Preferably, it contains 3-12 ring atoms, more preferably 3-10 ring atoms, or 3-8 ring atoms, or 3-6 ring atoms, or 4-6 ring atoms, or 5-6 ring atoms. The number of heteroatoms is preferably 1-4, more preferably 1-3 (namely 1, 2, or 3). Examples of monocyclic heterocyclyl include pyrrolidinyl, imidazolinyl, tetrahydrofuryl, dihydropyrrolyl, piperidyl, piperazinyl, pyranyl, etc. Bicyclic or polycyclic heterocyclyl include spiro, fused and bridged heterocyclyl.

Unless otherwise specified, the term fused "heterocyclyl" or "fused heterocyclyl" refers to a 5-20 membered polycyclic heterocyclic group wherein each ring in the system shares an adjacent pair of atoms (carbon and carbon atoms or carbon and nitrogen atoms) with another ring, containing one or more heteroatoms selected from nitrogen, oxygen, or optionally oxidized sulfur as ring members, the remaining ring members being carbon. One or more rings of the fused heterocyclic group may contain one or more double bonds, wherein one ring does not have a fully conjugated $\pi$-electron aromatic system. Preferably, the fused heterocyclyl is 6-14 membered, preferably 7-12 membered and more preferably 7-10 membered. According to the number of member rings, fused heterocyclyl are divided into bicyclic, tricyclic, tetracyclic, or polycyclic fused heterocyclyl, preferably refers to bicyclic or tricyclic fused heterocyclyl, and more preferably 5-membered/5-membered, 5-membered/6-membered, 6-membered/5-membered, 4-membered/6-membered, 6-membered/4-membered, or 6-membered/6-membered bicyclic fused heterocyclyl.

Representative examples of fused heterocyclyl include, but are not limited to the following groups: octahydrocyclopenta[c]pyrrole (e.g., octahydrocyclopenta[c]pyrrol-2-yl), octahydropyrrolo[3,4-c]pyrrolyl, octahydroisoindolyl, isoindolinyl (e.g., isoindolin-2-yl or isoindolin-5-yl), octahydro-benzo[b][1,4]dioxine, dihydropyridooxazinyl (e.g., 2,3-dihydro-1H-pyrido[2,3-b][1,4]oxazinyl) or dihydrobenzooxazepinyl (e.g., 5-oxo-3,4-dihydrobenzo[f][1,4]oxazepinyl), benzoazepinyl (e.g., 2,3,4,5-tetrahydro-1-oxo-2-benzoazepin-6-yl), benzooxazepinyl (e.g., 5-oxo-2,3,4,5-tetrahydro-1,4-benzooxazepin-8-yl), dihydroisoquinolinyl (e.g., 1-oxo-2-methyl-3,4-dihydroisoquinolin-6-yl), tetrahydroisoquinolinyl (e.g., 2-methyl-1-oxo-1,2,3,4-tetrahydroisoquinolin-6-yl), dihydrobenzoxazine (e.g., 3,4-dihydro-2H-1,4-benzoxazin-6-yl), benzopiperazinyl, 7,8-dihydro-5H-pyrano[4,3-b]pyridinyl, 5,8-dihydro-6H-pyrano[3,4-b]pyridinyl, 7,8-dihydro-5H-thiopyrano [4,3-b]pyridinyl, 5,6,7,8-tetrahydro-1,6-naphthyridinyl, 5,6,7,8-tetrahydro-1,7-naphthyridinyl, 7,8-dihydro-5H-pyrano [4,3-d]pyridine, 3,4-dihydro-1H-pyrano[4,3-c]pyridine, 2H-1-benzopyranyl or 4H-1-benzopyranyl.

Unless otherwise specified, the term "heterocycloalkenyl" refers to a cycloalkenyl containing 1 to 5 (preferably 1, 2, 3, or 4) heteroatoms selected from N, O, and S, wherein the nitrogen and sulfur atoms are optionally oxidized, and the nitrogen atoms are optionally quaternized. Heterocycloalkenyl can be a monocyclic, bicyclic, or polycyclic system (including fused, spiro, bridged, etc.). Generally, the heterocycloalkenyl typically includes 4-12 ring atoms (i.e., 4-12 membered heterocycloalkenyl), preferably, includes 4-10 (such as 4, 5, 6, 7, 8) ring atoms (e.g., 4-7 membered heterocycloalkenyl, or 4-6 membered heterocycloalkenyl) and contains 1, 2, 3, or 4 (preferably 1 or 2) heteroatoms.

Unless otherwise specified, the term "aryl" refers to monocyclic, bicyclic and tricyclic aromatic carbocyclic ring systems containing 6-16, 6-14, 6-12 or 6-10 carbon atoms, preferably 6-10 carbon atoms. The term "aryl" may be used interchangeably with the term "aromatic ring". Examples of aryl may include but are not limited to phenyl, naphthyl, anthryl,

phenanthryl or pyrenyl, etc.

Unless otherwise specified, the term "heteroaryl" refers to an aromatic monocyclic, bicyclic or polycyclic ring system having a 5-16, 5-14, 5-12, 5-10, 5-8, or 5-6 membered structure, in which 1, 2, 3 or more ring atoms are heteroatoms and the remaining atoms are carbon, the heteroatoms are independently selected from O, N and S, and the number of heteroatoms is preferably 1, 2 or 3. Examples of heteroaryl may include but are not limited to furyl, thienyl, oxazolyl, thiazolyl, isoxazolyl, oxadiazolyl, thiadiazolyl, pyrrolyl, pyrazolyl, imidazolyl, triazolyl, tetrazolyl, pyridyl, pyrimidyl, pyrazinyl, pyridazinyl, thiadiazolyl, triazinyl, phthalazinyl, quinolinyl, isoquinolinyl, pteridinyl, purinyl, indolyl, isoindolyl, indazolyl, benzofuryl, benzothienyl, benzopyridyl, benzopyrimidyl, benzopyrazinyl, benzimidazolyl, benzophthalazinyl, pyrrolo[2,3-b]pyridyl, imidazo[1,2-a]pyridyl, pyrazolo[1,5-a]pyridyl, pyrazolo[1,5-a]pyrimidyl, imidazo [1,2-b]pyridazinyl, [1,2,4]triazolo[4,3-b]pyridazinyl, [1,2,4]triazolo[1,5-a]pyrimidyl, [1,2,4]triazolo[1,5-a]pyridyl, etc.

Unless otherwise specified, the term "fused-heteroaryl" refers to an unsaturated aromatic fused ring structure containing 5-14 ring atoms (including at least one heteroatom) and formed by connecting two or more ring structures that share two adjacent atoms with each other, wherein 1, 2, 3 or more ring atoms are heteroatoms and the remaining atoms are carbon. The heteroatoms are independently selected from O, N, and S, and the number of heteroatoms is preferably 1, 2, or 3. Preferably, it is 5- 12 membered fused-heteroaryl, 7-12 membered fused-heteroaryl, 9-12 membered fused-heteroaryl, etc., preferably 5-membered/5-membered fused-heteroaryl, 5-membered/6-membered fused-heteroaryl, 6-membered/5-membered fused-heteroaryl, 6-membered/6-membered bicyclic fused-heteroaryl. Examples of fused-heteroaryl may include but are not limited to benzofuryl, benzoisofuryl, benzothienyl, indolyl, isoindole, benzoxazolyl, benzimidazolyl, indazolyl, benzotriazolyl, quinolinyl, 2-quinolinone, 4-quinolinone, 1-isoquinolinone, isoquinolinyl, acridinyl, phenanthridinyl, benzopyridazinyl, phthalazinyl, quinazolinyl, quinoxalinyl, phenazinyl, pteridinyl, purinyl, naphthyridinyl, phenazine, phenothiazine, etc.

Unless otherwise specified, the term "pharmaceutically acceptable salt" or similar expressions refers to a salt that is suitable for use in contact with the tissues of mammals, especially humans, without undue toxicity, irritation, allergic response or the like and commensurates with a reasonable benefit/risk ratio within the range of reasonable medical judgment. For example, the pharmaceutically acceptable salts of amines, carboxylic acids and other types of compounds are well-known in the art. The salts can be prepared in situ during the final isolation and purification of the compounds of this invention, or separately by reacting the free base or acid with a suitable reagent.

Compounds of the invention also include isotopic derivatives thereof. Unless otherwise specified, the term "isotope derivative" means that the compounds of this invention can exist in isotope-tagged or enriched form containing one or more atoms having an atomic mass or mass number different from the atomic mass or mass number most abundantly found in nature. Isotopes can be radioactive or nonradioactive isotopes. Isotopes commonly used as isotopic labels are: hydrogen isotopes: $^{2}H$ and $^{3}H$; carbon isotopes: $^{13}C$ and $^{14}C$; chlorine isotopes: $^{35}Cl$ and $^{37}Cl$; fluorine isotope: $^{18}F$; iodine isotopes: $^{123}I$ and $^{125}I$; nitrogen isotopes: $^{13}N$ and $^{15}N$; oxygen isotopes: $^{15}O$, $^{17}O$ and $^{18}O$; and sulfur isotope: $^{35}S$. These isotope-labelled compounds can be used to study the distribution of pharmaceutical molecules in tissues. In particular, $^{3}H$ and $^{13}C$ are more widely used due to their ease of labelling and ease of detection. The substitution with certain heavy isotopes, such as heavy hydrogen ($^{2}H$), can enhance the stability of metabolism, and prolong the half-life, to achieve the purpose of reducing dosage and providing therapeutic advantages. Isotope-labelled compounds are generally synthesized starting from labelled starting materials in the same way as non-isotope-labelled compounds using known synthetic techniques. Compounds of the invention also include their solvates. Unless otherwise specified, the term "solvate" or similar expressions refer to the physical association of the present compound with one or more solvent molecules, regardless of whether organic or inorganic. This physical association includes hydrogen bonds. In some cases, for example, when one or more solvent molecules are incorporated into the crystal lattice of a crystalline solid, the solvates can be isolated. Solvent molecules in the solvates may exist in regular and/or disordered arrangements. The solvates may contain stoichiometric or non-stoichiometric amounts of solvent molecules. The "solvate" encompasses both solution-phase and isolatable solvate. Exemplary solvates include but are not limited to hydrates, ethanolates, methanolates and isopropanolates. Solvation methods are well-known in the art.

Unless otherwise specified, the term "stereoisomers" refers to compounds which have identical chemical constitutions, but differ about the arrangement of the atoms or groups in space. Stereoisomers include enantiomer, diastereomers, conformer (rotamer), geometric (cis/trans) isomers, atropisomers, etc. A mixture of any resulting stereoisomers can be separated into pure or substantially pure geometric isomers, enantiomers, and diastereomers, according to physicochemical differences of constituents, for example, by chromatography and/or fractional crystallization.

Unless otherwise indicated, structure formulae depicted in this invention include all isomeric forms (e.g. enantiomers, diastereomers, and geometric isomers (or conformational isomers)): e.g., R and S configurations with the asymmetric centre, (Z) and (E) isomers of double bond, and (Z) and (E) conformational isomers. Therefore, individual stereochemical isomers or mixtures of enantiomers, diastereomers, or geometric isomers (or conformational isomers) of the present compounds are within the scope of this invention.

Compounds of the invention also include their co-crystals. Unless otherwise specified, the term "co-crystal" is used to describe the situation where neutral molecular components are present within a crystalline compound in a definite

stoichiometric ratio. The preparation of pharmaceutical co-crystals enables modifications to be made to the crystalline form of an active pharmaceutical ingredient, which in turn can alter its physicochemical properties without compromising its intended biological activity (see Pharmaceutical Salts and Co-crystals, ed. J. Wouters & L. Quere, RSC Publishing, 2012).

Compounds of the invention also include their polymorphs. Unless otherwise specified, the term "polymorph" refers to the different arrangement of chemical drug molecules, which is generally presented as the existing form of the drug's raw materials in the solid state. A drug may exist in a variety of crystal forms, and different crystal forms of the same drug may have different dissolution and absorption properties in vivo, thereby affecting the dissolution and release of the formulation.

Compounds of the invention also include their metabolites. Unless otherwise specified, the term "metabolite" refers to a product afforded through metabolism in the body of a specific compound or a salt thereof. Metabolite of a compound may be identified using routine techniques known in the art and its activity may be characterized using tests as those described herein. Such product may be obtained through routes such as oxidation, reduction, hydrolysis, amidation, deamidation, esterification, deesterification, and enzymatic cleavage of the administered compound. Accordingly, this invention includes the metabolite of a compound, including a metabolite afforded by sufficiently contacting a compound of this invention with a mammal for some time.

Compounds of the invention also include their prodrugs. Unless otherwise specified, the term "prodrug" refers to a drug that is converted into the parent drug in vivo. The prodrug is usually useful, which may improve certain undesirable physical or biological properties. The physical properties usually refer to related solubility (excessive or insufficient solubility in lipid or water) or stability, while problematic biological properties include overquick metabolism or poor bioavailability, which may itself be related to physical and chemical properties. For example, they are bioavailable via oral administration, whereas the parent drug cannot. Compared with the parent drug, the solubility of the prodrug in a pharmaceutical composition is also improved. An example of prodrug may be but is not limited to, any compound of this invention, which is administered as an ester ("prodrug") to facilitate transport through cell membranes, wherein the water solubility is detrimental to mobility but beneficial once it gets into the cell, and which is subsequently metabolically hydrolyzed to a carboxylic acid (i.e. active entity). Another example of the prodrug may be a short peptide (polyamino acid) bound to an acid group, wherein the peptide is metabolized to reveal the active moiety.

Unless otherwise specified, the term "optionally substituted" means that the hydrogen at the substitutable site of the group is unsubstituted, or substituted by one or more substituents, which are preferably selected from the group consisting of halogen, hydroxy, mercapto, cyano, nitro, amino, azido, oxo, carboxyl, $C_2$-$_6$alkenyl, $C_2$-$_6$alkynyl, $C_{1-6}$alkyl, $C_{1-6}$alkoxy, $C_3$-$_{10}$cycloalkyl, $C_3$-$_{10}$cycloalkylsulfonyl, 3-10 membered heterocycloalkyl, $C_{6-14}$aryl, and 5-10 membered heteroaryl, wherein the $C_2$-$_6$alkenyl, $C_2$-$_6$alkynyl, $C_{1-6}$alkyl, $C_{1-6}$alkoxy, $C_3$-$_{10}$cycloalkyl, $C_3$-$_{10}$cycloalkylsulfonyl, 3-10 membered heterocycloalkyl, $C_{6-14}$aryl or 5-10 membered heteroaryl may be optionally substituted by one or more substituents selected from halogen, hydroxy, amino, cyano, $C_{1-6}$alkyl, and $C_{1-6}$alkoxy, the oxo refers to the formation of a double bond by replacing two hydrogen atoms at the same substitution site with the same oxygen atom. "More substituents " refers to 2, 3, 4, 5, 6, 7, or 8 substituents; preferably 2, 3, or 4 substituents.

Unless otherwise specified, the term "pharmaceutically acceptable carrier" refers to a medium generally recognized in the art for delivering biologically active agents to animals (specifically, mammals). Pharmaceutically acceptable carriers are formulated taking into account many factors well within the purview of the ordinary skilled artisan. These include (but are not limited to) the type and nature of the active agent being formulated, the subject to whom the composition containing the pharmaceutical agent is to be administered, the intended route of administration of the composition, and the targeted therapeutic indication. Pharmaceutically acceptable carriers include aqueous and non-aqueous liquid media, as well as various solid and semi-solid dosage forms. Such carriers may include many different ingredients and additives besides the active agent, and such additional ingredients are included in the formulation for various reasons well known to the ordinary skilled artisan (e.g., stability of the active agent, binder, etc.). Descriptions of suitable pharmaceutically acceptable carriers and factors involved in their selection can be found in various readily available sources.

The above technical features of the present invention may be combined arbitrarily, and the embodiments obtained from the combinations also belong to the embodiments of the present invention.

**Beneficial Effects of the Invention**

Through extensive research, the applicant has obtained structurally novel 5-$HT_{2A}$ receptor agonists, particularly 5-$HT_{2A}$ receptor partial agonists. In vitro and in vivo experiments have shown that the compounds of the present application possess excellent antidepressant effects. On this basis, it is possible to achieve the effect of being non-hallucinogenic.

## Brief Description of the Drawings

Figure 1 shows the pharmacodynamic evaluation of the compounds of the present invention in the mouse head-twitch model.

Figure 2 shows the pharmacodynamic evaluation of the compound of the present invention in the mouse depression model.

## Detailed description

This invention is further described below in connection with specific examples. It should be understood that these examples are intended to illustrate this invention only and are not intended to limit the scope of this invention. Experimental methods for which no specific conditions are indicated in the following examples are generally by conventional conditions or in accordance with the conditions recommended by the manufacturers. Unless otherwise defined, all professional and scientific terms used herein have the same meaning as those familiar to those skilled in the art. In addition, any methods and materials similar or equivalent to those described herein may be used in the methods of this invention. The preferred embodiments and materials shown in this disclosure are illustrative only.

The structures of the compounds according to this invention are determined by nuclear magnetic resonance (NMR) or/and liquid chromatography-mass spectrometry (LC-MS) or/and liquid chromatography (HPLC). The NMR measurement instrument used is Bruker 400MHz or/and Varian 400MHz; the LC-MS instrument used is Agilent 1260 Infinity II-6120/6125MSD; the HPLC instrument used is Waters Acquity UPLC 2 or/and Shimadzu LC2030 or/and Agilent 1260 Infinity II. Chiral compound resolution is performed with an SFC-150 (Waters) instrument; chiral column: DAICEL CHIRALCEL®OD; column dimensions: 20×250 mm (10 μm particle size packing).

The starting materials in the examples of this invention are known and commercially available, or can be synthesized using those methods known in the art or according to those methods known in the art. This invention provides processes for preparing the compounds. The compounds can be prepared through the following steps.

In the following examples, abbreviations have the following meanings:

| | |
|---|---|
| Ac | Acetyl |
| ACN | Acetonitrile |
| AcOH | Acetic acid |
| Bn | Benzyl |
| Boc | Tert-butoxycarbonyl |
| $Boc_2O$ | Di-tert-butyl dicarbonate |
| BTC | Bis(trichloromethyl)carbonate (triphosgene) |
| CbzCl | Benzyl chloroformate |
| DCE | Dichloroethane |
| DCM | Dichloromethane |
| DEG | Diethylene glycol |
| DIBAL-H | Diisobutylaluminium hydride |
| DIEA | N,N-diisopropylethylamine |
| Dioxane | 1,4-Dioxacyclohexane |
| DMA | N,N-dimethylformamide dimethyl acetal |
| DMAP | 4-Dimethylaminopyridine |
| DMF | N,N-dimethylformamide |
| DPPF-$PdCl_2$ | [1,1'-Bis(diphenylphosphino)ferrocene]palladium dichloride |
| EA | Ethyl acetate |
| Et | Ethyl |

(continued)

| | |
|---|---|
| EtOH | Ethanol |
| HATU | O-(7-azabenzotriazol-1-yl)-N,N,N',N'-tetramethyluronium hexafluorophosphate |
| LAH | Lithium aluminium hydride |
| Me | Methyl |
| MeOH | Methanol |
| MsCl | Methanesulfonyl chloride |
| $Pd_2(dba)_2$ | Tris(dibenzylideneacetone)dipalladium |
| PE | Petroleum ether |
| PMB | Para-methoxybenzyl |
| STAB | Sodium triacetoxyborohydride |
| TEA | Triethylamine |
| TES | Triethylsilane |
| TFA | Trifluoroacetic acid |
| THF | Tetrahydrofuran |
| TsCl | 4-Toluenesulfonyl chloride |

Example 1 (Compound 1)

Preparation of 3-(1-(2-(2-methoxyphenyl)ethyl)pyrrolidin-3-yl)-1H-indol-4-ol:

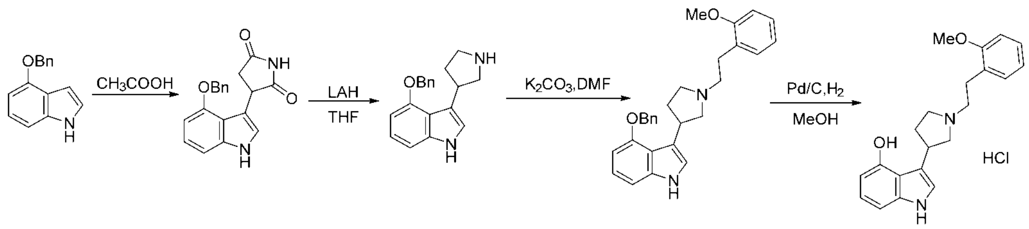


**Step 1:** Preparation of 3-(4-(benzyloxy)-1H-indol-3-yl)pyrrolidine-2,5-dione:

Pyrrolidine-2,5-dione (2eq) and 4-benzyloxy-1H-indole (4.5g, 1eq) were added to acetic acid (100mL). The mixture was stirred at 125°C for 48 hours. The reaction mixture was concentrated under a reduced pressure. The residue was purified by column chromatography to produce the target compound (4.18g). LCMS (ESI) $[M+H]^+$=321.4.

**Step 2:** Preparation of 4-(benzyloxy)-3-(pyrrolidin-3-yl)-1H-indole:

At 0°C, 3-(4-(benzyloxy)-1H-indol-3-yl)pyrrolidine-2,5-dione (450mg, 1eq) was added in batch to tetrahydrofuran (10mL) containing lithium tetrahydroaluminate (5 eq). The mixture was stirred at 75°C for 5 hours, cooled to room temperature, quenched with water (1mL), diluted with ethyl acetate (10mL), and filtered through diatomite. The filtrate was concentrated under vacuum. The residue was diluted with ethyl acetate, washed with brine, dried over anhydrous sodium sulfate, and concentrated under a reduced pressure. The residue was purified by reverse-phase chromatography to produce the target compound (190mg).

LCMS (ESI) $[M+H]^+$=293.2.

**Step 3:** Preparation of 4-(benzyloxy)-3-(1-(2-(2-methoxyphenyl)ethyl)pyrrolidin-3-yl)-1H-indole:

4-(benzyloxy)-3-(pyrrolidin-3-yl)-1H-indole (200mg, 1eq), and 1-(2-bromoethyl)-2-methoxybenzene (1eq) were dissolved in N,N-dimethylformamide (4mL), and potassium carbonate (1.5eq) was added.

The reaction system was stirred at 80°C for 16 hours. The reaction mixture was diluted with water (15mL), and extracted with ethyl acetate (15mL×3). The organic phases were combined, washed with saturated brine (20mL×2), dried over anhydrous sodium sulfate, and filtered. The filtrate was concentrated to give a crude product. The crude product was purified by reverse-phase chromatography to produce the target compound (65mg).
LCMS (ESI) $[M+H]^+$=427.5.

**Step 4:** Preparation of 3-(1-(2-(2-methoxyphenyl)ethyl)pyrrolidin-3-yl)-1H-indol-4-ol:

4-(benzyloxy)-3-(1-(2-(2-methoxyphenyl)ethyl)pyrrolidin-3-yl)-1H-indole (230mg, 1eq) was dissolved in methanol (5mL), and palladium/carbon (2eq) was added. At room temperature, the reaction system was stirred under a hydrogen atmosphere for 1 hour. The reaction mixture was filtered. The filtrate was concentrated to give a crude product. The crude product was purified by preparative HPLC (0.1% trifluoroacetic acid in water:acetonitrile) to produce the target compound. Said target compound was lyophilized, followed by adding 0.5% aqueous hydrochloric acid solution, and repeatedly lyophilized to give the hydrochloride salt of the target compound (14.29mg).
LCMS (ESI) $[M+H]^+$=337.1; $^1H$ NMR(400 MHz, DMSO-$d_6$)δ 10.84(s, 1H), 10.41(s, 1H), 9.57(d, J=29.4 Hz, 1H), 7.30-7.22(m, 2H), 7.13(s, 1H), 7.04-6.99(m, 1H), 6.95-6.90(m, 1H), 6.88-6.79(m, 2H), 6.38(d, J=7.2 Hz, 1H), 4.12-3.86(m, 2H), 3.84-3.79(m, 3H), 3.76-3.66(m, 1H), 3.63-3.55(m, 1H), 3.39(s, 2H), 3.25-3.13(m, 1H), 2.99(s, 2H), 2.40-2.09(m, 2H).

Examples 2-3 (Compounds 2 and 3)

With reference to the preparation of Example 1, compounds 2 and 3 were prepared:

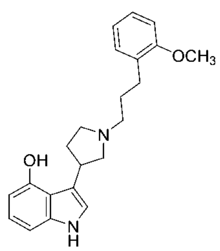
(2)
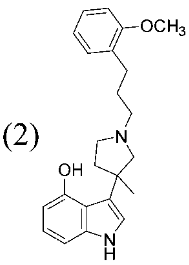
(3)

Compound 2: LCMS (ESI) $[M+H]^+$=351.2;
Compound 3: LCMS (ESI) $[M+H]^+$=365.2;

Example 4 (Compound 4)

Preparation of 3-(1-(3-(2-methoxyphenyl)propyl)piperidin-3-yl)-1H-indol-4-ol:

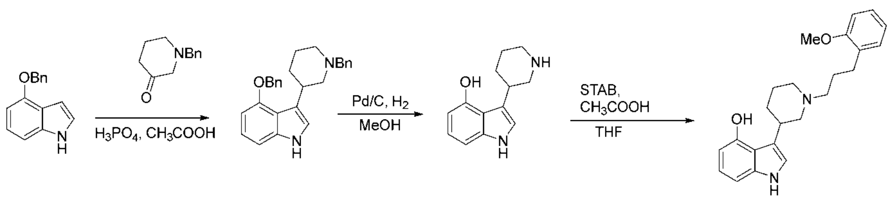


**Step 1:** Preparation of 4-(benzyloxy)-3-(1-benzylpiperidin-3-yl)-1H-indole:

1-benzylpiperidin-3-one (2g, 1eq) and 4-(benzyloxy)-1H-indole (1eq) were dissolved in acetic acid (20mL), and an 85% aqueous phosphoric acid solution (10eq) was added. The reaction system was stirred at 90°C for 2 hours. The reaction mixture was diluted with ethyl acetate (30mL). The organic phase was washed with water three times, and concentrated to give a crude product. The crude product was purified by reverse-phase chromatography (C18, 0.01% formic acid in water:acetonitrile) to produce the target compound (300mg).
LCMS (ESI) $[M+H]^+$=397.2.

**Step 2:** Preparation of 3-(piperidin-3-yl)-1H-indol-4-ol:

Under a hydrogen atmosphere, palladium/carbon (67mg, 1eq) was added to a solution of 4-(benzyloxy)-3-(1-benzylpiperidin-3-yl)-1H-indole (250mg, 1eq) in methanol (5mL). The reaction system was stirred at room temperature for 1 hour. The reaction mixture was filtered through diatomite and washed with methanol. The filtrate was concentrated to produce the target compound (200mg, crude).
LCMS (ESI) $[M+H]^+$=217.2.

**Step 3:** Preparation of 3-(1-(3-(2-methoxyphenyl)propyl)piperidin-3-yl)-1H-indol-4-ol:

3-(piperidin-3-yl)-1H-indol-4-ol (150mg, crude) and 3-(2-methoxyphenyl)propanal (137mg) were dissolved in tetrahydrofuran (5mL). A drop of acetic acid was added dropwise. The reaction system was stirred at room temperature for 30 minutes. Then sodium triacetoxyborohydride (294mg) was added. The reaction mixture was stirred at room temperature for another 8 hours. The reaction system was diluted with water (10mL), and extracted with dichloromethane (15mL×3). The organic phases were combined, dried over anhydrous sodium sulfate, filtered, and concentrated to give a crude product. The crude product was purified by preparative HPLC (C18, 0.05% formic acid in water:acetonitrile) to produce the target compound (21.97mg).
LCMS (ESI) $[M+H]^+$=365.3; $^1H$ NMR(400 MHz, DMSO-d6)$\delta$ 10.62(s, 1H), 8.26(s, 1H), 7.15(dd, J=13.3, 7.2 Hz, 2H), 6.92(d, J=8.2 Hz, 2H), 6.85(t, J=7.3 Hz, 1H), 6.76(dd, J=14.6, 7.5 Hz, 2H), 6.30(d, J=7.1 Hz, 1H), 3.75(s, 3H), 3.38-3.27(m, 2H), 2.95(d, J=9.2 Hz, 1H), 2.58-2.53(m, 2H), 2.41(s, 2H), 2.09-1.95(m, 3H), 1.76-1.69(m, 3H), 1.64(d, J=12.5 Hz, 1H), 1.48-1.38(m, 1H).

Example 5 (Compound 5)

Preparation of 3-(1-(3-(2-methoxyphenyl)propyl)pyrrolidin-3-yl)-1H-indole:

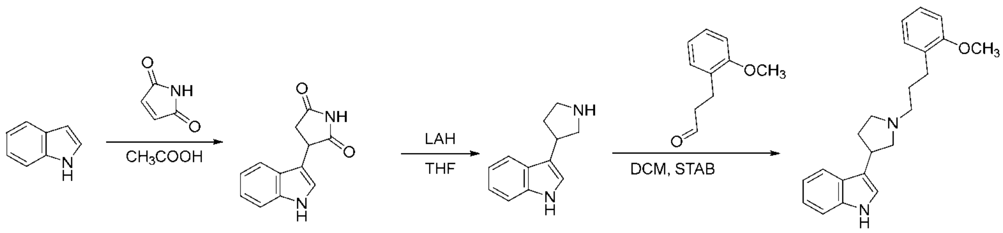


**Step 1:** Preparation of 3-(1H-indol-3-yl)pyrrolidine-2,5-dione:

Indole (15g, 1eq) was dissolved in acetic acid (150mL). 1H-pyrrole-2,5-dione (3eq) was added. The reaction mixture was reacted overnight at 120°C, and then concentrated to remove the solvent. Ethyl acetate (100mL) was added, and the pH was adjusted to 8 with saturated sodium bicarbonate. The aqueous phase was extracted with ethyl acetate (30mL×3). The organic phases were combined, washed with saturated brine (20mL×2), dried over anhydrous sodium sulfate, filtered, and concentrated to give a crude product. The crude product was purified by flash chromatography to produce the target compound (19g).
LCMS (ESI) $[M+H]^+$=215.3.

**Step 2:** Preparation of 3-(pyrrolidin-3-yl)-1H-indole:

3-(1H-indol-3-yl)pyrrolidine-2,5-dione (10g, 1eq) was dissolved in tetrahydrofuran (100mL). Lithium aluminium hydride (5eq) was added at 0°C. The reaction mixture was heated and stirred at 80°C for 3 hours. The reaction mixture was cooled to room temperature, quenched by adding water slowly under an ice bath, and diluted with ethyl acetate (100mL). The resulting mixture was filtered through diatomite. The filtrate was concentrated, diluted with ethyl acetate (100mL), washed with saturated brine (100mL×2), dried over anhydrous sodium sulfate, concentrated to dryness to give a crude product. The crude product was purified by reverse-phase flash chromatography (C18, 0.05% formic acid in water:acetonitrile) to produce the target compound (5g).
LCMS (ESI) $[M+H]^+$=187.1.

**Step 3:** Preparation of 3-(1-(3-(2-methoxyphenyl)propyl)pyrrolidin-3-yl)-1H-indole:

3-(2-methoxyphenyl)propanal (100mg, 1eq) was dissolved in dichloromethane (SmL). 3-(pyrrolidin-3-yl)-1H-indole (1eq) and acetic acid (0.2mL) were added. The reaction mixture was stirred at room temperature for 1 hour. Sodium triacetoxyborohydride (2eq) was added. The atmosphere was replaced with nitrogen gas. The mixture was stirred at room temperature for another 1 hour. Ammonium chloride (5mL) was added. The resulting mixture was extracted with dichloromethane (5mL×3). The combinded organic phases were washed with saturated brine (20mL×2), dried over anhydrous sodium sulfate, and concentrated to dryness to give a crude product. The crude product was purified by preparative HPLC (C18, 0.05% formic acid in water:acetonitrile) to produce the target compound (72.56mg).
LCMS (ESI) $[M+H]^+$=335.1; 1H NMR(400 MHz, MeOD-$d_4$)δ 8.49(s, 1H), 7.60(d, J=7.9 Hz, 1H), 7.39(d, J=8.1 Hz, 1H), 7.26-7.21(m, 2H), 7.20-7.13(m, 2H), 7.06(t, J=7.5 Hz, 1H), 6.97(d, J=8.2 Hz, 1H), 6.91(t, J=7.4 Hz, 1H), 3.94-3.83(m, 5H), 3.60(s, 2H), 3.41(s, 1H), 3.28(dd, J=11.4, 4.3 Hz, 2H), 2.76(t, J=7.4 Hz, 2H), 2.64-2.53(m, 1H), 2.36(ddd, J=13.1, 8.7, 4.7 Hz, 1H), 2.11-2.02(m, 2H).

Example 6 (Compound 6)

Preparation of 5-methoxy-1-(1-(3-(2-methoxyphenyl)propyl)pyrrolidin-3-yl)-1H-indole:

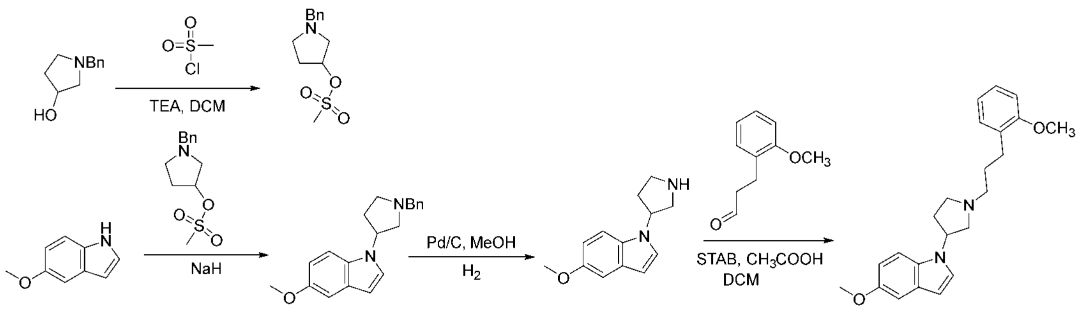


**Step 1:** Preparation of 1-benzylpyrrolidin-3-yl methanesulfonate:

1-benzylpyrrolidin-3-ol (4g, 1eq) was dissolved in dichloromethane (40mL). Triethylamine (4mL) was added, and methanesulfonyl chloride (1.5eq) was added under an ice bath. The reaction mixture was warmed up to room temperature and reacted for 1.5 hours. A saturated aqueous sodium bicarbonate solution was added to adjust pH > 10. The aqueous phase was extracted with dichloromethane (30mL×3). The organic phases were washed with saturated brine, then dried over anhydrous sodium sulfate, and concentrated to give the target product (4g).
LCMS (ESI) $[M+H]^+$=256.4.

**Step 2:** Preparation of 1-(1-benzylpyrrolidin-3-yl)-5-methoxy-1H-indole:

5-methoxy-1H-indole (1g, 1eq) was dissolved in tetrahydrofuran (10mL). Sodium hydride (3eq) was added. The reaction mixture was stirred at room temperature for 0.5 hours, then 1-benzylpyrrolidin-3-yl methanesulfonate (1.5eq) was added. The mixture was warmed up to 50°C, and reacted for another 16 hours. Water (20mL) was added. The mixture was extracted with ethyl acetate (30mL×2). The combined organic phases were washed with saturated brine (30mL×3), rotary-evaporated and dried over anhydrous sodium sulfate to give the target product (1.8g).
LCMS (ESI) $[M+H]^+$=307.5.

**Step 3:** Preparation of 5-methoxy-1-(pyrrolidin-3-yl)-1H-indole:

1-(1-benzylpyrrolidin-3-yl)-5-methoxy-1H-indole (1.7g, 1eq) was dissolved in methanol (30mL), and then palladium/carbon (500mg) was added. The resulting mixture was stirred in a hydrogen atmosphere at room temperature for 24 hours. The reaction mixture was filtered through diatomite. The filtrate was dried by rotatary evaporation to give a crude product. The crude product was purified by flash chromatography to produce the target compound (730mg).
LCMS (ESI) $[M+H]^+$=217.3.

**Step 4:** Preparation of 5-methoxy-1-(1-(3-(2-methoxyphenyl)propyl)pyrrolidin-3-yl)-1H-indole:

5-methoxy-1-(pyrrolidin-3-yl)-1H-indole (100mg, 1eq) and 3-(2-methoxyphenyl)propanal (1.3eq) were dissolved in dichloromethane (5mL). Acetic acid (0.1mL) was added, and then sodium triacetoxyborohydride (3eq) was slowly added. The mixture was stirred at room temperature, quenched by adding water, and extracted with ethyl acetate (10mL×3). The organic phases were combined, and dried over anhydrous sodium sulfate to produce a crude product. The crude product was purified by preparative HPLC (C18, 0.05% formic acid in water:acetonitrile) to produce the target compound (17.76mg).

LCMS (ESI) $[M+H]^+$=365.3; $^1H$ NMR(400 MHz, $CDCl_3$)δ 7.34(d, J=8.8 Hz, 2H), 7.19(d, J=1.4 Hz, 1H), 7.13(dd, J=7.4, 1.6 Hz, 1H), 7.08(d, J=2.4 Hz, 1H), 6.87(dt, J=10.9, 7.3 Hz, 3H), 6.44(d, J=3.1 Hz, 1H), 5.08(s, 1H), 3.84(s, 3H), 3.80(s, 3H), 3.12(s, 3H), 2.71(pd, J=13.5, 7.3 Hz, 4H), 2.54(s, 1H), 2.23(s, 1H), 1.93(d, J=6.8 Hz, 3H).

Examples 7-9 (Compounds 7, 8 and 9)

With reference to the preparation of Example 5, compounds 7-9 were prepared:

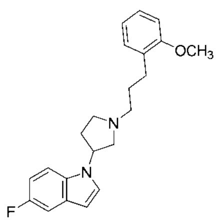 (7) 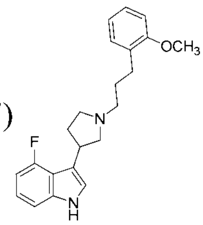 (8) 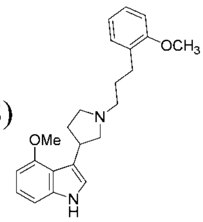 (9)

Compound 7: LCMS (ESI) $[M+H]^+$=353.2;
Compound 8: LCMS (ESI) $[M+H]^+$=353.2; $^1H$ NMR(400 MHz, $CDCl_3$)δ 8.79(s, 1H), 8.49(s, 1H), 7.25-7.15(m, 2H), 7.15-7.03(m, 3H), 6.94-6.82(m, 2H), 6.76(dd, J=11.6, 7.7 Hz, 1H), 4.03-3.93(m, 1H), 3.82(s, 3H), 3.66(s, 1H), 3.52-3.25(m, 3H), 3.11-3.04(m, 2H), 2.71(t, J=7.4 Hz, 2H), 2.47(dd, J=13.4,5.7 Hz, 1H), 2.33-2.23(m, 1H), 2.17-2.05(m, 2H);
Compound 9: LCMS (ESI) $[M+H]^+$=365.2; $^1H$ NMR(400 MHz, $CDCl_3$)δ 8.72(s, 1H), 8.27(s, 1H), 7.21-7.04(m, 3H), 6.99-6.80(m, 4H), 6.48(d, J=7.8 Hz, 1H), 4.00(s, 1H), 3.89(s, 3H), 3.80(s, 3H), 3.41(s, 1H), 3.18(s, 1H), 2.94(s, 1H), 2.78(d, J=9.2 Hz, 3H), 2.68(t, J=7.5 Hz, 2H), 2.37(td, J=13.4, 8.0 Hz, 1H), 2.07(dd, J=19.7, 7.6 Hz, 1H), 2.02-1.90(m, 2H).

Example 10 (Compound 10)

Preparation of 3-(2-(4-(2-methoxyphenyl)piperidin-1-yl)ethyl)-1H-indol-4-ol:

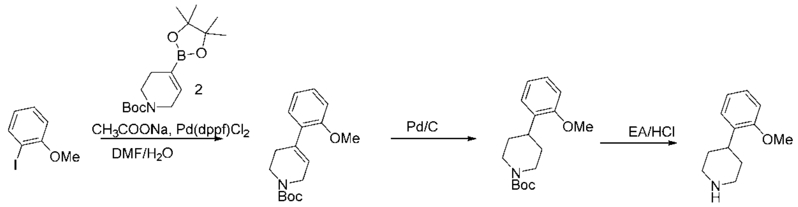


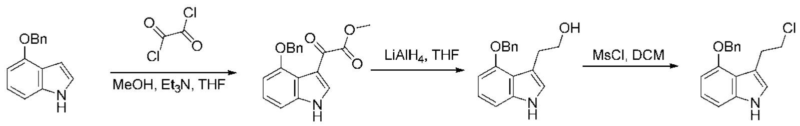


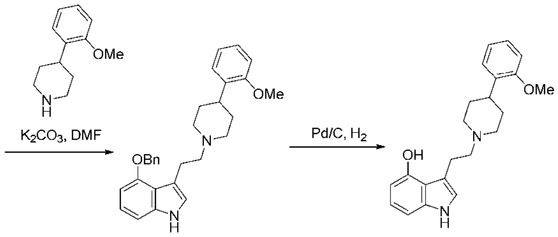


**Step** 1: Preparation of tert-butyl 4-(2-methoxyphenyl)-3,6-dihydropyridine-1-carboxylate:

1-iodo-2-methoxybenzene (5g, 1eq) was dissolved in a mixed solution of N,N-dimethylformamide (40mL) and water (3mL). Tert-butyl 4-(4,4,5,5-tetramethyl-1,3,2-dioxaborolan-2-yl)-3,6-dihydropyridine-1-carboxylate (1.2eq), sodium acetate (1.5eq) and (1,1'-bis(diphenylphosphino)ferrocene)palladium (II) dichloride (0.05eq) were added sequentially. The reaction mixture was stirred overnight at 95°C in a nitrogen atmosphere, then diluted with water (100mL), and extracted with ethyl acetate (50mL×3). The combined organic phases were washed with saturated brine (100mL×2), dried over anhydrous sodium sulfate, and concentrated to dryness to give a crude product. The crude product was purified by flash chromatography to give the target product (2.1g). LCMS (ESI) [M-100+H]+=190.2.

**Step 2:** Preparation of tert-butyl 4-(2-methoxyphenyl)piperidine-1-carboxylate:

Tert-butyl 4-(2-methoxyphenyl)-3,6-dihydropyridine-1-carboxylate (1.9g, 1eq) was dissolved in ethyl acetate (6mL). Palladium/carbon (0.5eq) was added. The atmosphere was replaced with hydrogen gas three times. Under a hydrogen atmosphere, the reaction mixture was stirred at room temperature for 3 hours. The mixture was filtered through diatomite and washed with methanol. The filtrate was concentrated to produce the target compound (1.2g, crude).
LCMS (ESI) [M-56+42]+=277.1.

**Step 3:** Preparation of 4-(2-methoxyphenyl)piperidine:

Tert-butyl 4-(2-methoxyphenyl)piperidine-1-carboxylate (1g, crude) was dissolved in ethyl acetate (6mL). Hydrochloric acid/ethyl acetate (6mL, 3M) was added. The reaction mixture was reacted at room temperature for 2 hours. A white solid precipitated, and LCMS indicated the completion of the reaction. The mixture was filtered, and the filter cake was dried to give the target product (600 mg).
LCMS (ESI) [M+H]+=192.1.

**Step 4:** Preparation of methyl 2-(4-(benzyloxy)-1H-indol-3-yl)-2-oxoacetate:

4-(benzyloxy)-1H-indole (5g, 1eq) was dissolved in anhydrous tetrahydrofuran (50mL). Oxalyl dichloride (1.2eq) was slowly added dropwise at 0°C. The reaction mixture was stirred at 0°C for 4 hours. At 0°C, methanol (6mL) was slowly added dropwise to the reaction mixture. Then triethylamine (4mL) was slowly added dropwise. The reaction mixture was refluxed at 70°C for 2 hours, then concentrated to remove the solvent, diluted with water (20mL), and extracted with ethyl acetate (20mL×3). The combinded organic phases were washed with saturated brine (50mL×2), dried and concentrated to give a crude product. The crude product was purified by flash chromatography to produce the target compound (3g).
LCMS (ESI) [M+H]+=310.1.

**Step 5:** Preparation of 2-(4-(benzyloxy)-1H-indol-3-yl)ethan-1-ol:

Methyl 2-(4-(benzyloxy)-1H-indol-3-yl)-2-oxoacetate (3g, 1eq) was dissolved in anhydrous tetrahydrofuran (30mL). A solution of lithium aluminium hydride in tetrahydrofuran (19.4mL, 1M) was slowly added dropwise at 0°C. The reaction mixture was stirred for 1 hour, and then heated and stirred overnight at 70°C. Water was slowly added dropwise at 0°C to quench the reaction. The reaction mixture was filtered. The filter cake was washed several times with ethyl acetate. The filtrate was washed with saturated brine (20mL×2). The organic phases were concentrated to give a crude product. The crude product was purified by flash chromatography to produce the target compound (2g).

LCMS (ESI) [M+H]+=268.1.

**Step 6:** Preparation of 4-(benzyloxy)-3-(2-chloroethyl)-1H-indole:

2-(4-(benzyloxy)-1H-indol-3-yl)ethan-1-ol (2.0g, 1eq) was dissolved in dichloromethane (30mL). Triethylamine (3eq) was added. Methanesulfonyl chloride (1.1eq) was slowly added at 0°C. The reaction mixture was stirred at 0°C for 4 hours. TLC detection showed the completion of the reaction. The mixture was diluted with water (10mL), separated into phases, and extracted with dichloromethane (20mL×3). The combinded organic phases were washed with saturated brine (50mL×2), dried and concentrated to give a crude product. The crude product was purified by flash chromatography to give the target product (300mg, yield: 14.0%).

**Step 7:** Preparation of 4-(benzyloxy)-3-(2-(4-(2-methoxyphenyl)piperidin-1-yl)ethyl)-1H-indole:

4-(benzyloxy)-3-(2-chloroethyl)-1H-indole (280mg, 1eq) was dissolved in N,N-dimethylformamide (5mL). Potassium carbonate (271mg, 2eq) and 4-(2-methoxyphenyl)piperidine (187mg, 1eq) were added. The reaction mixture was stirred at 80°C overnight, diluted with water (10mL), and extracted with ethyl acetate (15mL×3). The combinded organic phases were washed with saturated brine (20mL×2), dried and concentrated to give a crude product. The crude product was purified by flash chromatography to give the target product (210mg).

LCMS (ESI) [M+H]+=441.2.

**Step 8:** Preparation of 3-(2-(4-(2-methoxyphenyl)piperidin-1-yl)ethyl)-1H-indol-4-ol:

4-(benzyloxy)-3-(2-(4-(2-methoxyphenyl)piperidin-1-yl)ethyl)-1H-indole (260mg, 1eq) was dissolved in methanol (5mL). Palladium/carbon (1eq) was added. Under a hydrogen atmosphere, the reaction mixture was stirred at room temperature for 5 minutes, and filtered. The filtrate was concentrated to give a crude product. The crude product was purified by preparative HPLC (C18, 0.05% formic acid in water:acetonitrile) to produce the target compound (75.33mg).

LCMS (ESI) [M+H]+=351.1; 1H NMR(400 MHz, DMSO-d6)δ 10.63(s, 1H), 8.16(s, 1H), 7.19(t, J=7.0 Hz, 2H), 6.99-6.91(m, 3H), 6.84-6.74(m, 2H), 6.31(d, J=7.3 Hz, 1H), 3.79(s, 3H), 3.19-3.14(m, 2H), 3.04-2.89(m, 4H), 2.83-2.75(m, 2H), 2.43-2.35(m, 2H), 1.81-1.70(m, 4H).

Example 11 (Compound 11)

Preparation of 3-(1-(3-(2-methoxyphenyl)propyl)azetidin-3-yl)-1H-indole:

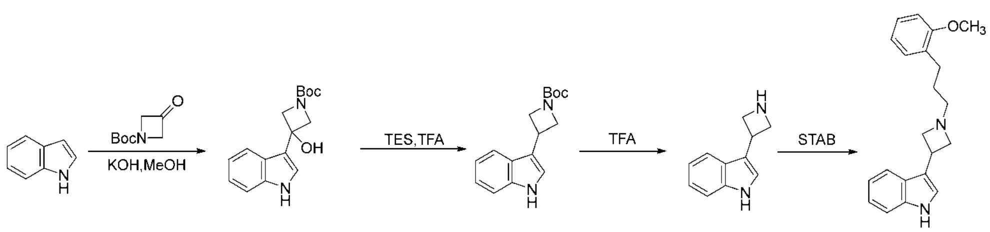


**Step** 1: Preparation of tert-butyl 3-hydroxy-3-(1H-indol-3-yl)azetidine-1-carboxylate:

Indole (0.5eq.) was added to a mixture of tert-butyl 3-oxoazetidine-1-carboxylate (1g, 1.0eq.) and potassium hydroxide (1.1eq.) in methanol (20mL). The reaction mixture was stirred at 50°C for 12 hours, then diluted with water, and extracted with ethyl acetate (20mL×3). The organic phases were combined, washed with saturated brine, dried over

anhydrous sodium sulfate, concentrated in vacuum, and purified by flash chromatography to produce the target compound (550mg).

LCMS (ESI) $[M+H-18]^+$=271.1.

**Step 2:** Preparation of tert-butyl 3-(1H-indol-3-yl)azetidine-1-carboxylate:

At 0°C, triethylsilane (7.5eq.) was added to a mixture of tert-butyl 3-hydroxy-3-(1H-indol-3-yl)azetidine-1-carboxylate (6g, 1.0eq.) in dichloromethane (60mL). Trifluoroacetic acid (3.3eq) was added. The mixture was stirred at 0°C for 10 minutes, warmed up to room temperature and stirred for another 20 minutes. The mixture was quenched with saturated sodium bicarbonate, and extracted with dichloromethane (100mL×3). The organic phases were combined, washed with saturated brine, dried over anhydrous sodium sulfate, concentrated in vacuum, and purified by flash chromatography to produce the target compound (2.2g).

LCMS (ESI) $[M+H-56+CH_3CN]^+$=258.2.

**Step 3:** Preparation of 3-(azetidin-3-yl)-1H-indole:

Trifluoroacetic acid (5mL) was added to a mixture of tert-butyl 3-(1H-indol-3-yl)azetidine-1-carboxylate (1g, 1.0eq.) in dichloromethane (20mL). The reaction mixture was stirred at room temperature for 0.5 hours. Dichloromethane (20mL) was added. The mixture was concentrated in vacuum, and purified by flash chromatography to produce the target compound (900mg, crude). LCMS (ESI) $[M+H]^+$=173.3.

**Step 4:** Preparation of 3-(1-(3-(2-methoxyphenyl)propyl)azetidin-3-yl)-1H-indole:

3-(2-methoxyphenyl)propanal (143mg, 1.0eq.) and 3-(azetidin-3-yl)-1H-indole (150mg, crude) were added to tetrahydrofuran (10mL). The reaction mixture was stirred at room temperature for 5 minutes. Sodium triacetoxyborohydride (2.0eq.) was added to the reaction system. The reaction mixture was stirred at room temperature for 2 hours, then diluted with water (20mL), and extracted with ethyl acetate (20mL×3). The organic phases were combined, dried over anhydrous sodium sulfate, concentrated in vacuum, and purified by preparative HPLC (C18, 0.1% formic acid in water:acetonitrile) to produce the target compound (22.99mg).

LCMS (ESI) $[M+H]^+$=321.2; $^1$H NMR(400 MHz, DMSO-d6)δ 11.02(s, 1H), 7.57(d, J=7.8 Hz, 1H), 7.36(dd, J=5.2, 2.8 Hz, 2H), 7.22-7.13(m, 2H), 7.11-7.06(m, 1H), 7.02-6.94(m, 2H), 6.88(td, J=7.4, 0.9 Hz, 1H), 4.11-4.05(m, 2H), 4.04-3.95(m, 1H), 3.78(s, 3H), 3.66(t, J=7.0 Hz, 2H), 2.86(t, J=7.4 Hz, 2H), 2.60(s, 2H), 1.72-1.62(m, 2H).

Examples 12-15 (Compounds 12-15)

With reference to the preparation of Example 4 or 5, compounds 12-15 were prepared:

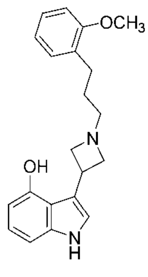 (12) 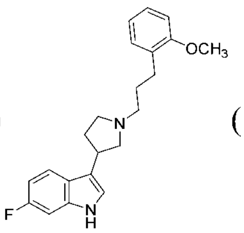 (13) 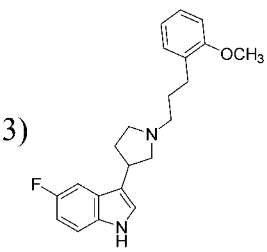 (14) 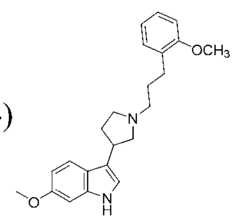 (15)

Compound 12: LCMS (ESI) $[M+H]^+$=337.2;
Compound 13: LCMS (ESI) $[M+H]^+$=353.2;
Compound 14: LCMS (ESI) $[M+H]^+$=353.2; $^1$H NMR(400 MHz, $CDCl_3$)δ 8.77(s, 1H), 8.61(s, 1H), 7.29(dd, J=8.8, 4.4 Hz, 1H), 7.24-7.15(m, 2H), 7.11(d, J=5.5 Hz, 2H), 6.98-6.82(m, 3H), 3.81(s, 3H), 3.76(d, J=8.3 Hz, 1H), 3.60(s, 1H), 3.35(d, J=27.4 Hz, 2H), 3.14(s, 1H), 3.00(dd, J=10.5, 5.8 Hz, 2H), 2.70(t, J=7.3 Hz, 2H), 2.45(dd, J=12.2, 5.3 Hz, 1H), 2.26-2.01(m, 3H).
Compound 15: LCMS (ESI) $[M+H]^+$=365.2; $^1$H NMR(400 MHz, DMSO-$d_6$)δ 10.62(s, 1H), 7.47(d, J=8.6 Hz, 1H), 7.21-7.12(m, 2H), 7.03(d, J=1.9 Hz, 1H), 6.94(d, J=8.1 Hz, 1H), 6.90-6.82(m, 2H), 6.63(dd, J=8.6, 2.2 Hz, 1H), 3.77(s, 3H), 3.74(s, 3H), 3.54(s, 1H), 3.24(d, J=8.8 Hz, 1H), 3.00-2.94(m, 1H), 2.86(d, J=5.4 Hz, 1H), 2.71(dt, J=15.7, 7.5 Hz, 3H), 2.63-2.58(m, 2H), 2.31-2.22(m, 1H), 1.93(dd, J=12.4, 7.6 Hz, 1H), 1.82-1.73(m, 2H).

Example 16 (Compound 16)

Preparation of 3-(7-(3-(2-methoxyphenyl)propyl)-7-azabicyclo[2.2.1]heptan-2-yl)-1H-indole:

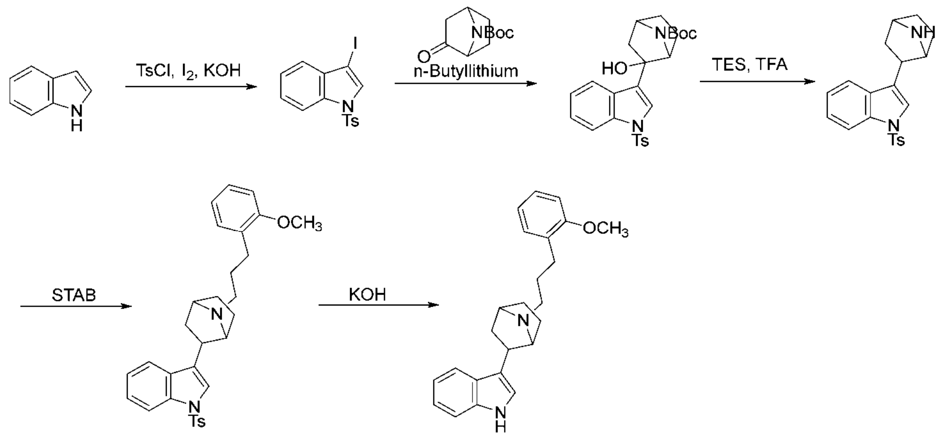


**Step** 1: Preparation of 3-iodo-1-(4-methylphenylsulfonyl)-1H-indole:

Indole (5g, 1.0eq.) was dissolved in N,N-dimethylformamide (70mL), and potassium hydroxide (2.5eq.) was added. The mixture was stirred at room temperature for 30 minutes. A solution of iodine (1.05 eq.) in N,N-dimethylformamide (70 mL) was added dropwise. The resulting mixture was stirred for another 1 hour. Then potassium hydroxide (2.5eq.) was added, followed by adding 4-methylbenzene-1-sulfonyl chloride (2.1eq.). The reaction system was stirred at room temperature for 15 hours. The reaction mixture was poured into water (500mL), and a solid precipitated. The solid was filtered off, washed with water, slurried with a small amount of isopropanol, then slurried with petroleum ether, filtered, and dried to produce the target compound (10 g).
$^1H$ NMR(400 MHz, $CDCl_3$)$\delta$ 7.97-7.93(m, 1H), 7.80-7.75(m, 2H), 7.69(s, 1H), 7.39-7.34(m, 2H), 7.31(dd, J=7.0, 1.4 Hz, 1H), 7.24(d, J=8.1 Hz, 2H), 2.35(s, 3H).

**Step 2:** Preparation of tert-butyl 2-hydroxy-2-(1-(4-methylphenylsulfonyl)-1H-indol-3-yl)-7-azabicyclo[2.2.1]heptane-7-carboxylate:

3-Iodo-1-(4-methylphenylsulfonyl)-1H-indole (50mg, 1.0eq.) was dissolved in tetrahydrofuran (1mL). At -78°C, n-butyllithium (1.6M, 0.01mL, 1.2eq.) was added dropwise. After addition, the mixture was kept at this temperature and stirred for 1 hour. A solution of tert-butyl 2-oxo-7-azabicyclo[2.2.1]heptane-7-carboxylate (1.1eq.) in tetrahydrofuran (1mL) was added dropwise. The reaction system was stirred at -78°C for 1 hour, diluted with water (5mL), extracted with ethyl acetate (10mL×3). The organic phases were combined, dried over anhydrous sodium sulfate, filtered, and concentrated to give a crude product. The crude product was purified by TLC to produce the target compound (20mg).
LCMS (ESI) $[M+H-56-H_2O]^+$=409.3.

**Step 3:** Preparation of 3-(7-azabicyclo[2.2.1]heptan-2-yl)-1-(4-methylphenylsulfonyl)-1H-indole:

Tert-butyl 2-hydroxy-2-(1-(4-methylphenylsulfonyl)-1H-indol-3-yl)-7-azabicyclo[2.2.1]heptane-7-carboxylate (300mg, 1.0eq.) was dissolved in anhydrous dichloromethane (3mL), and then trifluoroacetic acid (5.5eq.) and triethylsilane (2.5eq.) were added sequentially. The reaction system was stirred at room temperature for 1 hour. Additional trifluoroacetic acid (5.5eq.) and triethylsilane (2.5eq.) were added. The mixture was stirred for another 2 hours. The reaction mixture was concentrated to remove trifluoroacetic acid to give a crude product. The crude product was purified by flash chromatography (C18, 0.1% formic acid/water, ACN) to produce the target compound (150mg).
LCMS (ESI) $[M+H]^+$=367.3.

**Step 4:** Preparation of 3-(7-(3-(2-methoxyphenyl)propyl)-7-azabicyclo[2.2.1]heptan-2-yl)-1-(4-methylphenylsulfonyl)-1H-indole:

3-(2-Methoxyphenyl)propanal (1.5eq.) and 3-(7-azabicyclo[2.2.1]heptan-2-yl)-1-(4-methylphenylsulfonyl)-1H-indole (120mg, 1.0eq.) were dissolved in dichloromethane (5mL). Acetic acid (0.3mL) was added. The reaction mixture was stirred at room temperature for 30 minutes. Then sodium triacetoxyborohydride (3.0eq.) was added. The reaction system was stirred at room temperature for 2.5 hours, diluted with water (15mL), extracted with dichloromethane (15mL×3). The organic phases were combined, dried over anhydrous sodium sulfate, filtered, and concentrated to give a crude product. The crude product was purified by flash chromatography (C18, 0.1% formic acid in water:acetonitrile) to produce the target compound (140mg).

LCMS (ESI) $[M+H]^+$=515.5.

**Step 5:** Preparation of 3-(7-(3-(2-methoxyphenyl)propyl)-7-azabicyclo[2.2.1]heptan-2-yl)-1H-indole:

3-(7-(3-(2-methoxyphenyl)propyl)-7-azabicyclo[2.2.1]heptan-2-yl)-1-(4-methylphenylsulfonyl)-1H-indole (120mg, 1.0eq.) was dissolved in methanol (5mL). Potassium hydroxide (10eq.) was added. The reaction mixture was stirred at 70°C for 40 hours, diluted with dichloromethane and water (15mL), and extracted with dichloromethane and methanol (10:1, 15mL×3). The organic phases were combined, dried over anhydrous sodium sulfate, filtered, and concentrated to give a crude product, and purified by preparative HPLC (C18, 0.1% trifluoroacetic acid in water: acetonitrile) to produce the target compound (39mg).

LCMS (ESI) $[M+H]^+$=361.3; $^1$H NMR(400 MHz, $CDCl_3$)δ 8.80(s, 1H), 8.66(s, 1H), 7.59(s, 1H), 7.42(d, J=8.1 Hz, 1H), 7.24-7.21(m, 1H), 7.19(d, J=7.4 Hz, 1H), 7.11(t, J=7.0 Hz, 2H), 6.95(d, J=2.4 Hz, 1H), 6.90(d, J=7.4 Hz, 1H), 6.85(d, J=8.2 Hz, 1H), 4.01(s, 1H), 3.81(s, 3H), 3.13-3.08(m, 1H), 2.95-2.90(m, 1H), 2.70(dt, J=13.5, 6.7 Hz, 3H), 2.23-1.83(m, 9H).

Example 17 (Compound 17)

With reference to the preparation of Example 16, compound 17 was prepared:

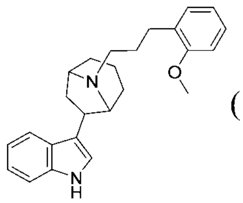

(17)

LCMS (ESI) $[M+H]^+$=375.2.

Example 18 (Compound 18)

Preparation of 3-(4-fluoro-1-(3-(2-methoxyphenyl)propyl)pyrrolidin-3-yl)-1H-indole:

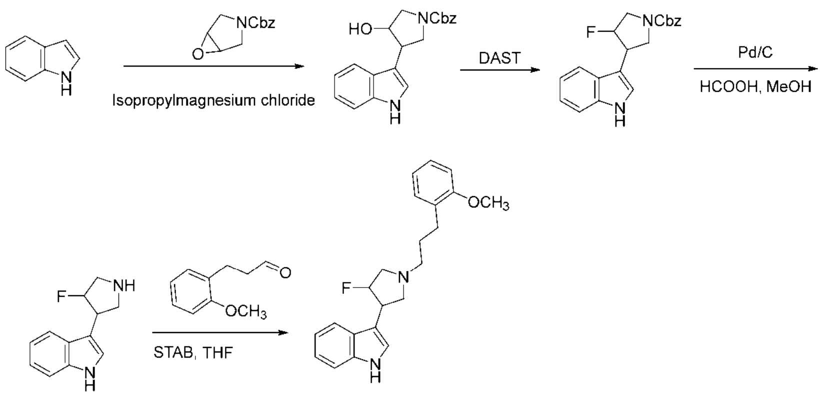


**Step 1:** Preparation of benzyl 3-hydroxy-4-(1H-indol-3-yl)pyrrolidine-1-carboxylate:

Indole (1.8g, 1.0eq) was dissolved in tetrahydrofuran (10mL). At 0°C, a 3M solution of isopropylmagnesium

chloride in tetrahydrofuran (1.0eq) was added dropwise, and the mixture was stirred at 0°C for 0.5 hours. 3-N-benzyloxycarbonyl-6-oxa-3-azabicyclo[3.1.0]hexane (1.0eq) was added. The reaction mixture was stirred at room temperature for 3.5 hours, diluted with saturated sodium bicarbonate solution (50mL), and extracted with ethyl acetate (50mL×3). The organic phases were combined, dried over anhydrous sodium sulfate, and concentrated to give a crude product. The crude product was purified by flash chromatography to produce the target compound (1.2g).

LCMS (ESI) $[M+Na]^+$=359.2.

**Step 2:** Preparation of benzyl 3-fluoro-4-(1H-indol-3-yl)pyrrolidine-1-carboxylate:

Benzyl 3-hydroxy-4-(1H-indol-3-yl)pyrrolidine-1-carboxylate (1g, 1.0eq) was dissolved in dichloromethane (SmL). At -78°C, diethylaminosulfur trifluoride (DAST, 1.2eq) was added. The reaction mixture was stirred at room temperature for 2 hours, diluted with water (200mL), extracted with ethyl acetate (200mL×3). The organic phases were combined, dried over anhydrous sodium sulfate, and concentrated to give a crude product. The crude product was purified by flash chromatography to produce the target compound (0.41g).

LCMS (ESI) $[M+Na]^+$=361.1.

**Step 3:** Preparation of 3-(4-fluoropyrrolidin-3-yl)-1H-indole:

Benzyl 3-fluoro-4-(1H-indol-3-yl)pyrrolidine-1-carboxylate (410mg, 1.0eq) was dissolved in methanol (5mL). Palladium/carbon (0.1eq) was added. Two drops of formic acid was added dropwise. The atmosphere was replaced with hydrogen gas. The reaction mixture was stirred at room temperature for 16 hours. LCMS detection showed the completion of the reaction. The reaction mixture was filtered by suction, and concentrated to give a crude product. The crude product was purified by reverse-phase flash chromatography (C18, 0.1% formic acid/$H_2O$, ACN) to produce the target compound (162mg).

LCMS (ESI) $[M+H]^+$=205.1.

**Step 4:** Preparation of 3-(4-fluoro-1-(3-(2-methoxyphenyl)propyl)pyrrolidin-3-yl)-1H-indole:

3-(4-Fluoropyrrolidin-3-yl)-1H-indole (100mg, 1.0eq) and 3-(2-methoxyphenyl)propanal (80mg, 1.0eq) were dissolved in tetrahydrofuran (5mL). Glacial acetic acid (3mg, 0.1eq) was added. The reaction mixture was stirred at room temperature for 1 hour. Sodium triacetoxyborohydride (208mg, 2.0eq) was added. The reaction mixture was stirred at room temperature for 3 hours, diluted with saturated sodium bicarbonate solution (50mL), and extracted with ethyl acetate (50mL×3). The organic phases were combined, washed with saturated brine, dried over anhydrous sodium sulfate, filtered by suction, and concentrated to give a crude product. The crude product was purified by preparative HPLC (C18, 0.05% formic acid in water:acetonitrile) to produce the target compound (49.70mg).

LCMS (ESI) $[M+H]^+$=353.2; $^1H$ NMR(400 MHz, DMSO-$d_6$)δ 10.92(s, 1H), 8.17(s, 1H), 7.56(d, J=7.9 Hz, 1H), 7.35(d, J=8.1 Hz, 1H), 7.24(d, J=2.2 Hz, 1H), 7.21-7.13(m, 2H), 7.12-7.06(m, 1H), 7.03-6.92(m, 2H), 6.86(td, J=7.4, 1.0 Hz, 1H), 5.15(d, J=55.8 Hz, 1H), 3.78(s, 3H), 3.65(s, 1H), 3.32(s, 1H), 3.07(d, J=12.7 Hz, 1H), 2.75-2.56(m, 4H), 2.47-2.37(m, 2H), 1.73(p, J=7.3 Hz, 2H).

Example 19 (Compound 19)

With reference to the preparation of Example 11, compound 19 was prepared:

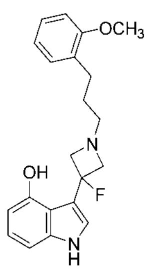

(19)

LCMS (ESI) $[M+H]^+$=355.2.

Example 20 (Compound 20)

Preparation of 3-(1-(3-(4,5-difluoro-2-methoxyphenyl)propyl)pyrrolidin-3-yl)-1H-indole:

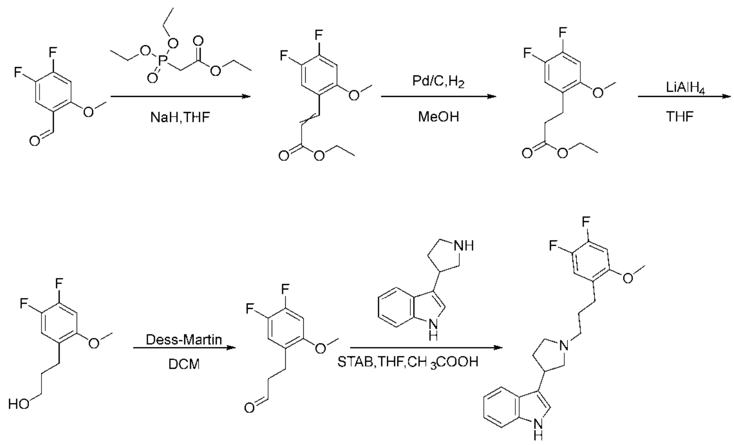


**Step** 1: Preparation of ethyl 3-(4,5-difluoro-2-methoxyphenyl)acrylate:

Sodium hydride (1.3eq.) was dissolved in tetrahydrofuran (30mL). At 0°C, a solution of ethyl 2-(diethoxyphosphoryl)acetate (1.1eq.) in tetrahydrofuran (70mL) was added dropwise. At 0°C, the mixture was stirred for 0.5 hours. Then a solution of 4,5-difluoro-2-methoxybenzaldehyde (4.7g, 1.0eq.) in tetrahydrofuran(50mL) was added dropwise. The mixture was reacted at 60°C for 12 hours, quenched by adding water, dried by rotary-evaporation, and purified by flash chromatography (C18, 0.1% formic acid in water:acetonitrile) to produce the target compound (3.8g).

LCMS (ESI) $[M+H]^+$=243.1.

**Step 2:** Preparation of ethyl 3-(4,5-difluoro-2-methoxyphenyl)propanoate:

Ethyl 3-(4,5-difluoro-2-methoxyphenyl)acrylate (3.5g, 1.0eq.) was dissolved in methanol (35mL). 10% palladium/carbon (1g) was added. Under a hydrogen atmosphere, the mixture was reacted at room temperature for 1 hour, and filtered. The filtrate was dried by rotary-evaporation to produce the target compound (3.2g).
LCMS (ESI) $[M+H]^+$=245.1.

**Step 3:** Preparation of 3-(4,5-difluoro-2-methoxyphenyl)propan-1-ol:

Ethyl 3-(4,5-difluoro-2-methoxyphenyl)propanoate (1.50g, 1.0eq.) was dissolved in tetrahydrofuran (15mL). Under an ice bath, lithium aluminium hydride (2.0eq.) was slowly added. the mixture was warmed up to room temperature and reacted for 1 hour, quenched by adding water (1mL), dried over anhydrous sodium sulfate, and filtered. The filtrate was dried by rotary-evaporation, purified by flash chromatography (Silica gel, petroleum ether:ethyl acetate=4: 1) to produce the target compound (800mg).
LCMS (ESI) $[M+H+ACN]^+$=244.2.

**Step 4:** Preparation of 3-(4,5-difluoro-2-methoxyphenyl)propanal:

3-(4,5-difluoro-2-methoxyphenyl)propan-1-ol (400mg, 1.0eq.) was dissolved in dichloromethane (10mL). Under an ice bath, Dess-Martin periodinane (2.0eq.) was slowly added. The mixture was warmed up to room temperature and reacted for 1 hour. TLC detection showed the completion of the reaction. The reaction mixture was quenched by adding a saturated aqueous ammonium chloride solution, filtered to remove the solid, dried by rotary-evaporation to remove dichloromethane, diluted with water, extracted with ethyl acetate three times. The organic phases were combined, washed with saturated brine, dried by rotary-evaporation, and purified by flash chromatography to produce the target compound (250mg).

**Step 5:** Preparation of 3-(1-(3-(4,5-difluoro-2-methoxyphenyl)propyl)pyrrolidin-3-yl)-1H-indole:

3-(4,5-difluoro-2-methoxyphenyl)propanal (100mg, 1.0eq.) and 3-(pyrrolidin-3-yl)-1H-indole (1.0eq.) were dissolved in tetrahydrofuran (SmL). Acetic acid (0.5mL) was added. Sodium triacetoxyborohydride (3.0eq.) was added. The reaction mixture was stirred at room temperature for 1 hour, diluted with water (10mL), extracted with ethyl acetate (5mL×3). The organic phases were combined, washed with saturated brine, dried over anhydrous sodium sulfate, dried by rotary-evaporation, and purified by preparative HPLC (C18, 0.05% formic acid in water:acetonitrile) to produce the target compound (9.78mg).

LCMS (ESI) $[M+H]^+$=371.2; $^1H$ NMR(400 MHz, $CDCl_3$)δ 8.59(s, 1H), 8.31(s, 1H), 7.57(d, J=7.9 Hz, 1H), 7.40(d, J=8.1 Hz, 1H), 7.20(s, 1H), 7.14(dd, J=11.0, 3.9 Hz, 2H), 6.94(dd, J=10.5, 9.0 Hz, 1H), 6.66(dd, J=12.1, 6.7 Hz, 1H), 3.97-3.87(m, 1H), 3.77(s, 3H), 3.72(s, 1H), 3.49(s, 1H), 3.31(s, 1H), 3.16(s, 1H), 3.04-2.96(m, 2H), 2.64(t, J=7.5 Hz, 2H), 2.53(d, J=7.5 Hz, 1H), 2.30(dd, J=13.0, 9.1 Hz, 1H), 2.16-2.05(m, 2H).

Example 21 (Compound 21)

Preparation of 3-(1-(3-(2-methoxyphenyl)propyl)pyrrolidin-3-yl)-1H-pyrrolo[3,2-b]pyridine:

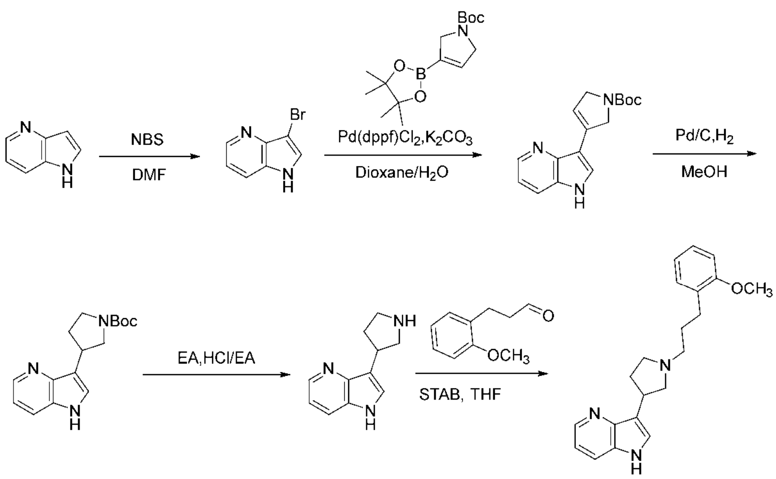


**Step 1:** Preparation of 3-bromo-1H-pyrrolo[3,2-b]pyridine:

At 0°C, N-bromosuccinimide (NBS, 1.51g, 1.0eq.) was added to a mixture of 1H-pyrrolo[3,2-b]pyridine (1.0eq) in N,N-dimethylformamide (20mL). The resulting mixture was stirred for 0.5 hours. Water (20mL) was added, and a large amount of solid precipitated. The solid was filtered and collected to give the target compound (989mg).

LCMS (ESI) $[M+H]^+$=199.1.

**Step 2:** Preparation of tert-butyl 3-(1H-pyrrolo[3,2-b]pyridin-3-yl)-2,5-dihydro-1H-pyrrole-1-carboxylate:

At room temperature, (1,1'-bis(diphenylphosphino)ferrocene)palladium dichloride (II) (0.1eq.) was added to a mixture of 3-bromo-1H-pyrrolo[3,2-b]pyridine (600mg, 1.0eq), tert-butyl 3-(4,4,5,5-tetramethyl-1,3,2-dioxaborolan-2-yl)-2,5-dihydro-1H-pyrrole-1-carboxylate (1.0eq.), and potassium carbonate (3.0eq.) in 1,4-dioxane (20mL) and water (2mL). In a nitrogen atmosphere, the resulting mixture was warmed up to 90°C and stirred for 6 hours, diluted with water (30mL), extracted with ethyl acetate (30mL×3). The organic phases were combined, washed with saturated brine, dried over anhydrous sodium sulfate, concentrated in vacuum, and purified by flash chromatography (Silica gel, PE:EA=1:1) to produce the target compound (400mg).

LCMS (ESI) $[M+H]^+$=286.2.

**Step 3:** Preparation of tert-butyl 3-(1H-pyrrolo[3,2-b]pyridin-3-yl)pyrrolidine-1-carboxylate:

Palladium/carbon (100mg) was added to a mixture of tert-butyl 3-(1H-pyrrolo[3,2-b]pyridin-3-yl)-2,5-dihydro-1H-pyrrole-1-carboxylate (400mg, 1.0eq.) in methanol (10mL). In a hydrogen atmosphere, the reaction mixture was stirred at

room temperature for 3 hours, and filtered. The filtrate was diluted with water (30mL), and extracted with ethyl acetate (20mL×3). The organic phases were combined, washed with saturated brine, dried over anhydrous sodium sulfate, filtered, concentrated in vacuum, and purified by flash chromatography (Silica gel, PE:EA=3:1) to produce the target compound (145mg).

LCMS (ESI) $[M+H]^+$=288.2.

**Step 4:** Preparation of 3-(pyrrolidin-3-yl)-1H-pyrrolo[3,2-b]pyridine:

At room temperature, tert-butyl 3-(1H-pyrrolo[3,2-b]pyridin-3-yl)pyrrolidine-1-carboxylate (140mg, 1.0eq.) was added to a solution of hydrochloric acid/ethyl acetate (4M, 2mL) and ethyl acetate (5mL). The mixture was stirred for 2 hours, filtered to remove the solid, dried to produce the target compound (100mg, crude).

LCMS (ESI) $[M+H]^+$=188.2.

**Step 5:** Preparation of 3-(1-(3-(2-methoxyphenyl)propyl)pyrrolidin-3-yl)-1H-pyrrolo[3,2-b]pyridine:

At room temperature, 3-(2-methoxyphenyl)propanal (88mg, 1.0eq.) was added to a solution of 3-(pyrrolidin-3-yl)-1H-pyrrolo[3,2-b]pyridine (1.0eq.) in tetrahydrofuran (SmL). The mixture was stirred for 5 minutes. Acetic acid (0.1mL) and sodium triacetoxyborohydride (2.0eq) were added. The resulting mixture was stirred for 2 hours, diluted with saturated sodium bicarbonate (15mL), and extracted with ethyl acetate (10mL×3). The organic phases were combined, washed with saturated brine, dried over anhydrous sodium sulfate, filtered, concentrated in vacuum, and purified by preparative HPLC (C18, 0.1% formic acid in water:acetonitrile) to produce the target compound (23.66mg).

LCMS (ESI) $[M+H]^+$=336.2; $^1$H NMR(400 MHz, DMSO-$d_6$)δ 11.11(s, 1H), 8.29(dd, J=4.6, 1.4 Hz, 1H), 7.72(dd, J=8.2,1.4 Hz, 1H), 7.50(d, J=2.4 Hz, 1H), 7.17(ddd, J=9.3, 8.3, 1.6 Hz, 2H), 7.09(dd, J=8.2, 4.6 Hz, 1H), 6.95(d, J=7.7 Hz, 1H), 6.87(td, J=7.4, 1.0 Hz, 1H), 3.77(s, 3H), 3.70(d, J=8.0 Hz, 1H), 3.37-3.32(m, 1H), 3.09-3.02(m, 1H), 2.97-2.87(m, 2H), 2.76(dd, J=13.9, 7.0 Hz, 2H), 2.65-2.59(m, 2H), 2.35-2.28(m, 1H), 2.07(dd, J=11.8, 7.3 Hz, 1H), 1.84-1.75(m, 2H).

Examples 22-23

With reference to the preparation of Example 21, compounds 22-23 were prepared:

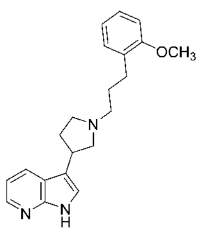 (22) 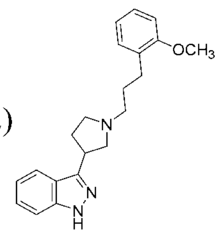 (23)

Compound 22: LCMS (ESI) $[M+H]^+$=336.2; $^1$H NMR(400 MHz, DMSO-$d_6$)δ 11.34(s, 1H), 8.22(s, 1H), 8.18(dd, J=4.6, 1.5 Hz, 1H), 8.07-8.02(m, 1H), 7.29(d, J=2.3 Hz, 1H), 7.16(ddd, J=9.4, 8.3, 1.6 Hz, 2H), 7.01(dd, J=7.9, 4.7 Hz, 1H), 6.94(d, J=7.6 Hz, 1H), 6.86(td, J=7.4, 1.0 Hz, 1H), 3.76(s, 3H), 3.58-3.52(m, 1H), 3.11(t, J=8.5 Hz, 1H), 2.87-2.76(m, 2H), 2.69-2.55(m, 5H), 2.34-2.22(m, 1H), 1.92(td, *J=15.5,* 8.1 Hz, 1H), 1.75(dq, J=15.1, 7.5 Hz, 2H).

Compound 23: LCMS (ESI) $[M+H]^+$=336.1; $^1$H NMR(400 MHz, $CDCl_3$)δ 8.65(s, 1H), 7.67(d, J=8.2 Hz, 1H), 7.47(d, J=8.4 Hz, 1H), 7.41-7.34(m, 1H), 7.23-7.07(m, 3H), 6.86(ddd, J=11.6, 9.0, 4.6 Hz, 2H), 4.15-4.01(m, 1H), 4.00-3.84(m, 1H), 3.80(s, 3H), 3.62(t, J=11.6 Hz, 1H), 3.34(dd, J=10.8, 8.3 Hz, 1H), 3.03(dd, J=8.7, 2.8 Hz, 3H), 2.69(t, J=7.4 Hz, 2H), 2.60(dd, J=13.0, 8.1 Hz, 1H), 2.28(td, J=6.3, 1.5 Hz, 1H), 2.08(dd, J=9.1, 7.5 Hz, 2H).

Examples 24-28

With reference to the preparation of Example 16, 18 or 21, compounds 24-28 were prepared:

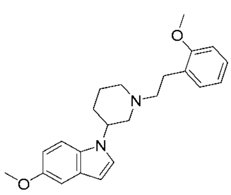 (24) 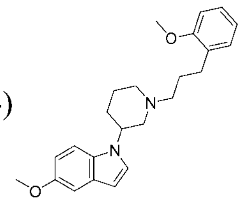 (25) 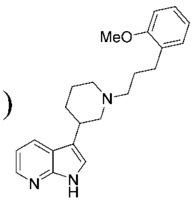 (26)

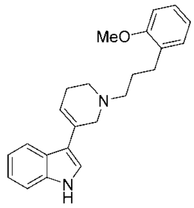 (27) 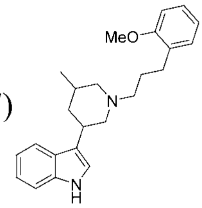 (28)

Compound 24: LCMS (ESI) $[M+H]^+$=365.2;
Compound 25: LCMS (ESI) $[M+H]^+$=379.2;
Compound 26: LCMS (ESI) $[M+H]^+$=350.2;
Compound 27: LCMS (ESI) $[M+H]^+$=347.2;
Compound 28: LCMS (ESI) $[M+H]^+$=363.2.

Example 29 (Compound 29)

Preparation of 2-(1H-indol-3-yl)-4-(3-(2-methoxyphenyl)propyl)morpholine:

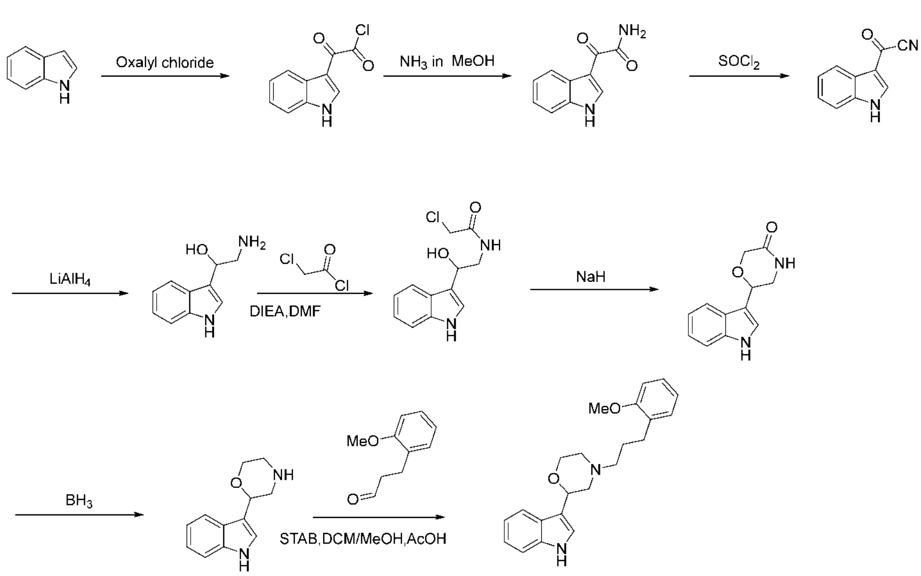


**Step 1:** Preparation of 2-(1H-indol-3-yl)-2-oxoacetyl chloride:

Indole (15.0g, 1.0eq.) was dissolved in tetrahydrofuran (300mL). The atmosphere was replaced with nitrogen gas. At 0°C, oxalyl dichloride (1.0eq.) was added dropwise.. After the completion of the addition, the reaction mixture was stirred at room temperature for 0.5 hours. A large amount of the solid precipitated. To the reaction mixture was added dropwise petroleum ether (300mL). After the completion of the addition, the reaction mixture was stirred at room temperature for 10 minutes, and filtered by suction. The filter cake was concentrated to remove the solvent to produce the target compound (21.7mg).

**Step 2:** Preparation of 2-(1H-indol-3-yl)-2-oxoacetamide:

2-(1H-indol-3-yl)-2-oxoacetyl chloride (11g, 1.0eq.) was dissolved in a solution of ammonia in methanol (19.82eq.). The reaction mixture was stirred at room temperature for 8 hours, concentrated, diluted with water (200mL), and extracted with ethyl acetate (200mL×3). The organic phases were combined, washed with saturated brine, dried over anhydrous sodium sulfate, and filtered by suction. The mother liquor was concentrated to produce the target compound (3.5g).
LCMS (ESI) $[M+H]^+$=189.2.

**Step 3:** Preparation of 1H-indole-3-carbonyl cyanide:

2-(1H-indol-3-yl)-2-oxoacetamide (5.6g, 1.0eq.) was dissolved in N,N-dimethylformamide (50mL). At 0°C, sulfurous dichloride (15.83eq.) was added. The reaction mixture was stirred at room temperature for 2 hours, diluted with water (150mL), and extracted with ethyl acetate (100mL×3). The organic phases were combined, washed with saturated brine, dried over anhydrous sodium sulfate, and filtered by suction. The mother liquor was concentrated to produce the target compound (4.5g).
LCMS (ESI) $[M+H]^+$=171.1.

**Step 4:** Preparation of 2-amino-1-(1H-indol-3-yl)ethan-1-ol:

1H-indole-3-carbonyl cyanide(1.5g, 1.0eq.) was dissolved in tetrahydrofuran (20mL). At -10°C, lithium aluminium hydride (2.0eq.) was added. At -10°C, the mixture was stirred for 4 hours, quenched by adding water (20mL), and extracted with ethyl acetate (50mL×3). The organic phases were combined, washed with saturated brine, dried over anhydrous sodium sulfate, and filtered by suction. The mother liquor was concentrated to give a crude product. The crude product was purified by flash chromatography to produce the target compound (0.8g).
LCMS (ESI) $[M+H-H_2O]^+$=159.2.

**Step 5:** Preparation of 2-chloro-N-(2-hydroxy-2-(1H-indol-3-yl)ethyl)acetamide:

2-amino-1-(1H-indol-3-yl)ethan-1-ol (500mg, 1.0eq.) was dissolved in N,N-dimethylformamide (20mL). Diisopropylethylamine (2.0eq.) was added. At 0°C, 2-chloroacetyl chloride (1.1eq.) was added dropwise. The reaction mixture was stirred at room temperature for 2 hours, diluted with saturated sodium bicarbonate solution (20mL) at room temperature, and extracted with dichloromethane (20mL×3). The organic phases were combined, washed with saturated brine, dried over anhydrous sodium sulfate, and filtered by suction. The mother liquor was concentrated to give a crude product. The crude product was purified by flash chromatography to produce the target compound (300mg).
LCMS (ESI) $[M+H-H_2O]^+$=235.1.

**Step 6:** Preparation of 6-(1H-indol-3-yl)morpholin-3-one:

2-Chloro-N-(2-hydroxy-2-(1H-indol-3-yl)ethyl)acetamide (170mg, 1.0eq.) was dissolved in tetrahydrofuran (SmL). At 0°C, sodium hydride (2.0eq.) was added. In a nitrogen atmosphere, the reaction mixture was stirred at room temperature for 16 hours, quenched with water (1mL), and purified by flash chromatography (C18, 0.05% formic acid/water, MeCN) to produce the target compound (26mg).
LCMS (ESI) $[M+H]^+$=217.1.

**Step 7:** Preparation of 3-(morpholin-2-yl)-1H-indole:

6-(1H-indol-3-yl)morpholin-3-one (500mg, 1eq.) was dissolved in tetrahydrofuran (5mL). Boranetetrahydrofuran complex (1M, 4.62mL, 2eq.) was added. The reaction mixture was stirred at room temperature for 3 hours. Methanol was slowly added dropwise until no more bubbles were generated. The reaction mixture was stirred thoroughly for 30 minutes, and concentrated to produce the target compound (400mg, crude).
LCMS (ESI) $[M+H]^+$=203.1.

**Step 8:** Preparation of 2-(1H-indol-3-yl)-4-(3-(2-methoxyphenyl)propyl)morpholine:

3-(2-methoxyphenyl)propanal (389.7mg) and 3-(morpholin-2-yl)-1H-indole (400mg, crude) were dissolved in dichloromethane (4mL) and methanol (0.4mL). Acetic acid (0.1mL) was added dropwise. Then sodium triacetoxyborohydride (838.33mg) was added. The reaction mixture was stirred at room temperature for 0.5 hours, diluted with water (20mL), extracted with dichloromethane/methanol (10:1, 15mL×3). The organic phases were combined, dried over anhydrous sodium sulfate, filtered, and concentrated to give a crude product, and purified by preparative HPLC (C18, 0.1% $NH_3$/water, ACN) to produce the target compound (10.43mg).
LCMS (ESI) $[M+H]^+$=351.3; $^1H$ NMR(400 MHz, $CDCl_3$)$\delta$ 8.08(s, 1H), 7.74(d, J=8.0 Hz, 1H), 7.36(d, J=8.1 Hz, 1H), 7.20(dd, J=4.0, 1.8 Hz, 2H), 7.18-7.13(m, 2H), 7.13-7.10(m, 1H), 6.86(ddd, J=12.6, 9.5, 4.6 Hz, 2H), 4.98(s, 1H), 4.04(d, J=10.0 Hz, 1H), 3.96(d, J=14.0 Hz, 1H), 3.80(s, 3H), 3.10(s, 1H), 2.86(s, 1H), 2.71-2.64(m, 2H), 2.48(s, 2H), 1.85(s, 2H), 1.57(s, 2H).

Examples 30-33

With reference to the preparation of Example 21 or 29, compounds 30-33 were prepared:

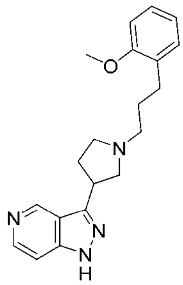
(30)
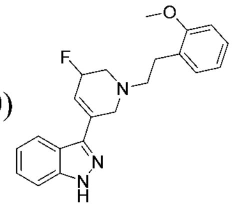
(31)
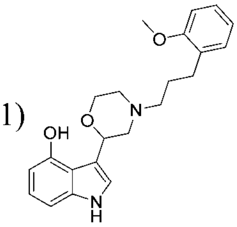
(32)
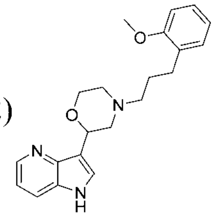
(33)

Compound 30: LCMS (ESI) $[M+H]^+$=337.4;
Compound 31: LCMS (ESI) $[M+H]^+$=352.2;
Compound 32: LCMS (ESI) $[M+H]^+$=367.2;
Compound 33: LCMS (ESI) $[M+H]^+$=352.2.

Example 34 (Compound 34)

Preparation of 4-(3-(2-methoxyphenyl)propyl)-2-(1H-pyrrolo[3,2-c]pyridin-3-yl)morpholine:

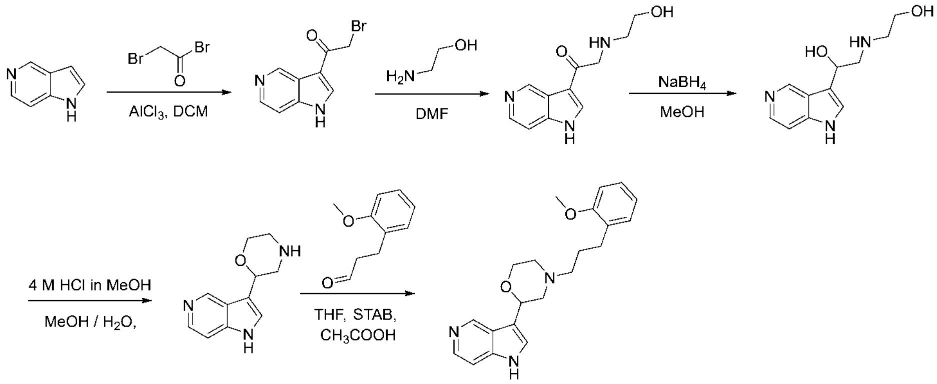


**Step 1:** Preparation of 2-bromo-1-(1H-pyrrolo[3,2-c]pyridin-3-yl)ethan-1-one:

1H-pyrrolo[3,2-c]pyridine (2g, 1eq) was dissolved in dichloromethane (20mL). Aluminum trichloride (3.5eq) was slowly added. The mixture was stirred at 40°C for 20 minutes. 2-bromoacetyl bromide (1eq) was added dropwise. The mixture was kept at this temperature and stirred for 40 minutes, cooled to room temperature, and added to ice-water (200mL). A white solid precipitated. The mixture was filtered. The filter cake was washed with water. The solid was collected, and dried to produce the target compound (1g).
LCMS (ESI) $[M+H]^+$=241.0.

**Step 2:** Preparation of 2-((2-hydroxyethyl)amino)-1-(1H-pyrrolo[3,2-c]pyridin-3-yl)ethan-1-one:

2-bromo-1-(1H-pyrrolo[3,2-c]pyridin-3-yl)ethan-1-one (100mg, 1eq) was dissolved in N,N-dimethylformamide (2mL). The reaction mixture was cooled down to 0°C. 2-aminoethan-1-ol (1eq) was added. The mixture was kept at this temperature, stirred and reacted for 5 minutes, then warmed up to room temperature, and stirred and reacted for 1 hour. A solid precipitated. The mixture was filtered. The filter cake was washed with acetonitrile twice (2*2mL), collected, and dried by rotary-evaporation to produce the target compound (80mg).
LCMS (ESI) $[M+H]^+$=220.1.

**Step 3:** Preparation of 2-((2-hydroxyethyl)amino)-1-(1H-pyrrolo[3,2-c]pyridin-3-yl)ethan-1-ol:

2-((2-hydroxyethyl)amino)-1-(1H-pyrrolo[3,2-c]pyridin-3-yl)ethan-1-one (80mg, 1eq) was dissolved in methanol (5mL). Sodium borohydride (8eq) was added. The reaction mixture was stirred and reacted at room temperature for 16 hours, cooled down to room temperature, quenched by adding 3N hydrochloric acid solution (3mL), dried by rotary-evaporation to remove the reaction solvent to produce the target compound (70mg, crude), which was directly used in the next step.

LCMS (ESI) $[M+H]^+$=222.2.

**Step 4:** Preparation of 2-(1H-pyrrolo[3,2-c]pyridin-3-yl)morpholine:

2-((2-hydroxyethyl)amino)-1-(1H-pyrrolo[3,2-c]pyridin-3-yl)ethan-1-ol (70mg, crude) was dissolved in methanol (3mL), and 3M hydrochloric acid/methanol solution (3mL) was added. The reaction mixture was stirred and reacted at room temperature for 1 hour, then warmed up to 80°C, stirred and reacted for 3 hours, filtered to remove the salt, and concentrated to produce the target compound (80mg, crude), which was directly used in the next step.

LCMS (ESI) $[M+H]^+$=204.1.

**Step 5:** Preparation of 4-(3-(2-methoxyphenyl)propyl)-2-(1H-pyrrolo[3,2-c]pyridin-3-yl)morpholine:

2-(1H-pyrrolo[3,2-c]pyridin-3-yl)morpholine(80mg, crude) was dissolved in tetrahydrofuran (6mL), 3-(2-methoxyphenyl)propanal (64.63mg) was added, and then 2 drops of acetic acid was added dropwise. The mixture was stirred and reacted at room temperature for 10 minutes. Sodium triacetoxyborohydride (166.05mg) was added. The resulting mixture was stirred and reacted for another 1 hour, diluted with water (5mL), and extracted with ethyl acetate (3×20mL). The organic phases were combined, washed with saturated brine, dried over anhydrous sodium sulfate, filtered, and concentrated. The resulting crude product was purified by preparative HPLC (0.1% ammonia in water: acetonitrile) to produce the target compound (31mg, yield: 22.6%).

LCMS (ESI) $[M+H]^+$=352.2; $^1$HNMR(400 MHz, MeOD-$d_4$)$\delta$ 8.91(s, 1H), 8.13(d, J=5.9 Hz, 1H), 7.46-7.32(m, 2H), 7.14(dd, J=13.9, 7.3 Hz, 2H), 6.91-6.81(m, 2H), 4.92(dd, J=10.3, 2.1 Hz, 1H), 4.01(d, J=10.1 Hz, 1H), 3.88(td, J=11.4, 2.1 Hz, 1H), 3.79(s, 3H), 3.06(d, J=11.5 Hz, 1H), 2.88(d, J=11.7 Hz, 1H), 2.66(t, J=7.6 Hz, 2H), 2.44(dt, J=22.0, 9.4 Hz, 3H), 2.31(td, J=11.5, 3.4 Hz, 1H), 1.90-1.76(m, 2H).

Examples 35-36

With reference to the preparation of Example 16 or 34, compounds 35-36 were prepared:

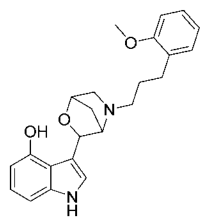
(35)
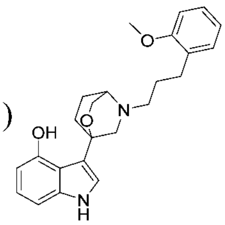
(36)

Compound 35: LCMS (ESI) $[M+H]^+$=379.2;
Compound 36: LCMS (ESI) $[M+H]^+$=393.2.
Example 37 (Compound 37)

Preparation of 3-(4-(3-(2-methoxyphenyl)propyl)morpholin-2-yl)-1H-indazole:

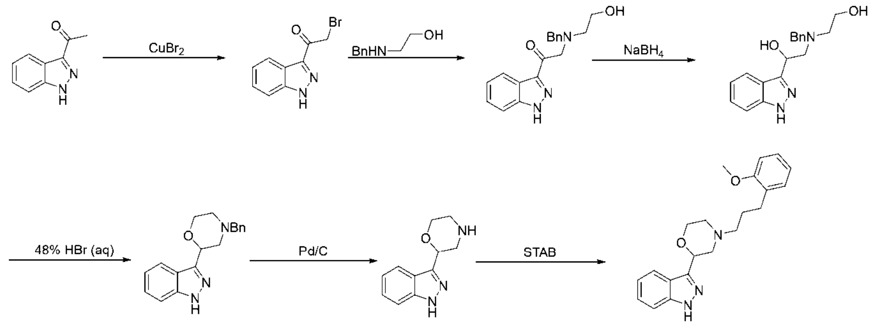


**Step** 1: Preparation of 2-bromo-1-(1H-indazol-3-yl)ethan-1-one:

Copper(II) bromide (13.39g, 2eq) was added to ethyl acetate (540mL). The mixture was heated to 82°C. Then 1-(1H-indazol-3-yl)ethan-1-one (4.8g, 1eq) was added. The reaction system was stirred at 82°C for 4 hours. The reaction mixture was washed with 20% sodium thiosulfate solution (450mL×2), then washed with saturated brine (100mL×2). The organic phase was dried over sodium sulfate, filtered, and concentrated to give a crude product. The crude product was slurried with ethyl acetate and petroleum ether (1:1) for purification to produce the target compound (5 g).
LCMS (ESI) $[M+H]^+$=239.0.

**Step 2:** Preparation of 2-(benzyl(2-hydroxyethyl)amino)-1-(1H-indazol-3-yl)ethan-1-one:

2-(benzylamino)ethan-1-ol (10eq) was dissolved in acetonitrile (20mL). Under an ice bath, 2-bromo-1-(1H-indazol-3-yl)ethan-1-one (2g, 1eq) was added in batch. The reaction system was stirred under an ice bath for 20 minutes, diluted with water (30mL), and extracted with dichloromethane (50mL×3). The organic phases were combined, dried over anhydrous sodium sulfate, filtered, and concentrated to give a crude product. The crude product was purified by flash chromatography to produce the target compound (1.8g).
LCMS (ESI) $[M+H]^+$=310.0.

**Step 3:** Preparation of 2-(benzyl(2-hydroxyethyl)amino)-1-(1H-indazol-3-yl)ethan-1-ol:

2-(benzyl(2-hydroxyethyl)amino)-1-(1H-indazol-3-yl)ethan-1-one (1.8g, 1eq) was dissolved in methanol (20mL). Sodium borohydride (2eq) was slowly added under an ice bath. After the completion of the addition, the reaction mixture was stirred at room temperature for 30 minutes. Water was added under an ice bath until no more bubbles were generated. The mixture was extracted with dichloromethane (30mL×3). The organic phases were combined, washed with saturated brine (20mL×2), dried over anhydrous sodium sulfate, filtered, and concentrated to produce the target compound (1.7g, crude), which was directly used in the next step.
LCMS (ESI) $[M+H]^+$=312.2.

**Step 4:** Preparation of 3-(4-benzylmorpholin-2-yl)-1H-indazole:

2-(benzyl(2-hydroxyethyl)amino)-1-(1H-indazol-3-yl)ethan-1-ol (1.5g, crude) was dissolved in 48% aqueous hydrobromic acid solution (15mL). The reaction system was stirred at 100°C for 3 hours, diluted with dichloromethane (15mL) and water (15mL), adjusted with saturated potassium carbonate to pH=10, and extracted with dichloromethane (30mL×3). The organic phases were combined, dried over anhydrous sodium sulfate, filtered, and concentrated to give a crude product. The crude product was purified by flash chromatography to produce the target compound (800mg).
LCMS (ESI) $[M+H]^+$=294.1.

**Step 5:** Preparation of 3-(morpholin-2-yl)-1H-indazole:

3-(4-benzylmorpholin-2-yl)-1H-indazole(750mg, 1eq) was dissolved in methanol (8mL). Under a hydrogen atmosphere, 10% palladium/carbon (0.28eq) was added, followed by 8 drops of acetic acid. The reaction system was stirred at room temperature for 24 hours. LCMS detection showed the completion of the reaction. The reaction mixture was concentrated to produce the target compound (500mg, crude), which was directly used in the next step.
LCMS (ESI) $[M+H]^+$=204.1.

**Step 6:** Preparation of 3-(4-(3-(2-methoxyphenyl)propyl)morpholin-2-yl)-1H-indazole:

3-(2-methoxyphenyl)propanal (121.19mg) and 3-(morpholin-2-yl)-1H-indazole (150mg, crude) were dissolved in dichloromethane (2mL). Acetic acid (0.1mL) was added. The mixture was stirred for 10 minutes, and then sodium triacetoxyborohydride (311.35mg) was added. The reaction system was stirred for 1 hour, diluted with water (10mL), and extracted with dichloromethane (15mL×3). The organic phases were combined, dried over anhydrous sodium sulfate, filtered, and concentrated to give a crude product. The crude product was purified by preparative HPLC (C18, 0.05% formic acid in water:acetonitrile) to produce the target compound (111.79mg).

LCMS (ESI) $[M+H]^+$=352.2; $^1H$ NMR(400 MHz, DMSO-$d_6$)δ 12.91(s, 1H), 8.15(s, 1H), 7.85(d, J=8.1 Hz, 1H), 7.49(d, J=8.4 Hz, 1H), 7.35-7.30(m, 1H), 7.19-7.12(m, 2H), 7.08(t, J=7.5 Hz, 1H), 6.92(d, J=8.0 Hz, 1H), 6.85(t, J=7.4 Hz, 1H), 4.91(dd, J=10.3, 2.3 Hz, 1H), 3.95(d, J=9.9 Hz, 1H), 3.80-3.73(m, 4H), 3.02(d, J=11.3 Hz, 1H), 2.81(d, J=11.2 Hz, 1H), 2.59(td, J=7.2, 3.4 Hz, 2H), 2.47-2.34(m, 3H), 2.20(td, J=11.3, 3.1 Hz, 1H), 1.77-1.67(m, 2H).

Examples 38-42

With reference to the preparation of Example 29 or 37, compounds 38-42 were prepared:

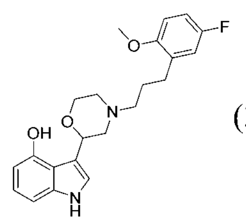
(38)
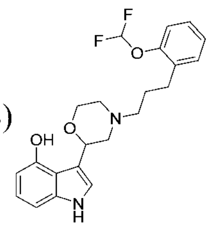
(39)
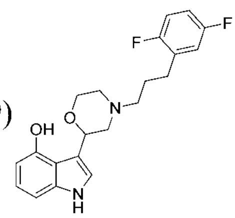
(40)

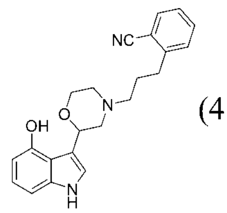
(41)
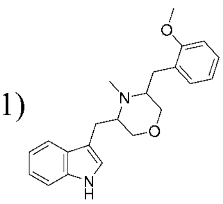
(42)

Compound 38: LCMS (ESI) $[M+H]^+$=385.2;
Compound 39: LCMS (ESI) $[M+H]^+$=403.2;
Compound 40: LCMS (ESI) $[M+H]^+$=373.2;
Compound 41: LCMS (ESI) $[M+H]^+$=362.2;
Compound 42: LCMS (ESI) $[M+H]^+$=351.2.

Examples 43-44

With reference to the preparation of Example 5, compounds 43-44 were prepared:

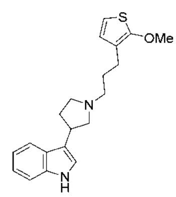
(43)
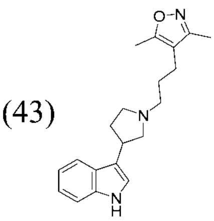
(44)

Compound 43: LCMS (ESI) $[M+H]^+$=341.1; $^1H$ NMR(400 MHz, $CDCl_3$)δ 7.98(s, 1H), 7.67(d, J=7.9 Hz, 1H), 7.35(d, J=8.1 Hz, 1H), 7.18(dd, J=8.1, 1.1 Hz, 1H), 7.10(ddd, J=8.0, 7.1, 1.0 Hz, 1H), 7.02(d, J=2.0 Hz, 1H), 6.67(d, J=5.8 Hz, 1H), 6.55(d, J=5.8 Hz, 1H), 3.87(s, 3H), 3.66(dd, J=16.9, 8.2 Hz, 1H), 3.18(t, J=8.4 Hz, 1H), 2.93(dd, J=14.7, 8.2 Hz, 1H), 2.66-2.49(m, 6H), 2.42-2.33(m, 1H), 2.01(ddd, J=12.6, 7.3, 2.7 Hz, 1H), 1.85-1.79(m, 2H).

Compound 44: LCMS (ESI) $[M+H]^+$=324.2; $^1H$ NMR(400 MHz, DMSO-$d_6$)δ 10.92(s, 1H), 8.18(s, 1H), 7.61(d, J=7.9 Hz, 1H), 7.35(d, J=8.1 Hz, 1H), 7.24(d, J=2.2 Hz, 1H), 7.11-7.05(m, 1H), 7.01-6.95(m, 1H), 3.69-3.64(m, 1H), 3.45(d,

J=8.4 Hz, 1H), 3.21-3.11(m, 2H), 2.97(t, J=9.4 Hz, 1H), 2.92-2.82(m, 2H), 2.41-2.33(m, 3H), 2.30(s, 3H), 2.16(s, 3H), 2.09-2.01(m, 1H), 1.80-1.71(m, 2H).

Example 45 (Compound 45)

Preparation of 3-(1-(3-(2-methyl-1,3-oxazol-4-yl)propyl)pyrrolidin-3-yl)-1H-indole:

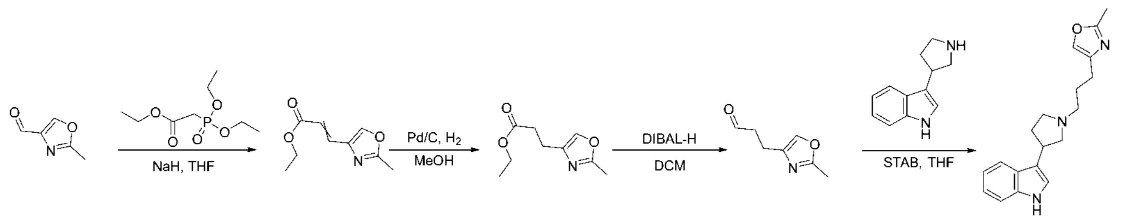


**Step 1:** Preparation of ethyl 3-(2-methyl-1,3-oxazol-4-yl)prop-2-enoate:

Sodium hydride (1.3eq) was dissolved in tetrahydrofuran (20mL). At 0°C, a solution of ethyl 2-(diethoxyphosphoryl)acetate (1.2eq) in tetrahydrofuran (30mL) was added dropwise. At 0°C, the mixture was stirred for 0.5 hours. Then a solution of 2-methyloxazole-4-carbaldehyde (950mg, 1eq)in tetrahydrofuran (10mL) was added dropwise. At room temperature, the mixture was reacted for 2 hours, quenched with water, and extracted with ethyl acetate three times. The organic phases were combined, washed with saturated brine, dried by rotary-evaporation to give a crude product. The crude product was purified by flash chromatography to produce the target compound (980mg).

LCMS (ESI) $[M+H]^+$=182.1.

**Step 2:** Preparation of ethyl 3-(2-methyl-1,3-oxazol-4-yl)propanoate:

Ethyl 3-(2-methyl-1,3-oxazol-4-yl)prop-2-enoate (800mg, 1eq) was dissolved in methanol (12mL). Palladium/-carbon (60mg, 10%) was added. Under a hydrogen atmosphere, the reaction mixture was stirred at room temperature for 3 hours. The detection showed the completion of the reaction. The mixture was filtered through diatomite. The resulting filtrate was dried by rotatary evaporation to produce the target compound (600mg).

$^1H$ NMR(400 MHz, $CDCl_3$) δ 7.29(s, 1H), 4.13(t, J=7.1 Hz, 2H), 2.81(t, J=7.4 Hz, 2H), 2.63(t, J=7.3 Hz, 2H), 2.41(s, 3H), 1.24(t, J=7.1 Hz, 3H).

**Step 3:** Preparation of 3-(2-methyl-1,3-oxazol-4-yl)propanal:

Ethyl 3-(2-methyl-1,3-oxazol-4-yl)propanoate (700mg, 1eq) was dissolved in dichloromethane (15mL). The mixture was cooled down to -78°C. While kept at this temperature, a solution of diisobutylaluminium hydride in toluene (1.5M, 3.8mL, 1.5eq) was slowly added dropwise. The mixture was reacted at -78°C for another 1 hour. The TLC detection (using 2,4-dinitrophenylhydrazine for staining) showed the completion of the reaction. The reaction was quenched by adding water (2mL). Anhydrous sodium sulfate was added, and then tetrahydrofuran solution was added. The mixture was stirred for 5 minutes, and filtered. The resulting filtrate was purified by flash chromatography to produce the target compound (300mg).

LCMS (ESI) $[M+H]^+$=140.2.

**Step 4:** Preparation of 3-(1-(3-(2-methyl-1,3-oxazol-4-yl)propyl)pyrrolidin-3-yl)-1H-indole:

3-(2-methyl-1,3-oxazol-4-yl)propanal (1.2eq.) and 3-(pyrrolidin-3-yl)-1H-indole (200mg, 1.0eq.) was dissolved in tetrahydrofuran (SmL). Acetic acid (0.5mL) and sodium triacetoxyborohydride (3.0eq.) were added. The reaction mixture was stirred at room temperature for 1 hour, diluted with water (10mL), and extracted with ethyl acetate (5mL×3). The organic phases were combined, washed with saturated brine, and dried over anhydrous sodium sulfate to produce a crude product. The crude product was purified by preparative HPLC (C18, 0.05% formic acid in water:acetonitrile) to produce the target compound (105.94mg).

LCMS (ESI) $[M+H]^+$ =310.2; $^1H$ NMR(400 MHz, $CDCl_3$)δ 8.54(s, 1H), 8.52(s, 1H), 7.56(d, J=7.9 Hz, 1H), 7.45-7.33(m, 2H), 7.24-7.18(m, 1H), 7.13(dd, J=11.5, 4.4 Hz, 2H), 3.90(t, J=8.5 Hz, 1H), 3.82-3.67(m, 1H), 3.50(d, J=7.9 Hz, 1H), 3.36(s, 1H), 3.20(s, 1H), 3.14-3.06(m, 2H), 2.59(t, J=7.1 Hz, 2H), 2.55-2.46(m, 1H), 2.42(s, 3H), 2.29(d, J=9.2 Hz,

1H), 2.16(dd, J=9.8, 6.4 Hz, 2H).

Examples 46-48

With reference to the preparation of Example 18 or 45, compounds 46-48 were prepared:

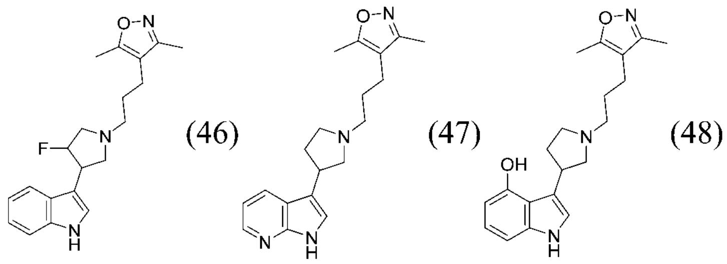

Compound 46: LCMS (ESI) $[M+H]^+$=342.2;
Compound 47: LCMS (ESI) $[M+H]^+$=325.2;
Compound 48: LCMS (ESI) $[M+H]^+$=340.2

Example 49 (Compound 49)

Preparation of 3-(1-[3-(1,2,4-oxadiazol-3-yl)propyl]pyrrolidin-3-yl)-1H-indole:

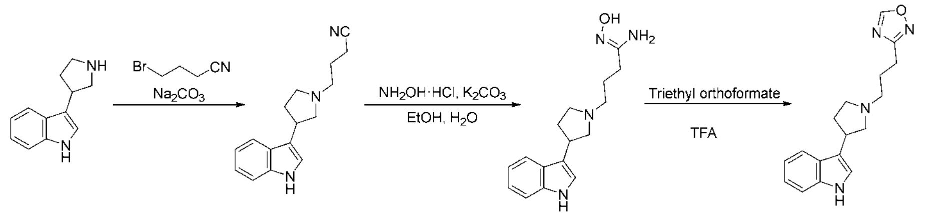


**Step 1:** Preparation of 4-[3-(1H-indol-3-yl)pyrrolidin-1-yl]butanenitrile:

At room temperature, 3-(pyrrolidin-3-yl)-1H-indole (4g, 1.0eq.) and sodium carbonate (3.0eq.) were dissolved in acetonitrile (40mL). Then 4-bromobutanenitrile (1.1eq.) was added. The reaction mixture was reacted at 80°C for 16 hours, diluted with water (30mL), and extracted with ethyl acetate (25mL×3). The organic phases were combined, washed with saturated brine, dried over anhydrous sodium sulfate, filtered, and concentrated to give a crude product. The crude product was purified by flash chromatography (Silica gel, PE:EA=3:1-2:1) to give the target product (3g).

LCMS (ESI) $[M+H]^+$=254.1.

**Step 2:** Preparation of (Z)-N'-hydroxy-4-[3-(1H-indol-3-yl)pyrrolidin-1-yl]butanimidamide:

At room temperature, 4-[3-(1H-indol-3-yl)pyrrolidin-1-yl]butanenitrile (2.2g, 1.0eq.) and hydroxylamine hydrochloride (3.5eq.) were dissolved in a mixed solvent of ethanol (28mL) and water (14mL). Potassium carbonate (3.0eq.) was added. The reaction mixture was reacted at 80°C for 12 hours, concentrated, and extracted with dichloromethane/methanol (10:1, 15mL×3). The organic phases were combined, washed with saturated brine, dried over anhydrous sodium sulfate, and filtered. The mother liquor was concentrated to give a crude product. The crude product was purified by flash chromatography (C18, 0.1% ammonia in water:acetonitrile) to give the target product (2g).

LCMS (ESI) $[M+H]^+$=287.2.

**Step 3:** Preparation of 3-(1-[3-(1,2,4-oxadiazol-3-yl)propyl]pyrrolidin-3-yl)-1H-indole:

At room temperature, (Z)-N'-hydroxy-4-[3-(1H-indol-3-yl)pyrrolidin-1-yl]butanimidamide (120mg, 1.0eq.) was

dissolved in triethyl orthoformate (2.25mL). A catalytic amount of trifluoroacetic acid (0.3mL) was added. The reaction mixture was stirred at room temperature for 10 minutes, then warmed up to 60°C and reacted for 30 minutes, concentrated, diluted with water (10mL), and extracted with ethyl acetate (15mL×3). The organic phases were combined, washed with saturated brine, dried over anhydrous sodium sulfate, filtered, concentrated, and purified by preparative HPLC (C18, 0.1% formic acid in water:acetonitrile) to produce the target compound (14.94mg).

LCMS (ESI) $[M+H]^+$=297.1; $^1H$ NMR(400 MHz, MeOD-$d_4$)δ 9.19(s, 1H), 8.39(s, 1H), 7.59(d, J=7.9 Hz, 1H), 7.37(d, J=8.1 Hz, 1H), 7.21(s, 1H), 7.12(dd, J=8.1, 1.0 Hz, 1H), 7.06(dd, J=7.9, 0.9 Hz, 1H), 3.91(d, J=4.9 Hz, 2H), 3.63(s, 2H), 3.50-3.35(m, 3H), 2.95(t, J=7.3 Hz, 2H), 2.59(td, J=13.1, 6.3 Hz, 1H), 2.46-2.31(m, 1H), 2.31-2.13(m, 2H).

Example 50 (Compound 50)

Preparation of 3-(1-(3-(1,3,4-thiadiazol-2-yl)propyl)pyrrolidin-3-yl)-1H-indole:

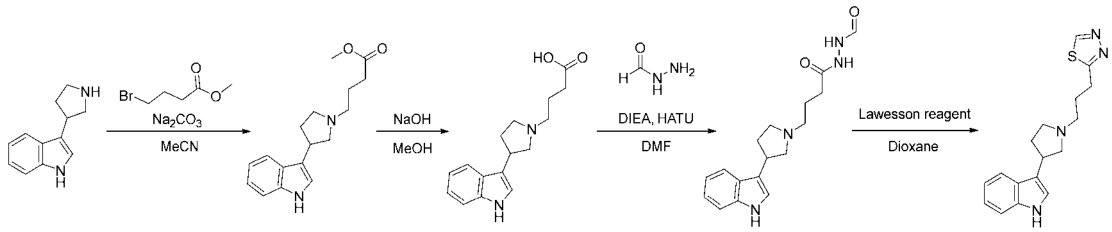


**Step 1:** Preparation of methyl 4-(3-(1H-indol-3-yl)pyrrolidin-1-yl)butanoate:

3-(pyrrolidin-3-yl)-1H-indole (4.00g, 1.0eq.) and methyl 4-bromobutanoate (1.1eq.) were dissolved in acetonitrile (40mL). Sodium carbonate (3.0eq.) was added. The mixture was stirred at 80°C for 16 hours, and filtered. The filter cake was washed with ethyl acetate. The filtrate was dried by rotary-evaporation to give a crude product. The crude product was purified by flash chromatography (Silica gel, DCM:MeOH=20:1) to produce the target compound (3.8g).

LCMS (ESI) $[M+H]^+$=287.2.

**Step 2:** Preparation of 4-(3-(1H-indol-3-yl)pyrrolidin-1-yl)butanoic acid:

Methyl 4-(3-(1H-indol-3-yl)pyrrolidin-1-yl)butanoate (2.6g, 1eq.) was dissolved in methanol (26mL). Sodium hydroxide (42.96eq.) was added. The reaction mixture was stirred at room temperature for 2 hours, adjusted with diluted hydrochloric acid to pH=7-8, dried by rotary-evaporation, and purified by flash chromatography (C18, 0.1% formic acid in water:acetonitrile) to produce the target compound (1.5g).

LCMS (ESI) $[M+H]^+$=273.1.

**Step 3:** Preparation of N'-formyl-4-(3-(1H-indol-3-yl)pyrrolidin-1-yl)butanehydrazide:

4-(3-(1H-indol-3-yl)pyrrolidin-1-yl)butanoic acid (1.5g, 1.0eq.) was dissolved in DMF(15mL). HATU (1.5eq.), N,N-diisopropylethylamine (3.0eq.) and formohydrazide (1.5eq.) were added. The reaction mixture was stirred at room temperature for 20 minutes, and purified by flash chromatography (C18, 0.1% formic acid in water:acetonitrile) to produce the target compound (550mg).

LCMS (ESI) $[M+H]^+$=315.2.

**Step 4:** Preparation of 3-(1-(3-(1,3,4-thiadiazol-2-yl)propyl)pyrrolidin-3-yl)-1H-indole:

N'-formyl-4-(3-(1H-indol-3-yl)pyrrolidin-1-yl)butanehydrazide (450mg, 1.0eq.) was dissolved in dioxane (10mL). Lawesson reagent (1.2eq.) was added. The mixture was stirred at 100°C for 1 hour, dried by rotary-evaporation, and purified by flash chromatography to give a crude product (300mg). The crude product was purified by preparative HPLC (C18, 0.05% ammonia in water:acetonitrile) to produce the target compound (9.88mg).

LCMS (ESI) $[M+H]^+$=313.2; $^1H$ NMR(400 MHz, $CDCl_3$-d)δ 9.04(s, 1H), 7.97(s, 1H), 7.67(d, J=7.9 Hz, 1H), 7.37(d, J=7.9 Hz, 1H), 7.20(t, J=7.5 Hz, 1H), 7.11(t, J=7.5 Hz, 1H), 7.06(s, 1H), 3.72(s, 1H), 3.30(t, J=7.3 Hz, 3H), 2.76(s, 5H), 2.41(s, 1H), 2.16(s, 3H).

Example 51 (Compound 51)

With reference to the preparation of Example 50, compound 51 was prepared:

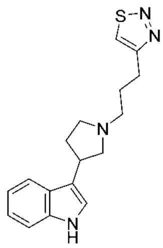

(51)

LCMS (ESI) $[M+H]^+$=313.1.

Example 52 (Compound 52)

Preparation of 3-(1-(3-(1,3,4-oxadiazol-2-yl)propyl)pyrrolidin-3-yl)-1H-indole:

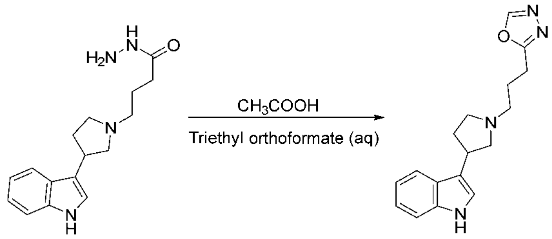


4-(3-(1H-indol-3-yl)pyrrolidin-1-yl)butanehydrazide (300mg, 1.0eq.) was dissolved in acetic acid (4mL). Triethyl orthoformate (8mL) was added. The atmosphere was replaced with nitrogen gas. The mixture was stirred and reacted at 110°C for 3 hours, dried by rotary-evaporation to remove the solvent, and purified by preparative HPLC (C18, 0.05% formic acid in water:acetonitrile) to produce the target compound (46.41mg).

LCMS (ESI) $[M+H]^+$=297.2; $^1H$ NMR(400 MHz, $CDCl_3$)$\delta$ 8.54(s, 1H), 8.35(s, 1H), 8.24(s, 1H), 7.61(d, J=7.9 Hz, 1H), 7.38(d, J=8.1 Hz, 1H), 7.22(t, J=7.5 Hz, 1H), 7.13(t, J=7.5 Hz, 1H), 7.09(d, J=2.2 Hz, 1H), 3.84(p, J=8.5 Hz, 1H), 3.67-3.58(m, 1H), 3.37(dd, J=18.1, 7.6 Hz, 1H), 3.25-3.14(m, 1H), 3.13-2.96(m, 5H), 2.49(ddd, J=15.8, 10.4, 6.6 Hz, 1H), 2.37-2.12(m, 3H).

Example 53 (Compound 53)

With reference to the preparation of Example 52, compound 53 was prepared:

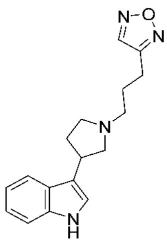

(53)

LCMS (ESI) $[M+H]^+$=297.2.

Example 54 (Compound 54)

Preparation of 3-(1-(3-(4-methyl-1,2,5-oxadiazol-3-yl)propyl)pyrrolidin-3-yl)-1H-indole:

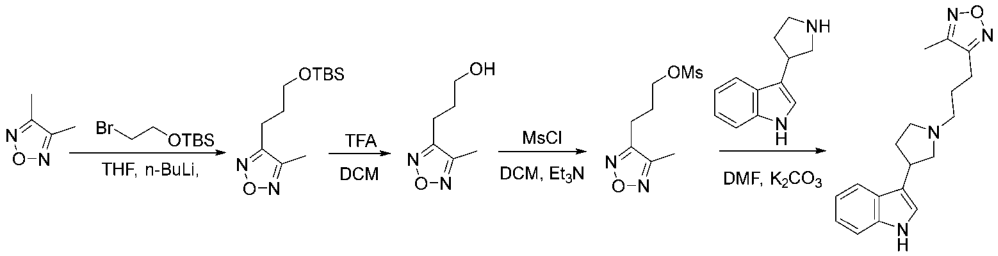


**Step** 1: Preparation of 3-(3-((tert-butyldimethylsilyl)oxy)propyl)-4-methyl-1,2,5-oxadiazole:

3,4-Dimethyl-1,2,5-oxadiazole (2g, 1eq) was dissolved in tetrahydrofuran (20mL). The reaction mixture was cooled down to -70°C. n-Butyllithium (12.7mL, 1eq, 1.6M, in THF) was slowly added dropwise. The addition was completed over 20 minutes. While kept at this temperature, the mixture was stirred and reacted for 40 minutes. (2-Bromoethoxy)(tert-butyl)dimethylsilane (1eq) was slowly added dropwise. While kept this temperature, the mixture was stirred and reacted for another 2 hours. The reaction mixture was diluted with water (20mL), and extracted with ethyl acetate (3×80mL). The organic phases were combined, washed with saturated brine (80mL), dried over anhydrous sodium sulfate, filtered, and concentrated. The resulting crude product was purified by flash chromatography to produce the target compound 3-(3-((tert-butyldimethylsilyl)oxy)propyl)-4-methyl-1,2,5-oxadiazole (1.3g).

LCMS (ESI) $[M+H]^+$=257.1.

**Step 2:** Preparation of 3-(4-methyl-1,2,5-oxadiazol-3-yl)propan-1-ol:

3-(3-((tert-butyldimethylsilyl)oxy)propyl)-4-methyl-1,2,5-oxadiazole (200mg, 1eq) was dissolved in dichloromethane (2mL). Trifluoroacetic acid (2mL) was added. The mixture was stirred and reacted at room temperature for 1 hour, and dried by rotary-evaporation to remove the solvent to produce the target compound (100mg, crude), which was not further purified and directly used in the next step.

LCMS (ESI) $[M+H]^+$=143.1.

**Step 3:** Preparation of 3-(4-methyl-1,2,5-oxadiazol-3-yl)propyl methanesulfonate:

3-(4-methyl-1,2,5-oxadiazol-3-yl)propan-1-ol (100mg, crude) was dissolved in dichloromethane (SmL). Triethylamine (213.54mg) was added. The reaction mixture was cooled down to 0°C. Methanesulfonyl chloride (96.69mg) in dichloromethane (1mL) was slowly added dropwise. The reaction mixture was stirred and reacted at room temperature for 1 hour, diluted with water (10mL), and extracted with dichloromethane (3×10mL). The organic phases were combined, washed with saturated brine, dried over anhydrous sodium sulfate, filtered, and concentrated to give a crude product, which was not further purified and directly used in the next step.

LCMS (ESI) $[M+H]^+$=221.1.

**Step 4:** Preparation of 3-(1-(3-(4-methyl-1,2,5-oxadiazol-3-yl)propyl)pyrrolidin-3-yl)-1H-indole:

3-(4-methyl-1,2,5-oxadiazol-3-yl)propyl methanesulfonate (80mg, crude, obtained from the previous step) was dissolved in N,N-dimethylformamide (5mL). 3-(Pyrrolidin-3-yl)-1H-indole (81.18mg) and potassium carbonate (150.6mg) were added. In an oil bath, the mixture was stirred and reacted at 70°C for 16 hours, diluted with water (SmL), and extracted with ethyl acetate (3×20mL). The organic phases were combined, washed with saturated brine, dried over anhydrous sodium sulfate, filtered, and concentrated. The resulting crude product was purified by preparative HPLC (C18, 0.1% formic acid in water:acetonitrile) to produce the target compound (22mg).

LCMS (ESI) $[M+H]^+$=311.3; $^1$HNMR(400 MHz, DMSO-$d_6$)δ 10.79(s, 1H), 8.19(s, 1H), 7.59(d, J=7.9 Hz, 1H), 7.33(d, J=8.1 Hz, 1H), 7.16(d, J=2.0 Hz, 1H), 7.06(t, J=7.5 Hz, 1H), 6.96(t, J=7.4 Hz, 1H), 3.57-3.51(m, 1H), 3.13(t, J=8.5 Hz, 1H), 2.90-2.84(m, 1H), 2.81(dd, J=13.5, 6.0 Hz, 2H), 2.73(dd, J=14.2, 8.7 Hz, 1H), 2.68(dd, J=7.0, 4.8 Hz, 1H), 2.61(dd, J=17.2, 8.2 Hz, 2H), 2.35(s, 3H), 2.31-2.19(m, 1H), 1.98-1.84(m, 3H).

Example 55 (Compound 55)

Preparation of 3-(1-(3-(1,2,4-thiadiazol-3-yl)propyl)pyrrolidin-3-yl)-1H-indole:

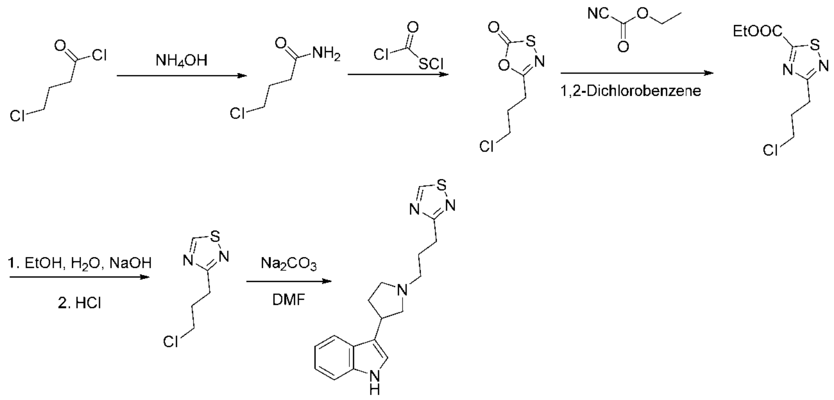


**Step 1:** Preparation of 4-chlorobutanamide:

4-Chlorobutanoyl chloride (15g, 1eq) was dissolved in tetrahydrofuran (150mL). Under an ice bath, ammonia water (75mL) was added dropwise. After the completion of the addition, the reaction mixture was stirred at room temperature for 16 hours, and extracted with ethyl acetate (100mL×3). The organic phases were combined, dried over anhydrous sodium sulfate, filtered, and concentrated to produce the target compound (5g, crude), which was directly used in the next step.

LCMS (ESI) $[M+H]^+$=122.1.

**Step 2:** Preparation of 5-(3-chloropropyl)-1,3,4-oxathiazol-2-one:

4-chlorobutanamide (5g, 1eq) was dissolved in 1,4-dioxane(2mL). Under an ice bath, carbonochloridic hypochlorous thioanhydride (1.1eq) was added. the reaction system was sealed in a tube, and reacted at 100°C for 1 hour. TLC detection showed the completion of the reaction, the reaction mixture was diluted with water (50mL), and extracted with ethyl acetate (50mL×3). The organic phases were combined, dried over anhydrous sodium sulfate, filtered, and concentrated to give a crude product. The crude product was purified by flash chromatography to produce the target compound (3g).

**Step 3:** Preparation of ethyl 3-(3-chloropropyl)-1,2,4-thiadiazole-5-carboxylate:

5-(3-chloropropyl)-1,3,4-oxathiazol-2-one(3g, 1eq) was dissolved in o-dichlorobenzene (30mL). Ethyl carbonocyanidate (5eq) was added. The reaction system was stirred at 140°C for 12 hours. The reaction mixture was directly purified by flash chromatography to produce the target compound (2.5g). LCMS (ESI) $[M+H]^+$=235.1.

**Step 4:** Preparation of 3-(3-chloropropyl)-1,2,4-thiadiazole:

ethyl 3-(3-chloropropyl)-1,2,4-thiadiazole-5-carboxylate (1g, 1eq) was dissolved in water (10mL) and ethanol (2mL). Sodium hydroxide (1.1eq) was added.. The reaction system was stirred at 100°C for 2 hours. The reaction mixture was placed under an ice bath, and concentrated hydrochloric acid (1.1eq) was added. The reaction system was stirred at 100°C for another 3 hours, diluted with water (20mL), and extracted with ethyl acetate (20mL×3). The organic phases were combined, dried over anhydrous sodium sulfate, filtered, and concentrated to give a crude product. The crude product was purified by reverse-phase chromatography (C18, 0.1%trifluoroacetic acid in water:acetonitrile) to produce the target compound (100mg).

LCMS (ESI) $[M+H]^+$=163.1.

**Step 5:** Preparation of 3-(1-(3-(1,2,4-thiadiazol-3-yl)propyl)pyrrolidin-3-yl)-1H-indole:

3-(pyrrolidin-3-yl)-1H-indole (91.61mg, 1eq) and 3-(3-chloropropyl)-1,2,4-thiadiazole (1eq) were dissolved in N,N-dimethylformamide (2mL). Sodium carbonate (2eq) was added. The reaction system was stirred at 70°C for 12 hours, diluted with water (10mL), and extracted with ethyl acetate (15mL×3). The organic phases were combined, washed with saturated brine (10mL×2), dried over anhydrous sodium sulfate, filtered, and concentrated to give a crude product. The crude product was purified by preparative HPLC (C18, 0.05% formic acid in water:acetonitrile) to produce the target compound (21.95mg).

LCMS (ESI) $[M+H]^+$=313.3; $^1H$ NMR(400 MHz, Chloroform-d)$\delta$ 9.79(s, 1H), 8.56(s, 1H), 8.35(s, 1H), 7.58(d,

J=7.8 Hz, 1H), 7.40(s, 1H), 7.22(t, J=7.5 Hz, 1H), 7.17-7.10(m, 2H), 3.89(dd, J=17.0, 8.5 Hz, 1H), 3.82-3.72(m, 1H), 3.58-3.47(m, 1H), 3.42-3.33(m, 1H), 3.22(d, J=9.9 Hz, 1H), 3.17(t, J=7.0 Hz, 4H), 2.52(dd, J=12.8, 4.9 Hz, 1H), 2.41(dd, J=15.5, 7.5 Hz, 2H), 2.28(dq, J=17.1, 8.5 Hz, 1H).

Example 56 (Compound 56)

With reference to the preparation of Example 55, compound 56 was prepared: LCMS (ESI) $[M+H]^+$=329.1.

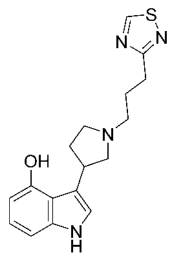

(56)

Example 57 (Compound 57)

Preparation of 3-(1-(3-(1-methyl-1H-pyrazol-4-yl)propyl)pyrrolidin-3-yl)-1H-indole:

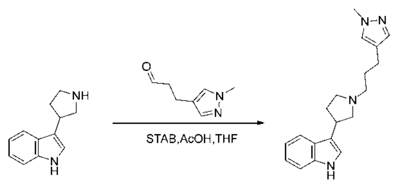


3-(1-methyl-1H-pyrazol-4-yl)propanal (80mg, 1.0eq.) and 3-(pyrrolidin-3-yl)-1H-indole (1.1eq.) were dissolved in tetrahydrofuran (10mL). The mixture was stirred, and then 2 drops of acetic acid was added dropwise thereto. The resulting mixture was reacted at 30°C for 0.5 hours. After adding sodium triacetoxyborohydride (2eq.), the resulting mixture was reacted for another 1 hour. The reaction mixture was diluted with water (10mL), and extracted with ethyl acetate (50mL×3). The organic phases were combined, washed with saturated brine (20mL×2), dried over anhydrous sodium sulfate, filtered, and concentrated, and purified by preparative HPLC (C18, 0.1% formic acid in water:acetonitrile) to produce the target compound (58.15mg).

LCMS (ESI) $[M+H]^+$=309.2; $^1$H NMR(400 MHz, DMSO-$d_6$)δ 10.77(s, 1H), 8.19(s, 1H), 7.60(d, J=7.5 Hz, 1H), 7.45(s, 1H), 7.32(d, J=8.0 Hz, 1H), 7.23(s, 1H), 7.14(d, J=2.3 Hz, 1H), 7.05(t, J=7.0 Hz, 1H), 6.95(t, J=7.0 Hz, 1H), 3.76(s, 3H), 3.55(d, J=8.3 Hz, 2H), 3.08(s, 1H), 2.82(d, J=6.9 Hz, 1H), 2.67(s, 1H), 2.57(d, J=7.9 Hz, 2H), 2.43(d, J=7.5 Hz, 2H), 2.25(s, 1H), 1.92(d, J=7.8 Hz, 1H), 1.70(dd, J=14.9, 7.4 Hz, 2H).

Examples 58-68 (Compound 58-68)

With reference to the preparation of Example 69, compounds 58-68 were prepared:

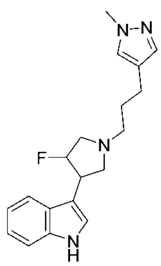

(58)

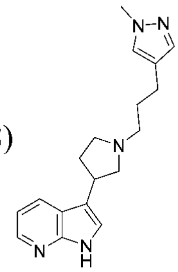

(59)

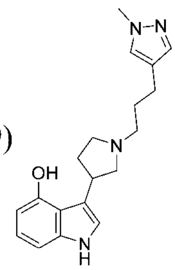

(60)

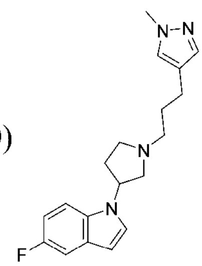

(61)

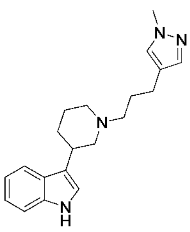 (62) 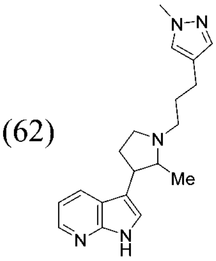 (63) 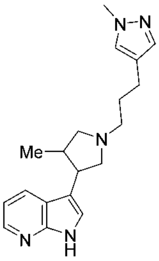 (64) 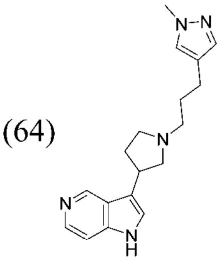 (65)

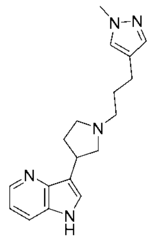 (66) 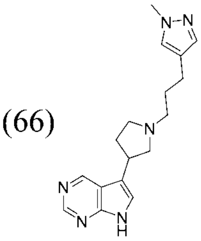 (67) 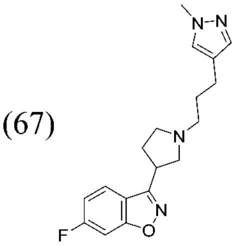 (68)

Compound 58: LCMS (ESI) $[M+H]^+$=327.2;
Compound 59: LCMS (ESI) $[M+H]^+$=310.2;
Compound 60: LCMS (ESI) $[M+H]^+$=325.2;
Compound 61: LCMS (ESI) $[M+H]^+$=327.2;
Compound 62: LCMS (ESI) $[M+H]^+$=323.2;
Compound 63: LCMS (ESI) $[M+H]^+$=324.2;
Compound 64: LCMS (ESI) $[M+H]^+$=324.2;
Compound 65: LCMS (ESI) $[M+H]^+$=310.2;
Compound 66: LCMS (ESI) $[M+H]^+$=310.2;
Compound 67: LCMS (ESI) $[M+H]^+$=311.2;
Compound 68: LCMS (ESI) $[M+H]^+$=329.2.

Example 69 (Compound 69)

Preparation of 3-(1-(3-(1-methyl-1H-pyrazol-4-yl)propyl)pyrrolidin-3-yl)-1H-indazole:

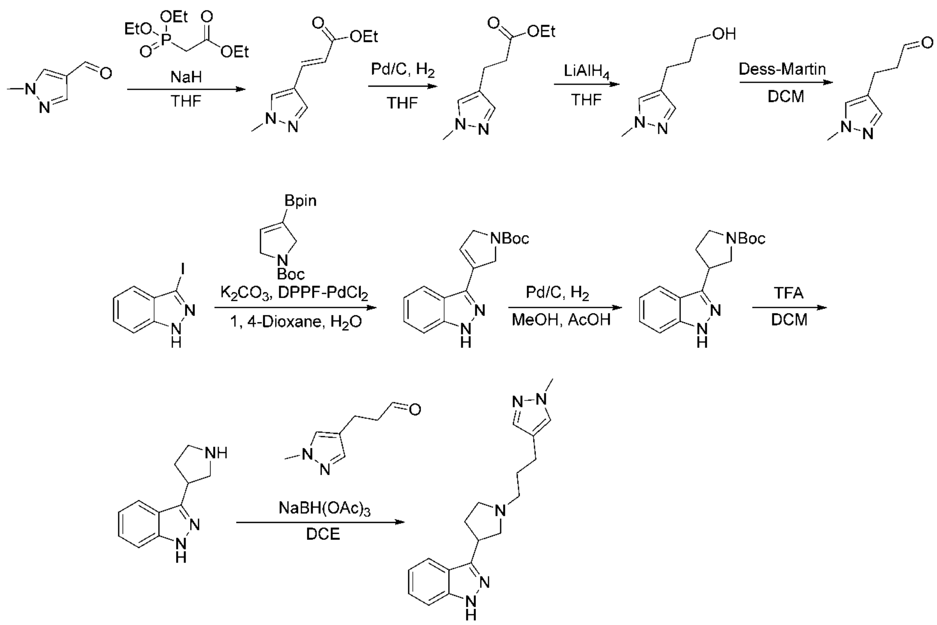

**Step** 1: Preparation of ethyl (E)-3-(1-methyl-1H-pyrazol-4-yl)acrylate:

At 0°C, to a suspension of NaH (60%, dispersed in mineral oil, 15.6g) in THF (260mL) was slowly added dropwise ethyl 2-(diethoxyphosphoryl)acetate (1.5eq). The resulting mixture was warmed to room temperature and stirred for 30 minutes. Then, at 0°C, to the mixture was added dropwise a solution of 1-methyl-1H-pyrazole-4-carbaldehyde (13g, 1eq) in THF(20mL), and then the mixture was warmed to room temperature and stirred for another 5 hours. At 0°C, to the mixture was slowly added dropwise water (100mL). The resulting mixture was extracted with ethyl acetate (100mL×4). The organic phases were combined, dried over anhydrous sodium sulfate, filtered, dried by rotary-evaporation, and purified by flash chromatography to produce the target compound (14.3g).

LCMS (ESI) [M+H]+=181.1.

**Step 2:** Preparation of ethyl 3-(1-methyl-1H-pyrazol-4-yl)propanoate:

At room temperature, to a solution of ethyl (E)-3-(1-methyl-1H-pyrazol-4-yl)acrylate (14.3g, 1eq) in THF (200mL) was added palladium/carbon (10%, 3.0g, 0.2eq). The atmosphere was replaced with hydrogen using a double-layer hydrogen balloon. Then the reaction mixture was reacted for 20 hours, and filtered through diatomite. The filter cake was washed with ethyl acetate (100mL). The filtrate was dried by rotary-evaporation to produce the target compound (14g, crude), which was directly used in the next step.

LCMS (ESI) $[M+H]^+$=183.1.

**Step 3:** Preparation of 3-(1-methyl-1H-pyrazol-4-yl)propan-1-ol:

Under the protection of nitrogen gas, under an ice bath, to a solution of ethyl 3-(1-methyl-1H-pyrazol-4-yl) propanoate (14g, 1eq) in THF (200mL) was slowly added $LiAlH_4$ (1.37eq). The resulting mixture was slowly warmed up to room temperature and reacted overnight. Under an ice bath, ethanol (20mL) and water (20mL) were sequentially slowly added dropwise to the above mixture to quench the reaction. Then the reaction mixture was diluted with ethyl acetate (100mL), dried over anhydrous magnesium sulfate (10g). The reaction mixture was stirred at room temperature for 5 minutes, and filtered. The filter cake was washed with ethyl acetate (100mL). The filtrate was dried by rotatary evaporation to produce the target compound (10.1g, crude), which was directly used in the next step.

LCMS (ESI) $[M+H]^+$=141.1.

**Step 4:** Preparation of 3-(1-methyl-1H-pyrazol-4-yl)propanal:

Under an ice bath, to a solution of 3-(1-methyl-1H-pyrazol-4-yl)propan-1-ol (10.1g, crude) in DCM (200mL) was slowly added to Dess-Martin periodinane (30.5g, 1eq). The resulting mixture was slowly warmed up to room temperature and reacted overnight. Then, petroleum ether (50mL) was added. The mixture was stirred at -78°C for 30 minutes and then filtered. The filtrate was dried by rotary-evaporation, and purified by silica gel column chromatography to produce the target compound (7.5g).

**Step 5:** Preparation of tert-butyl 3-(1H-indazol-3-yl)-2,5-dihydro-1H-pyrrole-1-carboxylate:

At room temperature, 3-iodo-1H-indazole (400mg, 1.0eq), tert-butyl 3-(3,3,4,4-tetramethylborolan-1-yl)-2,5-dihydro-1H-pyrrole-1-carboxylate (1.2eq), potassium carbonate (3.0eq), [1,1'-bis(diphenylphosphino)ferrocene]palladium dichloride (0.1eq) were added to a solution of 1,4-dioxane/water (10:1, 11mL). The mixture was reacted at 110°C in a nitrogen atmosphere for 3 hours and then filtered. The filtrate was concentrated, and purified by flash chromatography to produce the target compound (450mg).

LCMS (ESI) $[M+H]^+$=284.2.

**Step 6:** Preparation of tert-butyl 3-(1H-indazol-3-yl)pyrrolidine-1-carboxylate:

At room temperature, to a solution of tert-butyl 3-(1H-indazol-3-yl)-2,5-dihydro-1H-pyrrole-1-carboxylate (450mg, 1eq) and palladium/carbon (0.2eq) in methanol (30mL) was added dropwise three drops of acetic acid. Under a hydrogen atmosphere, the reaction mixture was stirred overnight, filtered through diatomite, then dried by rotary-evaporation to remove the solvent to produce the target compound (430mg).

LCMS (ESI) $[M+H]^+$=286.2.

**Step 7:** Preparation of 3-(pyrrolidin-3-yl)-1H-indazole:

At room temperature, to a solution of tert-butyl 3-(1H-indazol-3-yl)pyrrolidine-1-carboxylate (430mg, 1.0eq) in DCM(6mL) was added dropwise TFA (0.5mL). The mixture was stirred for 1 hour, dried by rotary-evaporation, then diluted with DCM (30mL). Under an ice bath, to the above solution was slowly added dropwise saturated sodium bicarbonate solution (30mL). After the phase separation, the aqueous phase was extracted with ethyl acetate (30mL×2). The organic phases were combined, dried over anhydrous sodium sulfate, filtered, dried by rotary-evaporation to remove the solvent to produce the target compound (275mg).

LCMS (ESI) $[M+H]^+$=188.1.

**Step 8:** Preparation of 3-(1-(3-(1-methyl-1H-pyrazol-4-yl)propyl)pyrrolidin-3-yl)-1H-indazole:

At room temperature, to a solution of 3-(pyrrolidin-3-yl)-1H-indazole(275mg, 1.0eq) and 3-(1-methyl-1H-pyrazol-4-yl)propanal (1.2eq) in 1,2-dichloroethane (5mL) was added sodium triacetoxyborohydride (1.4eq). The mixture was reacted at room temperature for 24 hours. Then dichloromethane (10mL) was added to the reaction mixture. Under an ice bath, to the above mixture was added dropwise saturated sodium bicarbonate solution (15mL). After the phase separation, the aqueous phase was extracted with ethyl acetate (10mL×2). The organic phases were combined, dried over anhydrous sodium sulfate, filtered, concentrated, and purified by prep-HPLC (C18, 0.05% formic acid in water:acetonitrile) to produce the target compound (75.36mg).

LCMS (ESI) $[M+H]^+$=310.2; $^1$H NMR(400 MHz, $d_6$-DMSO)δ 12.60(s, 1H), 7.83(d, J=8.1 Hz, 1H), 7.45(t, J=4.1 Hz, 2H), 7.48-7.41(m, 1H), 7.22(s, 1H), 7.05(t, J=7.8 Hz, 1H), 3.77-3.68(m, 4H), 3.01(t, J=8.5 Hz, 1H), 2.75-2.61(m, 3H), 2.55-2.51(m, 1H), 2.47-2.41(m, 3H), 2.34-2.22(m, 1H), 2.16-2.06(m, 1H), 1.73-1.64(m, 2H).

Example 70 (Compound 70)

With reference to the preparation of Example 57, compound 70 was prepared:

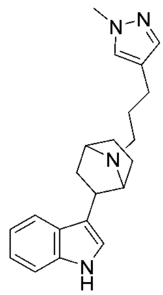

(70)

LCMS (ESI) $[M+H]^+$=335.2.

Example 71 (Compound 71)

Preparation of 3-(1-(3-(1-methyl-1H-pyrazol-4-yl)propyl)-2,5-dihydro-1H-pyrrol-3-yl)-1H-indole:

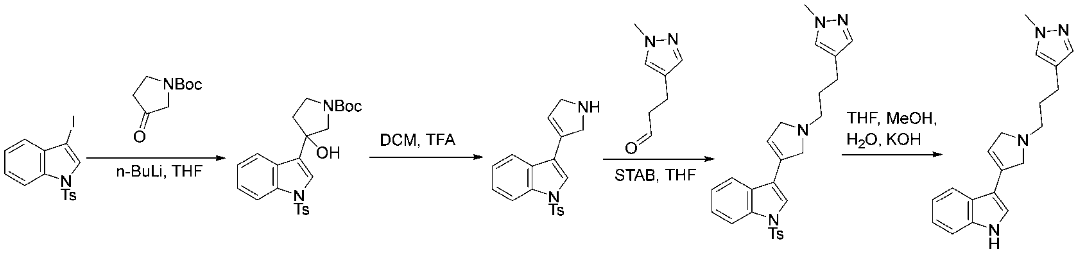


Step 1: Preparation of tert-butyl 3-hydroxy-3-(1-(4-methylphenylsulfonyl)-1H-indol-3-yl)pyrrolidine-1-carboxylate:

3-iodo-1-(4-methylphenylsulfonyl)-1H-indole (15g, 1.0eq) was dissolved in anhydrous tetrahydrofuran (150mL).

At -68°C, 1.6M n-butyllithium (1.2eq) was slowly added dropwise. While kept at this temperature, the mixture was reacted for 1 hour. Tert-butyl 3-oxopyrrolidine-1-carboxylate (1.1eq) was slowly added. The mixture was stirred for another 1 hour, warmed up to room temperature, diluted with water (500mL), extracted with ethyl acetate (500mL×3). The organic phases were dried by rotary-evaporation to give a crude product. The crude product was purified by flash chromatography (Silica gel, PE:EA=3:1) to give the target product (7g).

LCMS (ESI) $[M-56-H_2O+H]^+$=383.0.

**Step 2:** Preparation of 3-(2,5-dihydro-1H-pyrrol-3-yl)-1-(4-methylphenylsulfonyl)-1H-indole:

Tert-butyl 3-hydroxy-3-(1-(4-methylphenylsulfonyl)-1H-indol-3-yl)pyrrolidine-1-carboxylate (1g, 1.0eq) was dissolved in dichloromethane (12mL). Trifluoroacetic acid (2mL) was added. At room temperature, the mixture was reacted for 1 hour, and dried by rotary-evaporation to produce the target compound (800mg, crude).
LCMS (ESI) $[M+H]^+$=339.1.

**Step 3:** Preparation of 3-(1-(3-(1-methyl-1H-pyrazol-4-yl)propyl)-2,5-dihydro-1H-pyrrol-3-yl)-1-(4-methylphenylsulfonyl)-1H-indole:

3-(2,5-dihydro-1H-pyrrol-3-yl)-1-(4-methylphenylsulfonyl)-1H-indole (700mg, 1eq) and 3-(1-methyl-1H-pyrazol-4-yl)propanal (1.5eq) were dissolved in tetrahydrofuran (10mL). Sodium triacetoxyborohydride (2eq) was added. At room temperature, the mixture was reacted for 1 hour, diluted with water (10mL), and extracted with ethyl acetate (10mL×3). The organic phases were dried by rotary-evaporation to produce the target compound (800mg, crude).
LCMS (ESI) $[M+H]^+$=461.1.

**Step 4:** Preparation of 3-(1-(3-(1-methyl-1H-pyrazol-4-yl)propyl)-2,5-dihydro-1H-pyrrol-3-yl)-1H-indole:

3-(1-(3-(1-methyl-1H-pyrazol-4-yl)propyl)-2,5-dihydro-1H-pyrrol-3-yl)-1-(4-methylphenylsulfonyl)-1H-indole (500mg, crude) was dissolved in tetrahydrofuran (5mL). Methanol (1mL), water (1mL) and potassium hydroxide (304.34mg) were added. The reaction mixture was stirred at 70°C for 1 hour, dried by rotary-evaporation, diluted with water (10mL), and extracted with dichloromethane three times (10mL×3). The organic phases were combined to produce a crude product. The crude product was purified by prep-TLC to give a crude product (120mg). The crude product was purified by prep-HPLC (C18, 0.1% formic acid in water:acetonitrile) to produce the target compound (4.58mg).
LCMS (ESI) $[M+H]^+$=307.2; $^1H$ NMR(400 MHz, DMSO-$d_6$)δ 11.26(s, 1H), 8.21(s, 1H), 7.81(d, J=7.8 Hz, 1H), 7.48(s, 1H), 7.40(dd, J=5.1,2.6 Hz, 2H), 7.26(s, 1H), 7.11(dt, J=14.9, 7.1 Hz, 2H), 6.10(s, 1H), 3.90(s, 2H), 3.77(s, 3H), 3.71(s, 2H), 2.81-2.72(m, 2H), 2.46(d, J=7.6 Hz, 2H), 1.88-1.66(m, 2H).

Examples 72-73

With reference to the preparation of Example 29, compounds 72-73 were prepared:

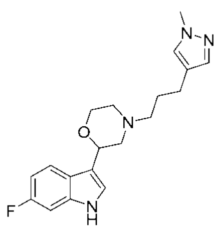
(72)
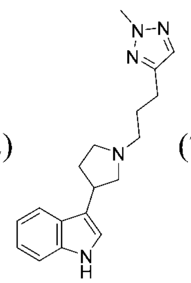
(73)

Compound 72: LCMS (ESI) $[M+H]^+$=343.2; $^1H$ NMR(400 MHz, DMSO-$d_6$)δ 11.09(s, 1H), 8.14(s, 1H), 7.65-7.56(m, 1H), 7.45(s, 1H), 7.30(s, 1H), 7.23(s, 1H), 7.12(d, J=10.1 Hz, 1H), 6.84(t, J=9.2 Hz, 1H), 4.78(d, J=9.9 Hz, 1H), 3.91(d, J=11.5 Hz, 1H), 3.73(d, J=17.7 Hz, 4H), 3.01(d, J=11.3 Hz, 1H), 2.86(d, J=11.2 Hz, 1H), 2.43(dd, J=13.7, 7.4 Hz, 5H), 2.29(t, J=11.0 Hz, 1H), 1.83-1.58(m, 2H).
Compound 73: LCMS (ESI) $[M+H]^+$=310.2.

Example 74 (Compound 74)

Preparation of 3-(1-(3-(4-methyl-4H-1,2,4-triazol-3-yl)propyl)pyrrolidin-3-yl)-1H-indole:

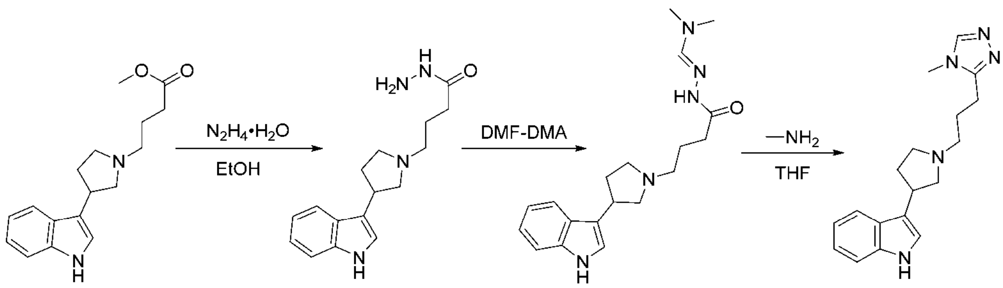


**Step 1:** Preparation of 4-(3-(1H-indol-3-yl)pyrrolidin-1-yl)butanehydrazide:

methyl 4-(3-(1H-indol-3-yl)pyrrolidin-1-yl)butanoate (3g, 1.0eq) was dissolved in ethanol (30mL). Under the protection of nitrogen gas, hydrazine hydrate (5.24g, 10eq) was added. The reaction mixture was stirred at 60°C for 12 hours, and concentrated to produce the target compound (3g, crude), which was directly used in the next step.

LCMS (ESI) $[M+H]^+$=287.1.

**Step 2:** Preparation of (E)-N'-(4-(3-(1H-indol-3-yl)pyrrolidin-1-yl)butanoyl)-N,N-dimethylformohydrazonamide:

4-(3-(1H-indol-3-yl)pyrrolidin-1-yl)butanehydrazide (3g, crude) was dissolved in methanol (30mL). N,N-dimethylformamide dimethyl acetal (3.12g) was added. In a nitrogen atmosphere, the reaction mixture was stirred at 80°C for 6 hours. Additional N,N-dimethylformamide dimethyl acetal (3.12g) is added. The reaction mixture was stirred at 80°C for another 6 hours, and concentrated to give a crude product. The crude product was purified by reverse-phase chromatography to produce the target compound (1g).

LCMS (ESI) $[M+H]^+$=342.2.

**Step 3:** Preparation of 3-(1-(3-(4-methyl-4H-1,2,4-triazol-3-yl)propyl)pyrrolidin-3-yl)-1H-indole:

(E)-N'-(4-(3-(1H-indol-3-yl)pyrrolidin-1-yl)butanoyl)-N,N-dimethylformohydrazonamide (300mg, 1eq) was dissolved in tetrahydrofuran (10mL). Under the protection of nitrogen gas, 1M methanamine tetrahydrofuran (20eq) was added. The reaction mixture was stirred at 100°C for 12 hours, and concentrated to give a crude product. This crude product was diluted with water (10mL), and extracted with ethyl acetate (20mL×3). The organic phases were combined, dried over anhydrous sodium sulfate, filtered, and concentrated to give another crude product. This crude product was purified by prep-HPLC (C18, 0.1%trifluoroacetic acid in water:acetonitrile), and converted to the hydrochloride salt by adding 1% hydrochloric acid to produce the target compound (47.22mg).

LCMS (ESI) $[M+H]^+$=310.2; $^1$H NMR(400 MHz, DMSO-$d_6$)δ 11.44(s, 1H), 11.05(s, 1H), 9.35(s, 1H), 7.60(s, 1H), 7.35(dd, J=17.4,5.0 Hz, 2H), 7.10(t, J=7.1 Hz, 1H), 7.01(t, J=7.4 Hz, 1H), 3.80(s, 6H), 3.34(d, J=6.6 Hz, 3H), 3.11(t, J=7.4 Hz, 3H), 2.47-2.42(m, 1H), 2.25(dt, J=14.5, 7.3 Hz, 3H).

Examples 75-76

With reference to the preparation of Example 74, compounds 75-76 were prepared:

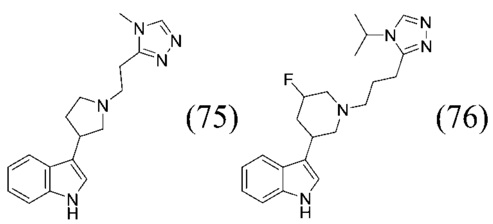
(75)
(76)

Compound 75: LCMS (ESI) $[M+H]^+$=296.1; $^1$H NMR(400 MHz, MeOD-$d_4$)δ 8.36(s, 1H), 7.56(d, J=7.8 Hz, 1H), 7.32(d, J=8.1 Hz, 1H), 7.11-7.04(m, 2H), 6.98(t, J=7.5 Hz, 1H), 3.66(d, J=8.5 Hz, 4H), 3.26(d, J=8.2 Hz, 1H), 3.09-2.95(m,

5H), 2.82(dd, J=14.9, 9.0 Hz, 1H), 2.73(t, J=8.9 Hz, 1H), 2.45-2.32(m, 1H), 2.15-2.01(m, 1H).

Compound 76: LCMS (ESI) $[M+H]^+$=370.2; $^1H$ NMR(400 MHz, DMSO-$d_6$)δ 10.88(s, 1H), 8.54(s, 1H), 7.56(d, J=7.9 Hz, 1H), 7.34(d, J=8.0 Hz, 1H), 7.19(d, J=2.2 Hz, 1H), 7.06(t, J=7.5 Hz, 1H), 6.97(t, J=7.3 Hz, 1H), 4.86-4.64(m, 1H), 4.37(dt, J=13.3, 6.7 Hz, 1H), 3.30(s, 2H), 3.25(s, 1H), 3.11-2.98(m, 2H), 2.73(t, J=7.4 Hz, 2H), 2.38(s, 1H), 2.06-1.94(m, 2H), 1.89(dd, J=14.5, 7.2 Hz, 2H), 1.74-1.62(m, 1H), 1.39(dd, J=6.6, 3.4 Hz, 6H).

Compound 77: LCMS (ESI) $[M+H]^+$=324.2.

Example 78 (Compound 78)

Preparation of 3-(8-(3-(4-(propan-2-yl)-4H-1,2,4-triazol-3-yl)propyl)-8-azabicyclo[3.2.1]octan-6-yl)-1H-indole:

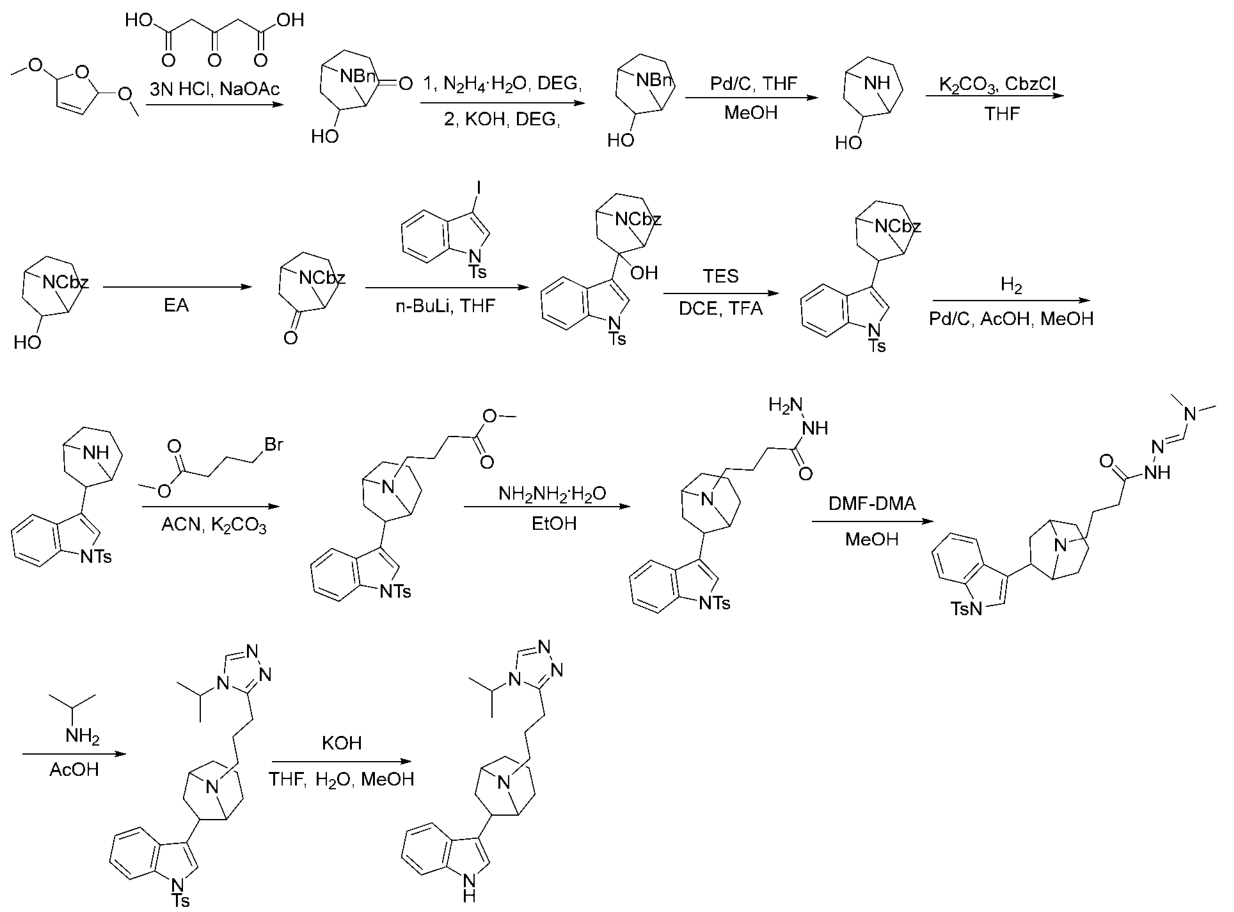


**Step** 1: Preparation of 8-benzyl-7-hydroxy-8-azabicyclo[3.2.1]octan-2-one:

2,5-dimethoxy-2,5-dihydrofuran (50g, 1eq) and 3-oxopentanedioic acid were dissolved in 3M aqueous hydrochloric acid solution (722mL). Under the protection of nitrogen gas, the reaction mixture was stirred at room temperature overnight. 6M aqueous sodium hydroxide solution (340mL) was added to adjust pH=7. The reaction mixture was added to sodium acetate buffer solution (2000mL) and stirred at room temperture overnight. Potassium carbonate (1.18eq) and sodium chloride (2.8eq) were added. The resulting mixture was stirred and reacted for another 1 hour, and then extracted with dichloromethane (4×300mL). The organic phases were combined, washed with saturated brine, dried over anhydrous sodium sulfate, filtered, and concentrated. The resulting crude product was purified by flash chromatography (Silica gel, PE:EA=3:1) to produce the target compound (14g).

LCMS (ESI) $[M+H]^+$=232.1.

**Step 2:** Preparation of 8-benzyl-8-azabicyclo[3.2.1]octan-6-ol:

Hydrazine hydrate (10eq) was added to a mixture of 8-benzyl-7-hydroxy-8-azabicyclo[3.2.1]octan-2-one

(15g,1eq) in diethylene glycol (250mL). The mixture was stirred at 120°C for 2 hours. LCMS detection showed the raw materials disappeared. Potassium hydroxide (20eq)/diethylene glycol were added in batch. The mixture was warmed up to 200°C and stirred for 4 hours, then cooled to room temperature. Water (1L) was added, and the mixture was stirred for 1 hour. A large amount of white solid precipitated, and the reaction mixture was filtered. The filter cake was washed with water. The solid was collected, and dried to produce the target compound (27g, crude).

LCMS (ESI) $[M+H]^+$=218.1.

**Step 3:** Preparation of 8-azabicyclo[3.2.1]octan-6-ol:

Palladium/carbon (300mg) was added to a mixture of 8-benzyl-8-azabicyclo[3.2.1]octan-6-ol (3g, crude) in methanol (30mL) and tetrahydrofuran (30mL). In the presence of palladium/carbon, the reaction mixture was stirred at room temperature for 12 hours, and then filtered through diatomite. The filter cake was washed with tetrahydrofuran. The filtrate was collected, and concentrated in vacuum to produce the target compound (1.2g).

LCMS (ESI) $[M+H]^+$=128.3.

**Step 4:** Preparation of benzyl 6-hydroxy-8-azabicyclo[3.2.1]octane-8-carboxylate:

At 0°C, benzyl chloroformate (1.1eq) was added to a mixture of 8-azabicyclo[3.2.1]octan-6-ol (1.2g, 1eq) and potassium carbonate (2eq)/tetrahydrofuran (10mL). The reaction mixture was stirred at room temperature for 2 hours, diluted with water (10mL), and extracted with ethyl acetate (10mL×3). The organic phases were combined, washed with saturated brine, dried over anhydrous sodium sulfate, and concentrated in vacuum. The residue was purified by flash chromatography to produce the target compound (1.47g).

LCMS (ESI) $[M+H]^+$=262.1.

**Step 5:** Preparation of benzyl 6-oxo-8-azabicyclo[3.2.1]octane-8-carboxylate:

Dess-Martin periodinane (3eq) was added to a mixture of benzyl 6-hydroxy-8-azabicyclo[3.2.1]octane-8-carboxylate (1g, 1eq) in ethyl acetate (20mL). The mixture was stirred at 80°C for 16 hours. The reaction mixture was cooled and filtered. The filtrate was concentrated in vacuum, and purified by reverse-phase chromatography (C18, 0.1% formic acid in water:acetonitrile) to give the target product (650mg).

LCMS (ESI) $[M+H]^+$=260.1.

**Step 6:** Preparation of benzyl 6-hydroxy-6-(1-(4-methylphenylsulfonyl)-1H-indol-3-yl)-8-azabicyclo[3.2.1]octane-8-carboxylate:

3-iodo-1-(4-methylphenylsulfonyl)-1H-indole (1.5eq) was dissolved in tetrahydrofuran (50mL). The atmosphere was replaced with nitrogen gas. At -78°C, a solution of 1.6M n-butyllithium in tetrahydrofuran (1.5eq) was added dropwise. After the completion of the addition, the mixture was stirred at -78°C for 0.5 hours. To the reaction mixture was added benzyl 6-oxo-8-azabicyclo[3.2.1]octane-8-carboxylate (5g, 1eq). After the completion of the addition, the mixture was stirred at -78°C for 1 hour. The reaction mixture was slowly diluted with saturated sodium carbonate solution (50mL) at -78°C, and extracted with ethyl acetate (50mL×3). The organic phases were combined, washed with saturated brine, dried over anhydrous sodium sulfate, and filtered by suction. The mother liquor was concentrated to give a crude product. The crude product was purified by flash chromatography to produce the target compound (2.6g).

LCMS (ESI) $[M+Na]^+$=553.1.

**Step 7:** Preparation of benzyl 6-(1-(4-methylphenylsulfonyl)-1H-indol-3-yl)-8-azabicyclo[3.2.1]octane-8-carboxylate:

Benzyl 6-hydroxy-6-(1-(4-methylphenylsulfonyl)-1H-indol-3-yl)-8-azabicyclo[3.2.1]octane-8-carboxylate (2.8g, 1eq) was dissolved in dichloroethane (20mL), and trifluoroacetic acid (10eq) and triethylsilane (10eq) were added. The atmosphere was replaced with nitrogen gas. The reaction mixture was stirred in a sealed container at 100°C for 3 hours, then diluted with saturated sodium carbonate solution (50mL), and extracted with dichloromethane (50mL×3). The organic phases were combined, washed with saturated sodium bicarbonate solution once and with saturated brine once, dried over anhydrous sodium sulfate, and filtered by suction. The mother liquor was concentrated to produce the target compound (2.3g, yield: 84.7%).

**Step 8:** Preparation of 3-(8-azabicyclo[3.2.1]octan-6-yl)-1-(4-methylphenylsulfonyl)-1H-indole:

Benzyl 6-(1-(4-methylphenylsulfonyl)-1H-indol-3-yl)-8-azabicyclo[3.2.1]octane-8-carboxylate (2.2g, 1eq) was

dissolved in methanol (20mL), and palladium/carbon (0.1eq) and acetic acid (1eq) were added. The atmosphere was replaced with hydrogen gas. The reaction mixture was stirred at room temperature for 16 hours, and filtered by suction. The mother liquor was concentrated to give a crude product. The crude product was purified to produce the target compound (560mg).

LCMS (ESI) $[M+H]^+$=381.2.

**Step 9:** Preparation of methyl 4-(6-(1-(4-methylphenylsulfonyl)-1H-indol-3-yl)-8-azabicyclo[3.2.1]octan-8-yl)butanoate:

3-(8-azabicyclo[3.2.1]octan-6-yl)-1-(4-methylphenylsulfonyl)-1H-indole (550mg, 1eq) was dissolved in acetonitrile (10mL), and methyl 4-bromobutanoate (1.2eq) and potassium carbonate (1.5eq) were added. The reaction mixture was stirred at 80°C for 16 hours, diluted with water (20mL), and extracted with ethyl acetate (20mL×3). The organic phases were combined, washed with saturated brine, dried over anhydrous sodium sulfate, and filtered by suction. The mother liquor was concentrated to give a crude product. The crude product was purified by flash chromatography to produce the target compound (560mg).

LCMS (ESI) $[M+H]^+$=481.2.

**Step 10:** Preparation of 4-(6-(1-(4-methylphenylsulfonyl)-1H-indol-3-yl)-8-azabicyclo[3.2.1]octan-8-yl)butanehydrazide:

Methyl 4-(6-(1-(4-methylphenylsulfonyl)-1H-indol-3-yl)-8-azabicyclo[3.2.1]octan-8-yl)butanoate (100mg, 1eq) was dissolved in ethanol (5mL), and hydrazine hydrate (10eq) was added. The reaction mixture was stirred at 60°C for 16 hours, and concentrated to produce the target compound (90mg).

LCMS (ESI) $[M+H]^+$=481.2.

**Step 11:** Preparation of (E)-N,N-dimethyl-N'-(4-(6-(1-(4-methylphenylsulfonyl)-1H-indol-3-yl)-8-azabicyclo[3.2.1]octan-8-yl)butanoyl)formohydrazonamide:

4-(6-(1-(4-methylphenylsulfonyl)-1H-indol-3-yl)-8-azabicyclo[3.2.1]octan-8-yl)butanehydrazide (400mg, 1eq) was dissolved in methanol (10mL), and N,N-dimethylformamide dimethyl acetal (10eq) was added. The mixture was reacted at 80°C for 16 hours, and concentrated to give a crude product. The crude product was purified to produce the target compound (400mg).

LCMS (ESI) $[M+H]^+$=536.3.

**Step 12:** Preparation of 1-(4-methylphenylsulfonyl)-3-(8-(3-(4-(propan-2-yl)-4H-1,2,4-triazol-3-yl)propyl)-8-azabicyclo [3.2.1]octan-6-yl)-1H-indole:

(E)-N,N-dimethyl-N'-(4-(6-(1-(4-methylphenylsulfonyl)-1H-indol-3-yl)-8-azabicyclo[3.2.1]octan-8-yl)butanoyl) formohydrazonamide (240mg, 1eq) was dissolved in acetic acid (5mL), and propan-2-amine (10eq) was added. The mixture was reacted in a sealed tube at 110°C for 16 hours, and concentrated to give a crude product. The crude product was purified to produce the target compound (170mg).

LCMS (ESI) $[M+H]^+$=532.2.

**Step 13:** 3-(8-(3-(4-(propan-2-yl)-4H-1,2,4-triazol-3-yl)propyl)-8-azabicyclo[3.2.1]octan-6-yl)-1H-indole:

1-(4-methylphenylsulfonyl)-3-(8-(3-(4-(propan-2-yl)-4H-1,2,4-triazol-3-yl)propyl)-8-azabicyclo[3.2.1]octan-6-yl)-1H-indole (90mg, 1eq) was dissolved in tetrahydrofuran (5mL), potassium hydroxide (249.5eq) was added, then methanol (1mL) and water (1mL) were added. The mixture was stirred at 70°C for 6 hours, diluted with water (20mL), and extracted with ethyl acetate (20mL×3). The organic phases were combined, washed with saturated brine, dried over anhydrous sodium sulfate, and filtered by suction. The mother liquor was concentrated to give a crude product. The crude product was purified by prep-HPLC (C18, 0.05% ammonia in water:acetonitrile) to produce the target compound (17.85mg).

LCMS (ESI) $[M+H]^+$=378.4; $^1H$ NMR(400 MHz, MeOD-$d_4$)$\delta$ 8.69(s, 1H), 8.48(s, 1H), 7.53(d, J=7.9 Hz, 1H), 7.45-7.34(m, 2H), 7.14(t, J=7.6 Hz, 1H), 7.04(t, J=7.4 Hz, 1H), 4.54-4.41(m, 2H), 4.19(s, 2H), 3.49(s, 2H), 3.14-2.98(m, 2H), 2.83(dd, J=20.3, 13.1 Hz, 1H), 2.52(dd, J=13.7, 6.8 Hz, 1H), 2.41(s, 2H), 2.18(s, 1H), 1.91(d, J=8.7 Hz, 3H), 1.54(d, J=6.7 Hz, 6H), 1.45(s, 2H).

Examples 79-84

With reference to the preparation of Example 4, 5, 74, or 78, compounds 79-84 were prepared:

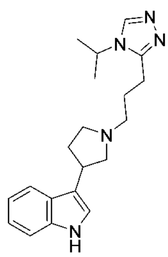
(79)
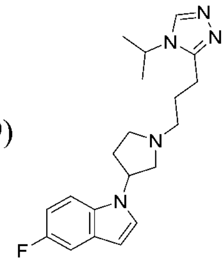
(80)
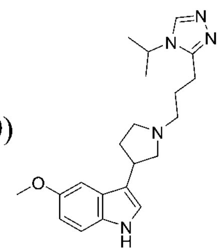
(81)

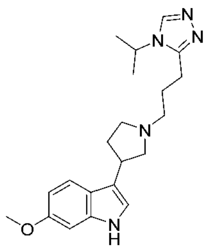
(82)
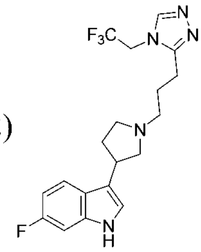
(83)
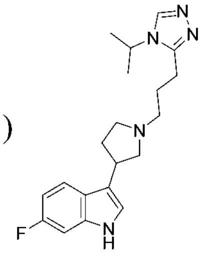
(84)

Compound 79: LCMS (ESI) $[M+H]^+$=338.2; $^1$H NMR(400 MHz, DMSO-$d_6$)δ 10.80(s, 1H), 8.55(s, 1H), 8.22(s, 1H), 7.61(d, J=7.8 Hz, 1H), 7.33(d, J=8.1 Hz, 1H), 7.17(d, J=2.2 Hz, 1H), 7.06(t, J=7.5 Hz, 1H), 6.96(t, J=7.4 Hz, 1H), 4.40-4.29(m, 1H), 3.61-3.51(m, 1H), 3.14(t, J=8.5 Hz, 1H), 2.87(dd, J=16.3, 7.6 Hz, 1H), 2.79(dd, J=14.6, 7.3 Hz, 3H), 2.74-2.59(m, 3H), 2.28(ddd, J=21.5, 10.4, 6.6 Hz, 1H), 2.00-1.87(m, 3H), 1.37(t, J=6.4 Hz, 6H).

Compound 80: LCMS (ESI) $[M+H]^+$=356.2;

Compound 81: LCMS (ESI) $[M+H]^+$=368.3; $^1$H NMR(400 MHz, Chloroform-d)δ 8.15(s, 1H), 7.99(s, 1H), 7.24(s, 1H), 7.11(d, J=2.3 Hz, 1H), 7.02(d, J=2.2 Hz, 1H), 6.86(dd, J=8.8, 2.4 Hz, 1H), 4.39-4.22(m, 1H), 3.86(s, 3H), 3.74-3.59(m, 1H), 3.24(t, J=8.5 Hz, 1H), 3.00(d, J=8.2 Hz, 1H), 2.85(t, J=7.5 Hz, 4H), 2.76(d, J=8.1 Hz, 2H), 2.44-2.34(m, 1H), 2.12(ddd, J=21.0, 12.0, 6.6 Hz, 3H), 1.46(dd, J=6.7, 4.8 Hz, 6H).

Compound 82: LCMS (ESI) $[M+H]^+$=368.2;

Compound 83: LCMS (ESI) $[M+H]^+$=396.1; $^1$H NMR(400 MHz, DMSO-ds)δ 10.92(s, 1H), 8.51(s, 1H), 8.24(s, 1H), 7.60(dd, J=8.7, 5.5 Hz, 1H), 7.18(d, J=2.0 Hz, 1H), 7.11(dd, J=10.2, 2.3 Hz, 1H), 6.83(ddd, J=9.8, 8.8, 2.4 Hz, 1H), 5.12(q, J=9.2 Hz, 2H), 3.61-3.51(m, 1H), 3.21(t, J=8.6 Hz, 1H), 2.93(dt, J=14.8, 7.4 Hz, 1H), 2.88-2.68(m, 6H), 2.29(ddd, J=21.5, 10.4, 6.6 Hz, 1H), 2.03-1.88(m, 3H).

Compound 84: LCMS (ESI) $[M+H]^+$=356.1; $^1$H NMR(400 MHz, Chloroform-d)δ 8.15(s, 1H), 8.07(s, 1H), 7.62-7.54(m, 1H), 7.07-6.99(m, 2H), 6.91-6.81(m, 1H), 4.40-4.26(m, 1H), 3.66(s, 1H), 3.18(s, 1H), 2.83(dd, J=34.4, 26.9 Hz, 7H), 2.39(s, 1H), 2.15(s, 2H), 2.04(dd, J=17.0, 7.1 Hz, 1H), 1.47(dd, J=6.6, 5.6 Hz, 6H).

Example 85 (Compound 85)

Preparation of 3-(1-(3-(4-(difluoromethyl)-4H-1,2,4-triazol-3-yl)propyl)pyrrolidin-3-yl)-1H-indole:

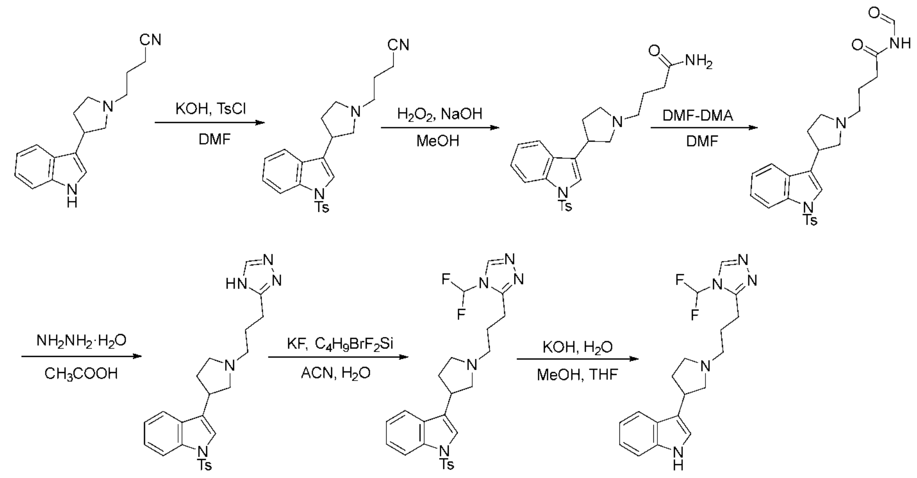


**Step** 1: Preparation of 4-(3-(1-(4-methylphenylsulfonyl)-1H-indol-3-yl)pyrrolidin-1-yl)butanenitrile:

4-(3-(1H-indol-3-yl)pyrrolidin-1-yl)butanenitrile (5g, 1eq) was dissolved in N,N-dimethylformamide (50mL), and potassium hydroxide (4eq) was added. The reaction system was stirred for 30 minutes, and a solution of 4-methylbenzenesulfonyl chloride (2.5eq) in N,N-dimethylformamide (25mL) was added dropwise. After the completion of the addition, the reaction system was stirred for 12 hours, diluted with water (150mL), and extracted with ethyl acetate (50mL×3). The organic phases were combined, washed with saturated brine twice, dried over anhydrous sodium sulfate, filtered, and concentrated to give a crude product. The crude product was purified by flash chromatography to produce the target compound (5g).

LCMS (ESI) $[M+H]^+$=408.2.

**Step 2:** Preparation of 4-(3-(1-(4-methylphenylsulfonyl)-1H-indol-3-yl)pyrrolidin-1-yl)butanamide:

4-(3-(1-(4-methylphenylsulfonyl)-1H-indol-3-yl)pyrrolidin-1-yl)butanenitrile (4.5g, 1eq) was dissolved in methanol (45mL). 30% hydrogen peroxide (5eq), and 1N sodium hydroxide (2eq) were added sequentially. The solution became turbid. Dimethyl sulfoxide (4.5 mL) was then added. The reaction system was stirred at room temperature for 3 hours, extracted with ethyl acetate (50mL×3). The organic phases were combined, dried over anhydrous sodium sulfate, filtered, and concentrated to produce the target compound (4.1g).

LCMS (ESI) $[M+H]^+$=426.3.

**Step 3:** Preparation of N-formyl-4-(3-(1-(4-methylphenylsulfonyl)-1H-indol-3-yl)pyrrolidin-1-yl)butanamide:

4-(3-(1-(4-methylphenylsulfonyl)-1H-indol-3-yl)pyrrolidin-1-yl)butanamide (3.6g, 1eq) was dissolved in a mixed solution of N,N-dimethylformamide dimethyl acetal (36mL) and N,N-dimethylformamide (3.6mL). The reaction system was stirred at 80°C for 12 hours, and concentrated to give a crude product.

The crude product was purified to produce the target compound (3.2g).

LCMS (ESI) $[M+H]^+$=454.1.

**Step 4:** Preparation of 1-(4-methylphenylsulfonyl)-3-(1-(3-(4H-1,2,4-triazol-3-yl)propyl)pyrrolidin-3-yl)-1H-indole:

N-formyl-4-(3-(1-(4-methylphenylsulfonyl)-1H-indol-3-yl)pyrrolidin-1-yl)butanamide (3g, 1eq) was dissolved in acetic acid (30mL). Hydrazine hydrate (2eq) was added under an ice bath. The reaction system was stirred at 90°C for 2 hours, and concentrated to give a crude product. The crude product was purified to produce the target compound (2.2g).

LCMS (ESI) $[M+H]^+$=450.1.

**Step 5:** Preparation of 3-(1-(3-(4-(difluoromethyl)-4H-1,2,4-triazol-3-yl)propyl)pyrrolidin-3-yl)-1-(4-methylphenylsulfonyl)-1H-indole:

1-(4-methylphenylsulfonyl)-3-(1-(3-(4H-1,2,4-triazol-3-yl)propyl)pyrrolidin-3-yl)-1H-indole (2g, 1eq) was dissolved in acetonitrile (20mL) and saturated aqueous potassium fluoride solution (4mL). (Bromodifluoromethyl)trimethylsilane (5eq) was added. The reaction system was stirred at room temperature for 16 hours, concentrated, diluted with water (20mL), and extracted with dichloromethane (30mL×3). The organic phases were combined, dried over anhydrous

sodium sulfate, filtered, and concentrated to give a crude product. The crude product was purified by prep-HPLC (C18, 0.1% ammonia in water:acetonitrile) to produce the target compound (200mg).

LCMS (ESI) $[M+H]^+$=500.2.

**Step 6:** Preparation of 3-(1-(3-(4-(difluoromethyl)-4H-1,2,4-triazol-3-yl)propyl)pyrrolidin-3-yl)-1H-indole:

3-(1-(3-(4-(difluoromethyl)-4H-1,2,4-triazol-3-yl)propyl)pyrrolidin-3-yl)-1-(4-methylphenylsulfonyl)-1H-indole (200mg, 1eq) was dissolved in a mixed solution of methanol (0.1mL), tetrahydrofuran (0.5mL) and water (0.1mL). Then potassium hydroxide (50eq) was added. The reaction system was stirred at 70°C for 3 hours, diluted with water (10mL), extracted with ethyl acetate (15mL×3). The organic phases were combined, dried over anhydrous sodium sulfate, filtered, and concentrated to give a crude product. The crude product was purified by prep-HPLC (C18, 0.05% ammonia in water:acetonitrile) to produce the target compound (18.73mg).

LCMS (ESI) $[M+H]^+$=345.9; $^1$H NMR(400 MHz, Chloroform-d)δ 8.40(s, 1H), 7.96(s, 1H), 7.67(d, J=7.9 Hz, 1H), 7.37-7.32(m, 1H), 7.19(dd, J=8.9, 6.2 Hz, 2H), 7.10(t, J=7.5 Hz, 1H), 7.03(s, 1H), 3.69(dd, J=16.7, 8.3 Hz, 1H), 3.21(t, J=7.9 Hz, 1H), 2.94(s, 1H), 2.87(t, J=7.5 Hz, 2H), 2.74-2.59(m, 4H), 2.42-2.32(m, 1H), 2.04(dd, J=14.2, 6.6 Hz, 3H).

Example 86 (Compound 86)

Preparation of 3-(1-(3-(5-methyl-4-(propan-2-yl)-4H-1,2,4-triazol-3-yl)propyl)pyrrolidin-3-yl)-1H-indole:

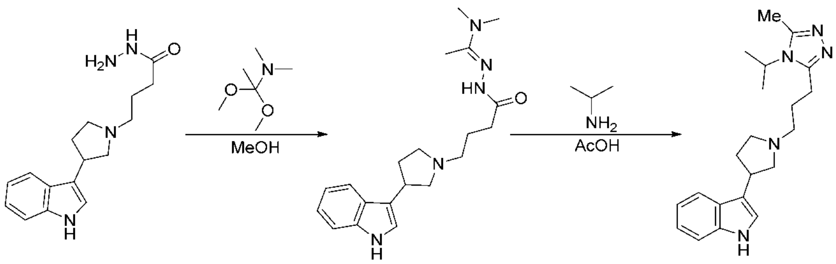


**Step** 1: Preparation of (E)-N'-(4-(3-(1H-indol-3-yl)pyrrolidin-1-yl)butanoyl)-N,N-dimethylacetohydrazonamide:

4-(3-(1H-indol-3-yl)pyrrolidin-1-yl)butanehydrazide (600mg, 1.0eq) was dissolved in methanol (10mL), and 1,1-dimethoxy-N,N-dimethylethan-1-amine (3.0eq) was added. The reaction mixture was stirred at 80°C for 3 hours, concentrated to give a crude product. The crude product was purified by flash chromatography to produce the target compound (240mg).

LCMS (ESI) $[M+H]^+$=356.2.

**Step 2:** Preparation of 3-(1-(3-(5-methyl-4-(propan-2-yl)-4H-1,2,4-triazol-3-yl)propyl)pyrrolidin-3-yl)-1H-indole:

(E)-N'-(4-(3-(1H-indol-3-yl)pyrrolidin-1-yl)butanoyl)-N,N-dimethylacetohydrazonamide(220mg, 1.0eq) was dissolved in acetic acid (5mL), and Isopropylamine (10eq) was added. The reaction mixture was stirred at 130°C for 48 hours, and concentrated to give a crude product, and purified by prep-HPLC (C18, 0.05% formic acid in water:acetonitrile) to produce the target compound (19.57mg).

LCMS (ESI) $[M+H]^+$=352.2; $^1$H NMR(400 MHz, DMSO-$d_6$)δ 10.79(s, 1H), 8.17(s, 1H), 7.61(d, J=7.9 Hz, 1H), 7.33(d, J=8.2 Hz, 1H), 7.17(s, 1H), 7.06(t, J=7.5 Hz, 1H), 6.96(t, J=7.5 Hz, 1H), 4.43(dd, J=14.0, 7.0 Hz, 1H), 3.57(s, 1H), 3.14(s, 1H), 2.86(s, 1H), 2.82-2.75(m, 3H), 2.73-2.62(m, 3H), 2.38(, 3H), 2.28(s, 1H), 1.94(dt, J=14.1, 7.2 Hz, 3H), 1.39(d, J=6.9 Hz, 6H).

Example 87 (Compound 87)

Preparation of 3-(1-(3-(4-methyl-5-(trifluoromethyl)-4H-1,2,4-triazol-3-yl)propyl)pyrrolidin-3-yl)-1H-indole:

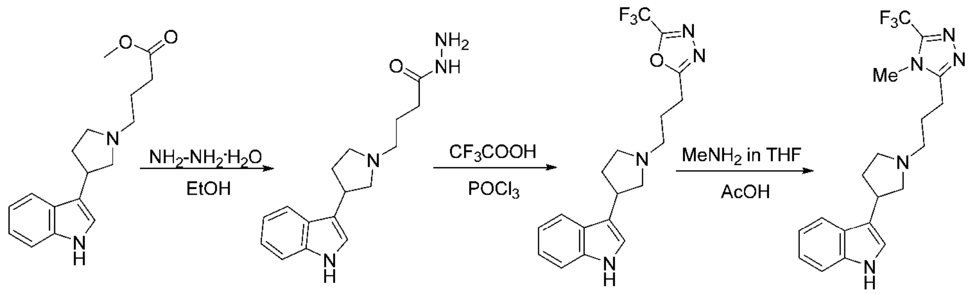


**Step 1:** Preparation of 4-(3-(1H-indol-3-yl)pyrrolidin-1-yl)butanehydrazide:

methyl 4-(3-(1H-indol-3-yl)pyrrolidin-1-yl)butanoate (800mg, 1eq) was dissolved in ethanol (5mL), and hydrazine hydrate (1398.47mg, 10eq) was added. The reaction mixture was stirred at 60°C for 16 hours, and concentrated to produce the target compound (720mg, crude), which was directly used in the next step.

LCMS (ESI) $[M+H]^+$=287.1.

**Step 2:** Preparation of 2-(3-(3-(1H-indol-3-yl)pyrrolidin-1-yl)propyl)-5-(trifluoromethyl)-1,3,4-oxadiazole:

4-(3-(1H-indol-3-yl)pyrrolidin-1-yl)butanehydrazide (1.5g, crude) was dissolved in phosphoryl trichloride (10mL), and trifluoroacetic acid (0.9g) was added. The atmosphere was replaced with nitrogen gas. The reaction mixture was stirred at 80°C for 16 hours, concentrated to remove phosphoryl trichloride, and then cooled down to 0°C. At 0°C, saturated sodium carbonate solution (50mL) was slowly added dropwise to the reaction mixture. After the completion of the addition, the reaction mixture was stirred at room temperature for 20 minutes, extracted with ethyl acetate (50mL×3), and the phases were separated. The organic phases were combined, washed with saturated sodium carbonate solution (25mL) once and with saturated sodium chloride solution (25mL) once, dried over anhydrous sodium sulfate, and filtered by suction. The mother liquor was concentrated to give a crude product. The crude product was purified by prep-HPLC (C18, 0.1% formic acid in water:acetonitrile) to produce the target compound (150mg).
LCMS (ESI) $[M+H]^+$=365.0.

**Step 3:** Preparation of 3-(1-(3-(4-methyl-5-(trifluoromethyl)-4H-1,2,4-triazol-3-yl)propyl)pyrrolidin-3-yl)-1H-indole:

2-(3-(3-(1H-indol-3-yl)pyrrolidin-1-yl)propyl)-5-(trifluoromethyl)-1,3,4-oxadiazole (500mg, 1eq) was dissolved in acetic acid (10mL), and a 2M solution of methanamine in tetrahydrofuran (10eq) was added. The reaction mixture was stirred in a sealed tube at 130°C for 48 hours, concentrated to remove acetic acid, diluted with saturated sodium carbonate solution (20mL), and extracted with ethyl acetate (20mL×3). The organic phases were combined, washed with saturated sodium carbonate solution (20mL) once and with saturated brine (20mL) once, and filtered by suction. The mother liquor was concentrated to give a crude product. The crude product was first purified by prep-HPLC (C18, 0.1% formic acid in water:acetonitrile) and then by prep-HPLC(C18, 0.05% ammonia in water:acetonitrile) to produce the target compound (53.63mg).
LCMS (ESI) $[M+H]^+$=378.2; $^1$H NMR(400 MHz, MeOD-$d_4$)$\delta$ 7.56(d, J=7.9 Hz, 1H), 7.32(d, J=8.1 Hz, 1H), 7.10-7.04(m, 2H), 6.98(t, J=7.4 Hz, 1H), 3.76(s, 3H), 3.69-3.60(m, 1H), 3.27-3.20(m, 1H), 3.01-2.91(m, 3H), 2.77-2.62(m, 4H), 2.36(dt, J=17.2, 7.9 Hz, 1H), 2.12-2.00(m, 3H).

Examples 88-89 (Compounds 88-89)

With reference to the preparation of Example 18 or 74, compounds 88-89 were prepared:

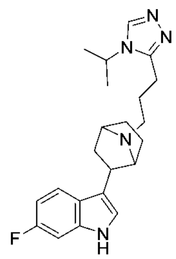
(88)

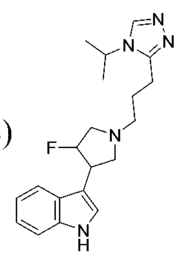
(89)

Compound 88: LCMS (ESI) $[M+H]^+$=382.2;
Compound 89: LCMS (ESI) $[M+H]^+$=356.2; $^1$H NMR(400 MHz, DMSO-$d_6$)δ 10.95(s, 1H), 8.55(s, 1H), 8.16(s, 1H), 7.56(d, J=7.8 Hz, 1H), 7.36(d, J=8.1 Hz, 1H), 7.25(d, J=2.1 Hz, 1H), 7.10(dd, J=11.0, 4.0 Hz, 1H), 7.00(dd, J=11.0, 3.9 Hz, 1H), 5.31-5.09(m, 1H), 4.45-4.29(m, 1H), 3.73-3.54(m, 1H), 3.34(t, J=8.3 Hz, 1H), 3.10(dd, J=24.3, 11.7 Hz, 1H), 2.84-2.66(m, 3H), 2.61-2.53(m, 2H), 2.46(t, J=8.8 Hz, 1H), 2.00-1.86(m, 2H), 1.39(dd, J=6.7, 3.5 Hz, 6H).

Example 90 (Compound 90)

Preparation of 6-fluoro-3-(4-methyl-1-(3-(4-(propan-2-yl)-4H-1,2,4-triazol-3-yl)propyl)pyrrolidin-3-yl)-1H-indole:

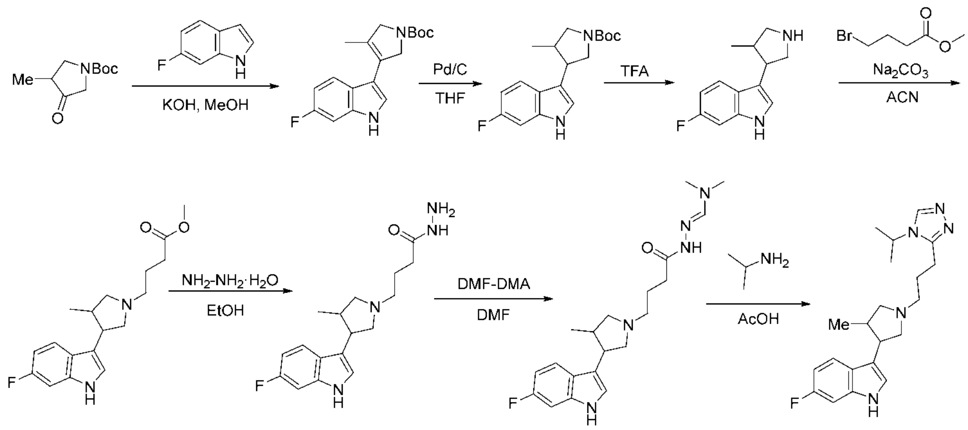


**Step 1**: Preparation of tert-butyl 3-(6-fluoro-1H-indol-3-yl)-4-methyl-2,5-dihydro-1H-pyrrole-1-carboxylate:

tert-butyl 3-methyl-4-oxopyrrolidine-1-carboxylate (1.7eq) and 6-fluoro-1H-indole (6g, 1eq) were dissolved in methanol (100mL). Potassium hydroxide (10eq) was added. The mixture was reacted at 70°C for 16 hours, dried by rotary-evaporation to remove methanol, diluted with water (50mL), extracted with ethyl acetate (100mL×3). The organic phases were dried by rotary-evaporation to give a crude product. The crude product was purified by flash chromatography (Silica gel, PE:EA=5:1) to give the target product (3g, crude).
LCMS (ESI) $[M-56+MeCN+H]^+$=302.2.

**Step 2:** Preparation of tert-butyl 3-(6-fluoro-1H-indol-3-yl)-4-methylpyrrolidine-1-carboxylate:

tert-butyl 3-(6-fluoro-1H-indol-3-yl)-4-methyl-2,5-dihydro-1H-pyrrole-1-carboxylate (2g, 1eq) was dissolved in tetrahydrofuran (20mL). Palladium/carbon (500mg, 10%) was added. Under a hydrogen atmosphere, the reaction mixture was stirred at room temperature for 1 hour, and filtered through diatomite. The filtrate was dried by rotary-evaporation to produce the target compound (2g, crude).
LCMS (ESI) $[M-56+MeCN+H]^+$=304.2.

**Step 3:** Preparation of 6-fluoro-3-(4-methylpyrrolidin-3-yl)-1H-indole:

tert-butyl 3-(6-fluoro-1H-indol-3-yl)-4-methylpyrrolidine-1-carboxylate (2g, crude) was dissolved in dichloromethane (20mL).Trifluoroacetic acid (5mL) was added. At room temperature, the mixture was reacted for 1 hour. LCMS detection showed the completion of the reaction. The reaction mixture was dried by rotary-evaporation and purified by reverse-phase chromatography (C18, 0.1% formic acid in water:acetonitrile) to produce the target compound (1g).
LCMS (ESI) $[M+H]^+$=219.1.

**Step 4:** Preparation of methyl 4-(3-(6-fluoro-1H-indol-3-yl)-4-methylpyrrolidin-1-yl)butanoate:

6-fluoro-3-(4-methylpyrrolidin-3-yl)-1H-indole (800mg, 1eq) was dissolved in acetonitrile (10mL). Methyl 4-bromobutanoate (0.8eq) and sodium carbonate (4eq) were added. The mixture was reacted at 70°C for 16 hours, then filtered. The filtrate was dried by rotary-evaporation to give a crude product. The crude product was purified by flash chromatography (Silica gel, DCM:MeOH=20:1) to give the target product (400mg, yield: 34.3%).

LCMS (ESI) $[M+H]^+$=319.3.

**Step 5:** Preparation of 4-(3-(6-fluoro-1H-indol-3-yl)-4-methylpyrrolidin-1-yl)butanehydrazide:

Methyl 4-(3-(6-fluoro-1H-indol-3-yl)-4-methylpyrrolidin-1-yl)butanoate (360mg, 1eq) was dissolved in ethanol (4mL). Hydrazine hydrate (10eq) was added. The mixture was reacted at 60°C for 4 hours. TLC detection showed the raw materials disappeared, LCMS detection showed the completion of the reaction. The reaction mixture was dried by rotary-evaporation to produce the target compound (360mg, crude).

LCMS (ESI) $[M+H]^+$=319.2.

**Step 6:** Preparation of (E)-N'-(4-(3-(6-fluoro-1H-indol-3-yl)-4-methylpyrrolidin-1-yl)butanoyl)-N,N-dimethylformohydrazonamide:

4-(3-(6-fluoro-1H-indol-3-yl)-4-methylpyrrolidin-1-yl)butanehydrazide (360mg, crude) was dissolved in N,N-dimethylformamide (1mL). N,N-dimethylformamide dimethyl acetal (22.26eq) was added. The mixture was reacted at 70°C for 16 hours. LCMS detection showed the completion of the reaction. The reaction mixture was dried by rotary-evaporation. The resulting crude product purified by reverse-phase chromatography (C18, 0.1%$NH_3 \cdot H_2O$ in water, MeOH) to produce the target compound (250mg).

LCMS (ESI) $[M+H]^+$=374.5.

**Step 7:** Preparation of 6-fluoro-3-(4-methyl-1-(3-(4-(propan-2-yl)-4H-1,2,4-triazol-3-yl)propyl)pyrrolidin-3-yl)-1H-indole:

(E)-N'-(4-(3-(6-fluoro-1H-indol-3-yl)-4-methylpyrrolidin-1-yl)butanoyl)-N,N-dimethylformohydrazonamide (250mg, 1eq) was dissolved in acetic acid (6mL). Under an ice bath, isopropylamine (10eq) was added dropwise. The mixture was reacted at 110°C for 16 hours, dried by rotary-evaporation to remove most of the acetic acid, adjusted with saturated potassium carbonate solution to pH=10, extracted with ethyl acetate (20mL×3) to remove the aqueous phases. The organic phases were dried by rotary-evaporation to produce a crude product. The crude product was purified by prep-HPLC (C18, 0.05%$NH_3 \cdot H_2O$ in water, MeCN) to give the target product (33.13mg).

LCMS (ESI) $[M+H]^+$=370.6; $^1H$ NMR(400 MHz, Chloroform-d)δ 8.16(s, 1H), 8.13(s, 1H), 7.50(dd, J=8.6, 5.3 Hz, 1H), 7.10-6.95(m, 2H), 6.87(td, J=9.4, 2.3 Hz, 1H), 4.36(dt, J=13.4, 6.8 Hz, 1H), 3.76(d, J=8.1 Hz, 1H), 3.23(s, 2H), 2.86(t, J=7.5 Hz, 2H), 2.70(dd, J=14.7, 7.1 Hz, 4H), 2.24(s, 1H), 2.13(s, 2H), 1.50(d, J=6.7 Hz, 6H), 0.62(d, J=7.1 Hz, 3H).

Examples 91-92 (Compounds 91-92)

With reference to the preparation of Example 18 or 74, compounds 91-92 were prepared:

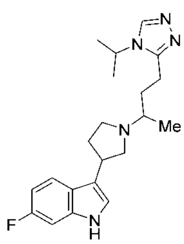
(91)

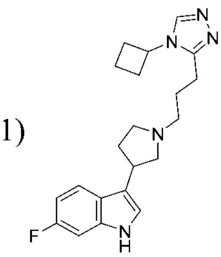
(92)

Compound 91: LCMS (ESI) $[M+H]^+$=370.2;

Compound 92: LCMS (ESI) $[M+H]^+$=368.1; $^1H$ NMR(400 MHz, DMSO-$d_6$)δ 8.19(s, 2H), 7.59(dd, J=8.7, 5.4 Hz, 1H), 7.03(dd, J=9.7, 2.2 Hz, 1H), 6.99(d, J=1.3 Hz, 1H), 6.86(m, 1H), 4.59-4.46(m, 1H), 3.65-3.54(m, 1H), 3.07(t, J=8.4 Hz, 1H), 2.82(dt, J=15.3, 8.1 Hz, 3H), 2.72-2.50(m, 6H), 2.38-2.29(m, 3H), 2.07-1.97(m, 3H), 1.89(m, 2H).

Examples 93-96 (Compounds 93-96)

With reference to the preparation of Example 74 or 86, compounds 93-96 were prepared:

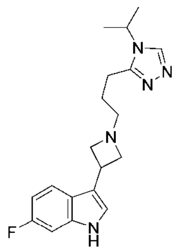 (93) 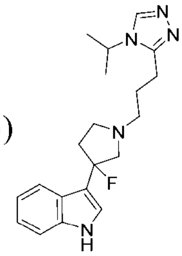 (94) 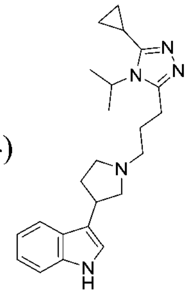 (95) 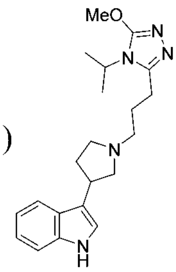 (96)

Compound 93: LCMS (ESI) $[M+H]^+$=342.2;
Compound 94: LCMS (ESI) $[M+H]^+$=356.2;
Compound 95: LCMS (ESI) $[M+H]^+$=378.2;
Compound 96: LCMS (ESI) $[M+H]^+$=368.2.

Example 97 (Compound 97)

Preparation of 6-fluoro-3-(1-(3-fluoro-3-(5-methyl-4-(propan-2-yl)-4H-1,2,4-triazol-3-yl)propyl)pyrrolidin-3-yl)-1H-indole:

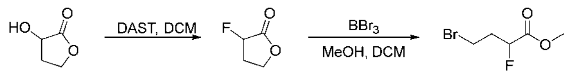


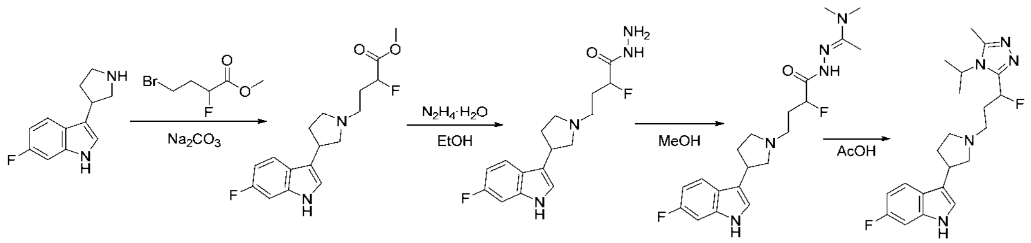


**Step 1:** Preparation of 3-fluorodihydrofuran-2(3H)-one:

3-hydroxydihydrofuran-2(3H)-one (14g, 1eq) was dissolved in dichloromethane (140mL). Under the protection of nitrogen gas, the mixture was cooled down to 0°C, and diethylaminosulfur trifluoride (1.5eq) was slowly added dropwise. The resulting mixture was warmed up to room temperature and reacted for another 16 hours. TLC detection showed the completion of the reaction. The reaction mixture was poured into saturated aqueous sodium bicarbonate solution (100mL), and extracted with dichloromethane (100mL×3). The organic phases were dried by rotary-evaporation to give a crude product. The crude product was purified by flash chromatography to produce the target compound (8g).

**Step 2:** Preparation of methyl 4-bromo-2-fluorobutanoate:

3-fluorodihydrofuran-2(3H)-one(8.4g, 1eq) was dissolved in dichloromethane (80mL). Under an ice bath, 1M tribromoborane (1.1eq) was added. The reaction mixture was warmed up to room temperature and stirred for 16 hours, diluted with methanol (10mL), and reacted for another 2 hours. The detection showed the completion of the reaction. The reaction mixture was diluted with water (80mL), and extracted. The organic phase was washed with saturated sodium bicarbonate solution (100mL*2), collected, and dried by rotary-evaporation to give a crude product. The crude product was purified by flash chromatography to produce the target compound (5.5g).

**Step 3:** Preparation of methyl 2-fluoro-4-(3-(6-fluoro-1H-indol-3-yl)pyrrolidin-1-yl)butanoate:

6-fluoro-3-(pyrrolidin-3-yl)-1H-indole (3.5g, 1eq) was dissolved in acetonitrile (40mL). Methyl 4-bromo-2-fluorobutanoate (1eq) and sodium carbonate (3eq) was added. The mixture was stirred at 70°C for 5 hours, dried by rotary-evaporation to remove acetonitrile, diluted with water (150mL), extracted with ethyl acetate (100mL*3). The organic

phases were dried by rotary-evaporation to give a crude product. The crude product was purified by flash chromatography (Silica gel, PE:EA=1:9) to give the target product (3.2g).

LCMS (ESI) $[M+H]^+$=323.0.

**Step 4:** Preparation of 2-fluoro-4-(3-(6-fluoro-1H-indol-3-yl)pyrrolidin-1-yl)butanehydrazide:

Methyl 2-fluoro-4-(3-(6-fluoro-1H-indol-3-yl)pyrrolidin-1-yl)butanoate (3.2g, 1eq) was dissolved in ethanol (30mL). Hydrazine hydrate (1.49g, 3eq) was added. The reaction mixture was reacted at 50°C for 1 hour, dried by rotary-evaporation to remove ethanol to produce the target compound (3.2g, crude), which was directly used in the next step.

LCMS (ESI) $[M+H]^+$=323.1.

**Step 5:** Preparation of (E)-N'-(2-fluoro-4-(3-(6-fluoro-1H-indol-3-yl)pyrrolidin-1-yl)butanoyl)-N,N-dimethylacetohydrazonamide:

2-fluoro-4-(3-(6-fluoro-1H-indol-3-yl)pyrrolidin-1-yl)butanehydrazide (3.16g, crude) was dissolved in methanol (30mL). (1,1-Dimethoxyethyl)dimethanamine (6.53g) was added. The reaction mixture was reacted at 70°C for 1 hour, and dried by rotary-evaporation to give a crude product. The crude product was purified to produce the target compound (2g).

LCMS (ESI) $[M+H]^+$=392.2.

**Step 6:** Preparation of 6-fluoro-3-(1-(3-fluoro-3-(5-methyl-4-(propan-2-yl)-4H-1,2,4-triazol-3-yl)propyl)pyrrolidin-3-yl)-1H-indole:

To a sealed tube were added (E)-N'-(2-fluoro-4-(3-(6-fluoro-1H-indol-3-yl)pyrrolidin-1-yl)butanoyl)-N,N-dimethylacetohydrazonamide(300mg, 1eq) and acetic acid (5mL). Nitrogen gas was introduced. Under an ice bath, 2-aminopropane (20eq) was added dropwise. The mixture was reacted at 110°C for 48 hours, adjusted with saturated potassium carbonate solution to pH=10, extracted with ethyl acetate (10mL×3). The organic phases were dried by rotary-evaporation to give a crude product. The crude product was purified by prep-HPLC (C18, 0.1% formic acid in water:acetonitrile) to produce the target compound (19.52mg).

LCMS (ESI) $[M+H]^+$=388.2; $^1$H NMR(400 MHz, MeOD-$d_4$)$\delta$ 8.45(s, 1H), 7.57(dd, J=8.7, 5.2 Hz, 1H), 7.23(s, 1H), 7.07(dd, J=9.9, 2.2 Hz, 1H), 6.84(td, J=9.5, 2.3 Hz, 1H), 5.99(ddd, J=48.9, 8.7, 3.4 Hz, 1H), 4.77-4.66(m, 1H), 3.92(dd, J=13.3, 7.0 Hz, 2H), 3.63(dd, J=16.7, 8.6 Hz, 3H), 3.50(ddd, J=22.4, 14.1, 9.7 Hz, 2H), 2.86-2.66(m, 2H), 2.64-2.54(m, 4H), 2.43-2.25(m, 1H), 1.56(d, J=6.9 Hz, 6H).

Examples 98-111 (Compounds 98-111)

With reference to the preparation of Example 18 or 74, compounds 98-111 were prepared:

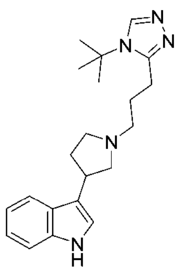
(98)
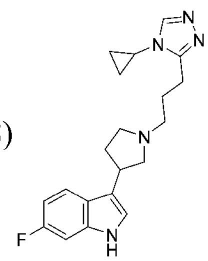
(99)
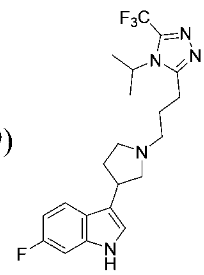
(100)
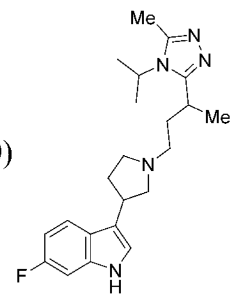
(101)

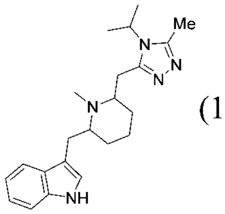 (102) 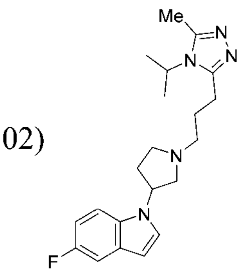 (103) 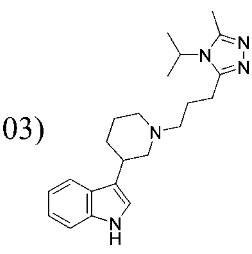 (104) 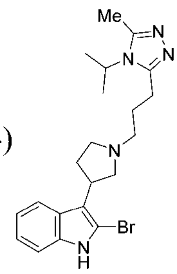 (105)

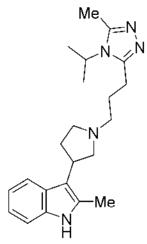 (106) 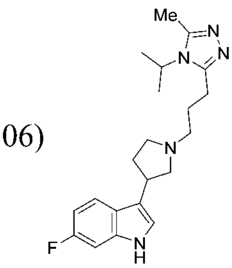 (107) 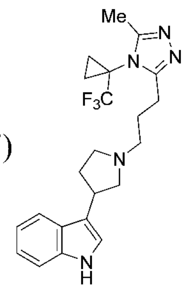 (108) 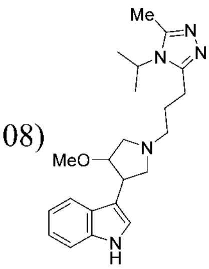 (109)

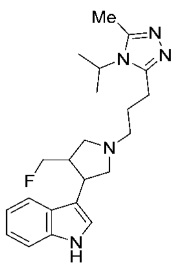 (110) 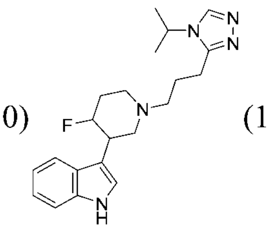 (111)

Compound 98: LCMS (ESI) $[M+H]^+$=352.1; $^1H$ NMR(400 MHz, DMSO-$d_6$)δ 10.74(s, 1H), 8.39(s, 1H), 7.61(d, J=7.9 Hz, 1H), 7.32(d, J=8.1 Hz, 1H), 7.13(d, J=1.9 Hz, 1H), 7.05(t, J=7.5 Hz, 1H), 6.94(t, J=7.4 Hz, 1H), 3.59-3.44(m, 1H), 2.95(dt, J=15.0, 8.1 Hz, 3H), 2.73(dd, J=15.2, 7.6 Hz, 1H), 2.63(dt, J=14.5, 7.5 Hz, 2H), 2.55(t, J=7.8 Hz, 2H), 2.29-2.16(m, 1H), 2.06-1.95(m, 2H), 1.93-1.83(m, 1H), 1.56(s, 9H). Compound 99: LCMS (ESI) $[M+H]^+$=353.9; $^1H$ NMR(400 MHz, MeOD-$d_4$)δ 8.38(s, 1H), 7.52(dd, J=8.7, 5.3 Hz, 1H), 7.06(s, 1H), 7.01(dd, J=10.0, 2.3 Hz, 1H), 6.81-6.74(m, 1H), 3.62(dd, J=16.8, 8.4 Hz, 1H), 3.34(dd, J=7.3,4.0 Hz, 1H), 3.24-3.18(m, 1H), 3.01-2.93(m, 3H), 2.70(ddt, J=17.7, 14.3, 7.5 Hz, 4H), 2.42-2.31(m, 1H), 2.12-1.99(m, 3H), 1.15-1.08(m, 2H), 1.07-1.00(m, 2H).
Compound 100: LCMS (ESI) $[M+H]^+$=424.2; $^1H$ NMR(400 MHz, Chloroform-d)δ 8.00(s, 1H), 7.58(dd, J=8.6, 5.4 Hz, 1H), 7.03(dd, J=13.4,3.8 Hz, 2H), 6.87(dd, J=12.8, 5.5 Hz, 1H), 4.64(dt, J=14.0, 6.9 Hz, 1H), 3.65(s, 1H), 3.17(s, 1H), 2.98(t, J=7.4 Hz, 3H), 2.77(d, J=32.3 Hz, 4H), 2.37(s, 1H), 2.22(s, 2H), 2.06(d, J=7.7 Hz, 1H), 1.57-1.52(m, 6H).
Compound 101: LCMS (ESI) $[M+H]^+$=384.3; $^1H$ NMR(400 MHz, DMSO-$d_6$)δ 10.83(s, 1H), 7.58(ddd, J=11.1, 8.8, 5.6 Hz, 1H), 7.14-7.06(m, 2H), 6.84-6.77(m, 1H), 4.53-4.35(m, 1H), 3.47(dt, J=15.9, 7.9 Hz, 1H), 3.15-3.07(m, 1H), 2.90(td, J=8.2, 4.6 Hz, 1H), 2.75-2.52(m, 2H), 2.47-2.30(m, 6H), 2.21(dt, J=21.9, 7.4 Hz, 1H), 2.02-1.73(m, 3H), 1.43(d, J=7.0 Hz, 3H), 1.31(dd, J=13.7, 7.0 Hz, 3H), 1.23(dd, J=6.8, 3.8 Hz, 3H).
Compound 102: LCMS (ESI) $[M+H]^+$=366.2;
Compound 103: LCMS (ESI) $[M+H]^+$=370.1; $^1H$ NMR(400 MHz, DMSO-$d_6$)δ 8.15(s, 2H), 7.65(dd, J=9.0, 4.5 Hz, 1H), 7.57(d, J=3.2 Hz, 1H), 7.29(dd, J=9.9, 2.5 Hz, 1H), 6.95(td, J=9.2, 2.5 Hz, 1H), 6.43(d, J=3.1 Hz, 1H), 5.12(dd, J=9.6, 6.2 Hz, 1H), 4.40(dt, J=13.9, 7.0 Hz, 1H), 3.06(t, J=6.8 Hz, 1H), 2.89(dd, J=8.7, 4.6 Hz, 1H), 2.79-2.73(m, 3H), 2.68-2.52(m, 2H), 2.48-2.40(m, 2H), 2.37(s, 3H), 2.02-1.81(m, 3H), 1.38(dd, J=7.0, 2.4 Hz, 6H).
Compound 104: LCMS (ESI) $[M+H]^+$=384.1;
Compound 105: LCMS (ESI) $[M+H]^+$=430.2;
Compound 106: LCMS (ESI) $[M+H]^+$=366.2;
Compound 107: LCMS (ESI) $[M+H]^+$=370.3; $^1H$ NMR(400 MHz, DMSO-$d_6$)δ 10.95(s, 1H), 8.27(s, 1H), 7.61(dd, J=8.7, 5.5 Hz, 1H), 7.20(d, J=1.9 Hz, 1H), 7.11(dd, J=10.2, 2.3 Hz, 1H), 6.83(ddd, J=9.8, 8.8, 2.4 Hz, 1H), 4.41(d, J=7.0 Hz, 2H), 3.66-3.54(m, 1H), 3.28(d, J=8.3 Hz, 1H), 2.96(d, J=7.0 Hz, 2H), 2.79(dd, J=16.4, 9.2 Hz, 5H), 2.38(s, 3H), 2.35-2.26(m, 1H), 1.97(dd, J=14.4, 7.0 Hz, 3H), 1.39(d, J=7.0 Hz, 6H).
Compound 108: LCMS (ESI) $[M+H]^+$=418.2;
Compound 109: LCMS (ESI) $[M+H]^+$=382.2;

Compound 110: LCMS (ESI) $[M+H]^+$=384.2;
Compound 111: LCMS (ESI) $[M+H]^+$=370.2.

Example 112 (Compound 112)

Preparation of 1-methyl-3-(1-(3-(5-methyl-4-(propan-2-yl)-4H-1,2,4-triazol-3-yl)propyl)pyrrolidin-3-yl)-1H-indole:

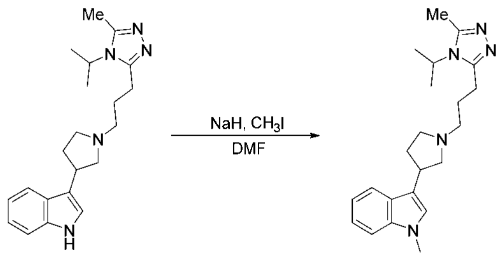


3-(1-(3-(5-methyl-4-(propan-2-yl)-4H-1,2,4-triazol-3-yl)propyl)pyrrolidin-3-yl)-1H-indole (50mg, 1eq) was dissolved in N,N-dimethylformamide (2mL). The reaction mixture was cooled down to 0°C. Sodium hydride (2eq) was added. The mixture was kept at this temperature and stirred and reacted for 5 minutes. Iodomethane (1.1eq) was added, and the mixture was warmed up to room temperature and stirred and reacted for 1 hour. The reaction mixture was diluted with water (3mL), extracted with ethyl acetate (5mL×3). The organic phases were combined, dried over anhydrous sodium sulfate, and filtered. The filtrate was concentrated, and purified by prep-HPLC (C18, 0.05% ammonia in water: acetonitrile) to produce the target compound (12.8mg).

LCMS (ESI) $[M+H]^+$=366.2; $^1$HNMR(400 MHz, MeOD-$d_4$)δ 7.57(d, J=7.9 Hz, 1H), 7.29(d, J=8.2 Hz, 1H), 7.14(t, J=7.5 Hz, 1H), 7.06-6.95(m, 2H), 4.53(m, 1H), 3.72(s, 3H), 3.68-3.57(m, 1H), 3.18(t, J=8.5 Hz, 1H), 2.94(dd, J=15.3, 8.0 Hz, 1H), 2.90-2.82(m, 2H), 2.74-2.58(m, 4H), 2.49(s, 3H), 2.40-2.29(m, 1H), 2.08-1.92(m, 3H), 1.50(d, J=7.0 Hz, 6H).

Examples 113-121 (Compounds 113-121)

With reference to the preparation of Example 74, compounds 113-121 were prepared:

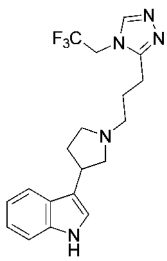 (113)

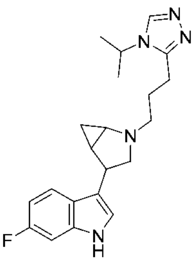 (114)

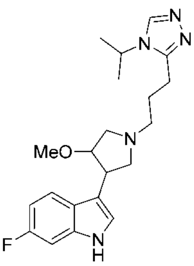 (115)

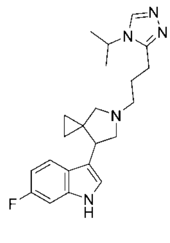 (116)

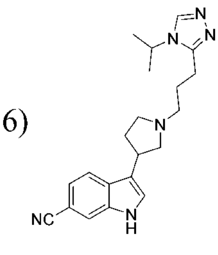 (117)

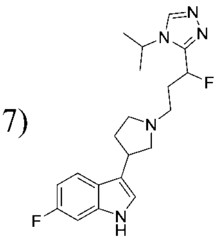 (118)

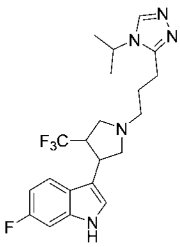 (119) 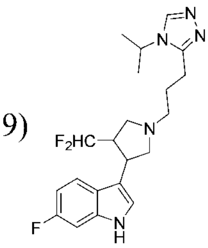 (120) 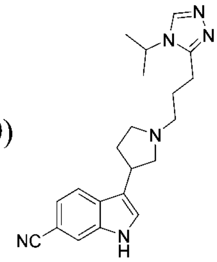 (121)

Compound 113: LCMS (ESI) [M+H]$^+$=378.1;
Compound 114: LCMS (ESI) [M+H]$^+$=368.2;
Compound 115: LCMS (ESI) [M+H]$^+$=386.2;
Compound 116: LCMS (ESI) [M+H]$^+$=382.2;
Compound 117: LCMS (ESI) [M+H]$^+$=363.2;
Compound 118: LCMS (ESI) [M+H]$^+$=374.2;
Compound 119: LCMS (ESI) [M+H]$^+$=424.2;
Compound 120: LCMS (ESI) [M+H]$^+$=406.2;
Compound 121: LCMS (ESI) [M+H]$^+$=363.2.

Example 122 (Compound 122)

Preparation of 3-(1-(3-(3,5-dimethyl-1H-1,2,4-triazol-1-yl)propyl)pyrrolidin-3-yl)-1H-indole:

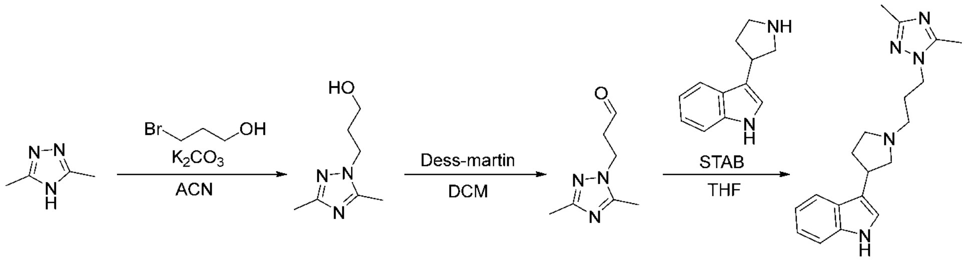


**Step 1:** Preparation of 3-(3,5-dimethyl-1H-1,2,4-triazol-1-yl)propan-1-ol:

Potassium carbonate (3.0eq) was added to a mixture of 3,5-dimethyl-4H-1,2,4-triazole (5g, 1.0eq) and 3-bromopropan-1-ol (1.2eq) in acetonitrile (200mL). The mixture was stirred at 50°C for 16 hours, and filtered. The filtrate was concentrated in vacuum to produce the target compound (6g).

LCMS (ESI) [M+H]$^+$=156.1.

**Step 2:** Preparation of 3-(3,5-dimethyl-1H-1,2,4-triazol-1-yl)propanal:

Dess-Martin periodinane (1.5eq) was added to a mixture of 3-(3,5-dimethyl-1H-1,2,4-triazol-1-yl)propan-1-ol (1.00g, 1.0eq) in dichloromethane (20mL). The reaction mixture was stirred at room temperature for 3 hours, quenched with saturated sodium bicarbonate, extracted with ethyl acetate (20mL×3). The aqueous phase was concentrated in vacuum. The residue was added to dichloromethane (20mL). The resulting mixture was stirred for 10 minutes, and filtered by suction. The filtrate was concentrated in vacuum to produce the target compound (200mg, crude).

LCMS (ESI) [M+H+18]$^+$=172.2;

**Step 3:** Preparation of 3-(1-(3-(3,5-dimethyl-1H-1,2,4-triazol-1-yl)propyl)pyrrolidin-3-yl)-1H-indole:

3-(3,5-dimethyl-1H-1,2,4-triazol-1-yl)propanal (150mg, crude) and 3-(pyrrolidin-3-yl)-1H-indole (182mg) was added to tetrahydrofuran (10mL). The mixture was stirred for 10 minutes, and sodium triacetoxyborohydride (415mg) and acetic acid (0.1mL) were added to the reaction system. The reaction mixture was stirred at room temperature for 2 hours, quenched with saturated sodium bicarbonate solution (10mL), extracted with dichloromethane (10mL×3). The organic

phases were combined, dried, concentrated. The resulting crude product was purified by prep-HPLC (C18, 0.1% ammonia in water: acetonitrile) to produce the target compound (20.9mg).

LCMS (ESI) $[M+H]^+$=324.2; $^1H$ NMR(400 MHz, DMSO-$d_6$)δ 10.75(s, 1H), 7.58(d, J=7.9 Hz, 1H), 7.32(d, J=8.1 Hz, 1H), 7.13(d, J=2.0 Hz, 1H), 7.05(t, J=7.5 Hz, 1H), 6.95(t, J=7.4 Hz, 1H), 4.04(t, J=6.8 Hz, 2H), 3.57-3.46(m, 1H), 2.96(t, J=8.3 Hz, 1H), 2.71(dd, J=14.9, 7.9 Hz, 1H), 2.57(dd, J=14.2, 8.6 Hz, 1H), 2.49-2.33(m, 3H), 2.32(s, 3H), 2.27-2.18(m, 1H), 2.15(s, 3H), 1.95-1.83(m, 3H).

Example 123

With reference to the preparation of Example 74, compound 123 was prepared:

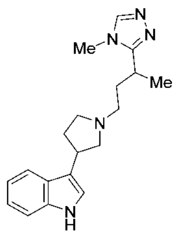

(123)

LCMS (ESI) $[M+H]^+$=324.2.

Example 125 (Compound 125)

Preparation of 3-(1-(3-(2H-1,2,3-triazol-2-yl)propyl)pyrrolidin-3-yl)-1H-indole:

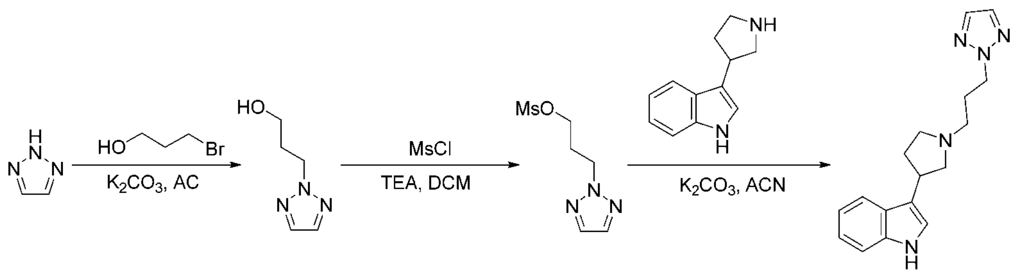


**Step 1:** Preparation of 3-(2H-1,2,3-triazol-2-yl)propan-1-ol:

2H-1,2,3-triazole (500mg, 1eq) and 3-bromopropan-1-ol (1eq) were dissolved in acetone (AC, 5mL). Potassium carbonate (1eq) was added. The reaction mixture was stirred at 50°C for 6 hours, filtered by suction. The mother liquor was concentrated to give a crude product. The crude product was purified by flash chromatography (Silica gel, DCM:MeOH=20:1) to give the target product (300mg).

LCMS (ESI) $[M+H]^+$=128.2.

**Step 2:** Preparation of 3-(2H-1,2,3-triazol-2-yl)propyl methanesulfonate:

3-(2H-1,2,3-triazol-2-yl)propan-1-ol (300mg, 1eq) was dissolved in dichloromethane (5mL), triethylamine (2.4eq) was added. At 0°C, methanesulfonyl chloride (1.2eq) was added dropwise. After the completion of the addition, the reaction mixture was stirred for 1 hour at 0°C, and concentrated to give a crude product. The crude product was purified by flash chromatography (Silica gel, DCM:MeOH=15:1) to give the target product (230mg).

LCMS (ESI) $[M+H]^+$=206.1.

**Step 3:** Preparation of 3-(1-(3-(2H-1,2,3-triazol-2-yl)propyl)pyrrolidin-3-yl)-1H-indole:

3-(2H-1,2,3-triazol-2-yl)propyl methanesulfonate (100mg, 1eq) and 3-(pyrrolidin-3-yl)-1H-indole (1eq) were dissolved in acetonitrile (5mL). Potassium carbonate (1.5eq) was added. The reaction mixture was stirred at 70°C for

16 hours, diluted with water (10mL), and extracted with ethyl acetate (10mL×2). The organic phases were combined, washed with saturated brine, dried over anhydrous sodium sulfate, and filtered by suction. The mother liquor was concentrated to give a crude product. The crude product was purified by prep-HPLC (C18, 0.1% formic acid in water:acetonitrile) to produce the target compound (40.36mg).

LCMS (ESI) $[M+H]^+$=296.2; $^1H$ NMR(400 MHz, MeOD-$d_4$)δ 8.50(s, 1H), 7.70(s, 2H), 7.57(d, J=7.9 Hz, 1H), 7.35(s, 1H), 7.19(s, 1H), 7.12(t, J=7.3 Hz, 1H), 7.04(t, J=7.4 Hz, 1H), 4.59(t, J=6.5 Hz, 2H), 3.92-3.79(m, 2H), 3.60-3.52(m, 2H), 3.36(t, J=9.6 Hz, 1H), 3.30(d, J=1.5 Hz, 1H), 3.27(d, J=8.2 Hz, 1H), 2.54(td, J=13.3, 6.6 Hz, 1H), 2.45-2.37(m, 2H), 2.30(ddd, J=17.4, 10.9, 6.5 Hz, 1H).

Examples 124 and 126-129 (Compounds 124 and 126-129)

With reference to the preparation of Example 122, or 125, compounds 124 and 126-129 were prepared:

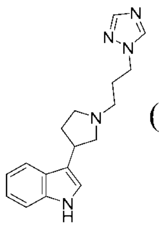
(124)
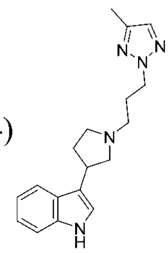
(126)
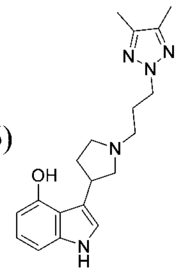
(127)

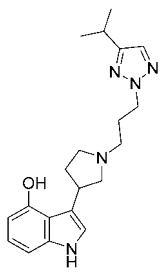
(128)
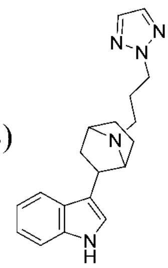
(129)

Compound 124: LCMS (ESI) $[M+H]^+$=296.1;
Compound 126: LCMS (ESI) $[M+H]^+$=310.2;
Compound 127: LCMS (ESI) $[M+H]^+$=340.2;
Compound 128: LCMS (ESI) $[M+H]^+$=354.2;
Compound 129: LCMS (ESI) $[M+H]^+$=322.2.

Examples 130-131 (Compounds 130-131)

With reference to the preparation of Example 1, or 5, compounds 130-131 were prepared:

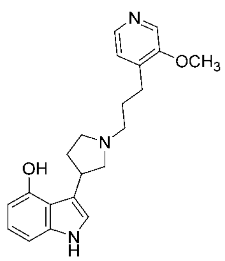
(130)
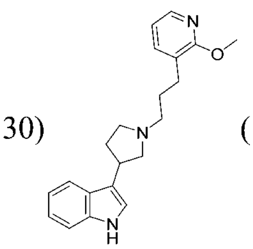
(131)

Compound 130: LCMS (ESI) $[M+H]^+$=352.1;
Compound 131: LCMS (ESI) $[M+H]^+$=336.2; $^1H$ NMR(400 MHz, DMSO-$d_6$)δ 10.89(s, 1H), 8.02(dd, J=4.9, 1.6 Hz, 1H), 7.61(d, J=7.9 Hz, 1H), 7.56(dd, J=7.1, 1.4 Hz, 1H), 7.35(d, J=8.1 Hz, 1H), 7.22(d, J=2.1 Hz, 1H), 7.08(s, 1H), 6.98(s, 1H), 6.93(dd, J=7.1,5.0 Hz, 1H), 3.87(s, 3H), 3.67-3.63(m, 1H), 3.41(d, J=8.4 Hz, 1H), 3.10(dd, J=18.7, 10.9 Hz, 2H), 2.94-2.82(m, 3H), 2.63-2.58(m, 2H), 2.39-2.29(m, 1H), 2.02(dd, J=12.4, 8.2 Hz, 1H), 1.92-1.82(m, 2H).

Example 132 (Compound 132)

Preparation of 3-(1-(3-(4-methoxypyrimidin-5-yl)propyl)pyrrolidin-3-yl)-1H-indole:

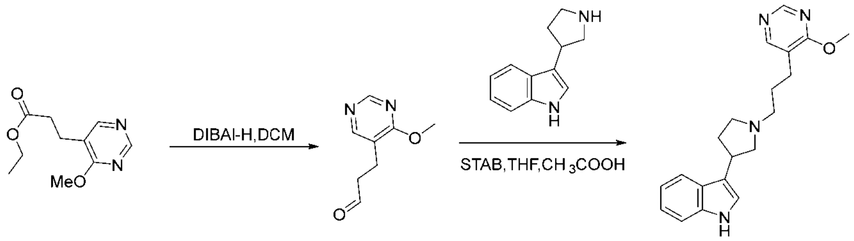


**Step 1:** Preparation of 3-(4-methoxypyrimidin-5-yl)propanal:

Ethyl 3-(4-methoxypyrimidin-5-yl)propanoate (700mg, 1.0eq) was dissolved in dichloromethane (10mL). The mixture was cooled down to -78°C, a solution of diisobutylaluminium hydride in toluene (2.0eq) was slowly added dropwise. The mixture was reacted at -78°C for another 1 hour. TLC detection showed the completion of the reaction. The reaction was quenched with water (2mL). Dichloromethane was added and the mixture was stirred for 5 minutes. Anhydrous sodium sulfate was added, and the mixture was filtered. The filtrate was concentrated, and purified to produce the target compound (250mg).
LCMS (ESI) $[M+H]^+$=167.1.

**Step 2:** Preparation of 3-(1-(3-(4-methoxypyrimidin-5-yl)propyl)pyrrolidin-3-yl)-1H-indole:

3-(4-methoxypyrimidin-5-yl)propanal (1.1eq) and 3-(pyrrolidin-3-yl)-1H-indole (150mg, 1.0eq) were dissolved in tetrahydrofuran (10mL). Acetic acid (0.5mL) was added. Sodium triacetoxyborohydride (3.0eq) was added. At room temperature, the mixture was reacted for 1 hour, quenched with water, extracted with ethyl acetate (10mL×3). The organic phases were combined, dried over anhydrous sodium sulfate, concentrated followed by adding aqueous hydrochloric acid solution (4mL, 1%), and purified by prep-HPLC (C18, 0.05%TFA/water, MeCN), to give the target product (36.02mg). LCMS (ESI) $[M+H]^+$=337.3; $^1$H NMR(400 MHz, MeOD-$d_4$)δ 9.16(s, 1H), 8.73(s, 1H), 7.60(d, J=8.0 Hz, 1H), 7.37(d, J=8.1 Hz, 1H), 7.23(s, 1H), 7.12(d, J=7.5 Hz, 1H), 7.05(d, J=7.7 Hz, 1H), 4.29(d, J=3.3 Hz, 3H), 4.15-4.00(m, 1H), 3.87(dd, J=18.9, 7.9 Hz, 2H), 3.72(t, J=8.0 Hz, 1H), 3.52-3.33(m, 3H), 3.22(t, J=11.1 Hz, 1H), 2.84(t, J=7.2 Hz, 2H), 2.66-2.53(m, 1H), 2.47-2.28(m, 1H), 2.16(d, J=3.8 Hz, 2H).

Example 133 (Compound 133)

With reference to the preparation of Example 20, or 132, compound 133 was prepared:

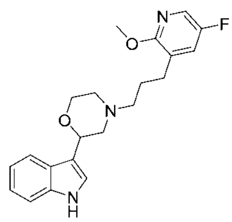

(133)

LCMS (ESI) $[M+H]^+$=370.3; $^1$H NMR(400 MHz, DMSO-$d_6$)δ 10.99(s, 1H), 8.14(s, 1H), 7.97(d, J=2.9 Hz, 1H), 7.63-7.54(m, 2H), 7.35(d, J=8.1 Hz, 1H), 7.29(d, J=2.3 Hz, 1H), 7.07(t, J=7.5 Hz, 1H), 6.98(t, J=7.4 Hz, 1H), 4.75(d, J=8.5 Hz, 1H), 3.93-3.83(m, 4H), 3.70(t, J=10.3 Hz, 1H), 2.98(d, J=10.6 Hz, 1H), 2.82(d, J=10.7 Hz, 1H), 2.61-2.55(m, 2H), 2.40(d, J=23.6 Hz, 3H), 2.23(s, 1H), 1.82-1.70(m, 2H).

Examples 134-138 (Compounds 134-138)

With reference to the preparation of Example 1, 5, 140, 146, or 147, compounds 134-138 were prepared:

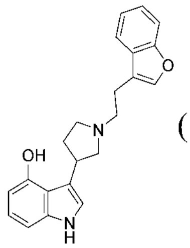 (134) 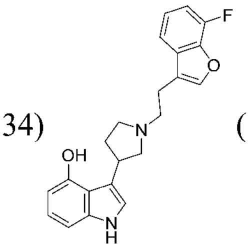 (135) 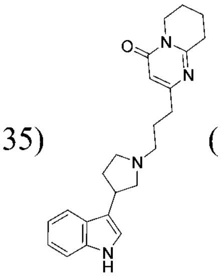 (136)

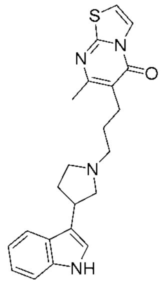 (137) 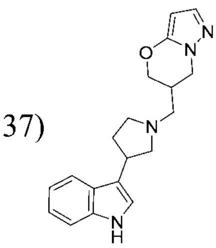 (138)

Compound 134: LCMS (ESI) $[M+H]^+$=347.1;
Compound 135: LCMS (ESI) $[M+H]^+$=365.1;
Compound 136: LCMS (ESI) $[M+H]^+$=377.2;
Compound 137: LCMS (ESI) $[M+H]^+$=393.2;
Compound 138: LCMS (ESI) $[M+H]^+$=323.2.

Example 139 (Compound 139)

Preparation of 7-((3-(1H-indol-3-yl)pyrrolidin-1-yl)methyl)-3-isopropyl-5,6,7,8-tetrahydro-[1,2,4]triazolo[4,3-a]pyridine:

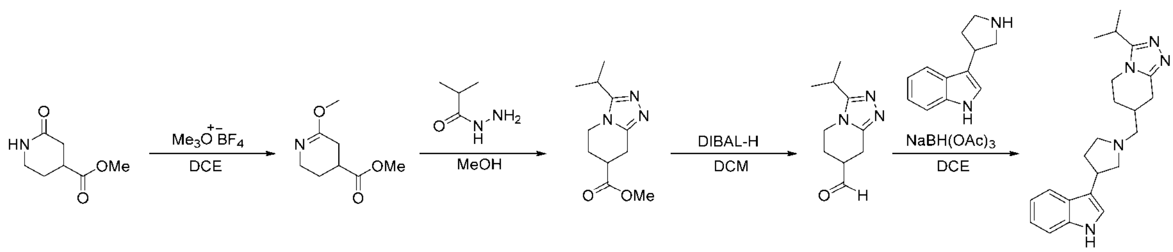


**Step 1:** Preparation of methyl 6-methoxy-2,3,4,5-tetrahydropyridine-4-carboxylate:

At room temperature, to a solution of methyl 2-oxopiperidine-4-carboxylate (2g, 1eq) in DCE(90mL) was added trimethyloxonium tetrafluoroborate (1.2eq). Then the mixture was warmed up to 60°C and reacted for 24 hours. Under an ice bath, to the above reaction mixture was slowly added saturated potassium carbonate solution (40mL). The mixture was stirred for 10 minutes and then separated into phases. The organic phase was washed with water (10mL×2), dried over anhydrous sodium sulfate, filtered, dried by rotary-evaporation to produce the target compound (2g, crude).
LCMS (ESI) $[M+H]^+$=172.0.

**Step 2:** Preparation of methyl 3-isopropyl-5,6,7,8-tetrahydro-[1,2,4]triazolo[4,3-a]pyridine-7-carboxylate:

At room temperature, to a solution of methyl 6-methoxy-2,3,4,5-tetrahydropyridine-4-carboxylate (2g, crude) in methanol (60mL) was added to isobutyrohydrazide (1.1g). The mixture was warmed up to 80°C and reacted for 24 hours. Then, the reaction mixture was dried by rotary-evaporation to produce the target compound (2.6g, crude).
LCMS (ESI) $[M+H]^+$=224.2.

**Step 3:** Preparation of 3-isopropyl-5,6,7,8-tetrahydro-[1,2,4]triazolo[4,3-a]pyridine-7-carbaldehyde:

At -78°C, in a nitrogen atmosphere, to a solution of methyl 3-isopropyl-5,6,7,8-tetrahydro-[1,2,4]triazolo[4,3-a]pyridine-7-carboxylate (1.4g, crude) in dichloromethane (10mL) was slowly added dropwise diisobutylaluminium hydride (1M, 6.3mL). The mixture was naturally warmed up to room temperature and reacted overnight. Then, under an ice bath, the reaction mixture was quenched with water (5mL), diluted with ethyl acetate (20mL), then dried over anhydrous magnesium sulfate, filtered. The filter cake was washed with ethyl acetate (30mL). The filtrate was dried by rotary-evaporation to produce the target compound (1.1g, crude).

**Step 4:** Preparation of 7-((3-(1H-indol-3-yl)pyrrolidin-1-yl)methyl)-3-isopropyl-5,6,7,8-tetrahydro-[1,2,4]triazolo[4,3-a]pyridine:

At room temperature, to a solution of 3-(pyrrolidin-3-yl)-1H-indole (198mg, 1.0eq) in 1,2-dichloroethane (5mL) were added sequentially 3-isopropyl-5,6,7,8-tetrahydro-[1,2,4]triazolo[4,3-a]pyridine-7-carbaldehyde (410mg, crude) and sodium triacetoxyborohydride (1.4eq). The reaction mixture was reacted at room temperature for 24 hours, diluted with acetonitrile (10mL), and then filtered. The filter cake was washed with acetonitrile (15mL). The filtrate was dried by rotatary evaporation, and purified by prep-HPLC (C18, 0.05% formic acid in water:acetonitrile) to produce the target compound (32.49mg).

LCMS (ESI) $[M+H]^+$=364.2; $^1$H NMR(400 MHz, $CDCl_3$)δ 8.08(s, 1H), 7.70(t, J=8.2 Hz, 1H), 7.36(d, J=8.1 Hz, 1H), 7.19(t, J=7.6 Hz, 1H), 7.11(t, J=7.5 Hz, 1H), 7.03(s, 1H), 4.06-3.97(m, 1H), 3.81-3.70(m, 1H), 3.70-3.60(m, 1H), 3.29-3.21(m, 1H), 3.11-2.91(m, 2H), 2.83-2.62(m, 3H), 2.58-2.49(m, 2H), 2.41-2.26(m, 2H), 2.18-2.00(m, 2H), 1.78-1.65(m, 2H), 1.41(d, J=6.9 Hz, 3H), 1.36(dd, J=6.9, 2.1 Hz, 3H).

Example 140 (Compound 140)

Preparation of 3-(1-(2-(3-(propan-2-yl)-5H,6H,7H-pyrrolo[2,1-c][1,2,4]triazol-7-yl)ethyl)pyrrolidin-3-yl)-1H-indole:

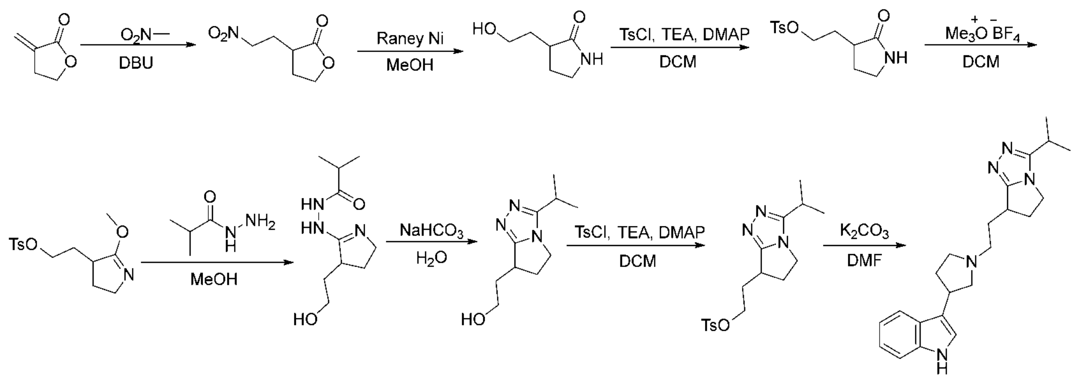


**Step** 1: Preparation of 3-(2-nitroethyl)dihydrofuran-2(3H)-one:

3-methylenedihydrofuran-2(3H)-one (25g, 1eq) was dissolved in nitromethane (240mL), and DBU (1,8-diazabicyclo[5.4.0]undec-7-ene) (0.13eq) was added. The reaction system was stirred at room temperature for 12 hours. TLC detection showed the completion of the reaction. The reaction mixture was concentrated to give a crude product. The crude product was diluted with dichloromethane (240mL), sequentially washed with 3M hydrochloric acid (240mL×3), water (240mL×3), saturated aqueous sodium bicarbonate solution (240mL×3) and brine (240mL×3). The organic phases were dried over anhydrous sodium sulfate, and filtered. The filtrate was concentrated to give a crude product. The crude product was purified by flash chromatography to produce the target compound (18.5g).

**Step 2:** Preparation of 3-(2-hydroxyethyl)pyrrolidin-2-one:

3-(2-nitroethyl)dihydrofuran-2(3H)-one (17.5g, 1eq) was dissolved in methanol (350mL), and Raney Ni (2eq), and magnesium sulfate (1eq) were added sequentially. In a hydrogen atmosphere, the reaction mixture was stirred at 30°C for 12 hours. TLC detection showed the completion of the reaction. The reaction mixture was filtered, and concentrated to give a crude product. The crude product was slurrized with dichloromethane, and filtered. The filtrate was concentrated to

produce the target compound (14g).

**Step 3:** Preparation of 2-(2-oxopyrrolidin-3-yl)ethyl 4-methylbenzenesulfonate:

3-(2-hydroxyethyl)pyrrolidin-2-one (5g, 1eq) was dissolved in dichloromethane (5mL). 4-methylbenzenesulfonyl chloride (1.1eq), N,N-dimethylpyridine-4-amine (0.2eq), and triethylamine (1.2eq) were added sequentially. The reaction system was stirred at room temperature for 16 hours, and concentrated to give a crude product. The crude product was purified by flash chromatography to produce the target compound (7.5g, yield: 68.4%).

LCMS (ESI) $[M+H]^+$=284.0.

**Step 4:** Preparation of 2-(5-methoxy-3,4-dihydro-2H-pyrrol-4-yl)ethyl 4-methylbenzenesulfonate:

2-(2-oxopyrrolidin-3-yl)ethyl 4-methylbenzenesulfonate (1g, 1eq) was dissolved in dichloromethane (10mL), and trimethyloxonium tetrafluoroborate (1.5eq) was added. The reaction system was stirred at room temperature for 3 hours, and concentrated to give a crude product. The crude product was purified to produce the target compound (800mg).

LCMS (ESI) $[M+H]^+$=298.1.

**Step 5:** Preparation of N'-(4-(2-hydroxyethyl)-3,4-dihydro-2H-pyrrol-5-yl)-2-methylpropanehydrazide:

2-(5-methoxy-3,4-dihydro-2H-pyrrol-4-yl)ethyl 4-methylbenzenesulfonate (300mg, 1eq) was dissolved in methanol (3mL), and isobutyrohydrazide (1.1eq) was added. The reaction system was stirred at 85°C for 12 hours, and concentrated to give a crude product. The crude product was purified by reverse-phase chromatography (C18, 0.1% ammonia in water:acetonitrile) to produce the target compound (150mg).

LCMS (ESI) $[M+H]^+$=214.0.

**Step 6:** Preparation of 2-(3-(propan-2-yl)-5H,6H,7H-pyrrolo[2,1-c][1,2,4]triazol-7-yl)ethan-1-ol:

N'-(4-(2-hydroxyethyl)-3,4-dihydro-2H-pyrrol-5-yl)-2-methylpropanehydrazide (150mg, 1eq) was dissolved in water (2mL), and sodium bicarbonate (10eq) was added. The reaction system was stirred at 100°C for 16 hours. The reaction mixture was purified to produce the target compound (30mg). LCMS (ESI) $[M+H]^+$=196.3.

**Step 7:** Preparation of 2-(3-(propan-2-yl)-5H,6H,7H-pyrrolo[2,1-c][1,2,4]triazol-7-yl)ethyl 4-methylbenzenesulfonate:

2-(3-(propan-2-yl)-5H,6H,7H-pyrrolo[2,1-c][1,2,4]triazol-7-yl)ethan-1-ol (30mg, 1eq) was dissolved in dichloromethane (1mL). 4-methylbenzenesulfonyl chloride (1.2eq), triethylamine (1.5eq), and N,N-dimethylpyridine-4-amine (0.1eq) were added sequentially. The reaction mixture was stirred at room temperature for 1 hour, diluted with water (5mL), extracted with dichloromethane (10mL×3). The organic phases were combined, dried over anhydrous sodium sulfate, filtered, and concentrated to give a crude product. The crude product was purified by reverse-phase chromatography (C18, 0.1 %trifluoroacetic acid in water:acetonitrile) to produce the target compound (30mg).

LCMS (ESI) $[M+H]^+$=350.0

**Step 8:** Preparation of 3-(1-(2-(3-(propan-2-yl)-5H,6H,7H-pyrrolo[2,1-c][1,2,4]triazol-7-yl)ethyl)pyrrolidin-3-yl)-1H-indole:

3-(pyrrolidin-3-yl)-1H-indole (13.32mg, 1eq) and 2-(3-(propan-2-yl)-5H,6H,7H-pyrrolo[2,1-c][1,2,4]triazol-7-yl) ethyl 4-methylbenzenesulfonate (1eq) was dissolved in N,N-dimethylformamide (1mL). Potassium carbonate (2eq) was added. The reaction system was stirred at 70°C for 12 hours, diluted with water (10mL), extracted with ethyl acetate (10mL×3). The organic phases were combined, dried over anhydrous sodium sulfate, filtered, and concentrated to give a crude product. The crude product was purified by prep-HPLC (C18, 0.05%ammonia in water:acetonitrile) to produce the target compound (4.8mg).

LCMS (ESI) $[M+H]^+$=364.3; $^1$H NMR(400 MHz, MeOD-$d_4$)δ 7.57(d, J=7.9 Hz, 1H), 7.32(d, J=8.1 Hz, 1H), 7.09-7.05(m, 2H), 6.98(t, J=7.5 Hz, 1H), 4.11(tdd, J=8.6, 4.3, 2.1 Hz, 1H), 4.00(ddd, J=7.3, 6.5, 2.4 Hz, 1H), 3.70-3.63(m, 1H), 3.29-3.23(m, 2H), 3.15-3.08(m, 1H), 3.03-2.84(m, 3H), 2.77-2.63(m, 3H), 2.51-2.34(m, 2H), 2.14-2.04(m, 2H), 1.93-1.84(m, 1H), 1.33(dd, J=7.0, 3.4 Hz, 6H).

Example 141 (Compound 141)

With reference to the preparation of Example 5, compound 141 was prepared:

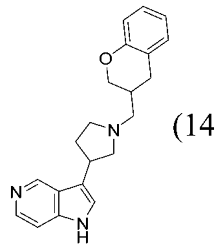

(141)

LCMS (ESI) $[M+H]^+$=334.1.

Example 142 (Compound 142)

Preparation of 3-(1-(2-((1,2,4)triazolo(4,3-a)pyridin-3-yl)ethyl)pyrrolidin-3-yl)-1H-indole:

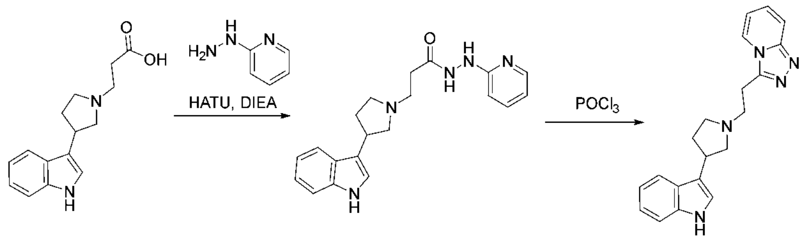


**Step 1:** Preparation of 3-(3-(1H-indol-3-yl)pyrrolidin-1-yl)-N'-(pyridin-2-yl)propanehydrazide:

3-(3-(1H-indol-3-yl)pyrrolidin-1-yl)propanoic acid (500mg, 1.0eq) and 2-hydrazineylpyridine (1.2eq) were dissolved in N,N-dimethylformamide (10mL). HATU (1.2eq) was added. At room temperature, diisopropylethylamine (2.0eq) was added dropwise. After the completion of the addition, the reaction mixture was stirred at room temperature for 4 hours. LCMS detection showed the completion of the reaction. Purification by flash chromatography (C18, 0.05% formic acid in water: acetonitrile) produced the target compound (350mg).

LCMS (ESI) $[M+H]^+$=350.2.

**Step 2:** Preparation of 3-(1-(2-((1,2,4)triazolo(4,3-a)pyridin-3-yl)ethyl)pyrrolidin-3-yl)-1H-indole:

3-(3-(1H-indol-3-yl)pyrrolidin-1-yl)-N'-(pyridin-2-yl)propanehydrazide (200mg, 1.0eq) was dissolved in phosphoryl trichloride (10mL). The mixture was reacted at 100°C for 16 hours, and dried by rotary-evaporation to remove phosphoryl trichloride. The residue was dissolved by adding dichloromethane and methanol, and the resulting mixture became clear. The reaction mixture was adjusted with saturated sodium carbonate to neutral pH, and filtered. The filtrate was dried by rotary-evaporation, and purified by prep-HPLC (C18, 0.1% formic acid in water:acetonitrile) to produce the target compound (4.46mg). LCMS (ESI) $[M+H]^+$=332.1; $^1H$ NMR(400 MHz, MeOD-$d_4$)$\delta$ 8.48(s, 1H), 8.39(d, J=7.0 Hz, 1H), 7.75(d, J=9.4 Hz, 1H), 7.58(d, J=7.9 Hz, 1H), 7.49(dd, J=9.0, 6.8 Hz, 1H), 7.35(d, J=8.1 Hz, 1H), 7.19(s, 1H), 7.07(ddd, J=21.6, 15.5, 7.6 Hz, 3H), 3.86(dt, J=18.0, 8.0 Hz, 2H), 3.72(d, J=7.1 Hz, 2H), 3.57(dd, J=15.7, 8.3 Hz, 4H), 3.35(s, 1H), 2.57(dd, J=12.8, 5.5 Hz, 1H), 2.39-2.24(m, 1H).

Example 143 (Compound 143)

Preparation of 3-(2-(3-(1H-indol-3-yl)pyrrolidin-1-yl)ethyl)thiazolo[2,3-c][1,2,4]triazole:

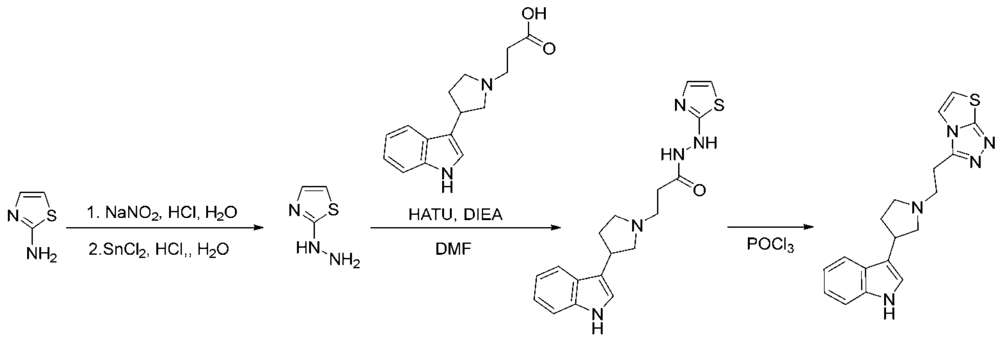


**Step** 1: Preparation of 2-hydrazineyl-1,3-thiazole:

1,3-Thiazole-2-amine (1g, 1.0eq) was dissolved in concentrated hydrochloric acid (36%, 10mL), and water (10mL) was added. At -10°C, sodium nitrite (1.0eq) was added. The mixture was stirred at -10°C for 10 minutes. A solution of tin(II) chloride (2.0eq) in hydrochloric acid (10mL) was added. The reaction mixture was stirred at -10°C for 0.5 hours, and filtered by suction. The residue was dried to the target compound (1.1g).

LCMS (ESI) $[M+H]^+$=116.1.

**Step 2:** Preparation of 3-(3-(1H-indol-3-yl)pyrrolidin-1-yl)-N'-(1,3-thiazol-2-yl)propanehydrazide:

2-hydrazineyl-1,3-thiazole (500mg, 1.0eq) was dissolved in N,N-dimethylformamide (10mL). 3-(3-(1H-indol-3-yl)pyrrolidin-1-yl)propanoic acid (1.0eq) and HATU (1.5eq) were added. At room temperature, diisopropylethylamine (3.0eq) was added dropwise. After the completion of the addition, the reaction mixture was stirred at room temperature for 4 hours, diluted with water (50mL), and extracted with ethyl acetate (50mL×3). The organic phases were combined, washed with saturated brine, dried over anhydrous sodium sulfate, and filtered by suction. The mother liquor was concentrated to give a crude product. The crude product was purified by flash chromatography to produce the target compound (344mg).

LCMS (ESI) $[M+H]^+$=356.2.

**Step 3:** Preparation of 3-(2-(3-(1H-indol-3-yl)pyrrolidin-1-yl)ethyl)thiazolo[2,3-c][1,2,4]triazole:

3-(3-(1H-indol-3-yl)pyrrolidin-1-yl)-N'-(1,3-thiazol-2-yl)propanehydrazide (320mg, 1.0eq) was dissolved in phosphoryl trichloride (13mL). The mixture was reacted at 100°C for 16 hours, dried by rotary-evaporation to remove phosphoryl trichloride. The residue was dissolved by adding dichloromethane and methanol, and the resulting mixture became clear. The reaction mixture was adjusted with saturated sodium carbonate to neutral pH, and filtered. The filtrate was dried by rotary-evaporation, and purified by prep-HPLC (C18, 0.1% formic acid in water:acetonitrile) to produce the target compound (27.32mg).

LCMS (ESI) $[M+H]^+$=338.1; $^1H$ NMR(400 MHz, DMSO-$d_6$)δ 10.75(s, 1H), 8.17(s, 1H), 7.98(d, J=4.4 Hz, 1H), 7.51(d, J=8.2 Hz, 1H), 7.38(d, J=4.3 Hz, 1H), 7.31(d, J=8.1 Hz, 1H), 7.11(s, 1H), 7.04(t, J=7.4 Hz, 1H), 6.93(d, J=7.8 Hz, 1H), 3.53-3.48(m, 1H), 3.19(d, J=7.3 Hz, 2H), 3.10(d, J=8.2 Hz, 1H), 2.93(dd, J=13.2, 7.0 Hz, 2H), 2.81(d, J=8.7 Hz, 1H), 2.72(d, J=5.6 Hz, 1H), 2.63-2.58(m, 1H), 2.23(s, 1H), 1.89(d, J=7.7 Hz, 1H).

Example 144 (Compound 144)

With reference to the preparation of Example 5, compound 144 was prepared:

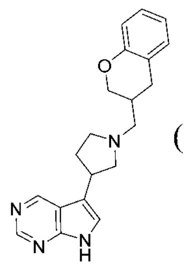
(144)

LCMS (ESI) $[M+H]^+$=335.1.

Example 145 (Compound 145)

Preparation of 3-(1-((3-cyclopropyl-SH,6H,7H,8H-[1,2,4]triazolo[4,3-a]pyridine-7-yl)methyl)pyrrolidin-3-yl)-1H-indole:

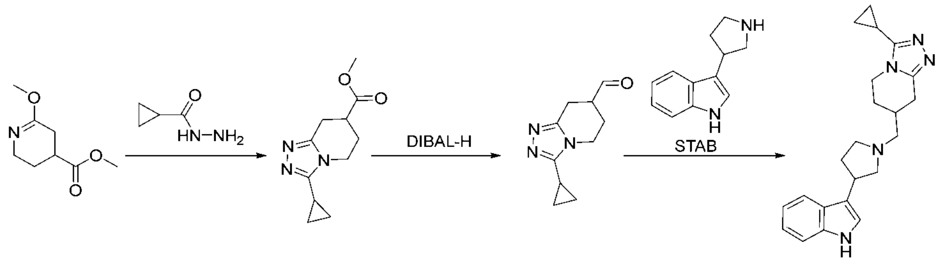


Step 1: Preparation of methyl 3-cyclopropyl-5,6,7,8-tetrahydro-[1,2,4]triazolo[4,3-a]pyridine-7-carboxylate:

Methyl 6-methoxy-2,3,4,5-tetrahydropyridine-4-carboxylate (2.8g, 1eq) was dissolved in methanol (30mL). Cyclopropanecarbohydrazide (1.2eq) was added. The mixture was stirred and reacted at 85°C for 16 hours, dried by rotary-evaporation to remove the solvent, purified by flash chromatography to produce the target compound (2.5g).
LCMS (ESI) $[M+H]^+$=222.0.

**Step 2:** Preparation of 3-cyclopropyl-5,6,7,8-tetrahydro-[1,2,4]triazolo[4,3-a]pyridine-7-carbaldehyde:

Methyl 3-cyclopropyl-5,6,7,8-tetrahydro-[1,2,4]triazolo[4,3-a]pyridine-7-carboxylate (200mg, 1.0eq) was dissolved in dichloromethane (8mL). At -70°C, diisobutylaluminium hydride (1M/Hexanes, 1.8mL, 2eq) was slowly added dropwise. While kept at this temperature, the mixture was reacted for 1 hour. The reaction mixture was diluted with an appropriate amount of dichloromethane, quenched by slowly adding dropwise water (0.4mL), stirred at room temperature for 15 minutes. An appropriate amount of anhydrous magnesium sulfate was added, and the mixture was stirred for another 15 minutes, and filtered. The filtrate was concentrated to produce the target compound (100mg).
LCMS (ESI) $[M+H]^+$=192.0.

**Step 3:** Preparation of 3-(1-((3-cyclopropyl-SH,6H,7H,8H-[1,2,4]triazolo[4,3-a]pyridine-7-yl)methyl)pyrrolidin-3-yl)-1H-indole:

3-cyclopropyl-5,6,7,8-tetrahydro-[1,2,4]triazolo[4,3-a]pyridine-7-carbaldehyde (100mg, 1.0eq) was dissolved in tetrahydrofuran (8mL). 3-(pyrrolidin-3-yl)-1H-indole (1.0eq) was added, then 2 drops of acetic acid was added dropwise. The mixture was stirred and reacted at room temperature for 20 minutes. Sodium triacetoxyborohydride (2eq) was added. The resulting mixture was stirred and reacted for another 1 hour, diluted with water (20mL), and extracted with ethyl acetate (10mL×3). The organic phases were combined, washed with saturated brine, dried over anhydrous sodium sulfate, filtered, and concentrated. Purification by prep-HPLC (C18, 0.05% formic acid in water:acetonitrile) produced the target compound (21.07mg).
LCMS (ESI) $[M+H]^+$=362.2; $^1$HNMR(400 MHz, DMSO-$d_6$)δ 10.77(s, 1H), 7.62(d, J=7.9 Hz, 1H), 7.33(d, J=8.1 Hz, 1H), 7.16(s, 1H), 7.06(t, J=7.5 Hz, 1H), 6.96(t, J=7.4 Hz, 1H), 4.16-4.06(m, 1H), 3.83(dt, J=11.7, 5.5 Hz, 1H), 3.55(dd, J=16.3, 8.2 Hz, 1H), 3.09(s, 1H), 3.04-2.94(m, 1H), 2.83(s, 1H), 2.73(s, 1H), 2.58(dd, J=16.9, 8.5 Hz, 3H), 2.40(dd, J=16.4, 10.3 Hz, 1H), 2.34-2.22(m, 1H), 2.16(d, J=12.5 Hz, 2H), 1.98-1.84(m, 2H), 1.70-1.58(m, 1H), 0.98-0.78(m, 4H).

Example 146 (Compound 146)

Preparation of 7-((3-(1H-indol-3-yl)pyrrolidin-1-yl)methyl)-3-(difluoromethyl)-5,6,7,8-tetrahydro-[1,2,4]triazolo[4,3-a] pyridine:

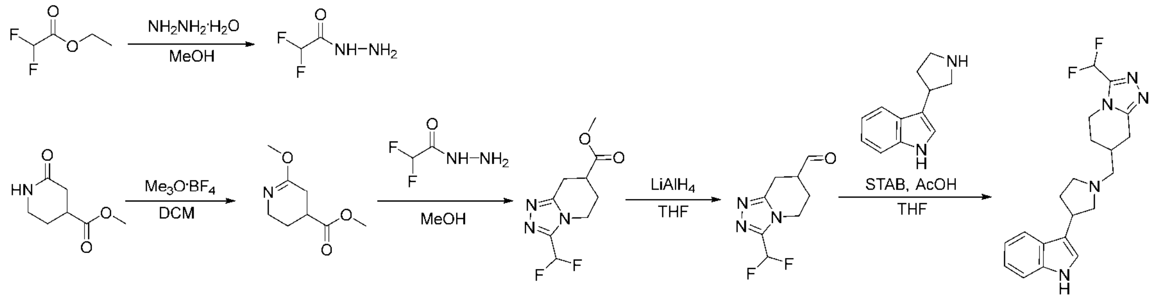


**Step** 1: Preparation of 2,2-difluoroacetohydrazide:

Ethyl 2,2-difluoroacetate (5g, 1.0eq) was dissolved in methanol (25mL). Hydrazine hydrate (10.0eq) was added. The reaction mixture was stirred at 80°C for 16 hours, and then concentrated to produce the target compound (4.2g).

$^1H$ NMR(400 MHz, DMSO-$d_6$)δ 6.19(t, J=53.5 Hz, 1H).

**Step 2:** Preparation of methyl 6-methoxy-2,3,4,5-tetrahydropyridine-4-carboxylate:

Methyl 2-oxopiperidine-4-carboxylate (500mg, 1.0eq) was dissolved in dichloromethane (5mL). Trimethyloxonium tetrafluoroborate (1.5eq) was added. The reaction mixture was stirred at room temperature for 12 hours, diluted with saturated sodium bicarbonate solution (5mL), and extracted with dichloromethane (10mL×3). The organic phases were combined, washed with saturated brine, dried over anhydrous sodium sulfate, and filtered by suction. The filtrate was concentrated to produce the target compound (370g).

LCMS (ESI) $[M+H]^+$=172.1.

**Step 3:** Preparation of methyl 3-(difluoromethyl)-5,6,7,8-tetrahydro-[1,2,4]triazolo[4,3-a]pyridine-7-carboxylate:

Methyl 6-methoxy-2,3,4,5-tetrahydropyridine-4-carboxylate (2g, 1.0eq) and 2,2-difluoroacetohydrazide (1.1eq) were dissolved in methanol (20mL). The mixture was stirred at 85°C for 24 hours. The reaction mixture was concentrated to give a crude product. The crude product was purified by flash chromatography to produce the target compound (700mg).

LCMS (ESI) $[M+H]^+$=232.0.

**Step 4:** Preparation of 3-(difluoromethyl)-5,6,7,8-tetrahydro-[1,2,4]triazolo[4,3-a]pyridine-7-carbaldehyde:

Methyl 3-(difluoromethyl)-5,6,7,8-tetrahydro-[1,2,4]triazolo[4,3-a]pyridine-7-carboxylate (700mg, 1.0eq) was dissolved in tetrahydrofuran (10mL). At -40°C, lithium aluminium hydride (1.5eq) as solid was added. Under the protection of nitrogen gas, the mixture was stirred at -40°C for 2 hours, quenched with water to consume lithium aluminium hydride, and filtered by suction. The mother liquor was concentrated to give a crude product. The crude product was purified by flash chromatography (C18, 0.05%trifluoroacetic acid in water:acetonitrile) to produce the target compound (100mg).

LCMS (ESI) $[M+H]^+$=202.1.

**Step 5:** Preparation of 7-((3-(1H-indol-3-yl)pyrrolidin-1-yl)methyl)-3-(difluoromethyl)-5,6,7,8-tetrahydro-[1,2,4]triazolo [4,3-a]pyridine:

3-(difluoromethyl)-5,6,7,8-tetrahydro-[1,2,4]triazolo[4,3-a]pyridine-7-carbaldehyde (80mg, 1.0eq) and 3-(pyrrolidin-3-yl)-1H-indole (1.5eq) were dissolved in tetrahydrofuran (5mL). Acetic acid (0.1eq) was added. The reaction mixture was stirred at room temperature for 30 minutes, and sodium triacetoxyborohydride (1.5eq) was added. The reaction mixture was stirred at room temperature for 2 hours, diluted with saturated sodium bicarbonate solution (5mL), and extracted with ethyl acetate (5mL×3). The organic phases were combined, washed with saturated brine, dried over anhydrous sodium sulfate, and filtered by suction. The mother liquor was concentrated to give a crude product. The crude product was purified by prep-HPLC (C18, 0.05% ammonia in water:acetonitrile) to produce the target compound (7.12mg).

LCMS (ESI) $[M+H]^+$=372.1; $^1H$ NMR(400 MHz, MeOD-$d_4$)δ 7.61(d, J=7.9 Hz, 1H), 7.31(d, J=8.1 Hz, 1H), 7.20-6.89(m, 4H), 4.37(d, J=12.6 Hz, 1H), 4.15-4.01(m, 1H), 3.71-3.60(m, 1H), 3.25(s, 1H), 3.17(t, J=8.4 Hz, 1H), 2.93(td, J=9.0, 2.8 Hz, 1H), 2.75(ddd, J=15.0, 8.7, 2.4 Hz, 1H), 2.70-2.53(m, 4H), 2.41-2.19(m, 3H), 2.14-1.99(m, 1H), 1.84-1.68(m, 1H).

Example 147 (Compound 147)

Preparation of 6-fluoro-3-(1-(2-(5-methoxy-(1,2,4)triazolo(4,3-a)pyridin-3-yl)ethyl)pyrrolidin-3-yl)-1H-indole:

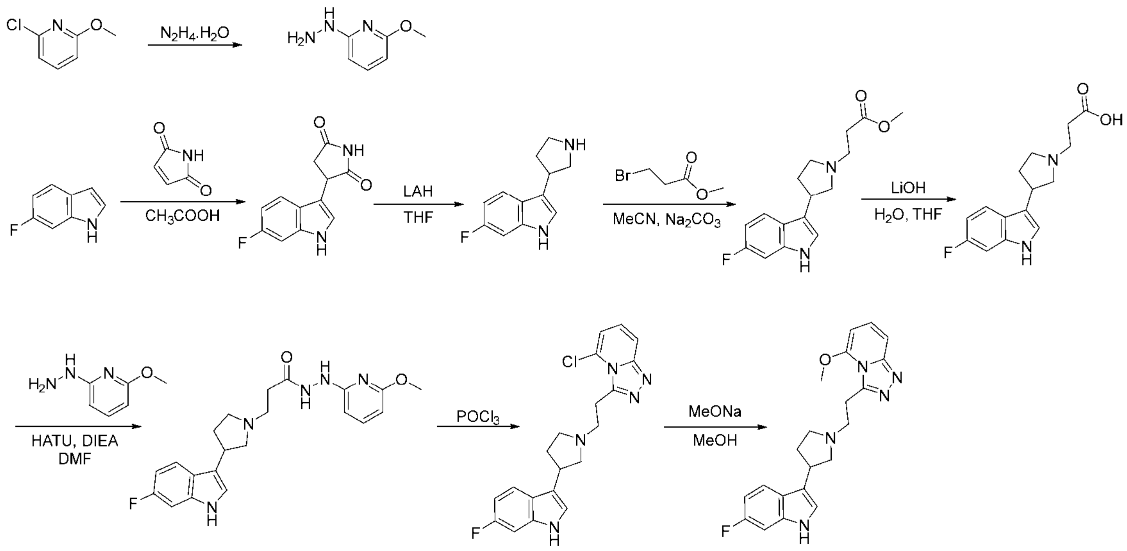


**Step 1:** Preparation of 2-hydrazineyl-6-methoxypyridine:

2-chloro-6-methoxypyridine (10g, 1eq) was added to hydrazine hydrate (35mL). The mixture was reacted at 100°C for 16 hours, then cooled to room temperature, and concentrated in vacuum. The residue was diluted with water (100mL), extracted with ethyl acetate (80mL×3). The organic phases were combined, dried over anhydrous sodium sulfate, concentrated in vacuum. The residue was purified by reversed-phase column chromatography (C18, 0.05% ammonia in water:acetonitrile) to produce 2-hydrazineyl-6-methoxypyridine (1.8g) as brown oil.
LCMS (ESI) $[M+H]^+$=140.1.

**Step 2:** Preparation of 3-(6-fluoro-1H-indol-3-yl)pyrrolidine-2,5-dione:

6-fluoro-1H-indole (20g, 1eq) was dissolved in acetic acid (200mL). 2,5-dihydro-1H-pyrrole-2,5-dione (2.5eq) was added. The reaction system was stirred at 120°C for 48 hours. The reaction mixture was concentrated, diluted with a small amount of ethyl acetate and a large amount of water, adjusted with 15% sodium hydroxide to pH=10, and extracted with ethyl acetate (500mL×3). The organic phases were combined, dried over anhydrous sodium sulfate, filtered, and concentrated to give a crude product. The crude product was slurrized with petroleum ether:ethyl acetate (3:1), filtered, dried to produce the target compound (18g).
LCMS (ESI) $[M+H]^+$=233.2.

**Step 3:** Preparation of 6-fluoro-3-(pyrrolidin-3-yl)-1H-indole:

At 0°C, lithium aluminium hydride (5eq) was added in batch to 3-(6-fluoro-1H-indol-3-yl)pyrrolidine-2,5-dione (24g, 1eq) in tetrahydrofuran (300mL). The mixture was warmed up to 80°C and stirred for 3 hours. The reaction mixture was cooled down to 0°C. Then water (20mL), 15% sodium hydroxide solution (20mL), and water (60mL) were slowly added dropwise. After the completion of the addtion, the mixture was dried over anhydrous sodium sulfate, filtered by suction. The filter cake was washed with tetrahydrofuran (100mL). The filtrate was collected, and concentrated in vacuum to produce the target compound (18.3g, crude), which was directly used in the next step.
LCMS (ESI) $[M+H]^+$=205.2.

**Step 4:** Preparation of methyl 3-(3-(6-fluoro-1H-indol-3-yl)pyrrolidin-1-yl)propanoate:

Methyl 3-bromopropanoate (6.54g, 1eq) was added to a mixture of 6-fluoro-3-(pyrrolidin-3-yl)-1H-indole (8g, crude) and sodium carbonate (3eq) in acetonitrile (100mL). The mixture was stirred at 70°C for 16 hours. The reaction mixture was cooled to room temperature, diluted with water (150mL), extracted with ethyl acetate (100mL×3). The organic phases were combined, washed with saturated brine, dried over anhydrous sodium sulfate, concentrated in vacuum. The

residue was purified to produce the target compound (7.5g).
LCMS (ESI) $[M+H]^+$=291.1.

**Step 5:** Preparation of 3-(3-(6-fluoro-1H-indol-3-yl)pyrrolidin-1-yl)propanoic acid:

Lithium hydroxide (1eq) was added to a mixture of methyl 3-(3-(6-fluoro-1H-indol-3-yl)pyrrolidin-1-yl)propanoate (6g, 1eq) in tetrahydrofuran (40mL) and water (20mL). The reaction mixture was stirred at room temperature for 16 hours, adjusted with acetic acid to pH=7, concentrated in vacuum. The residue was purified by reverse-phase chromatography (C18, 0.1% formic acid in water:acetonitrile) to give the target product (1.84g).
LCMS (ESI) $[M+H]^+$=277.2.

**Step 6:** Preparation of 3-(3-(6-fluoro-1H-indol-3-yl)pyrrolidin-1-yl)-N'-(6-methoxypyridin-2-yl)propanehydrazide:

HATU (1.5eq) and DIEA(3eq) were added to a mixture of 3-(3-(6-fluoro-1H-indol-3-yl)pyrrolidin-1-yl)propanoic acid (1.6g, 1eq), 2-hydrazineyl-6-methoxypyridine (1.2eq) in N,N-dimethylformamide (10mL). The reaction mixture was stirred at room temperature for 4 hours, diluted with water (20mL), extracted with ethyl acetate (20mL×3). The organic phases were combined, washed with saturated brine, dried, concentrated in vacuum. The residue was purified by reverse-phase chromatography (C18, 0.05% ammonia in water:acetonitrile) to give the target product (1.28g).
LCMS (ESI) $[M+H]^+$=398.2.

**Step** 7: Preparation of 3-(1-(2-(5-chloro-(1,2,4)triazolo(4,3-a)pyridin-3-yl)ethyl)pyrrolidin-3-yl)-6-fluoro-1H-indole:

3-(3-(6-fluoro-1H-indol-3-yl)pyrrolidin-1-yl)-N'-(6-methoxypyridin-2-yl)propanehydrazide (600mg, 1eq) was added to phosphoryl trichloride (10mL). The mixture was stirred at 100°C for 16 hours, concentrated in vacuum. The residue was purified to produce the target compound (230mg).
LCMS (ESI) $[M+H]^+$=384.4.

**Step 8:** Preparation of 6-fluoro-3-(1-(2-(5-methoxy-(1,2,4)triazolo(4,3-a)pyridin-3-yl)ethyl)pyrrolidin-3-yl)-1H-indole:

Under an ice bath, sodium methanolate (1.5eq) was added to a mixture of 3-(1-(2-(5-chloro-(1,2,4)triazolo(4,3-a)pyridin-3-yl)ethyl)pyrrolidin-3-yl)-6-fluoro-1H-indole (200mg, 1eq) in methanol (5mL). The mixture was stirred at 80°C for 1 hour. The reaction mixture was quenched with water (10mL), extracted with ethyl acetate (10mL×3). The organic phases were combined, and concentrated in vacuum. The residue was purified by prep-HPLC (C18, 0.05% ammonia in water:acetonitrile) to give the target product (26.30mg).
LCMS (ESI) $[M+H]^+$=380.1; $^1$H NMR(400 MHz, Chloroform-d)δ 8.20(s, 1H), 7.56(dd, J=8.6, 5.4 Hz, 1H), 7.31(d, J=9.1 Hz, 1H), 7.15(dd, J=9.1, 7.3 Hz, 1H), 7.06-6.99(m, 2H), 6.85(td, J=9.5, 2.2 Hz, 1H), 5.91(d, J=7.2 Hz, 1H), 4.03(s, 3H), 3.66(dt, J=23.9, 7.8 Hz, 3H), 3.26(t, J=8.1 Hz, 1H), 3.21-3.05(m, 2H), 3.01(d, J=6.6 Hz, 1H), 2.88-2.74(m, 2H), 2.45-2.35(m, 1H), 2.02(td, J=14.3, 7.7 Hz, 1H).

Example 148 (Compound 148)

Preparation of 6-fluoro-3-(1-(2-(8-fluoro-5-methoxy-(1,2,4)triazolo(4,3-a)pyridin-3-yl)ethyl)azetidin-3-yl)-1H-indole:

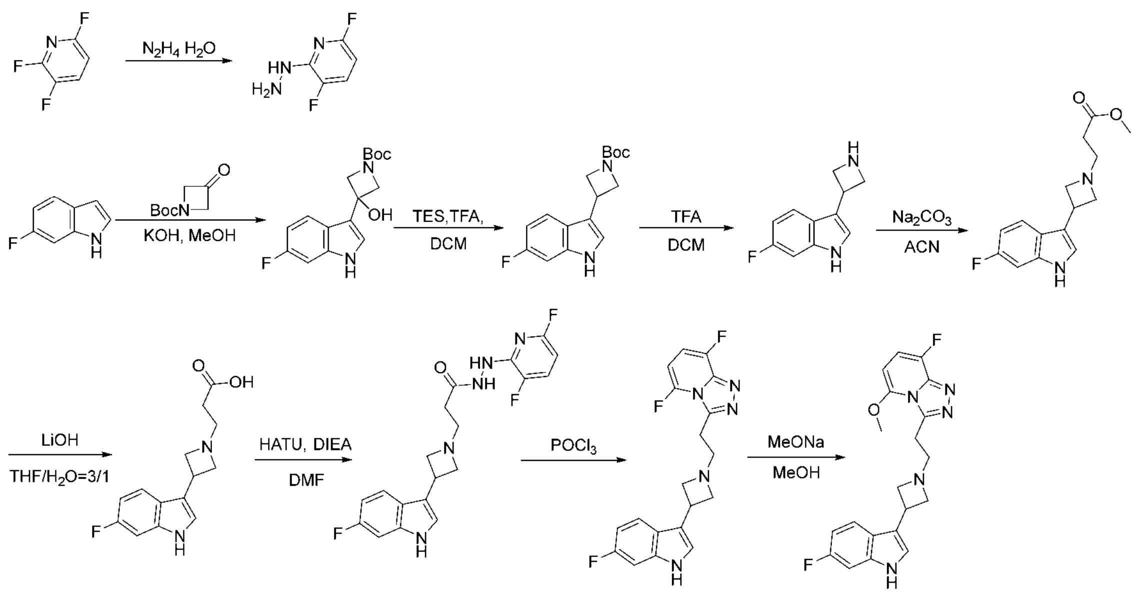


**Step 1:** Preparation of 3,6-difluoro-2-hydrazineylpyridine:

hydrazine hydrate (2.5eq) was added to a mixture of 2,3,6-trifluoropyridine (1g, 1eq), triethylamine (3eq) in N-methylpyrrolidinone (10mL). The mixture was stirred at 100°C for 1 hour, cooled to room temperature, diluted with water (20mL), extracted with ethyl acetate (20mL). The organic phase was washed with saturated brine, dried over anhydrous sodium sulfate, concentrated in vacuum to produce the target compound (850mg).

LCMS (ESI) $[M+H]^+$=146.2.

**Step 2:** Preparation of tert-butyl 3-(6-fluoro-1H-indol-3-yl)-3-hydroxyazetidine-1-carboxylate:

Potassium hydroxide (1.1eq) was added to a mixture of 6-fluoro-1H-indole (15g, 1eq) in methanol (300mL). The mixture was stirred until became dissolved and clear, then tert-butyl 3-oxoazetidine-1-carboxylate (20.9g, 1.1eq) was added. The resulting mixture was stirred at 50°C for 12 hours, cooled to room temperature, and ethyl acetate (500mL) was added. The mixture was washed with water (300mL×3) and washed with saturated brine (500mL×3). The organic phase was dried over anhydrous sodium sulfate, filtered. The filtrate was concentrated under vacuum. The residue was purified to produce the target compound (8.6g).
LCMS (ESI) $[M+H-18-Boc]^+$=189.1.

**Step 3:** Preparation of tert-butyl 3-(6-fluoro-1H-indol-3-yl)azetidine-1-carboxylate:

At 0°C, triethylsilane (10eq) was added to a mixture of tert-butyl 3-(6-fluoro-1H-indol-3-yl)-3-hydroxyazetidine-1-carboxylate (11g, 1eq) in dichloromethane (150mL). After the reaction mixture became turbid, trifluoroacetic acid (3eq) was added. While kept at 0°C, the mixture was stirred for 3 hours, diluted with saturated sodium bicarbonate solution (300mL), extracted with dichloromethane (300mL×3). The organic phases were combined, dried, concentrated. The residue was purified to produce the target compound (4g).
LCMS (ESI) $[M-56+42]^+$=276.2.

**Step 4:** Preparation of 3-(azetidin-3-yl)-6-fluoro-1H-indole:

Trifluoroacetic acid (10mL) was added to a mixture of tert-butyl 3-(6-fluoro-1H-indol-3-yl)azetidine-1-carboxylate (4g, 1eq) in dichloromethane (30mL). The reaction mixture was stirred at room temperature for 3 hours, concentrated in vacuum to produce the target compound (4g, crude).
LCMS (ESI) $[M+H]^+$=191.2.

**Step 5:** Preparation of methyl 3-(3-(6-fluoro-1H-indol-3-yl)azetidin-1-yl)propanoate :

Methyl 3-bromopropanoate (3.51g, 1eq) was added to a mixture of 3-(azetidin-3-yl)-6-fluoro-1H-indole (4g,

crude) and sodium carbonate (5eq) in acetonitrile (40mL). The mixture was stirred at 70°C for 16 hours, cooled to room temperature, filtered. The filtrate was collected, concentrated in vacuum. The residue was purified to produce the target compound (4.17g).

LCMS (ESI) $[M+H]^+$=277.1.

**Step 6:** Preparation of 3-(3-(6-fluoro-1H-indol-3-yl)azetidin-1-yl)propanoic acid:

Lithium hydroxide (5eq) was added to a mixture of methyl 3-(3-(6-fluoro-1H-indol-3-yl)azetidin-1-yl)propanoate (4.17g, 1eq) in tetrahydrofuran (30mL) and water (10mL). The reaction mixture was stirred at room temperature for 16 hours, adjusted with acetic acid to pH=7, concentrated in vacuum. The residue was purified by reverse-phase chromatography (C18, 0.1% formic acid in water:acetonitrile) to produce the target compound (3.2g).

LCMS (ESI) $[M+H]^+$=263.2.

**Step 7:** Preparation of N'-(3,6-difluoropyridin-2-yl)-3-(3-(6-fluoro-1H-indol-3-yl)azetidin-1-yl)propanehydrazide :

HATU (1.5eq) was added to a mixture of 3-(3-(6-fluoro-1H-indol-3-yl)azetidin-1-yl)propanoic acid (3g, 1eq) in N,N-dimethylformamide (30mL). 3,6-difluoro-2-hydrazineylpyridine (1.2eq) and DIEA(3eq) were added. The reaction mixture was stirred at room temperature for 4 hours, diluted with water (40mL), extracted with ethyl acetate (40mL×3). The organic phases were combined, washed with saturated brine, dried, concentrated in vacuum. The residue was purified to produce the target compound (3.73g).

LCMS (ESI) $[M+H]^+$=390.1.

**Step 8:** Preparation of 3-(1-(2-(5,8-difluoro-(1,2,4)triazolo[4,3-a]pyridin-3-yl)ethyl)azetidin-3-yl)-6-fluoro-1H-indole:

N'-(3,6-difluoropyridin-2-yl)-3-(3-(6-fluoro-1H-indol-3-yl)azetidin-1-yl)propanehydrazide (3.73g, 1eq) was added to phosphoryl trichloride (30mL). The mixture was stirred at 100°C for 16 hours, concentrated in vacuum. The residue was purified by reverse-phase chromatography (C18, 0.05% ammonia in water:acetonitrile) to produce the target compound (2.5g) as colorless oil.

LCMS (ESI) $[M+H]^+$=372.1.

**Step 9:** Preparation of 6-fluoro-3-(1-(2-(8-fluoro-5-methoxy-(1,2,4)triazolo(4,3-a)pyridin-3-yl)ethyl)azetidin-3-yl)-1H-indole:

Sodium methanolate (1.5eq) was added to a mixture of 3-(1-(2-(5,8-difluoro-(1,2,4)triazolo[4,3-a]pyridin-3-yl)ethyl)azetidin-3-yl)-6-fluoro-1H-indole (220mg, 1eq) in methanol (10mL). The mixture was stirred at 80°C for 1 hour, concentrated in vacuum. The residue was purified with prep-HPLC (C18, 0.05% ammonia in water: acetonitrile) to produce the target compound (86.63mg).

LCMS (ESI) $[M+H]^+$=384.2; $^1H$ NMR(400 MHz, DMSO-$d_6$)$\delta$ 10.94(s, 1H), 7.57(dd, J=8.6, 5.6 Hz, 1H), 7.21(t, J=9.4 Hz, 2H), 7.10(dd, J=10.2, 2.2 Hz, 1H), 6.85-6.75(m, 1H), 6.15(dd, J=8.2, 3.0 Hz, 1H), 4.04(s, 3H), 3.73(t, J=9.1 Hz, 3H), 3.29(d, J=7.0 Hz, 2H), 3.17(s, 2H), 2.92(d, J=7.3 Hz, 2H).

Example 149 (Compound 149)

Preparation of 6-fluoro-3-(1-(2-(8-fluoro-(1,2,4)triazolo[4,3-a]pyridin-3-yl)ethyl)pyrrolidin-3-yl)-1H-indole:

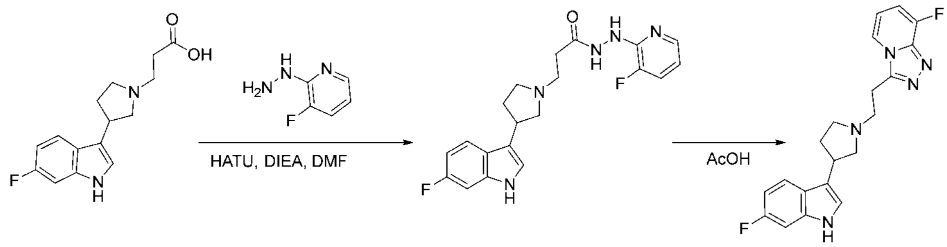

**Step 1:** Preparation of 3-(3-(6-fluoro-1H-indol-3-yl)pyrrolidin-1-yl)-N'-(3-fluoropyridin-2-yl)propanehydrazide:

3-(3-(6-fluoro-1H-indol-3-yl)pyrrolidin-1-yl)propanoic acid (3.5g, 1eq) was dissolved in DMF (N,N-dimethylformamide) (30mL). (3-fluoropyridin-2-yl)hydrazine (1eq) and HATU (1.2eq) were added. The mixture was stirred and reacted for 5 minutes, and then N,N-diisopropylethylamine (2eq) was added. The mixture was stirred and reacted at room temperature for 16 hours, diluted with water (100mL), and extracted with ethyl acetate (3×100mL). The organic phases were combined, dried over anhydrous sodium sulfate, and filtered. The filtrate was concentrated, and the resulting crude product was purified by reverse-phase column chromatography (C18, 0.1% ammonia in water:acetonitrile) to give the target product (700mg).

LCMS (ESI) $[M+H]^+$=386.1.

**Step 2:** Preparation of 6-fluoro-3-(1-(2-(8-fluoro-(1,2,4)triazolo[4,3-a]pyridin-3-yl)ethyl)pyrrolidin-3-yl)-1H-indole:

3-(3-(6-fluoro-1H-indol-3-yl)pyrrolidin-1-yl)-N'-(3-fluoropyridin-2-yl)propanehydrazide (300mg, 1eq) was dissolved in acetic acid (10mL). The atmosphere was replaced with nitrogen gas three times. In an oil bath, the mixture was stirred and reacted at 110°C for 16 hours, dried by rotary-evaporation to remove the solvent, diluted with water (20mL), adjusted with saturated sodium carbonate solution to pH=10, extracted with ethyl acetate (3×80mL). The organic phases were dried by rotary-evaporation to give a crude product. The crude product was purified by prep-HPLC (C18, 0.05% ammonia in water:acetonitrile) to produce the target compound (60mg).

LCMS (ESI) $[M+H]^+$=368.1; $^1$HNMR(400 MHz, DMSO-$d_6$)$\delta$ 10.81(s, 1H), 8.37(d, J=6.9 Hz, 1H), 7.47(dd, J=8.5, 5.7 Hz, 1H), 7.24(dd, J=11.1, 7.4 Hz, 1H), 7.13-7.02(m, 2H), 6.99-6.84(m, 1H), 6.74(td, J=9.8, 2.3 Hz, 1H), 3.55-3.40(m, 1H), 3.35(t, J=7.2 Hz, 2H), 3.11-2.90(m, 3H), 2.76(t, J=10.6 Hz, 2H), 2.61(d, J=8.5 Hz, 1H), 2.21(tt, J=13.2, 6.6 Hz, 1H), 1.90-1.77(m, 1H).

Example 150 (Compound 150)

With reference to the preparation of Example 149, compound 150 was prepared:

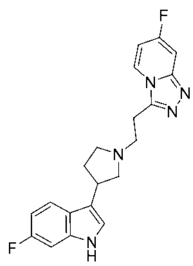

(150)

LCMS (ESI) $[M+H]^+$=368.1.

Example 151 (Compound 151)

Preparation of 6-fluoro-3-(1-(((3-trifluoromethyl)-5H,6H,7H,8H-(1,2,4)triazolo[4,3-a]pyridine-7-yl)methyl)pyrrolidin-3-yl)-1H-indole:

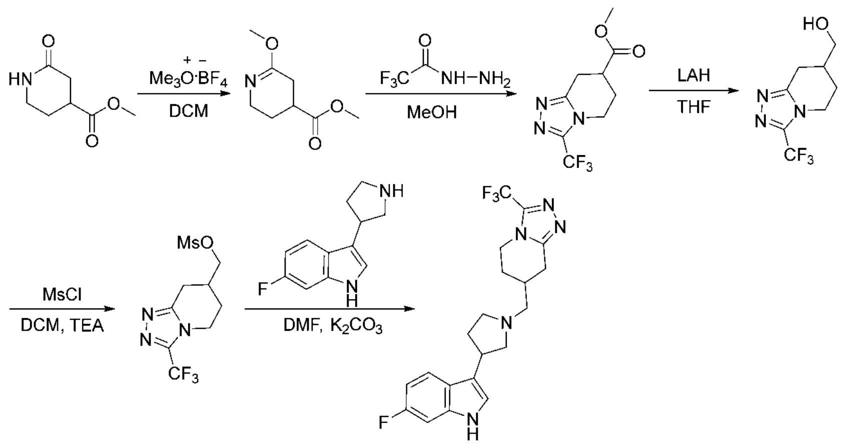


**Step** 1: Preparation of methyl 6-methoxy-2,3,4,5-tetrahydropyridine-4-carboxylate:

Methyl 2-oxopiperidine-4-carboxylate (1.5g, 1eq) was dissolved in dichloromethane (20mL), and trimethyloxonium tetrafluoroborate (1.5eq) was added. The reaction mixture was stirred at room temperature for 12 hours, diluted with saturated sodium bicarbonate solution (20mL), and extracted with dichloromethane (20mL×3). The organic phases were combined, washed with saturated brine, dried over anhydrous sodium sulfate, and filtered by suction. The mother liquor was concentrated to produce the target compound (1.46g).

LCMS (ESI) $[M+H]^+$=172.1.

**Step 2:** Preparation of methyl 3-(trifluoromethyl)-5H,6H,7H,8H-(1,2,4)triazolo[4,3-a]pyridine-7-carboxylate :

Methyl 6-methoxy-2,3,4,5-tetrahydropyridine-4-carboxylate (800mg, 1eq) was dissolved in methanol (10mL), and 2,2,2-trifluoroacetohydrazide (1.1eq) was added. The mixture was stirred at 85°C for 24 hours, concentrated to give a crude product. The crude product was purified to produce the target compound (180mg).

LCMS (ESI) $[M+H]^+$=250.1.

**Step 3:** Preparation of (3-(trifluoromethyl)-5H,6H,7H,8H-(1,2,4)triazolo[4,3-a]pyridine-7-yl)methanol:

Methyl 3-(trifluoromethyl)-5H,6H,7H,8H-(1,2,4)triazolo[4,3-a]pyridine-7-carboxylate (300mg, 1eq) was dissolved in tetrahydrofuran (5mL). The atmosphere was replaced with nitrogen gas. The mixture was cooled down to -60°C. A solution of lithium aluminium hydride in tetrahydrofuran (1M, 2eq) was slowly added. After the completion of the addition, the mixture was stirred at -60°C for 2 hours, and the target compound generated. Water (1mL) was slowly added dropwise to the reaction mixture until lithium aluminium hydride was consumed. The reaction mixture was warmed up to room temperature, diluted with water (20mL), and extracted with ethyl acetate (20mL×3). The organic phases were combined, washed with saturated brine, dried over anhydrous sodium sulfate, and filtered by suction. The mother liquor was concentrated to give a crude product. The crude product was purified to produce the target compound (260mg).

LCMS (ESI) $[M+H]^+$=222.0;

**Step 4:** Preparation of (3-(trifluoromethyl)-5H,6H,7H,8H-(1,2,4)triazolo[4,3-a]pyridine-7-yl)methyl methanesulfonate:

(3-(trifluoromethyl)-5H,6H,7H,8H-(1,2,4)triazolo[4,3-a]pyridine-7-yl)methanol (270mg, 1eq) was dissolved in dichloromethane (5mL), and triethylamine (2.2eq) was added. The mixture was cooled down to 0°C, and methanesulfonyl chloride (1.1eq) was slowly added dropwise. After the completion of the addition, the mixture was stirred at 0°C for 2 hours, and saturated sodium bicarbonate solution was added. The reaction mixture was stirred at room temperature for 5 minutes, diluted with water (5mL), extracted with dichloromethane (10mL×3). The organic phases were washed with saturated brine, dried over anhydrous sodium sulfate, and filtered by suction. The mother liquor was concentrated to produce the target compound (280mg, crude), which was directly used in the next step.

LCMS (ESI) $[M+H]^+$=300.0.

**Step 5:** Preparation of 6-fluoro-3-(1-(((3-trifluoromethyl)-5H,6H,7H,8H-(1,2,4)triazolo[4,3-a]pyridine-7-yl)methyl)pyrrolidin-3-yl)-1H-indole:

(3-(trifluoromethyl)-5H,6H,7H,8H-(1,2,4)triazolo[4,3-a]pyridine-7-yl)methyl methanesulfonate (270mg, 1eq) was dissolved in N,N-dimethylformamide (5mL), and 6-fluoro-3-(pyrrolidin-3-yl)-1H-indole (1eq) and potassium carbonate (1.5eq) were added. The mixture was stirred at 70°C for 72 hours, diluted with water (20mL), and extracted with ethyl acetate (20mL×3). The organic phases were combined, washed with saturated brine, dried over anhydrous sodium sulfate, and filtered by suction. The mother liquor was concentrated to give a crude product. The crude product was purified by prep-HPLC (C18, 0.05% formic acid in water:acetonitrile) to produce the target compound (17.5mg).

LCMS (ESI) $[M+H]^+$=408.3; $^1H$ NMR(400 MHz, DMSO-$d_6$)$\delta$ 10.85(s, 1H), 8.17(s, 1H), 7.67-7.57(m, 1H), 7.16(s, 1H), 7.10(dd, J=10.2, 2.1 Hz, 1H), 6.82(t, J=9.3 Hz, 1H), 4.22(d, J=10.5 Hz, 1H), 4.11-4.02(m, 1H), 3.54(dd, J=16.1, 7.9 Hz, 1H), 3.20-3.11(m, 1H), 3.10-3.02(m, 1H), 2.85-2.69(m, 2H), 2.58(dt, J=16.6, 8.8 Hz, 4H), 2.34-2.12(m, 3H), 1.90(dq, J=14.7, 7.5 Hz, 1H), 1.74(dd, J=18.0, 6.2 Hz, 1H).

Example 152 (Compound 152)

With reference to the preparation of Example 149, compound 152 was prepared:

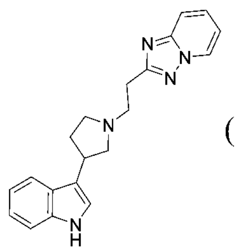

(152)

LCMS (ESI) $[M+H]^+$=332.2.

Example 153 (Compound 153)

Preparation of 3-(1-(2-(5-(trifluoromethyl)-(1,2,4)triazolo(4,3-a)pyridin-3-yl)ethyl)pyrrolidin-3-yl)-1H-indole:

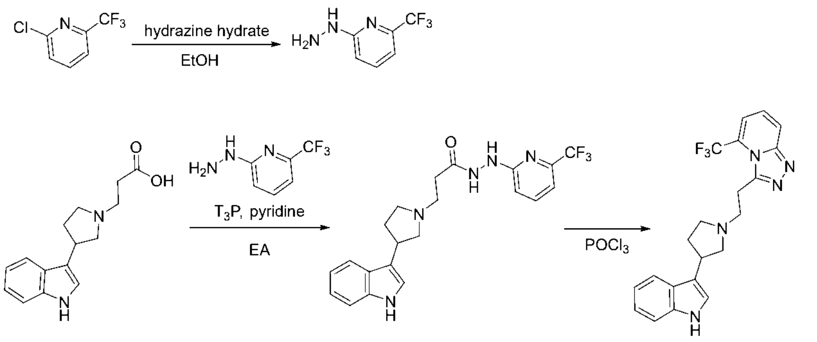


**Step** 1: Preparation of 2-hydrazineyl-6-(trifluoromethyl)pyridine:

2-chloro-6-(trifluoromethyl)pyridine (5g, 1eq) was dissolved in ethanol (50mL). Hydrazine hydrate (5eq) was added. The mixture was reacted at 80°C for 16 hours, dried by rotary-evaporation to remove ethanol, diluted with water (50mL), extracted with ethyl acetate (50mL×3). The organic phases were washed with saturated brine, dried over anhydrous sodium sulfate to produce the target compound (3.5g). LCMS (ESI) $[M+H]^+$=178.1.

**Step 2:** Preparation of 3-(3-(1H-indol-3-yl)pyrrolidin-1-yl)-N'-(6-(trifluoromethyl)pyridin-2-yl)propanehydrazide:

3-(3-(1H-indol-3-yl)pyrrolidin-1-yl)propanoic acid (1g, 1eq) and (6-(trifluoromethyl)pyridin-2-yl)hydrazine (1.2eq) were dissolved in ethyl acetate (10mL). Pyridine (5eq) and 50% propanephosphonic acid anhydride ($T_3P$)/ethyl

acetate solution (16.22eq) were added. The mixture was reacted at 50°C for 1 hour. The reaction mixture was diluted with water (30mL), extracted with ethyl acetate (20mL×3). The organic phases were washed with saturated brine, and dried over anhydrous sodium sulfate, then dried by rotary-evaporation to produce a crude product. The crude product was purified by to produce the target compound (800mg).

LCMS (ESI) $[M+H]^+$=418.2.

**Step 3:** Preparation of 3-(1-(2-(5-(trifluoromethyl)-(1,2,4)triazolo(4,3-a)pyridin-3-yl)ethyl)pyrrolidin-3-yl)-1H-indole:

3-(3-(1H-indol-3-yl)pyrrolidin-1-yl)-N'-(6-(trifluoromethyl)pyridin-2-yl)propanehydrazide (300mg, 1eq) was dissolved in phosphoryl trichloride (5mL). Under the protection of nitrogen gas, the mixture was reacted at 100°C for 16 hours, and purified to produce the target compound (15.31mg).

LCMS (ESI) $[M+H]^+$=400.3; $^1$H NMR(400 MHz, MeOD-$d_4$)δ 8.31(d, J=41.3 Hz, 2H), 8.10(d, J=9.2 Hz, 1H), 7.73(d, J=6.9 Hz, 1H), 7.65-7.51(m, 2H), 7.37(d, J=8.1 Hz, 1H), 7.24(s, 1H), 7.18-7.10(m, 1H), 7.09-6.98(m, 1H), 4.02(dt, J=18.5, 7.5 Hz, 4H), 3.75(s, 2H), 3.63(dd, J=24.3, 17.7 Hz, 3H), 2.64(dd, J=12.7, 6.8 Hz, 1H), 2.42(dt, J=9.0, 6.5 Hz, 1H).

Example 154 (Compound 154)

With reference to the preparation of Example 153, compound 154 was prepared:

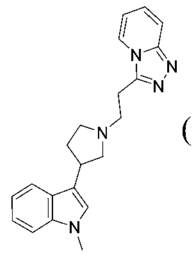

(154)

LCMS (ESI) $[M+H]^+$=346.2.

Example 155 (Compound 155)

Preparation of 3-(4-(2-(imidazo(1,5-a)pyridin-3-yl)ethyl)morpholin-2-yl)-1H-indole:

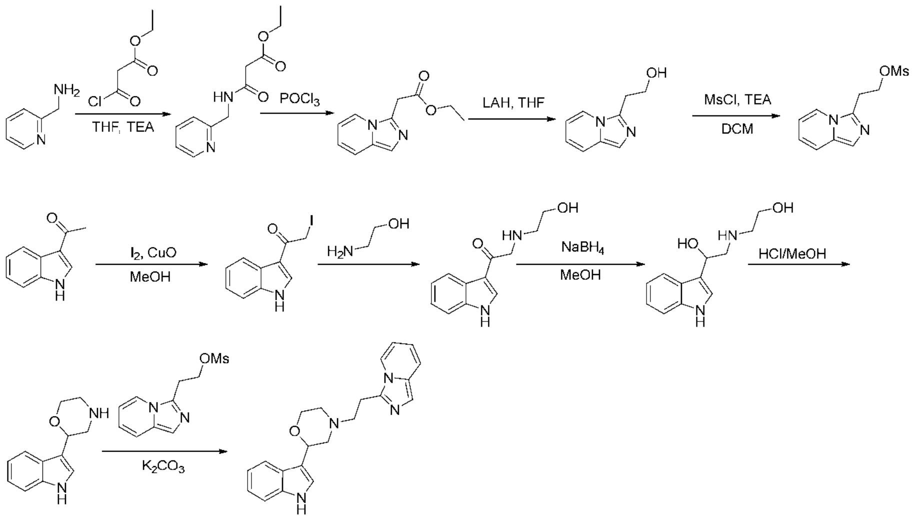


**Step** 1: Preparation of ethyl 2-(((pyridin-2-yl)methyl)aminoformyl)acetate:

1-(pyridin-2-yl)methanamine (10g, 1eq) was dissolved in anhydrous tetrahydrofuran (100mL). Under the protection of nitrogen gas, under an ice bath, ethyl 3-chloro-3-oxopropanoate (1eq) was added dropwise, then triethylamine (1.5eq) was added dropwise. The mixture was warmed up to room temperature, reacted for 2 hours, diluted with water (150mL), extracted with ethyl acetate (100mL×3). The organic phases were combined, washed with saturated brine, dried over anhydrous sodium sulfate. to produce the target compound (14g).

LCMS (ESI) $[M+H]^+$=223.2.

**Step 2:** Preparation of ethyl 2-(imidazo(1,5-a)pyridin-3-yl)acetate:

Ethyl 2-(((pyridin-2-yl)methyl)aminoformyl)acetate (13g, 1eq) was dissolved in phosphoryl trichloride (100mL). Under the protection of nitrogen gas, the mixture was reacted at 100°C for 16 hours, dried by rotary-evaporation to remove most of phosphoryl trichloride, quenched, washed to give a crude product. The crude product was purified to produce the target compound (9g).

LCMS (ESI) $[M+H]^+$=205.2.

**Step 3:** Preparation of 2-imidazo(1,5-a)pyridin-3-ylethanol:

Ethyl 2-(imidazo(1,5-a)pyridin-3-yl)acetate (2g, 1eq) was dissolved in tetrahydrofuran (20mL). Under an ice bath, lithium aluminium hydride (2eq) was added in batch. The mixture was warmed up to room temperature and reacted for 2 hours, and quenched with water (1mL). 15% sodium hydroxide (1mL) and water (1mL) were added. Anhydrous sodium sulfate was added. The mixture was stirred for 30 minutes, and filtered to produce the target compound (1.5g, crude).

LCMS (ESI) $[M+H]^+$=163.0.

**Step 4:** Preparation of 2-(imidazo(1,5-a)pyridin-3-yl)ethyl methanesulfonate :

2-imidazo(1,5-a)pyridin-3-ylethanol (800mg, crude) was dissolved in dichloromethane (10mL). Triethylamine (1497.35mg) was added. Methanesulfonyl chloride (847.52mg) was slowly added dropwise. At room temperature, the mixture was reacted for 2 hours, diluted with water (10mL). The aqueous phase was extracted with dichloromethane (10mL×3). The organic phases were washed with saturated brine, dried over anhydrous sodium sulfate to give a crude product. The crude product was purified to produce the target compound (700mg).

LCMS (ESI) $[M+H]^+$=241.1.

**Step 5:** Preparation of 1-(1H-indol-3-yl)-2-iodoethan-1-one:

1-(1H-indol-3-yl)ethan-1-one (1g, 1eq) was dissolved in methanol (10mL). Copper(II) oxide (1.1eq) and iodine (1.1eq) were added. The mixture was reacted at 70°C for 3 hours, cooled to room temperature. The reaction mixture was diluted with water (30mL), stirred for 20 minutes, and filtered. The filter cake was dissolved in tetrahydrofuran (50mL). Copper oxide was removed by filtering. The filtrate was dried by rotary-evaporation to produce the target compound (1.7g).

LCMS (ESI) $[M+H]^+$=286.0.

**Step 6:** Preparation of 2-(2-hydroxyethylamino)-1-(1H-indol-3-yl)ethan-1-one:

Under an ice bath, 1-(1H-indol-3-yl)-2-iodoethan-1-one (15g, 1eq) was added in batch to ethan-1-ol-2-amine (80mL, 24.9eq), which was being stirred. Under an ice bath, the mixture was reacted for 30 minutes, diluted with water (100mL), and a solid precipitated. The mixture was filtered to give a crude product. The crude product was slurrized with acetonitrile to produce the target compound (3.2g).

LCMS (ESI) $[M+H]^+$=219.1.

**Step 7:** Preparation of 2-((2-hydroxyethyl)amino)-1-(1H-indol-3-yl)ethan-1-ol:

2-(2-hydroxyethylamino)-1-(1H-indol-3-yl)ethan-1-one (1.3g, 1eq) was dissolved in methanol (10mL). Under an ice bath, sodium borohydride (5eq) was slowly added. The mixture was warmed up to room temperature and reacted for 16 hours. Under an ice bath, 4M hydrochloric acid /methanol (15mL) was added to quench the reaction until the pH became neutral. The mixture was filtered to remove the salt, and the filtrate was directly used in the next step.

LCMS (ESI) $[M-18+H]^+$=203.2.

**Step 8:** Preparation of 3-(morpholin-2-yl)-1H-indole:

Under an ice bath, 4M hydrochloric acid/methanol solution (10mL) was added dropwise to 2-((2-hydroxyethyl)amino)-1-(1H-indol-3-yl)ethan-1-ol (the quenched reaction mixture from the previous step) until the pH became 2. The reaction mixture was stirred at room temperature for 30 minutes, dried by rotary-evaporation, filtered, and washed to give a crude product. The crude product was dissolved in methanol, and ammonia water was added dropwise until the pH became 9. The mixture was filtered, dried by rotary-evaporation to produce the target compound (1.5g).

LCMS (ESI) $[M+H]^+$=203.1.

**Step 9:** Preparation of 3-(4-(2-(imidazo(1,5-a)pyridin-3-yl)ethyl)morpholin-2-yl)-1H-indole:

3-(morpholin-2-yl)-1H-indole (300mg, 1eq) and 2-(imidazo(1,5-a)pyridin-3-yl)ethyl methanesulfonate (1eq) were dissolved in acetonitrile (10mL) and N,N-dimethylformamide (5mL). Potassium carbonate (3eq) was added. The mixture was reacted at 70°C for 24 hours, dried by rotary-evaporation to remove acetonitrile, diluted with water (20mL) and extracted with ethyl acetate (10mL×3). The organic phase was washed with saturated brine, dried over anhydrous sodium sulfate to produce a crude product. The crude product was purified by prep-HPLC to produce the target compound (21.03mg).

LCMS (ESI) $[M+H]^+$=347.1; $^1$H NMR(400 MHz, DMSO-$d_6$)δ 10.98(s, 1H), 8.19(d, J=7.0 Hz, 1H), 7.61(d, J=7.9 Hz, 1H), 7.49(d, J=9.1 Hz, 1H), 7.34(d, J=8.1 Hz, 1H), 7.30-7.24(m, 2H), 7.07(t, J=7.3 Hz, 1H), 6.98(t, J=7.2 Hz, 1H), 6.70(dd, J=9.0, 6.4 Hz, 1H), 6.61(t, J=6.3 Hz, 1H), 4.72(d, J=8.1 Hz, 1H), 3.89(d, J=11.5 Hz, 1H), 3.68(dd, J=11.1, 9.1 Hz, 1H), 3.19(t, J=7.5 Hz, 2H), 3.04(d, J=11.1 Hz, 1H), 2.84(dt, J=14.4, 9.5 Hz, 3H), 2.40(d, J=10.7 Hz, 1H), 2.33-2.25(m, 1H).

Example 156 (Compound 156)

Preparation of (3-(4-(2-imidazo[1,2-a]pyridin-3-yl)ethyl)morpholin-2-yl)-1H-indole:

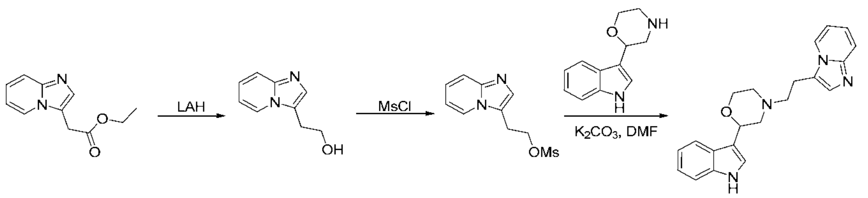


**Step 1:** Preparation of 2-(imidazo[1,2-a]pyridin-3-yl)ethan-1-ol:

ethyl (2-imidazo[1,2-a]pyridin-3-yl)acetate (1g, 1eq) was dissolved in tetrahydrofuran (30mL). The reaction mixture was cooled down to -10°C, and lithium aluminium hydride (2eq) was slowly added in batch. The mixture was slowly warmed up to room temperature, and stirred for 3 hours. To the reaction mixture was added water (0.4mL), then added 15% sodium hydroxide solution (0.4mL), and then added water (1.2mL). The resulting mixture was stirred at room temperature for 15 minutes, and an appropriate amount of anhydrous magnesium sulfate was added. The mixture was stirred for another 15 minutes, and filtered. The filtrate was dried by rotary-evaporation to produce the target compound (790mg).

LCMS (ESI) $[M+H]^+$=163.1.

**Step 2:** Preparation of 2-(imidazo[1,2-a]pyridin-3-yl)ethyl methanesulfonate:

2-(imidazo[1,2-a]pyridin-3-yl)ethan-1-ol (600mg, 1eq) was dissolved in dichloromethane (15mL), and triethylamine (3eq) was added. The reaction mixture was cooled down to 0°C, and methanesulfonyl chloride (1.2eq) in dichloromethane (3mL) was slowly added dropwise. The reaction mixture was stirred and reacted at room temperature for 1 hour, diluted with water (30mL), extracted with dichloromethane (3×50mL). The organic phases were combined, dried over anhydrous sodium sulfate, and filtered. The filtrate was concentrated to produce the target compound (600mg, crude), which was directly used in the next step.

LCMS (ESI) $[M+H]^+$=241.1.

**Step 3:** Preparation of (3-(4-(2-imidazo[1,2-a]pyridin-3-yl)ethyl)morpholin-2-yl)-1H-indole:

2-(imidazo[1,2-a]pyridin-3-yl)ethyl methanesulfonate (500mg, crude) was dissolved in N,N-dimethylformamide (20mL). 3-morpholin-2-yl-1H-indole (420.86mg, 1eq) and potassium carbonate (3eq) were added. In an oil bath, the mixture was stirred and reacted at 70°C for 16 hours, diluted with water (100mL), extracted with ethyl acetate (3×100mL).

The organic phases were combined, washed with saturated brine, dried over anhydrous sodium sulfate, filtered, and concentrated. The resulting crude product was purified by prep-HPLC (C18, 10mmol ammonia in water: acetonitrile) to produce the target compound (95.41mg).

LCMS (ESI) $[M+H]^+$=347.2; $^1$HNMR(400 MHz, MeOD-$d_4$)$\delta$ 8.31(t, J=8.1 Hz, 1H), 7.65(d, J=7.8 Hz, 1H), 7.53(d, J=9.1 Hz, 1H), 7.44(d, J=3.8 Hz, 1H), 7.38-7.20(m, 3H), 7.09(t, J=7.5 Hz, 1H), 6.98(dt, J=12.4, 6.8 Hz, 2H), 4.90(d, J=10.3 Hz, 1H), 4.02(d, J=11.5 Hz, 1H), 3.95-3.76(m, 1H), 3.18(dd, J=16.6, 8.5 Hz, 3H), 2.99(d, J=9.7 Hz, 1H), 2.90-2.75(m, 2H), 2.65-2.52(m, 1H), 2.45(dd, J=11.3, 7.9 Hz, 1H).

Examples 157-165

With reference to the preparation of Example 1, 5, or 156, compounds 157-165 were prepared:

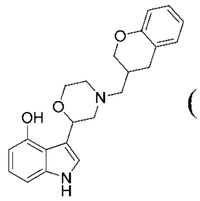 (157) 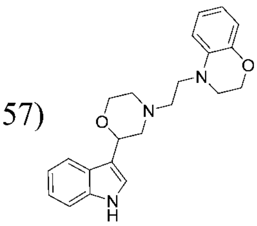 (158) 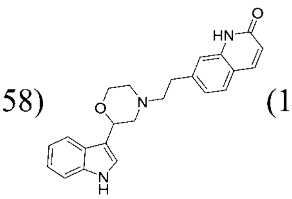 (159)

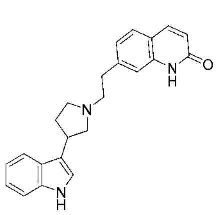 (160) 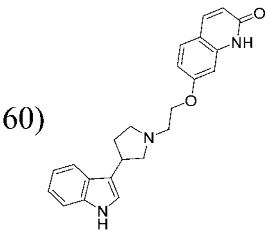 (161) 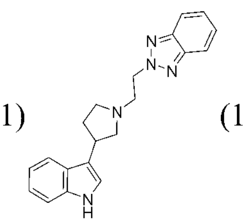 (162)

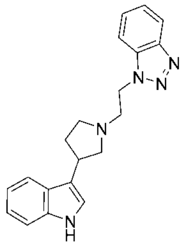 (163) 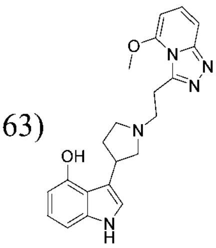 (164) 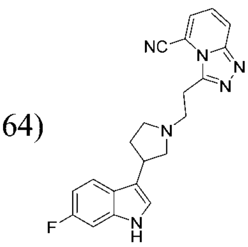 (165)

Compound 157: LCMS (ESI) $[M+H]^+$=365.1;
Compound 158: LCMS (ESI) $[M+H]^+$=364.2;
Compound 159: LCMS (ESI) $[M+H]^+$=374.1;
Compound 160: LCMS (ESI) $[M+H]^+$=358.2;
Compound 161: LCMS (ESI) $[M+H]^+$=374.2;
Compound 162: LCMS (ESI) $[M+H]^+$=332.2;
Compound 163: LCMS (ESI) $[M+H]^+$=332.2;
Compound 164: LCMS (ESI) $[M+H]^+$=378.2;
Compound 165: LCMS (ESI) $[M+H]^+$=375.1.

Example 166 (Compound 166)

With reference to the preparation of Example 20, 29, or 132, compound 166 was prepared:

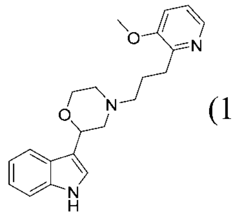

(166)

LCMS (ESI) $[M+H]^+$=375.1; $^1H$ NMR(400 MHz, MeOD-$d_4$)$\delta$ 7.98(dd, J=4.8, 1.1 Hz, 1H), 7.64(d, J=7.9 Hz, 1H), 7.35(t, J=8.1 Hz, 2H), 7.23(dd, J=8.9, 4.2 Hz, 2H), 7.09(dd, J=11.2, 4.0 Hz, 1H), 7.01(t, J=7.1 Hz, 1H), 4.87(d, J=2.1 Hz, 1H), 3.97(dd, J=11.5, 1.8 Hz, 1H), 3.89-3.80(m, 4H), 3.05(d, J=11.6 Hz, 1H), 2.86(t, J=7.7 Hz, 3H), 2.47(dt, J=22.1, 9.2 Hz, 3H), 2.29(td, J=11.5, 3.4 Hz, 1H), 1.97-1.86(m, 2H).

Examples 167-169 (Compounds 167-169)

With reference to the preparation of Example 1 or 5, compounds 167-169 were prepared:

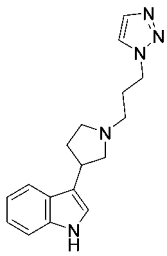

(167)

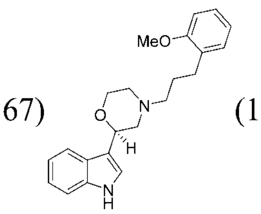

(168)

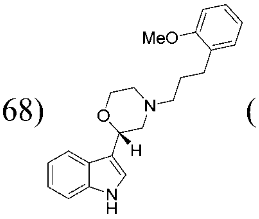

(169)

Compound 167: LCMS (ESI) $[M+H]^+$=296.1;
Compound 168: LCMS (ESI) $[M+H]^+$=351.2;
Compound 169: LCMS (ESI) $[M+H]^+$=351.2.

Example 170 (Compound 170)

Preparation of 3-(1-(3-(1,2,4-oxadiazol-3-yl)propyl)azetidin-3-yl)-1H-indole:

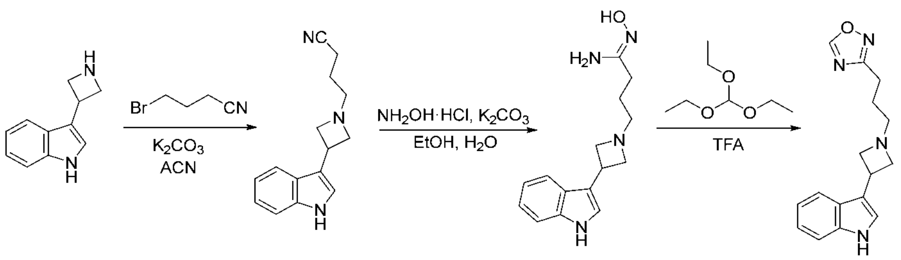


**Step 1:** Preparation of 4-(3-(1H-indol-3-yl)azetidin-1-yl)butanenitrile:

4-bromobutanenitrile (0.9eq) was added to a solution of 3-(azetidin-3-yl)-1H-indole (4.85g, 1eq) and potassium carbonate (1eq) in acetonitrile (50mL). The mixture was stirred at 70°C for 16 hours, diluted with water (100mL), and extracted with dichloromethane (100mL×3). The organic phases were combined, washed with saturated brine (200mL), dried over anhydrous sodium sulfate, concentrated. The residue was purified by flash chromatography (Silica gel, PE/EA=1/1) to give the target product (1g).
LCMS (ESI) $[M+H]^+$=240.1.

**Step 2:** Preparation of (Z)-N'-hydroxy-4-(3-(1H-indol-3-yl)azetidin-1-yl)butanimidamide:

Potassium carbonate (3eq) was added to a mixture of 4-(3-(1H-indol-3-yl)azetidin-1-yl)butanenitrile (1g, 1eq)

and hydroxylamine hydrochloride (3.5eq) in ethanol (10mL) and water (5mL). The mixture was stirred at 80°C for 16 hours. The reaction mixture was concentrated in vacuum. The residue was purified by reversed-phase column chromatography (C18, 0.1%$NH_3 \cdot H_2O$/water, MeCN) to give the target product (260mg).

LCMS (ESI) $[M+H]^+$=273.1.

**Step 3:** Preparation of 3-(1-(3-(1,2,4-oxadiazol-3-yl)propyl)azetidin-3-yl)-1H-indole:

(Z)-N'-hydroxy-4-(3-(1H-indol-3-yl)azetidin-1-yl)butanimidamide (250mg, 1eq) was added to triethyl orthoformate (5mL). A catalytic amount of trifluoroacetic acid was added, the reaction mixture was stirred at room temperature for 10 minutes and at 60°C for 0.5 hours. The reaction mixture was concentrated in vacuum. The residue was diluted with water (10mL), and extracted with dichloromethane (10mL×3). The organic phases were combined, washed with saturated brine (20mL), dried over anhydrous sodium sulfate, concentrated. The residue was purified with prep-HPLC (C18, 0.1%$NH_3 \cdot H_2O$/water, MeCN) to produce the target compound (60.47mg).

LCMS (ESI) $[M+H]^+$=283.1. $^1H$ NMR(400 MHz, DMSO-$d_6$)δ 10.87(s, 1H), 9.49(s, 1H), 7.60(d, J=7.9 Hz, 1H), 7.33(d, J=8.1 Hz, 1H), 7.21(d, J=2.2 Hz, 1H), 7.09-7.03(m, 1H), 6.99-6.94(m, 1H), 3.79(dd, J=14.9, 7.2 Hz, 1H), 3.71(t, J=7.0 Hz, 2H), 3.14(q, J=6.7 Hz, 2H), 2.80(t, J=7.5 Hz, 2H), 2.53(d, J=7.0 Hz, 2H), 1.78-1.68(m, 2H).

Example 171 (Compound 171) and Example 172 (Compound 172)

Preparation of (R)-3-(1-[3-(1,2,4-oxadiazol-3-yl)propyl]pyrrolidin-3-yl)-1H-indole (Compound 171) and (S)-3-(1-[3-(1,2,4-oxadiazol-3-yl)propyl]pyrrolidin-3-yl)-1H-indole (Compound 172):

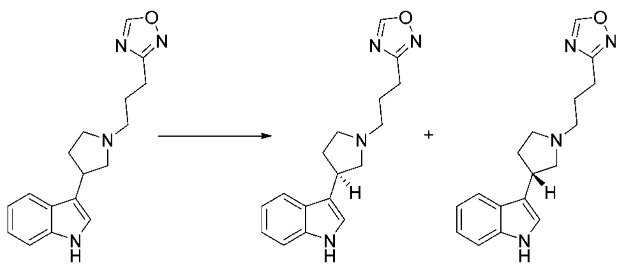

compound 49 compound 171 compound 172

Compound 49 (2.7g) was chirally resolved to afford compound 171 and compound 172. Chiral resolution conditions: Temperature: room temperature; Mobile phase: $CO_2$/MeOH[0.1%a solution of ammonia in methanol (7M) =86/14]; Flow rate: 130mL/min; Detection wavelength: 214nm. The target compound P1 (171 or 172)($t_{major}$=3.108min, ee>99%, 1.274g) and P2 (172 or 171)($t_{major}$=3.757min, ee>96%, 0.933g) were obtained.

Example 173 (Compound 173)

Preparation of 3-(1-(3-(1,2,4-oxadiazol-3-yl)propyl)pyrrolidin-3-yl)-1H-indol-4-ol:

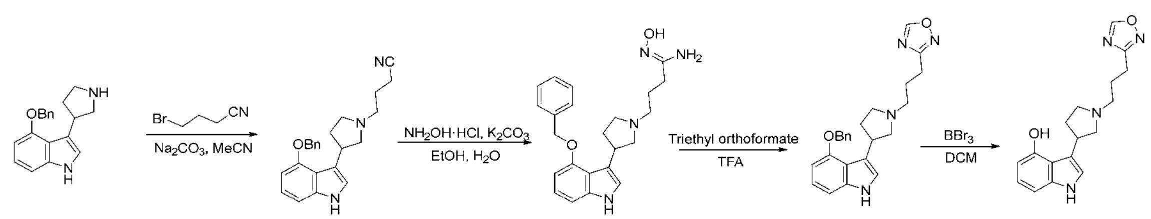


**Step** 1: Preparation of 4-(3-(4-(benzyloxy)-1H-indol-3-yl)pyrrolidin-1-yl)butanenitrile:

4-(Benzyloxy)-3-(pyrrolidin-3-yl)-1H-indole (5g, 1eq) was dissolved in acetonitrile (50mL). 4-Bromobutanenitrile (0.9eq) and sodium carbonate (3eq) was added. The mixture was reacted at 70°C for 16 hours, filtered. The filter cake was

washed with ethyl acetate. The filtrate was dried by rotatary evaporation and purified by flash chromatography (Silica gel, DCM:MeOH=30:1) to give the target product (5.5g).

LCMS (ESI) $[M+H]^+$=360.2.

Step 2: Preparation of (Z)-4-(3-(4-(benzyloxy)-1H-indol-3-yl)pyrrolidin-1-yl)-N'-hydroxybutanimidamide:

4-(3-(4-(benzyloxy)-1H-indol-3-yl)pyrrolidin-1-yl)butanenitrile (5.4g, 1eq) was dissolved in water (50mL) and ethanol (50mL). Hydroxylamine hydrochloride (3eq) and potassium carbonate (3eq) was added. The mixture was reacted at 80°C for 16 hours, dried by rotary-evaporation to remove ethanol, diluted with water (50mL), extracted with ethyl acetate (50mL×3). The organic phases were dried by rotary-evaporation to give a crude product. The crude product was purified by reverse-phase chromatography (C18, 0.1% formic acid/water, MeCN) to give the target product (3.2g).

LCMS (ESI) $[M+H]^+$=393.2.

**Step 3:** Preparation of 4-(benzyloxy)-3-(1-(3-(1,2,4-oxadiazol-3-yl)propyl)pyrrolidin-3-yl)-1H-indole:

(Z)-4-(3-(4-(benzyloxy)-1H-indol-3-yl)pyrrolidin-1-yl)-N'-hydroxybutanimidamide(700mg, 1eq) was dissolved in triethyl orthoformate (6mL). Trifluoroacetic acid (0.5mL) was added dropwise. At room temperature, the mixture was reacted for 30 minutes, then quenched with saturated aqueous sodium bicarbonate solution (3mL), diluted with water (30mL), extracted with ethyl acetate (20mL×3). The organic phase was washed with saturated brine, dried over anhydrous sodium sulfate to give a crude product. The crude product was purified by flash chromatography (Silica gel, DCM:MeOH=30:1) to give the target product (200mg).

LCMS (ESI) $[M+H]^+$=403.2.

**Step 4:** Preparation of 3-(1-(3-(1,2,4-oxadiazol-3-yl)propyl)pyrrolidin-3-yl)-1H-indol-4-ol:

4-(benzyloxy)-3-(1-(3-(1,2,4-oxadiazol-3-yl)propyl)pyrrolidin-3-yl)-1H-indole (200mg, 1eq) was dissolved in anhydrous dichloromethane (20mL). The mixture was cooled down to -70°C, and a solution boron tribromide in dichloromethane (1M, 2.5mL, 5eq) was slowly added dropwise.

The mixture was reacted at -70°C for 30 minutes. LCMS detection showed the generation of the product. The reaction mixture was quenched by adding saturated sodium bicarbonate solution (20mL)dropwise at -70°C, warmed up to room temperature, stirred for another 15 minutes, diluted with water (20mL), and extracted with dichloromethane (20mL×3). The combined organic phases were dried by rotary-evaporation, purified by Prep-HPLC (C18, 0.1% formic acid/water, MeCN) to give the target product (10.74mg).

LCMS (ESI) $[M+H]^+$=313.2; $^1H$ NMR(400 MHz, MeOD-$d_4$)δ 9.17(s, 1H), 8.54(s, 1H), 7.02(s, 1H), 6.97-6.81(m, 2H), 6.39(d, J=7.2 Hz, 1H), 4.09-3.98(m, 1H), 3.81(d, J=9.1 Hz, 1H), 3.53(dd, J=20.6, 10.3 Hz, 3H), 3.31-3.22(m, 2H), 2.93(t, J=7.0 Hz, 2H), 2.52-2.37(m, 2H), 2.26-2.15(m, 2H).

Example 174 (Compound 174)

Preparation of 3-(1-(3-(5-isopropyl-1,2,4-oxadiazol-3-yl)propyl)pyrrolidin-3-yl)-1H-indol-4-ol:

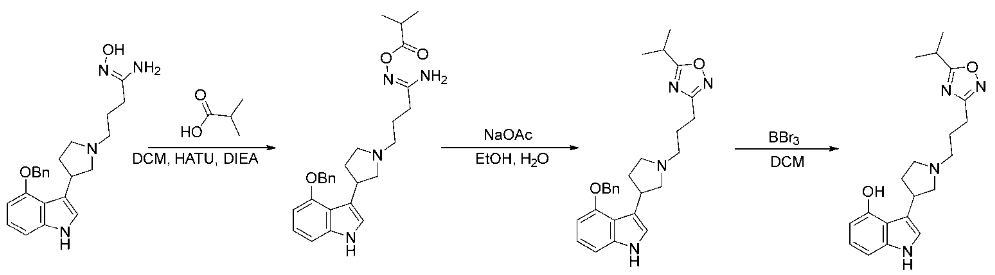


**Step** 1: Preparation of (Z)-4-(3-(4-(benzyloxy)-1H-indol-3-yl)pyrrolidin-1-yl)-N'-(isobutyryloxy)butanimidamide:

2-methylpropanoic acid (134.69mg, 1eq) was dissolved in dichloromethane (10mL). HATU (1eq) and N,N-diisopropylethylamine (1.3eq) were added. The reaction mixture was stirred at room temperature for 30 minutes. (Z)-4-(3-(4-(benzyloxy)-1H-indol-3-yl)pyrrolidin-1-yl)-N'-hydroxybutanimidamide (1eq) was added. The mixture was

reacted for another 2 hours, diluted with water (20mL), extracted with dichloromethane (10mL×3). The organic phases were washed with saturated brine, dried over anhydrous sodium sulfate to give a crude product. The crude product was purified by flash chromatography (Silica gel, DCM:MeOH=20:1) to give the target product (400mg).

LCMS (ESI) $[M+H]^+$=463.2.

**Step 2:** Preparation of 4-(benzyloxy)-3-(1-(3-(5-(propan-2-yl)-1,2,4-oxadiazol-3-yl)propyl)pyrrolidin-3-yl)-1H-indole:

(Z)-4-(3-(4-(benzyloxy)-1H-indol-3-yl)pyrrolidin-1-yl)-N'-(isobutyryloxy)butanimidamide(350mg, 1eq) was dissolved in ethanol (20mL) and water (10mL). Sodium acetate (5eq) was added. The mixture was reacted at 80°C for 16 hours, dried by rotary-evaporation to remove ethanol, diluted with water (20mL) and extracted with ethyl acetate (15mL×3) to give a crude product. The crude product was purified by flash chromatography (Silica gel, DCM:MeOH=15:1) to give the target product (250mg). LCMS (ESI) $[M+H]^+$=445.2.

**Step 3:** Preparation of 3-(1-(3-(5-isopropyl-1,2,4-oxadiazol-3-yl)propyl)pyrrolidin-3-yl)-1H-indol-4-ol:

4-(benzyloxy)-3-(1-(3-(5-(propan-2-yl)-1,2,4-oxadiazol-3-yl)propyl)pyrrolidin-3-yl)-1H-indole (75mg, 1eq) was dissolved in anhydrous dichloromethane (20mL). The mixture was cooled down to -70°C, and a solution of boron tribromide in dichloromethane (1M, 5eq) was slowly added dropwise. The mixture was reacted at -70°C for 30 minutes. LCMS detection showed the generation of the product. The reaction mixture was quenched by adding methanol (1mL) dropwise at -70°C, then stirred for another 5 minutes, warmed up to room temperature, adjusted with saturated sodium bicarbonate to pH=9, diluted with water (20mL), extracted with dichloromethane (20mL×3). The combined organic phases were dried by rotary-evaporation, purified by Prep-HPLC (C18, 0.1% formic acid/water, MeCN) to give the target product (8.36mg).

LCMS (ESI) $[M+H]^+$=355.2; $^1H$ NMR(400 MHz, MeOD-$d_4$)δ 8.52(s, 1H), 7.03(s, 1H), 6.96-6.77(m, 2H), 6.39(d, J=7.3 Hz, 1H), 4.03(d, J=8.3 Hz, 1H), 3.82(s, 1H), 3.68-3.44(m, 3H), 3.31(s, 2H), 3.27-3.16(m, 1H), 2.86(t, J=6.9 Hz, 2H), 2.59-2.38(m, 2H), 2.32-2.11(m, 2H), 1.37(d, J=6.9 Hz, 6H).

Example 175 (Compound 175)

Preparation of 3-(3-(-3-(1H-indazol-3-yl)pyrrolidin-1-yl)propyl)-5-isopropyl-1,2,4-oxadiazole:

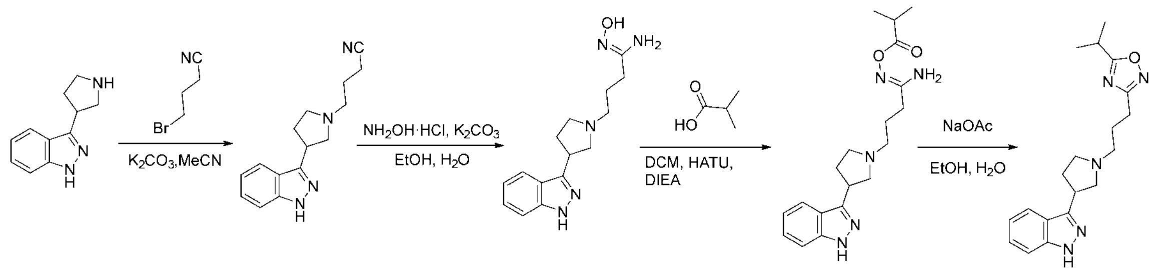


**Step 1:** Preparation of 4-(3-(1H-indazol-3-yl)pyrrolidin-1-yl)butanenitrile:

3-(pyrrolidin-3-yl)-1H-indazole(1.6g, 1eq) was dissolved in acetonitrile (20mL) and N,N-dimethylformamide (10mL), and 4-bromobutanenitrile (1eq) and potassium carbonate (3eq) were was added. The mixture was reacted at 80°C for 5 hours, dried by rotary-evaporation to remove acetonitrile, diluted with water (30mL), extracted with ethyl acetate (40mL×3). The organic phases were washed with saturated brine, dried over anhydrous sodium sulfate to produce the target compound (2g).

LCMS (ESI) $[M+H]^+$=255.2.

**Step 2:** Preparation of (Z)-N'-hydroxy-4-(3-(1H-indazol-3-yl)pyrrolidin-1-yl)butanimidamide:

4-(3-(1H-indazol-3-yl)pyrrolidin-1-yl)butanenitrile (2.2g, 1eq) was dissolved in water (20mL) and ethanol (30mL). Hydroxylamine hydrochloride (3eq) and potassium carbonate (3eq) were added. The mixture was reacted at 70°C for 16 hours, dried by rotary-evaporation to remove ethanol, diluted with water (30mL), extracted with ethyl acetate (30mL×3). The organic phases were dried by rotary-evaporation to give a crude product. The crude product was purified

by reverse-phase chromatography (C18, 0.1%TFA/water, MeCN) to give the target product (2g).

LCMS (ESI) $[M+H]^+$=288.2.

**Step 3:** Preparation of (Z)-N'-(isobutyryloxy)-4-(3-(1H-indazol-3-yl)pyrrolidin-1-yl)butanimidamide:

2-methylpropanoic acid (306.62mg, 1eq) was dissolved in dichloromethane (20mL). HATU (1eq) and N,N-diisopropylethylamine (1.3eq) were added. The reaction mixture was stirred at room temperature for 30 minutes. (Z)-N'-hydroxy-4-(3-(1H-indazol-3-yl)pyrrolidin-1-yl)butanimidamide (1eq) was added. The mixture was reacted for another 2 hours, diluted with water (30mL), and extracted with dichloromethane (20mL×3). The organic phases were washed with saturated brine, dried over anhydrous sodium sulfate to give a crude product. The crude product was purified by flash chromatography (Silica gel, DCM:MeOH=20:1) to give the target product (250mg).

LCMS (ESI) $[M+H]^+$=358.2.

**Step 4:** Preparation of 3-(3-(-3-(1H-indazol-3-yl)pyrrolidin-1-yl)propyl)-5-isopropyl-1,2,4-oxadiazole:

(Z)-N'-(isobutyryloxy)-4-(3-(1H-indazol-3-yl)pyrrolidin-1-yl)butanimidamide (150mg, 1eq) was dissolved in ethanol (5mL) and water (5mL). Sodium acetate (3eq) was added. The mixture was reacted at 80°C for 16 hours, dried by rotary-evaporation to remove ethanol, diluted with water (20mL), extracted with ethyl acetate (15mL×3) to produce a crude product. The crude product was purified by prep-HPLC (C18, 0.1% formic acid/water, MeCN) to give the target product (11.6mg).

LCMS (ESI) $[M+H]^+$=340.2; $^1H$ NMR(400 MHz, MeOD-$d_4$)δ 8.56(s, 1H), 7.79(d, J=8.1 Hz, 1H), 7.49(d, J=8.4 Hz, 1H), 7.38(t, J=7.7 Hz, 1H), 7.14(t, J=7.5 Hz, 1H), 4.13-4.00(m, 1H), 3.74-3.63(m, 1H), 3.56-3.46(m, 1H), 3.43-3.34(m, 2H), 3.18(dd, J=16.4, 9.2 Hz, 3H), 2.85(t, J=7.3 Hz, 2H), 2.58(dd, J=13.5, 6.9 Hz, 1H), 2.35(dd, J=13.4, 7.0 Hz, 1H), 2.22-2.06(m, 2H), 1.37(d, J=7.0 Hz, 6H).

Example 176 (Compound 176)

Preparation of 3-(1-(3-(2H-1,2,3-triazol-2-yl)propyl)azetidin-3-yl)-1H-indole:

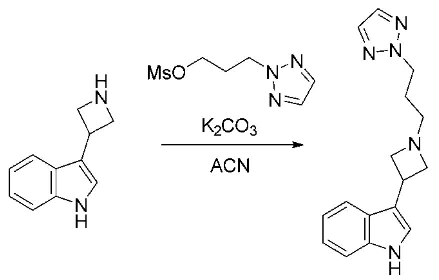


Preparation of 3-(1-(3-(2H-1,2,3-triazol-2-yl)propyl)azetidin-3-yl)-1H-indole:

3-(2H-1,2,3-triazol-2-yl)propyl methanesulfonate (596mg, 1eq) was added to a mixture of 3-(azetidin-3-yl)-1H-indole (1eq) and potassium carbonate (6eq) in acetonitrile (10mL). The mixture was stirred at 70°C for 16 hours, cooled to room temperature, diluted with water (20mL), extracted with dichloromethane (20mL×3). The organic phases were combined, washed with saturated brine (30mL), dried over anhydrous sodium sulfate, and concentrated. The residue was purified with prep-HPLC (C18, 0.1%$NH_3$.$H_2O$/water/MeCN) to produce the target compound (50.34mg).

LCMS (ESI) $[M+H]^+$=282.4; $^1H$ NMR(400 MHz, DMSO-$d_6$)δ 10.86(s, 1H), 7.76(s, 2H), 7.60(d, J=7.9 Hz, 1H), 7.33(d, J=8.1 Hz, 1H), 7.19(d, J=2.1 Hz, 1H), 7.06(t, J=7.5 Hz, 1H), 6.96(t, J=7.4 Hz, 1H), 4.47(t, J=7.0 Hz, 2H), 3.77(dd, J=14.9, 7.4 Hz, 1H), 3.68(t, J=6.9 Hz, 2H), 3.09(t, J=6.9 Hz, 2H), 2.42(t, J=6.9 Hz, 2H), 1.89(dd, J=13.9, 7.0 Hz, 2H).

Example 177 (Compound 177) and Example 178 (Compound 178)

Preparation of (R)-3-(1-(3-(2H-1,2,3-triazol-2-yl)propyl)pyrrolidin-3-yl)-1H-indole (Compound 177) and (S)-3-(1-(3-(2H-1,2,3-triazol-2-yl)propyl)pyrrolidin-3-yl)-1H-indole (Compound 178):

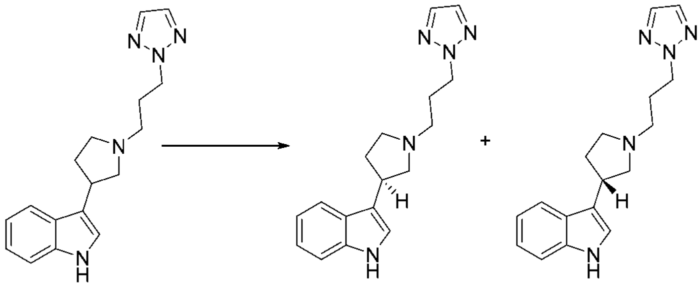

compound 125 compound 177 compound 178

Compound 125 (200mg) was chirally resolved to afford the target compound 177 and compound 178. Chiral resolution conditions: Temperature: room temperature; Mobile phase: $CO_2$/MeOH[0.1%DEA=75/25]; Flow rate: 120mL/min; Detection wavelength: 214nm, the target compound (P3(177 or 178)($t_{major}$=1.905min, ee>97%, 15.8mg), P4(178 or 177)($t_{major}$=2.437min, ee>95%, 14.5mg) were obtained.

Example 179 (Compound 179)

Preparation of 3-(1-(3-(2H-1,2,3-triazol-2-yl)propyl)-1,2,5,6-tetrahydropyridin-3-yl)-1H-indole:

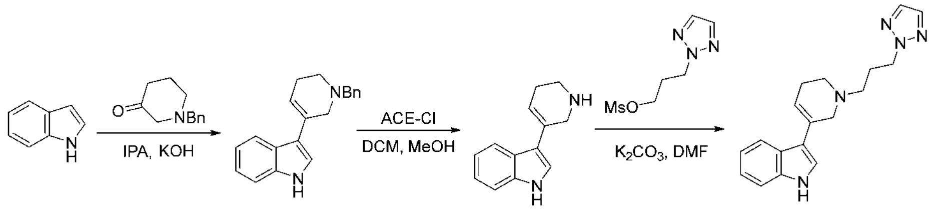


**Step 1:** Preparation of 3-(1-benzyl-1,2,5,6-tetrahydropyridin-3-yl)-1H-indole:

1H-indole (5g, 1eq) was dissolved in isopropanol (50mL). 1-benzylpiperidin-3-one (2eq) and potassium hydroxide (10eq) were added. The mixture was stirred at 80°C for 16 hours, dried by rotary-evaporation to remove isopropanol. Dichloromethane was added to dissolve the product. The resulting mixture was filtered. The filtrate was dried by rotary-evaporation to produce a crude product. The crude product was purified by flash chromatography (Silicagel, dichloromethane:ethyl acetate=5: 1) to produce the target compound (4.5g).

LCMS (ESI) $[M+H]^+$=289.3.

**Step 2:** Preparation of 3-(1,2,5,6-tetrahydropyridin-3-yl)-1H-indole:

3-(1-benzyl-1,2,5,6-tetrahydropyridin-3-yl)-1H-indole (450mg, 1eq) was dissolved in dichloromethane (10mL). Under the protection of nitrogen gas, 1-chloroethyl chloroformate (ACE-Cl) (1.1eq) was added. At room temperature, the mixture was reacted for 5 hours. LCMS detection showed the raw materials disappeared. Methanol (10mL) was added. The mixture was reacted at 70°C for another 5 hours, dried by rotary-evaporation to remove the solvent, purified by reverse-phase chromatography (C18, 0.1%TFA/water, MeCN) to give the target product (70mg).

LCMS (ESI) $[M+H]^+$=199.1.

**Step 3:** Preparation of 3-(1-(3-(2H-1,2,3-triazol-2-yl)propyl)-1,2,5,6-tetrahydropyridin-3-yl)-1H-indole:

3-(1,2,5,6-tetrahydropyridin-3-yl)-1H-indole (60mg, 1eq) was dissolved in N,N-dimethylformamide (10mL). 3-(2H-1,2,3-triazol-2-yl)propyl methanesulfonate (1eq) and potassium carbonate (2eq) were added. The mixture was reacted at 60°C for 16 hours, diluted with water (30mL), extracted with ethyl acetate (30mL×2). The organic phases were washed with saturated brine three times, dried over anhydrous sodium sulfate, dried by rotary-evaporation, purified by Prep-HPLC (C18, 10mmol/L$NH_4HCO_3$/water, MeCN) to give the target product (6.46mg).

LCMS (ESI) $[M+H]^+$=308.2; $^1$H NMR(400 MHz, MeOD-$d_4$)δ 7.78(d, J=8.0 Hz, 1H), 7.70(s, 2H), 7.37(d, J=8.1 Hz, 1H), 7.28(s, 1H), 7.12(t, J=7.5 Hz, 1H), 7.06(d, J=7.2 Hz, 1H), 6.26(d, J=3.8 Hz, 1H), 4.58(t, J=6.7 Hz, 2H), 3.67(d, J=1.7

Hz, 2H), 2.98(t, J=6.0 Hz, 2H), 2.86(dd, J=9.2, 6.6 Hz, 2H), 2.52(d, J=3.7 Hz, 2H), 2.37(dd, J=15.2, 7.2 Hz, 2H).

Example 180 (Compound 180)

Preparation of 3-(1-(3-(2H-1,2,3-triazol-2-yl)propyl)pyrrolidin-3-yl)-1H-indol-4-ol:

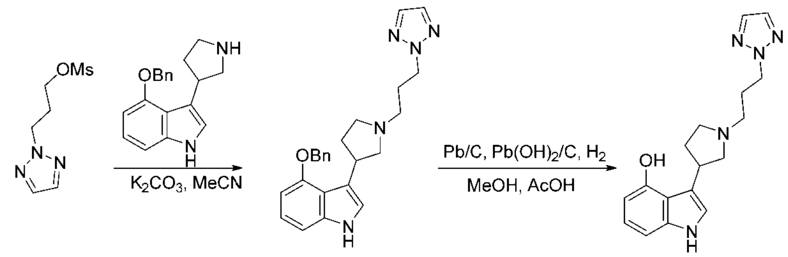


**Step** 1: Preparation of 4-(benzyloxy)-3-(1-(3-(2H-1,2,3-triazol-2-yl)propyl)pyrrolidin-3-yl)-1H-indole:

4-(benzyloxy)-3-(pyrrolidin-3-yl)-1H-indole (400mg, 1eq) and potassium carbonate (3eq) was added to acetonitrile (20mL). 3-(2H-1,2,3-triazol-2-yl)propyl methanesulfonate (1eq) was added. The mixture was reacted at 70°C for 16 hours, and filtered. The filtrate was dried by rotary-evaporation, purified by flash chromatography (Silica gel, dichloromethane:methanol =10:1, volume ratio) to produce the target compound (400mg).
LCMS (ESI) $[M+H]^+$=402.2.

**Step 2:** Preparation of 3-(1-(3-(2H-1,2,3-triazol-2-yl)propyl)pyrrolidin-3-yl)-1H-indol-4-ol:

4-(benzyloxy)-3-(1-(3-(2H-1,2,3-triazol-2-yl)propyl)pyrrolidin-3-yl)-1H-indole (320mg, 1eq) was dissolved in methanol (10mL). Acetic acid (1mL), 10% palladium/carbon (50mg) and 20% palladium hydroxide/carbon (50mg) were added. Under a hydrogen atmosphere, the mixture was reacted at room temperature for 1 hour, filtered, dried by rotary-evaporation to give a crude product. The crude product was purified by Prep-HPLC (C18, 0.1% formic acid/water, MeCN) to give the target product (59.45mg). LCMS (ESI) $[M+H]^+$=312.1; $^1$H NMR(400 MHz, MeOD-$d_4$)δ 8.49(s, 1H), 7.70(s, 2H), 7.02(s, 1H), 6.87(dd, J=18.8, 7.7 Hz, 2H), 6.39(d, J=7.3 Hz, 1H), 4.59(t, J=6.4 Hz, 2H), 4.10-3.95(m, 1H), 3.84-3.72(m, 1H), 3.62-3.45(m, 3H), 3.29-3.21(m, 2H), 2.52-2.33(m, 4H).

Example 181 (Compound 181) and Example 182 (Compound 182)

Preparation of (R)-3-(1-(3-(2H-1,2,3-triazol-2-yl)propyl)pyrrolidin-3-yl)-1H-indol-4-ol (Compound 181) and (S)-3-(1-(3-(2H-1,2,3-triazol-2-yl)propyl)pyrrolidin-3-yl)-1H-indol-4-ol (Compound 182):

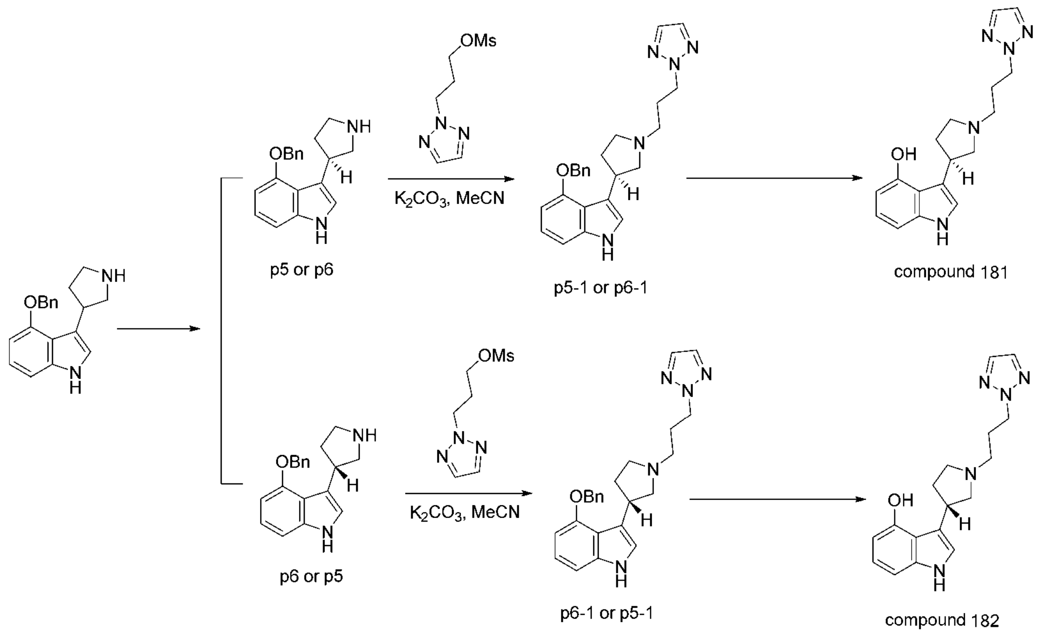

p5 or p6
p5-1 or p6-1
compound 181
p6 or p5
p6-1 or p5-1
compound 182

**Step 1:** Preparation of (R)-4-(benzyloxy)-3-(pyrrolidin-3-yl)-1H-indole and (S)-4-(benzyloxy)-3-(pyrrolidin-3-yl)-1H-indole:

4-(benzyloxy)-3-(pyrrolidin-3-yl)-1H-indole (7g) was chirally resolved. Chiral resolution conditions: Temperature: room temperature; Mobile phase: $CO_2$/IPA[0.1% ammonia/ethanol solution (7M)=85/15]; Flow rate: 120mL/min; Detection wavelength: 214nm. Compounds P5 ($t_{major}$=1.770min, ee>99%, 3.32g) and P6 ($t_{major}$=2.276min, ee>99%, 3.26g) were obtained.

**Step 2:** Preparation of (R)-4-(benzyloxy)-3-(1-(3-(2H-1,2,3-triazol-2-yl)propyl)pyrrolidin-3-yl)-1H-indole and (S)-4-(benzyloxy)-3-(1-(3-(2H-1,2,3-triazol-2-yl)propyl)pyrrolidin-3-yl)-1H-indole:

The compound (P5: 400mg, 1eq) obtained in step 1 and potassium carbonate (3eq) were added to acetonitrile (20mL). 3-(2H-1,2,3-triazol-2-yl)propyl methanesulfonate (1eq) was added. The mixture was reacted at 70°C for 16 hours, and filtered. The filtrate was dried by rotary-evaporation, and purified by flash chromatography (Silica gel, dichloromethane:methanol=10:1) to afford the compound (P5-1: 450mg). P5-1: LCMS (ESI) $[M+H]^+$=402.2.

Using the same preparation method except for replacing the compound P5 with the compound P6 (400mg, 1eq), the compound (P6-1: 440mg) was obtained. P6-1: LCMS (ESI) $[M+H]^+$=402.2.

**Step 3:** Preparation of (R)-3-(1-(3-(2H-1,2,3-triazol-2-yl)propyl)pyrrolidin-3-yl)-1H-indol-4-ol and (S)-3-(1-(3-(2H-1,2,3-triazol-2-yl)propyl)pyrrolidin-3-yl)-1H-indol-4-ol:

The compound (P5-1: 400mg, 1eq) obtained in step 2 was dissolved in methanol (10mL). Acetic acid (1mL), 10% palladium/carbon (50mg) and 20% palladium hydroxide/carbon (50mg) were added. Under a hydrogen atmosphere, the mixture was reacted at room temperature for 1 hour. LCMS detection showed the completion of the reaction. The mixture was filtered, and dried by rotary-evaporation to give a crude product. The crude product was purified by Prep-HPLC (C18, 0.1% formic acid/water, MeCN) to give the target product (181 or 182: 125mg). (181 or 182): LCMS (ESI) $[M+H]^+$=312.2.

Using the same preparation method except for replacing the compound P5-1 with the compound P6-1 (400mg, 1eq), the target compound (182 or 181: 120mg) was obtained. (182 or 181): LCMS (ESI) $[M+H]^+$=312.2.

Example 183 (Compound 183)

Preparation of 2-methyl-3-(1-(3-(2H-1,2,3-triazol-2-yl)propyl)pyrrolidin-3-yl)-1H-indol-4-ol:

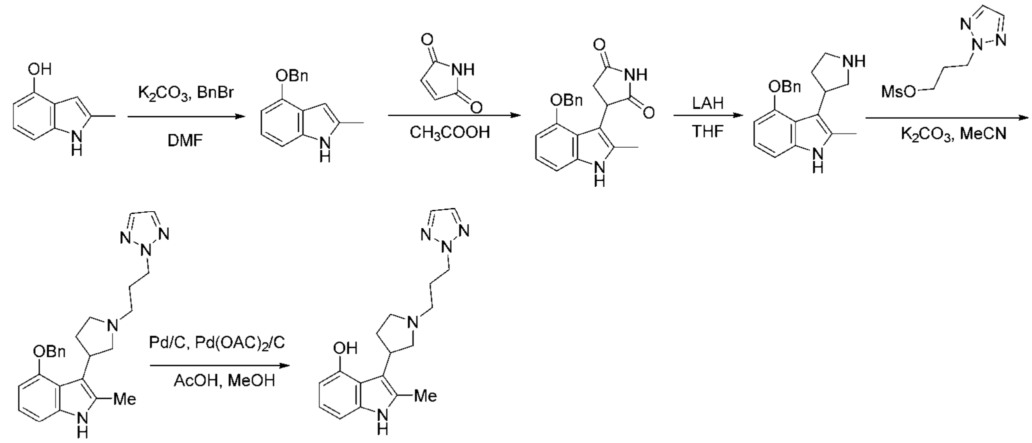


**Step 1:** Preparation of 4-(benzyloxy)-2-methyl-1H-indole:

2-methyl-1H-indol-4-ol (900mg, 1eq), potassium carbonate (2.5g, 3eq) was dissolved in N,N-dimethylformamide (15mL). Benzyl bromide (1.5eq) was added dropwise. The mixture was reacted at room temperature for 16 hours, diluted with water (40mL), extracted with ethyl acetate (40mL×2). The organic phases were washed with saturated brine (30mL×3), dried over anhydrous sodium sulfate to give a crude product. The crude product was purified by flash chromatography (Silica gel, DCM:MeOH=15:1) to give the target product (1.5g, crude).
LCMS (ESI) $[M+H]^+$=238.1.

**Step 2:** Preparation of 3-(4-(benzyloxy)-2-methyl-1H-indol-3-yl)pyrrolidine-2,5-dione:

4-(benzyloxy)-2-methyl-1H-indole (1.5g, crude) and 2,5-dihydro-1H-pyrrole-2,5-dione (1,53g) were dissolved in acetic acid (20mL). The mixture was reacted at 125°C for 16 hours, dried by rotary-evaporation to remove acetic acid, diluted with water (30mL), adjusted with saturated sodium carbonate to pH=9, extracted with ethyl acetate (30mL×3). The organic phases were dried by rotary-evaporation, and slurrized with dichloromethane to produce the target compound (1g). LCMS (ESI) $[M+H]^+$=335.1.

**Step 3:** Preparation of 4-(benzyloxy)-2-methyl-3-(pyrrolidin-3-yl)-1H-indole:

3-(4-(benzyloxy)-2-methyl-1H-indol-3-yl)pyrrolidine-2,5-dione (1g, 1eq) was dissolved in tetrahydrofuran (20mL). Under an ice bath, lithium aluminium hydride (5eq) was slowly added. The mixture was warmed up to 75°C, and reacted for another 16 hours. The reaction mixture was cooled down to room temperature. Under an ice bath, water (1mL), 15% sodium hydroxide solution (1mL), and water (3mL) were added dropwise. Then anhydrous sodium sulfate was added. The resulting mixture was stirred for 20 minutes, and filtered. The filter cake was washed with tetrahydrofuran. The filtrate was dried by rotatary evaporation to give a crude product. The crude product was purified by reverse-phase chromatagraphy (C18, 0.1%TFA/water, MeCN) to produce the target compound (600mg). LCMS (ESI) $[M+H]^+$=307.1.

**Step 4:** Preparation of 4-(benzyloxy)-2-methyl-3-(1-(3-(2H-1,2,3-triazol-2-yl)propyl)pyrrolidin-3-yl)-1H-indole:

4-(benzyloxy)-2-methyl-3-(pyrrolidin-3-yl)-1H-indole (210mg, 1eq) was dissolved in acetonitrile (10mL). 3-(2H-1,2,3-triazol-2-yl)propyl methanesulfonate (1eq) and potassium carbonate (3eq) were added. The mixture was reacted at 70°C for 16 hours, and filtered. The filtrate was dried by rotary-evaporation, and purified by flash chromatography (Silica gel, dichloromethane:methanol=10:1) to give the target product (160mg).
LCMS (ESI) $[M+H]^+$=416.2.

**Step 5:** Preparation of 2-methyl-3-(1-(3-(2H-1,2,3-triazol-2-yl)propyl)pyrrolidin-3-yl)-1H-indol-4-ol:

4-(benzyloxy)-2-methyl-3-(1-(3-(2H-1,2,3-triazol-2-yl)propyl)pyrrolidin-3-yl)-1H-indole (150mg, 1eq) was dissolved in methanol (10mL). Acetic acid (0.5mL), 10% palladium/carbon (10mg) and 20% palladium hydroxide/carbon (10mg) were added. Under a hydrogen atmosphere, the mixture was reacted at room temperature for 20 minutes, filtered, dried by rotary-evaporation, and purified by Prep-HPLC (C18, 0.1% formic acid/water, MeCN) to give the target product (32.44mg).

LCMS (ESI) $[M+H]^+$=326.1; $^1H$ NMR(400 MHz, MeOD-$d_4$)$\delta$ 8.53(s, 1H), 7.72(s, 2H), 6.85(t, J=7.8 Hz, 1H), 6.81-6.75(m, 1H), 6.39(dd, J=7.5, 0.8 Hz, 1H), 4.61(t, J=6.5 Hz, 2H), 3.83(dd, J=11.4, 7.8 Hz, 2H), 3.66-3.47(m, 3H), 3.30-3.21(m, 2H), 2.62-2.48(m, 1H), 2.46-2.38(m, 2H), 2.37-2.27(m, 4H).

Example 184 (Compound 184)

Preparation of 6-fluoro-3-(1-[3-(2H-1,2,3-triazol-2-yl)propyl]pyrrolidin-3-yl)-1H-indol-4-ol:

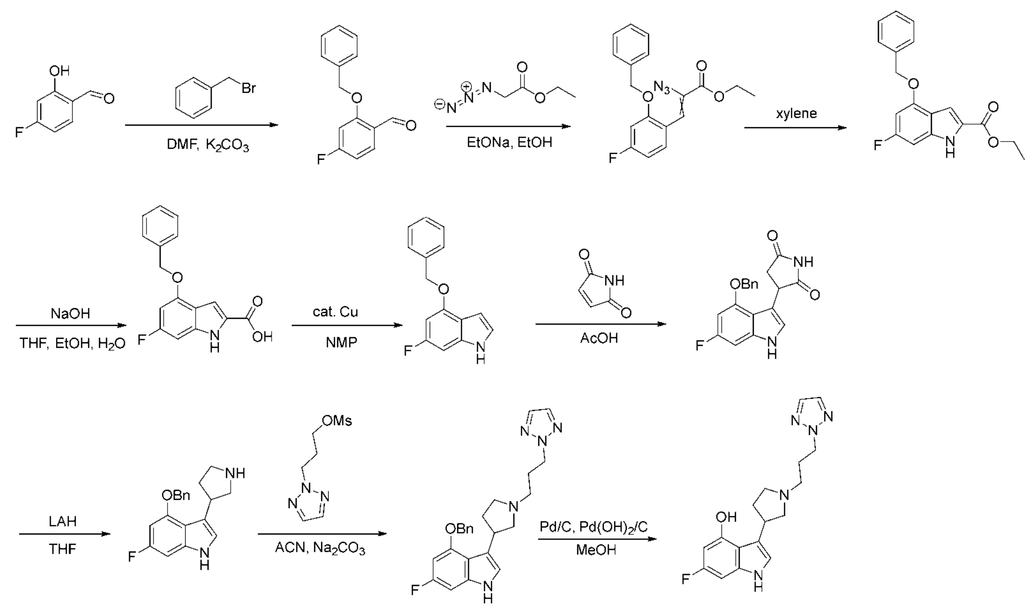


**Step 1:** Preparation of 4-fluoro-2-(phenylmethoxy)benzaldehyde:

4-fluoro-2-hydroxybenzaldehyde (25g, 1eq) was dissolved in N,N-dimethylformamide (300mL). (Bromomethyl) benzene (1.1eq) and potassium carbonate (1.5eq) were added. The mixture was stirred at 80°C for 6 hours. LCMS detection showed the completion of the reaction, and a large amount of the target compound generated. The reaction mixture was slowly poured into ice-water (2L), and a grey solid formed. The mixture was stirred for 20 minutes, and filtered. The filter cake was washed with water (2×100mL) twice, collected, and then dissolved in ethyl acetate (800mL). The organic phases were combined, washed with saturated brine (3x200mL), dried over anhydrous sodium sulfate, filtered, and concentrated to produce the target compound (two parallel batches)(70g).
LCMS (ESI) $[M+H]^+$=231.0.

**Step 2:** Preparation of ethyl 2-azido-3-(2-(benzyloxy)-4-fluorophenyl)acrylate:

Sodium ethoxide (295.57g, 5eq, 20wt% in ethanol) was dissolved in ethanol (500mL). The reaction mixture was cooled down to -20°C. Ethyl 2-azidoacetate (5eq) was slowly added. While kept at this temperature, the mixture was stirred and reacted for 20 minutes. 4-fluoro-2-(phenylmethoxy)benzaldehyde (40g, 1eq) was slowly added dropwise over 60 minutes After the completion of the addition, the mixture was reacted at -8°C for 3 hours. TLC detection showed disappearance of the starting material spot and appearance of a less polar spot. The reaction mixture was slowly added to ice-water, filtered. The filter cake was washed with water (3×200mL), collected (45g, crude), dissolved in xylene (1200mL) and washed with saturated brine. The organic phases were combined, dried over anhydrous sodium sulfate, filtered, and directly used in the next step without further purification, and used directly in the next step without further purification.

**Step 3:** Preparation of ethyl 4-(benzyloxy)-6-fluoro-1H-indole-2-carboxylate:

Ethyl 2-azido-3-(2-(benzyloxy)-4-fluorophenyl)acrylate (45g, crude) was dissolved in xylene (1000mL). The mixture was reacted at 150°C for 3 hours, cooled down to room temperature, and concentrated. A small amount of xylene

was added to the obtained solid. The mixture was filtered. The filter cake was washed with xylene, dried to produce the target compound (20g).

LCMS (ESI) $[M+H]^+$=314.1.

**Step 4:** Preparation of 4-(benzyloxy)-6-fluoro-1H-indole-2-carboxylic acid:

Ethyl 4-(benzyloxy)-6-fluoro-1H-indole-2-carboxylate (12g, 1eq) was dissolved in ethanol /tetrahydrofuran /water (30mL/30mL/30mL). Sodium hydroxide (5eq) was added. The mixture was stirred and reacted for 10 hours. LCMS detection showed the completion of the reaction. The reaction mixture was adjusted with 1N hydrochloric acid to pH=2, and extracted with ethyl acetate (100mL×3). The organic phases were combined, washed with saturated brine, dried over anhydrous sodium sulfate, filtered by suction. The mother liquor was concentrated to produce the target compound (9g).

LCMS (ESI) $[M+H]^+$=286.1.

**Step 5:** Preparation of 4-(benzyloxy)-6-fluoro-1H-indole:

4-(benzyloxy)-6-fluoro-1H-indole-2-carboxylic acid (9g, 1eq) was dissolved in N-methylpyrrolidinone (200mL). Activated copper powder (4eq) was added. The atmosphere was replaced with nitrogen gas three times. In an oil bath, the mixture was stirred and reacted at 200°C for 10 hours. LCMS detection showed the formation of the target compound. The reaction mixture was cooled down to room temperature, cooled with ice-water (300mL), and extracted with ethyl acetate (200mL×3). The organic phases were combined, washed with saturated brine, dried over anhydrous sodium sulfate, and filtered by suction. The mother liquor was concentrated to give a crude product. The crude product was purified by flash chromatography (Silica gel, PE:EtOAc=10:1) to produce the target compound (7g).

LCMS (ESI) $[M+H]^+$=242.1.

**Step 6:** Preparation of 3-(4-(benzyloxy)-6-fluoro-1H-indol-3-yl)pyrrolidine-2,5-dione:

4-(benzyloxy)-6-fluoro-1H-indole (6.7g, 1eq) was dissolved in acetic acid (80mL), and maleimide (4eq) was added. In an oil bath, the mixture was stirred and reacted for 72 hours. LCMS detection showed the completion of the reaction. The reaction mixture was cooled down to room temperature, dried by rotary-evaporation to remove the solvent, diluted with water (80mL), adjusted with saturated sodium carbonate solution (40mL) to pH=9, extracted with ethyl acetate (100mL×3). The organic phases were dried by rotary-evaporation to give a crude product. An appropriate amount of anhydrous dietheylether (30mL) was added. The mixture was stirred and reacted for 30 minutes, and filtered. The filter cake was washed with diethylether (3×5mL), collected, dried by rotary-evaporation to give the target product (5g).

LCMS (ESI) $[M+H]^+$=339.1.

**Step 7:** Preparation of 4-(benzyloxy)-6-fluoro-3-(pyrrolidin-3-yl)-1H-indole:

3-(4-(benzyloxy)-6-fluoro-1H-indol-3-yl)pyrrolidine-2,5-dione (4.5g, 1eq) was dissolved in tetrahydrofuran (15mL). The reaction mixture was cooled down to -10°C. Lithium aluminium hydride (5eq) was slowly added in batch. The mixture was slowly warmed up to 70°C and stirred for 3 hours. LCMS detection showed the completion of the reaction. The reaction mixture was cooled down to - 10°C. Ice-water (2.5mL) was slowly added, then 15% sodium hydroxide solution (2.5mL) was added, and then water (8mL) was added. The mixture was stirred at room temperature for 15 minutes. An appropriate amount of anhydrous magnesium sulfate was added. The mixture was stirred for another 15 minutes, and filtered. The filtrate was dried by rotary-evaporation to give a crude product. The crude product was slurrized with anhydrous diethylether (20mL). The mixture was stirred at room temperature for 10 minutes, and filtered. The filter cake was washed with diethylether (3×5mL) three times, collected, dried by rotary-evaporation to give the target product (2.5g).

LCMS (ESI) $[M+H]^+$=311.1.

**Step 8:** Preparation of 4-(benzyloxy)-6-fluoro-3-(1-[3-(2H-1,2,3-triazol-2-yl)propyl]pyrrolidin-3-yl)-1H-indole:

4-(benzyloxy)-6-fluoro-3-(pyrrolidin-3-yl)-1H-indole (900mg, 1eq) was dissolved in acetonitrile (30mL). Sodium carbonate (3eq) and 3-(2H-1,2,3-triazol-2-yl)propyl methanesulfonate (1eq) were added. The atmosphere was replaced with nitrogen gas. In an oil bath, the mixture was stirred and reacted at 70°C for 3 hours. LCMS detection showed the formation of the target compound. The reaction mixture was cooled down to room temperature, diluted with water (30mL), and extracted with ethyl acetate (3x80mL). The organic phases were combined, washed with saturated brine, dried over anhydrous sodium sulfate, and filtered by suction. The mother liquor was concentrated to give a crude product. The crude product was purified by flash chromatography (Silica gel, DCM:MeOH=10:1) to give the target product (400mg).

LCMS (ESI) $[M+H]^+$=420.2.

**Step 9:** Preparation of 6-fluoro-3-(1-[3-(2H-1,2,3-triazol-2-yl)propyl]pyrrolidin-3-yl)-1H-indol-4-ol:

4-(benzyloxy)-6-fluoro-3-(1-[3-(2H-1,2,3-triazol-2-yl)propyl]pyrrolidin-3-yl)-1H-indole (120mg, 1eq) was dissolved in methanol (10mL). Palladium/carbon (0.1eq, 10% purity) and palladium hydroxide/carbon (0.1eq, 10% purity) were added. The reaction mixture was stirred at room temperature for 1 hour. LCMS detection showed the completion of the reaction. The mixture was filtered. The filtrate was dried by rotary-evaporation to give a crude product. The crude product was concentrated, purified by Prep-HPLC (C18, 10mmol/L $NH_4HCO_3$/water, MeCN) to produce the target compound (19.41mg). LCMS (ESI) $[M+H]^+$=330.2; $^1H$ NMR(400 MHz, $CDCl_3$)$\delta$ 13.58(s, 1H), 7.80(d, J=26.1 Hz, 1H), 7.59(s, 2H), 6.77(d, J=2.3 Hz, 1H), 6.51(dd, J=9.0, 2.2 Hz, 1H), 6.31(dd, J=11.7, 2.2 Hz, 1H), 4.64-4.44(m, 2H), 3.59(dd, J=15.0, 9.3 Hz, 1H), 3.37(dd, J=14.6, 6.8 Hz, 1H), 3.17(d, J=9.6 Hz, 1H), 2.62(dt, J=19.2, 9.8 Hz, 2H), 2.52(s, 1H), 2.43-2.26(m, 3H), 2.19(s, 1H), 2.15-2.04(m, 1H); $^{19}F$ NMR(377 MHz, $CDCl_3$)$\delta$ -119.97(s).

Example 185 (Compound 185) and Example 186 (Compound 186)

Preparation of (R)-3-(1-(3-(4,5-dimethyl-2H-1,2,3-triazol-2-yl)propyl)pyrrolidin-3-yl)-1H-indol-4-ol (Compound 185) and (S)-3-(1-(3-(4,5-dimethyl-2H-1,2,3-triazol-2-yl)propyl)pyrrolidin-3-yl)-1H-indol-4-ol (Compound 186):

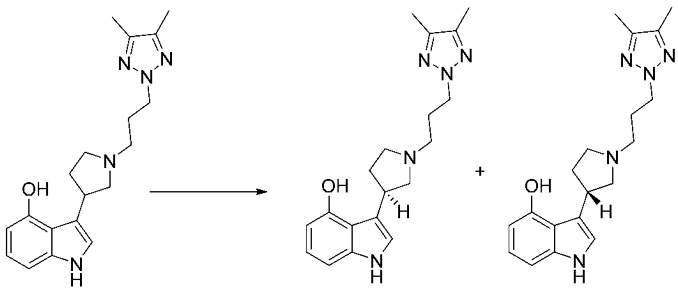

Compound 127 Compound 185 Compound 186

Compound 127 (300mg) was chirally resolved to afford the target compounds 185 and 186. Chiral resolution conditions: Temperature: room temperature; Mobile phase: $CO_2$/IPA[0.1% ammonia in methanol solution (7M)=80/20]; Flow rate: 120mL/min; Detection wavelength: 214nm. P7 (185 or 186)($t_{major}$=2.611min, ee>99%, 57mg) and P8 (186 or 185)($t_{major}$=3.490min, ee>99%, 66.5mg) were obtained.

Example 187 (Compound 187)

Preparation of 3-(1-(3-(4-methyl-2H-1,2,3-triazol-2-yl)propyl)pyrrolidin-3-yl)-1H-indol-4-ol:

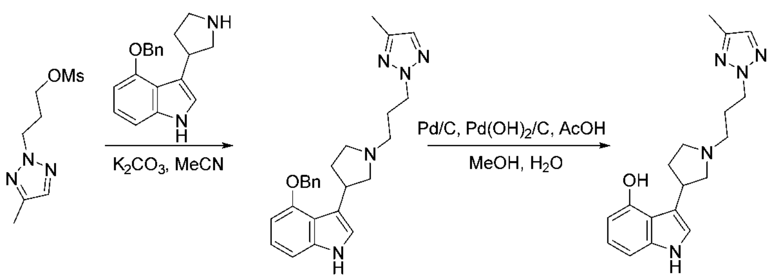


**Step 1:** Preparation of 4-(benzyloxy)-3-(1-(3-(4-methyl-2H-1,2,3-triazol-2-yl)propyl)pyrrolidin-3-yl)-1H-indole:

3-(4-methyl-2H-1,2,3-triazol-2-yl)propyl methanesulfonate (300mg, 1eq) was added to a mixture of 4-(benzyloxy)-3-(pyrrolidin-3-yl)-1H-indole (1eq) and potassium carbonate (3eq) in acetonitrile (10mL). The reaction mixture was

stirred at 70°C for 16 hours. The reaction mixture was cooled to room temperature, and filtered. The filtrate was collected and concentrated in vacuum. The residue was purified by reversed-phase column chromatography (C18, 0.1% formic acid/water, MeCN) to give the target product (300mg).

LCMS (ESI) $[M+H]^+$=416.2.

**Step 2:** Preparation of 3-(1-(3-(4-methyl-2H-1,2,3-triazol-2-yl)propyl)pyrrolidin-3-yl)-1H-indol-4-ol:

Palladium/carbon (20mg) and palladium hydroxide/carbon (20mg) was added to a mixture of 4-(benzyloxy)-3-(1-(3-(4-methyl-2H-1,2,3-triazol-2-yl)propyl)pyrrolidin-3-yl)-1H-indole (200mg, 1eq) in methanol (5mL) and water (5mL). The reaction mixture was stirred at 25°C in a hydrogen atmosphere for 0.5 hours. The reaction was detected by LCMS. The reaction mixture was filtered through diatomite. The filtrate was adjusted to pH=9 with saturated sodium bicarbonate solution, and concentrated in vacuum. The residue was added to dichloromethane (20 mL) to form a slurry. The slurry was filtered. The filtrate was concentrated, and the residue was purified with prep-HPLC (C18, 0.1% formic acid/water, MeCN) to give the target product (12.98mg).

LCMS (ESI) $[M+H]^+$=326.2; $^1$H NMR(400 MHz, DMSO-$d_6$)δ 10.60(s, 1H), 8.22(s, 1H), 7.50(s, 1H), 6.95(d, J=2.3 Hz, 1H), 6.81(t, J=7.8 Hz, 1H), 6.73(dd, J=8.0, 0.9 Hz, 1H), 6.25(dd, J=7.5, 0.8 Hz, 1H), 4.40(t, J=7.0 Hz, 2H), 3.69(d, J=9.0 Hz, 1H), 3.01(t, J=7.4 Hz, 1H), 2.79(d, J=6.5 Hz, 2H), 2.60-2.53(m, 2H), 2.45(s, 1H), 2.33-2.26(m, 1H), 2.22(s, 3H), 2.08(dt, J=14.1, 7.2 Hz, 2H), 1.85(dt, J=15.7, 8.6 Hz, 1H).

Example 188 (Compound 188)

Preparation of 3-(1-(3-(4-cyclopropyl-2H-1,2,3-triazol-2-yl)propyl)pyrrolidin-3-yl)-1H-indol-4-ol:

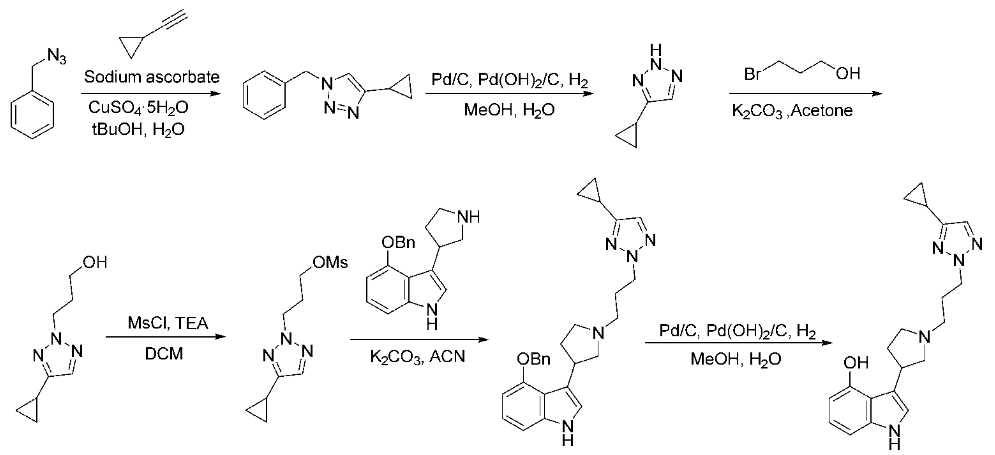


**Step 1:** Preparation of 1-benzyl-4-cyclopropyl-1H-1,2,3-triazole:

Ethynylcyclopropane (1.5eq) was added to a mixture of (azidomethyl)benzene (6g, 1eq), sodium ascorbate (0.1eq), copper sulfate pentahydrate (0.01eq) in tert-butanol (50mL) and water (50mL). The reaction mixture was stirred at room temperature for 16 hours. The reaction was detected by LCMS. The reaction mixture was extracted with water (50mL) and ethyl acetate (100mL×3). The organic phases were combined, washed with saturated brine (200mL), dried, and concentrated. The residue was purified by flash chromatography (Silica gel, PE/EA=3/1) to give the target product (8.93g).

LCMS (ESI) $[M+H+ACN]^+$=241.2.

**Step 2:** Preparation of 4-cyclopropyl-2H-1,2,3-triazole:

Palladium/carbon (1g) and palladium hydroxide/carbon (1g) were added to a mixture of 1-benzyl-4-cyclopropyl-1H-1,2,3-triazole (8g, 1eq) in methanol (40mL) and water (40mL). The resulting mixure was stirred at 60°C in a hydrogen atmosphere for 16 hours. The reaction was detected by LCMS. The reaction mixture was filtered through diatomite. The filtrate was adjusted to pH=9 with saturated sodium bicarbonate solution, and concentrated in vacuum. The residue was added to dichloromethane (50 mL) to form a slurry. The slurry was filtered. The filtrate was concentrated to

produce the target compound (4.05g).

LCMS (ESI) $[M+H]^+$=110.1.

**Step 3:** Preparation of 3-(4-cyclopropyl-2H-1,2,3-triazol-2-yl)propan-1-ol:

3-bromopropan-1-ol (5.09g, 1eq) was added to a mixture of 4-cyclopropyl-2H-1,2,3-triazole (4g, 1eq) and potassium carbonate (1.5eq) in acetone (50mL). The reaction mixture was stirred at 50°C for 8 hours. The reaction was detected by LCMS. The reaction mixture was filtered. The filtrate was concentrated in vacuum. The residue was purified by flash chromatography (Silica gel, DCM/MeOH=10/1) to give the target product (3.12g).

LCMS (ESI) $[M+H]^+$=168.1.

**Step 4:** Preparation of 3-(4-cyclopropyl-2H-1,2,3-triazol-2-yl)propyl methanesulfonate:

At 0°C, methanesulfonyl chloride (1.2eq) was added dropwise to a mixture of 3-(4-cyclopropyl-2H-1,2,3-triazol-2-yl)propan-1-ol (500mg, 1eq) and triethylamine (1.2eq) in dichloromethane (10mL). The reaction mixture was stirred at room temperature for 10 minutes. The reaction was detected by LCMS. The reaction mixture was extracted with water (20mL) and dichloromethane (20mL×3). The organic phases were combined, washed with saturated brine (30mL), dried over anhydrous sodium sulfate, and concentrated to produce the target compound (634mg, crude).

LCMS (ESI) $[M+H]^+$=246.5.

**Step 5:** Preparation of 4-(benzyloxy)-3-(1-(3-(4-cyclopropyl-2H-1,2,3-triazol-2-yl)propyl)pyrrolidin-3-yl)-1H-indole:

3-(4-cyclopropyl-2H-1,2,3-triazol-2-yl)propyl methanesulfonate (537mg, 1eq) was added to a mixture of 4-(benzyloxy)-3-(pyrrolidin-3-yl)-1H-indole (1eq) and potassium carbonate (3eq) in acetonitrile (10mL). The reaction mixture was stirred at 70°C for 16 hours. The reaction was detected by LCMS. The reaction mixture was extracted with water (20mL) and dichloromethane (15mL×3). The organic phases were combined, washed with saturated brine (30mL), dried over anhydrous sodium sulfate, and concentrated. The residue was purified by flash chromatography (Silica gel, DCM/MeOH=10/1) to give the target product (588mg).

LCMS (ESI) $[M+H]^+$=442.7.

**Step 6:** Preparation of 3-(1-(3-(4-cyclopropyl-2H-1,2,3-triazol-2-yl)propyl)pyrrolidin-3-yl)-1H-indol-4-ol:

Palladium/carbon (50mg) and palladium hydroxide/carbon (50mg) were added to a mixture of 4-(benzyloxy)-3-(1-(3-(4-cyclopropyl-2H-1,2,3-triazol-2-yl)propyl)pyrrolidin-3-yl)-1H-indole (500mg, 1eq) in methanol (5mL) and water (5mL). Acetic acid (0.1mL) was added. The reaction mixture was stirred at room temperature in a hydrogen atmosphere for 1 hour. The reaction was detected by LCMS. The reaction mixture was filtered by suction. The filtrate was adjusted to pH 9 with saturated sodium bicarbonate solution, and then extracted with dichloromethane (15mL×2). The organic phases were combined, and concentrated in vacuum. The residue was purified with prep-HPLC (C18, 0.1% $NH_3.H_2O$/water, MeCN) to give the target product (101.69mg).

LCMS (ESI) $[M+H]^+$=352.3; $^1H$ NMR(400 MHz, DMSO-$d_6$)δ 11.49(s, 1H), 10.58(s, 1H), 7.48(s, 1H), 6.93(d, J=2.3 Hz, 1H), 6.82(t, J=7.8 Hz, 1H), 6.73(dd, J=8.0, 0.9 Hz, 1H), 6.25(dd, J=7.5, 0.9 Hz, 1H), 4.41-4.33(m, 2H), 3.72-3.61(m, 1H), 2.97(dd, J=8.5, 6.6 Hz, 1H), 2.75(dd, J=9.1,3.9 Hz, 1H), 2.68(t, J=8.8 Hz, 1H), 2.51(d, J=1.5 Hz, 1H), 2.47(s, 1H), 2.40-2.23(m, 2H), 2.12-2.00(m, 2H), 1.93(tt, J=8.4,5.0 Hz, 1H), 1.88-1.75(m, 1H), 0.95-0.87(m, 2H), 0.72-0.67(m, 2H).

Example 189 (Compound 189)

Preparation of 3-(1-(3-(2H-1,2,3-triazol-2-yl)propyl)pyrrolidin-3-yl)-1H-indazole:

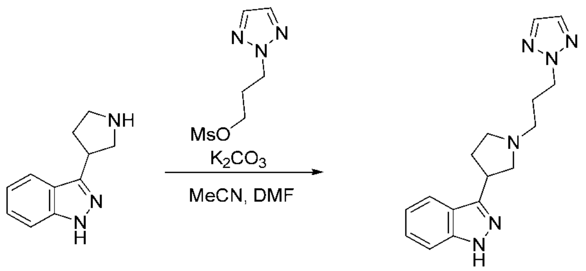


3-(pyrrolidin-3-yl)-1H-indazole (350mg, 1eq) and potassium carbonate (3eq) were dissolved in acetonitrile (15mL) and N,N-dimethylformamide (3mL). 3-(2H-1,2,3-triazol-2-yl)propyl methanesulfonate (1eq) was added. The mixture was reacted at 70°C for 16 hours. The reaction mixture was extracted with water (30mL) and ethyl acetate (20mL×3). The organic phase was washed with saturated brine, rotary-evaporated and dried over anhydrous sodium sulfate, and purified by flash chromatography (Silica gel, DCM:MeOH=20:1) to produce a crude product, which was then purified by Prep-HPLC (C18, 0.1% formic acid/water, MeCN) to give the target product (92.44mg).

LCMS (ESI) $[M+H]^+$=297.1; $^1H$ NMR(400 MHz, MeOD-$d_4$)δ 8.47(s, 1H), 7.78(d, J=8.2 Hz, 1H), 7.71(s, 2H), 7.50(d, J=8.5 Hz, 1H), 7.45-7.33(m, 1H), 7.21-7.08(m, 1H), 4.61(t, J=6.5 Hz, 2H), 4.20-4.07(m, 1H), 3.79(dd, J=11.2, 7.9 Hz, 1H), 3.69(dd, J=11.2, 7.4 Hz, 1H), 3.49(dd, J=14.7, 7.4 Hz, 2H), 3.29-3.23(m, 2H), 2.61(dt, J=14.9, 7.3 Hz, 1H), 2.48-2.30(m, 3H).

## Biological assay

### Assay 1: 5-$HT_{2A}$ receptor isotope competition affinity assay

A radiolabeled ligand (agonist or antagonist) incubated with receptor protein could form a receptor-ligand complex. An added test compound could compete with the isotopically labeled ligand for the receptor binding, eventually forming a relative steady-state equilibrium. The affinity of the test compound for the receptor was indirectly evaluated by measuring the dissociation of the isotopically labeled ligand from the receptor.

Assay procedure

A 100x serial gradient dilution of compounds was prepared in DMSO, starting from 1 mM with 4-fold serial dilutions, resulting in 10 concentration points. Using a pipettor, 1 μL of each gradient concentration of the test compound (final concentration starting at 10 μM, 4-fold serial dilutions, 10 concentration points) was transferred to a 96-well assay plate, to each well of which 49 μL of 5-$HT_{2A}$ receptor protein (102 μg/mL, prepared in an assay buffer: 50 mM Tris-HCl, pH 7.4) had been added in advance (final concentration 5 μg/well). This was followed by the addition of 50 μL of isotopically labeled [$^3$H]-Ketanserin (2 nM, 2× final concentration, prepared in the assay buffer). The plate was sealed and incubated on a shaker at room temperature for 1 hour. The reaction mixture was filtered through a Unifilter-96 GF/C filter plate pre-treated with 0.3% PEI. The filter plate was washed with a precooled (4°C) assay buffer and then dried in an incubator at 50°C. A scintillation liquid was added, and the plate was read using a MicroBeta2 instrument. The percentage inhibition was calculated using the equation: $\%Inhibition=(Count_{High\ Signal\ Control}-Count_{Sample})/(Count_{High\ Signal\ Control}-Count_{Low\ Signal\ Control})*100$. A four-parameter logistic fit was used to calculate the $IC_{50}$ value. High Signal Control: No compound group (the same volume of DMSO substituted). Low Signal Control: Ketanserin group (non-isotopically labeled, 2× final concentration).

Table 1: Results of 5-$HT_{2A}$ receptor isotope competition affinity assay, Ki Values

| Compound | 5-$HT_{2A}$ receptor/Ki (nM) | Compound | 5-$HT_{2A}$ receptor/Ki (nM) |
|---|---|---|---|
| 1 | +++ | 79 | +++ |
| 2 | +++ | 80 | +++ |
| 4 | +++ | 83 | +++ |
| 5 | +++ | 84 | +++ |
| 7 | +++ | 86 | +++ |
| 8 | +++ | 90 | +++ |
| 9 | +++ | 91 | +++ |

(continued)

| Compound | 5-$HT_{2A}$ receptor/Ki (nM) | Compound | 5-$HT_{2A}$ receptor/Ki (nM) |
|---|---|---|---|
| 10 | +++ | 92 | +++ |
| 11 | +++ | 99 | +++ |
| 16 | +++ | 100 | +++ |
| 22 | +++ | 103 | +++ |
| 23 | +++ | 122 | +++ |
| 29 | +++ | 125 | +++ |
| 30 | +++ | 132 | +++ |
| 34 | +++ | 134 | +++ |
| 37 | +++ | 135 | +++ |
| 40 | +++ | 139 | +++ |
| 49 | +++ | 141 | +++ |
| 50 | +++ | 142 | +++ |
| 54 | +++ | 143 | +++ |
| 55 | +++ | 145 | +++ |
| 56 | +++ | 147 | +++ |
| 57 | +++ | 148 | +++ |
| 60 | +++ | 149 | +++ |
| 61 | +++ | 173 | +++ |
| 68 | +++ | 174 | +++ |
| 69 | +++ | 175 | +++ |
| 177 | +++ | 178 | +++ |
| 179 | +++ | 180 | +++ |
| 181 | +++ | 182 | +++ |
| 184 | +++ | 185 | +++ |
| 186 | +++ | 187 | +++ |
| 188 | +++ | 189 | +++ |
| Note: Ki: +++≤100nM, 100nM<++≤500 nM, +>500 nM. | | | |

**Assay 2: 5-$HT_{2A}$ β-arrestin recruitment assay**

Human osteosarcoma cells (U2OS) co-expressing a ProLink™ (PK)-tagged GPCR receptor and an enzyme acceptor (EA)-tagged β-Arrestin were used. Activation of the GPCR-PK induced the recruitment of β-arrestin-EA, forcing the complementation of the two enzyme fragments. The resulting functional enzyme hydrolyzed the substrate, generating a chemiluminescent signal. These cells had been modified using proprietary compounds that did not significantly affect the detection performance, to prevent their long-term propagation and amplification.

Cell Preparation: AssayComplete Cell Plating Reagent was pre-warmed in a 37°C water bath for 15 minutes. 11.5 mL of the pre-warmed AssayComplete Cell Plating Reagent was transferred to a new sterile 15 mL centrifuge tube. 5-$HT_{2A}$ U2OS cells were taken from -80°C or liquid nitrogen and placed on dry ice before thawing. Immediately, 0.5 mL of pre-warmed AssayComplete Cell Plating Reagent was added to the cell cryovial to thaw the cells. 0.6 mL of the thawed cell suspension was transferred to a centrifuge tube to which 23.5 mL of pre-warmed AssayComplete Cell Plating Reagent had been added. The cells were gently mixed and transferred to a disposable sterile dispensing reservoir. 20 μL of the cell suspension was added to each well of a 384-well plate. The 384-well plate was placed in an incubator and cultured for 48 hours at 37°C under 5% $CO_2$.

β-Arrestin2 Assay: Compounds were diluted in a dilution plate using Cell Plating Reagent 0, with 4-fold dilutions

across 10 points. The concentration for each point should be 5× the final concentration. 5 μL of the 5× compound was added per well to the aforementioned 384-well plate containing the cell suspension. The plate was incubated at 37°C for 90 minutes. 19 parts of Cell Assay Buffer, 5 parts of Substrate Reagent 1, and 1 part of Substrate Reagent 2 were added to a 15 mL centrifuge tube and mixed to prepare the detection reagent; 12.5 μL of the detection reagent was added per well; the plate was incubated in the dark at room temperature for 1 hour; readings were taken (Envision).

Day 1: Seeding cells into 10 cm dishes:

HEK293 cells were seeded into 10 cm dishes at $4\times10^6$ cells/dish and cultured in DMEM medium containing 10% dialyzed FBS and 1% PS.

Day 2: Plasmid transfection:

Plasmid A (5-$HT_{2A}$) and plasmid B (β-arrestin-2) were added at a 1:1 ratio to a serum-free Opti-MEM medium;
FuGENE HD reagent was further added to the above Opti-MEM medium and left at room temperature for 15 minutes;
The above mixture was added to the 10 cm dish containing HEK293 cells and gently mixed;
The dish was incubated for 24 hours at 37°C under 5% $CO_2$.

Day 3: Compound treatment and cell harvesting:

Test compounds were prepared as solutions in DMSO and diluted to different gradient concentrations with the assay buffer, then added to the 384-well assay plate;
Cells after 24 hours of transfection were collected from the 10 cm dish, washed twice with the serum-free DMEM medium, and resuspended. The cell concentration was adjusted to $5\times10^5$ cells/mL.

20 μL of the cell suspension (containing $1\times10^4$ cells) was aspirated and seeded into the 384-well assay plate to which the compounds had been added. The assay plate was centrifuged at 1000 rpm for 5 seconds, and then incubated for 24 hours at 37°C under 5% $CO_2$.

Day 4: Chemiluminescence detection:

The required amount of reconstituted Nano-Glo viable cell reagent was prepared. The Nano-Glo viable cell substrate was mixed with Nano-Glo LCS dilution buffer at a 1:19 volume ratio (20× dilution). 5 μL per well of the diluted detection reagent was added to the 384-well assay plate.

Chemiluminescence signals from the assay plate were read, and percentage activity was calculated: %Activity= $(RLU_{Sample}-RLU_{Low\ Signal\ Control})/(RLUHigh_{\ Signal\ Control}-RLU_{Low\ Signal\ Control})*100$, and the $EC_{50}$ value was calculated by fitting. Low Signal Control: DMSO group with no compound, High Signal Control: 1 μM 5-hydroxytryptamine group as positive control.

Assay 3: 5-$HT_{2A}$R calcium flux activation assay

1. Assay materials

1.1 Culture Medium

DMEM medium (Invitrogen, Cat# #11965)
Dialyzed Fetal Bovine Serum (BI, Cat# #040111A)
Hygromycin (Invitrogen, Cat# # 10687010)

1.2 Reagents

Fluo-4 Direct Kit, (Invitrogen, Cat# F10471)

1.3 Instruments

384-well Cell Plate (Greiner, Cat# 781090)
384-well Compound Plate (Greiner, Cat# 781280)
FLIPR Instrument (Molecular Device)
EHCO Instrument (Labcyte)
Cell Counter (Beckman, Vi-cell XR)

2. Compound detection

2.1 Cell preparation

1) For cultured cells (HEK293 cells stably expressing human 5-$HT_{2A}$ receptor), they were first washed twice with pre-warmed DPBS, then an appropriate amount of trypsin was added for digestion at 37°C for 1 minute; an appropriate amount of the culture medium was then added to stop digestion, and 1 mL was taken for counting and vitality determination using Vi-CELL™.
2) Cells were centrifuged at 1000 rpm for 5 minutes.
3) The supernatant was gently discarded, being careful not to disturb the cell pellet.
4) Cells were diluted with the culture medium to $1\times10^6$ cells/mL (20,000 cells/20 μL) and seeded into 384-well poly-lysine coated cell plates. They were cultured at 37°C under 5% $CO_2$ for 16-20 hours.

2.2 Reagent preparation

1) Preparation of 250 mM Probenecid solution: According to the kit instructions, 77 mg of probenecid was added to 1 mL of FLIPR buffer salt solution. Prepared fresh for immediate use.
2) Preparation of Fluo-4 Direct™ (2×, 8 μM) loading buffer: According to the kit instructions, the required amount of Fluo-4 Direct™ was thawed in advance. 10 mL of FLIPR buffer salt solution and 0.2 mL of 250 mM Probenecid solution were added to each vial. It was vortexed in the dark for >5 minutes. Prepared fresh for immediate use.

2.3 Compound testing method

1) Preparation of control and test compound plates: Compounds were gradient diluted to 10 points using Echo, and 750 nL was transferred to the corresponding compound plate.
2) 30 μL of assay buffer salt solution was added to the corresponding compound plate, which was then centrifuged at 1000 rpm for 1 minute;
3) The cell plate prepared the day before was taken from the incubator, and 20 μL of 2× Fluo-4 Direct™ buffer was added to each well, making a total volume of 40 μL.
4) The plate was incubated in a 5% $CO_2$, 37°C incubator for 50 minutes, then left at room temperature for 10 minutes.
5) The compound plate, the cell plate, and pipette tips were placed into the instrument;
6) The FLIPR instrument software was run according to the set program, adding 10 μL of the specified concentrations of test compounds and control compounds to the cell plate, and fluorescence signals were read. After reading, data was exported using the "Max-Min", "Read 1 to 90" method in the software.
7) Data analysis:

$$\text{Agonist Activation\%}=(\text{RLU}-\text{DMSO})/(\text{HC}-\text{DMSO})*100$$

RLU: Relative Light Units, read values 1 to 90;
HC: Average fluorescence signal of the highest concentration point of the agonist;
DMSO: Average fluorescence signal of the DMSO group;

8) Data was fitted using Prism 5.0.

For agonist: $EC_{50}$ was fitted using "log(agonist) vs. response -- Variable slope".

Table 2: 5-$HT_{2A}$R Calcium Flux Activation Assay

| Compound | $EC_{50}$(nM) | Max Dose(nM) | $E_{max}$(%) |
|---|---|---|---|
| 2 | 16.85 | 100000 | 51.46 |
| 54 | 86.53 | 100000 | 46.71 |
| 56 | 8.598 | 100000 | 67.67 |
| 60 | 33.31 | 100000 | 29.94 |
| 49 | 33.98 | 100000 | 47.59 |
| 122 | 24.84 | 100000 | 38.59 |
| 125 | 23.06 | 100000 | 62.89 |
| 126 | 27.67 | 100000 | 49.73 |
| 127 | 3.937 | 100000 | 53.37 |
| 128 | 7.090 | 100000 | 63.11 |
| 134 | 43.34 | 100000 | 68.85 |
| 142 | 45.93 | 100000 | 41.87 |
| 171 | 63.52 | 100000 | 40.25 |
| 180 | 9.954 | 100000 | 59.34 |
| 182 | 21.11 | 100000 | 57.42 |
| 184 | 8.721 | 100000 | 52.44 |
| 187 | 4.175 | 100000 | 63.68 |
| 188 | 10.66 | 100000 | 48.47 |
| 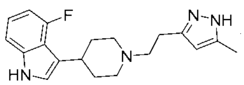 WO9727186A1 | >100000 | 100000 | 0.90 |
| 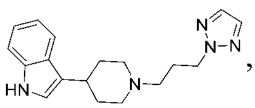, with reference to Examples 4 and 179, replacing 1-benzylpiperidin-3-one with N-benzylpiperidinone | >100000 | 100000 | 25.47 |

Through the testing of compounds for 5-$HT_{2A}$R calcium flux activation, the results indicated that the compounds of the invention moderately activated calcium flux, thereby exerting antidepressant efficacy. The control compound showed no calcium flux activity, indicating no activation effect.

Assay 4: Efficacy evaluation of the compounds of the invention in mouse head-twitch response model and depression model

1. Assay conditions and materials:

1.1 Animal source: Mice (strain C57BL/6, purchased from Shanghai Lingchang Biotechnology Co., Ltd., 8-9 weeks old, 20-24 grams, male)

1.2 Conditions: Temperature (20-25°C), relative humidity (40-70%), light cycle (12-hour light cycle (7:00-19:00)), free access to food and water

1.3 Marking: Animals were marked on the tail.

2. Assay procedure:

Assay 4.1: Effect of compounds in head-twitch response test

A) Adaptation: Starting from the second day after the animals arrived, they were conciliated by stroking and acclimated for seven consecutive days to reduce their stress response.

B) Grouping: One hour before the assay test, animals were placed in the testing room for adaptation. On the test day, animals were randomly grouped according to body weight. Each group contained 12 animals.

C) Assay Dosing: Animal body weight was measured, and compounds were administered according to the group, with a dosing volume of 10 mL/kg for all.

D) Testing: After dosing, animals were placed in transparent observation chambers, and head-twitch behaviors were recorded over 60 minutes.

E) Assay test indicator: Total number of head-twitches within 60 minutes after dosing.

| Compound | Dose (mpk) |
|---|---|
| Vehicle | 10mL/kg |
| DOI | 3 |
| Psilocybin | 2 |
| Compound 49 | 30 |
| Compound 180 | 30 |
| Compound 182 | 30 |

Assay results: The mouse head-twitch response (HTR) test reflected the hallucinogenic effect of compounds. DOI was used as a positive control tool compound; after administration, mice showed significant head-twitch behavior, indicating a hallucinogenic effect. Psilocybin was used as a reference drug; after administration, mice also showed significant head-twitch behavior, indicating a hallucinogenic effect.

Compounds 49, 180 and 182, when administered at doses higher than that of Psilocybin, did not cause significant head-twitch behavior in mice, indicating no hallucinogenic effect. Results are shown in Figure 1.

Assay 4.2: Antidepressant effect of the compound 7 days after single administration in Naive Mice

A) Adaptation: Starting from the second day after the animals arrived, they were conciliated by stroking and acclimated for seven consecutive days to reduce their stress response.

B) Behavioral test (FST test): Compound and Psilocybin groups (15 animals per group) continued to be housed for 7 days after dosing. On day 7, the Ketamine group (15 animals per group) was dosed 30 minutes before being placed, together with the compound and Psilocybin groups, into forced swim observation chambers for 6 minutes. Immobility time during the last 4 minutes was calculated.

C) Assay test indicator: Immobility time of animals during 2-6 minutes of the forced swim test.

The assay results showed that compared to the vehicle control group, compound 180 significantly reduced immobility time in the mouse forced swim test ($p<0.05$). Results are shown in Figure 2. Moreover, in two separate trials, compared to the vehicle group, compound 180 consistently and significantly shortened immobility time in the mouse forced swim test ($p<0.05$), demonstrating stable efficacy.

## Claims

1. A compound represented by the following formula (I), or a stereoisomer thereof, a pharmaceutically acceptable salt thereof, or a mixture thereof:

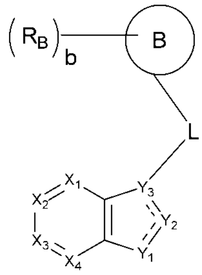

(I)

wherein,

- - - represents a single bond or a double bond, with one and only one being a double bond;
$X_1$ is selected from N or NH, and $CR_{X1}$; $X_2$ is N, or $CR_{X2}$; $X_3$ is N, or $CR_{X3}$; $X_4$ is N, or $CR_{X4}$; and $X_1$, $X_2$, $X_3$, and $X_4$ are not N at the same time;
$Y_1$ is selected from O, S, $NR_{Y11}$, and $CR_{Y12}$;
$Y_2$ is N, or $CR_{Y22}$;
$Y_3$ is N, or C;
and when $Y_3$ is N, a single bond is present between $Y_3$ and $Y_2$; when $Y_3$ is C, a double bond is present between $Y_3$ and $Y_2$;
$R_{X1}$ is selected from hydrogen, deuterium, halogen, -OH, -CN, $-NH_2$, and the following groups that are optionally substituted: $C_1$-$_4$alkyl, $C_1$-$_4$oxaalkyl, and $C_1$-$_4$thiaalkyl; wherein optionally substituted means being unsubstituted or being substituted by one or more substituents selected from deuterium, halogen, oxo, -CN, -OH, and $-NH_2$;
$R_{X2}$ is selected from hydrogen, deuterium, halogen, -OH, -CN, $-NH_2$, and the following groups that are optionally substituted: $C_1$-$_4$alkyl, $C_1$-$_4$oxaalkyl, and $C_1$-$_4$thiaalkyl; wherein optionally substituted means being unsubstituted or being substituted by one or more substituents selected from deuterium, halogen, oxo, -CN, -OH, and $-NH_2$;
$R_{X3}$ is selected from hydrogen, deuterium, halogen, -OH, -CN, $-NH_2$, and the following groups that are optionally substituted: $C_1$-$_4$alkyl, $C_1$-$_4$oxaalkyl, and $C_1$-$_4$thiaalkyl; wherein optionally substituted means being unsubstituted or being substituted by one or more substituents selected from deuterium, halogen, oxo, -CN, -OH, and $-NH_2$;
$R_{X4}$ is selected from hydrogen, deuterium, halogen, and optionally substituted $C_1$-$_4$alkyl; wherein optionally substituted means being unsubstituted or being substituted by one or more substituents selected from deuterium, halogen, oxo, -CN, -OH, and $-NH_2$;
$R_{Y11}$ is selected from hydrogen, deuterium, and optionally substituted $C_{1-3}$alkyl, wherein optionally substituted means being unsubstituted or being substituted by one or more substituents selected from deuterium, halogen, oxo, -CN, -OH, and $-NH_2$;
$R_{Y12}$ is selected from hydrogen, deuterium, and optionally substituted $C_{1-3}$alkyl, wherein optionally substituted means being unsubstituted or being substituted by one or more substituents selected from deuterium, halogen, oxo, -CN, -OH, and $-NH_2$;
$R_{Y22}$ is selected from hydrogen, deuterium, halogen, and optionally substituted $C_{1-3}$alkyl, wherein optionally substituted means being unsubstituted or being substituted by one or more substituents selected from deuterium, halogen, oxo, -CN, -OH, and $-NH_2$;
L is -Z-L'-, -L'-Z-, or -L'-Z-L'-;
L' is selected from the following groups that are optionally substituted: $-C_1$-$_4$alkylene-, $-C_1$-$_4$oxaalkylene-, $-C_1$-$_4$thiaalkylene-, and $-C_1$-$_4$azaalkylene-, wherein optionally substituted means being unsubstituted or being substituted by one or more substituents selected from deuterium, halogen, -CN, -OH, $-NH_2$, $C_{1-3}$alkyl, $C_{1-3}$alkoxy, $C_{1-3}$haloalkyl, and $C_{1-3}$haloalkoxy;
Z is selected from the following groups that are optionally substituted by $R_A$ group(s) in an amount of a: 4-12 membered heterocyclyl, and 4-12 membered heterocycloalkenyl; wherein each $R_A$ is independently selected from deuterium, halogen, -OH, -CN, $-NH_2$, $C_{1-3}$alkyl, $C_{1-3}$alkoxy, halo$C_{1-3}$alkyl, and halo$C_{1-3}$alkoxy;
ring B is $C_{6-14}$aryl, 5-16 membered heterocyclyl, or 5-16 membered heteroaryl;
each $R_B$ is independently selected from deuterium, halogen, -OH, -CN, $-NH_2$, oxo, and the following groups that

are optionally substituted: $C_1$-$_4$alkyl, $C_2$-$_6$alkenyl, $C_2$-$_6$alkynyl, $C_1$-$_4$oxaalkyl, $C_1$-$_4$thiaalkyl, $C_3$-$_6$cycloalkyl, $C_3$-$_6$cycloalkenyl, wherein optionally substituted means being unsubstituted or being substituted by one or more substituents selected from deuterium, halogen, oxo, -CN, -OH, $-NH_2$, $C_1$-$_4$alkyl, and $C_1$-$_4$haloalkyl;
a is 0, 1, 2, or 3;
b is selected from 0, 1, 2, 3, and 4;
unless otherwise specified, the heteroatoms in the above heterocyclyl and heteroaryl are independently selected from O, N and S, with the number of heteroatoms being 1, 2, 3, or 4;
provided that Z is neither

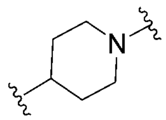 nor 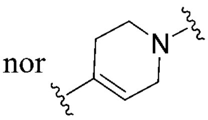 ;

and when $Y_1$ is NH, and $X_4$ is N, L is not

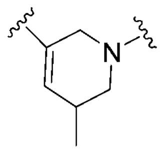 .

2. The compound according to claim 1, or a stereoisomer thereof, a pharmaceutically acceptable salt thereof, or a mixture thereof, wherein

$X_1$, $X_2$, $X_3$, $X_4$ are all not N; or,
$X_1$ is N, $X_2$ is $CR_{X2}$, $X_3$ is $CR_{X3}$, $X_4$ is $CR_{X4}$; or,
$X_2$ is N, $X_1$ is $CR_{X1}$, $X_3$ is $CR_{X3}$, $X_4$ is $CR_{X4}$; or,
$X_3$ is N, $X_1$ is $CR_{X1}$, $X_2$ is $CR_{X2}$, $X_4$ is $CR_{X4}$; or,
$X_4$ is N, $X_1$ is $CR_{X1}$, $X_2$ is $CR_{X2}$, $X_3$ is $CR_{X3}$; or,
$X_2$ and $X_4$ are N, $X_1$ is $CR_{X1}$, $X_3$ is $CR_{X3}$; or,
$X_1$ and $X_3$ are N, $X_2$ is $CR_{X2}$, $X_4$ is $CR_{X4}$.

3. The compound according to any one of claims 1-2, or a stereoisomer thereof, a pharmaceutically acceptable salt thereof, or a mixture thereof, wherein

$Y_1$ is $CR_{Y12}$, $Y_2$ is $CR_{Y22}$, $Y_3$ is N; or,
$Y_1$ is $NR_{Y11}$, $Y_2$ is N or $CR_{Y22}$, $Y_3$ is C; or,
$Y_1$ is $NR_{Y11}$, $Y_2$ is $CR_{Y22}$, $Y_3$ is C; or,
$Y_1$ is O, $Y_2$ is N, $Y_3$ is C.

4. The compound according to any one of claims 1-3, or a stereoisomer thereof, a pharmaceutically acceptable salt thereof, or a mixture thereof, wherein

$R_{X1}$ is selected from hydrogen, deuterium, halogen, -OH, -CN, $-NH_2$, and the following groups that are optionally substituted: $C_1$-$_4$oxaalkyl, and $C_1$-$_4$thiaalkyl; wherein optionally substituted means being unsubstituted or being substituted by one or more substituents selected from deuterium, halogen, oxo, - CN, -OH, and $-NH_2$;
preferably, $R_{X1}$ is selected from hydrogen, deuterium, -F, -Cl, -Br, -OH, -CN, $-NH_2$, and the following groups that are optionally substituted: methoxy, ethoxy, and methylthio; wherein optionally substituted means being unsubstituted or being substituted by one or more substituents selected from deuterium, halogen, oxo, -CN, -OH, and $-NH_2$;
more preferably, $R_{X1}$ is selected from hydrogen, -F, -OH, and $-OCH_3$;
$R_{X2}$ is selected from hydrogen, deuterium, halogen, -OH, -CN, $-NH_2$, and the following groups that are optionally substituted: $C_1$-$_4$oxaalkyl, and $C_1$-$_4$thiaalkyl; wherein optionally substituted means being unsubstituted or being substituted by one or more substituents selected from deuterium, halogen, oxo, - CN, -OH, and $-NH_2$;
preferably, $R_{X2}$ is selected from hydrogen, deuterium, -F, -Cl, -Br, -OH, -CN, $-NH_2$, and the following groups that are optionally substituted: methoxy, ethoxy, and methylthio; wherein optionally substituted means being unsubstituted or being substituted by one or more substituents selected from deuterium, halogen, oxo, -CN, -OH,

and $-NH_2$;
more preferably, $R_{X2}$ is selected from hydrogen, -F, and $-OCH_3$; $R_{X3}$ is selected from hydrogen, deuterium, halogen, -OH, -CN, $-NH_2$, and the following groups that are optionally substituted: $C_{1-4}$oxaalkyl, and $C_{1-4}$thiaalkyl; wherein optionally substituted means being unsubstituted or being substituted by one or more substituents selected from deuterium, halogen, oxo, -CN, -OH, and $-NH_2$; further preferably, $R_{X2}$ is selected from hydrogen, -F, and $-OCH_3$;
more further preferably, $R_{X2}$ is selected from hydrogen;
$R_{X3}$ is selected from hydrogen, deuterium, -F, -Cl, -Br, -OH, -CN, $-NH_2$, and the following groups that are optionally substituted: methoxy, ethoxy, and methylthio; wherein optionally substituted means being unsubstituted or being substituted by one or more substituents selected from deuterium, halogen, oxo, - CN, -OH, and $-NH_2$;
preferably, $R_{X3}$ is selected from hydrogen, -F, and $-OCH_3$;
more preferably, $R_{X3}$ is hydrogen;
$R_{X4}$ is selected from hydrogen, deuterium, halogen, and the following groups that are optionally substituted: methyl, and ethyl; wherein optionally substituted means being unsubstituted or being substituted by one or more substituents selected from deuterium, halogen, oxo, -CN, -OH, and $-NH_2$; preferably, $R_{X4}$ is selected from hydrogen, deuterium, -F, -Cl, and -Br;
more preferably, $R_{X4}$ is hydrogen.

**5.** The compound according to any one of claims 1-4, or a stereoisomer thereof, a pharmaceutically acceptable salt thereof, or a mixture thereof, wherein

$R_{Y11}$ is selected from hydrogen, deuterium, and the following groups that are optionally substituted: methyl, and ethyl, wherein optionally substituted means being unsubstituted or being substituted by one or more substituents selected from deuterium, halogen, oxo, -CN, -OH, and $-NH_2$; preferably, $R_{Y11}$ is selected from hydrogen and methyl;
preferably, $R_{Y11}$ is hydrogen; $R_{Y22}$ is selected from hydrogen, deuterium, -F, -Cl, -Br, and the following groups that are optionally substituted: methyl, and ethyl, wherein optionally substituted means being unsubstituted or being substituted by one or more substituents selected from deuterium, halogen, oxo, - CN, -OH, and $-NH_2$;
more preferably, $R_{Y22}$ is selected from hydrogen, -Br, and methyl;
further preferably, $R_{Y22}$ is hydrogen.

**6.** The compound according to any one of claims 1-5, or a stereoisomer thereof, a pharmaceutically acceptable salt thereof, or a mixture thereof,

wherein L is -Z-L'-, -L'-Z-, or -L'-Z-L'-;
L' is selected from the following groups that are optionally substituted: $-C_{1-4}$alkylene-, and $-C_{1-4}$oxaalkylene-; wherein optionally substituted means being unsubstituted or being substituted by one or more substituents selected from deuterium, halogen, -CN, -OH, $-NH_2$, $C_{1-3}$alkyl, $C_{1-3}$alkoxy, and halo$C_{1-3}$alkyl;
preferably, L' is

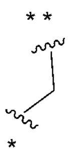 , 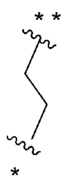 , 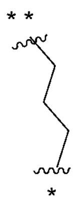 , 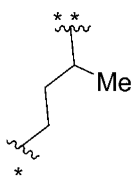 , 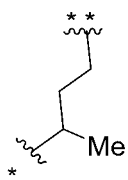 , 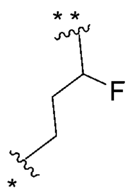 , or 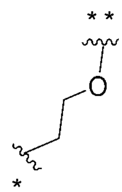 ;

** represents
the attachment point to ring B; * represents the attachment point to ring Z;
Z is selected from the following groups that are optionally substituted by $R_A$ group(s) in an amount of a: 4-10 membered heterocyclyl, and 4-10 membered heterocycloalkenyl; wherein $R_A$ is selected from deuterium, halogen, -CN, $-NH_2$, $C_{1-3}$alkyl, $C_{1-3}$alkoxy, halo$C_{1-3}$alkyl, and halo$C_{1-3}$alkoxy;
preferably, Z is selected from the following groups that are optionally substituted by $R_A$ group(s) in an amount of a:

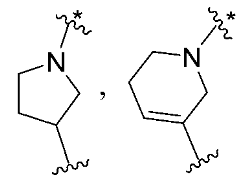;

* represents the attachment point to L'; or preferably, Z is

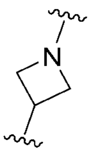, 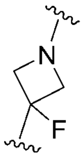, 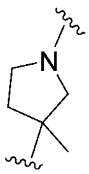, 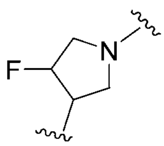, 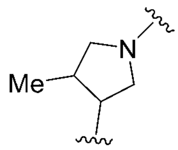, 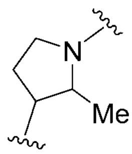, 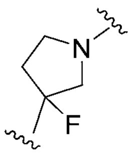,

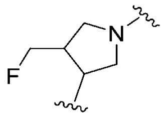, 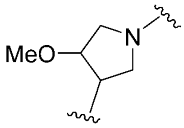, 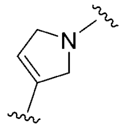, 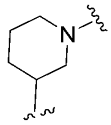, 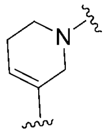, 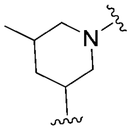, 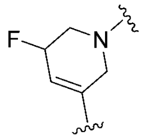,

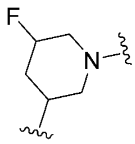, 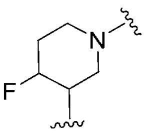, 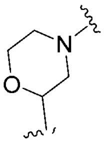, 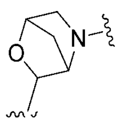, 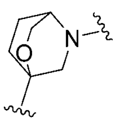, 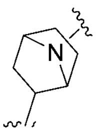, 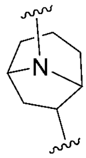, 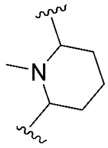,

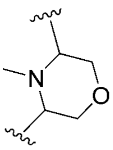, 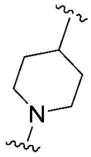, 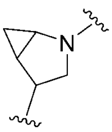, 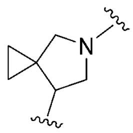, 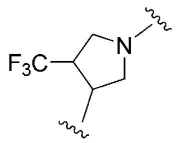, 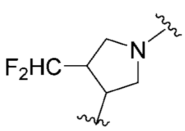, 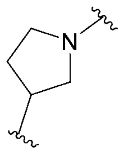,

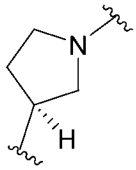, 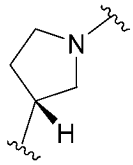, 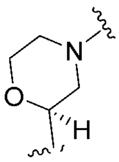, or 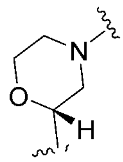;

further preferably, L is

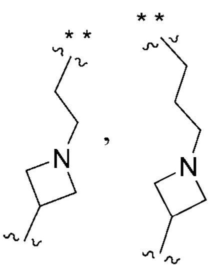, 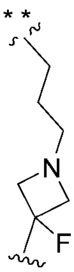, 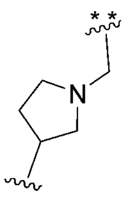, 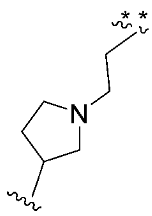, 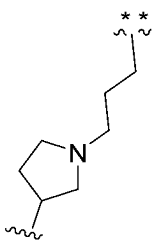, 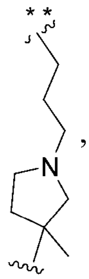,

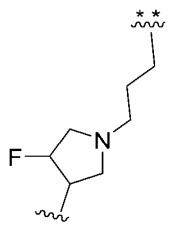, 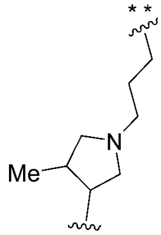, 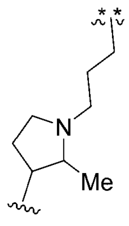, 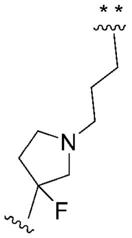, 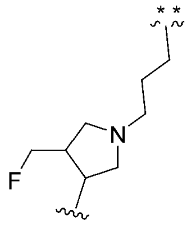, 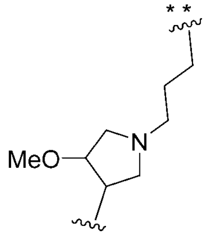,

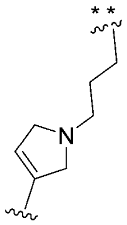, 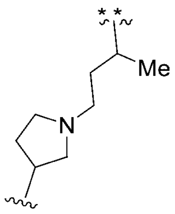, 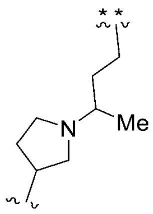, 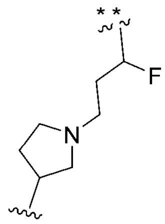, 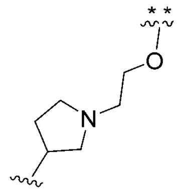, 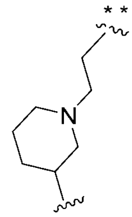,

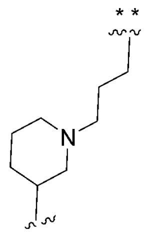, 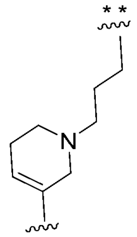, 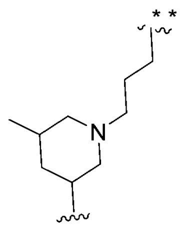, 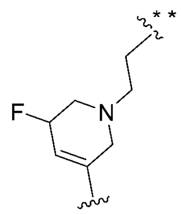, 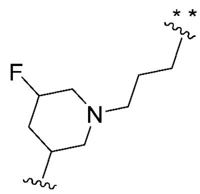, 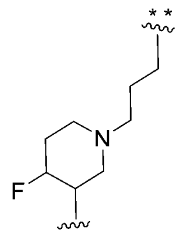,

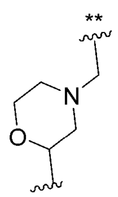, 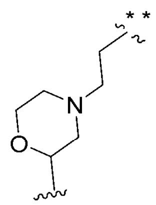, 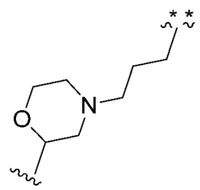, 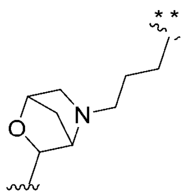, 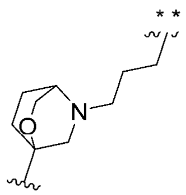, 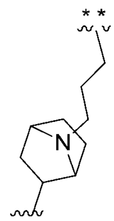,

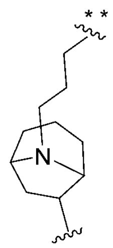, 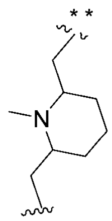, 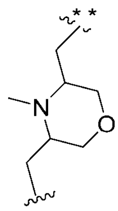, 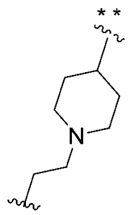, 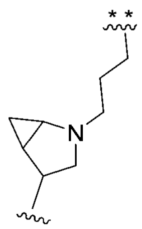, 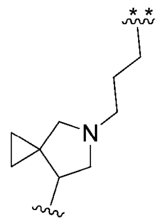, 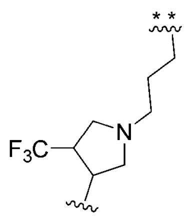,

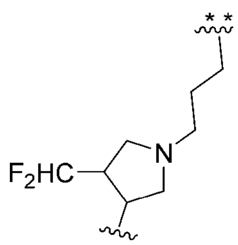, 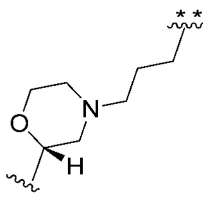, 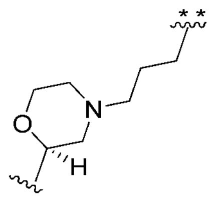, 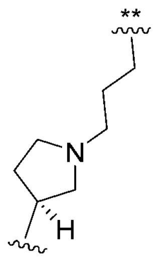, or 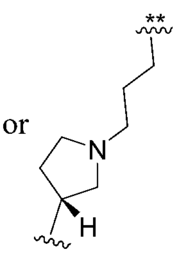;

** represents the attachment point to ring B.

**7.** The compound according to any one of claims 1-6, or a stereoisomer thereof, a pharmaceutically acceptable salt thereof, or a mixture thereof, wherein

ring B is phenyl, 5-12 membered heterocyclyl, or 5-12 membered heteroaryl; e.g., ring B is phenyl, or 5-membered monocyclic heteroaryl;
preferably, ring B is

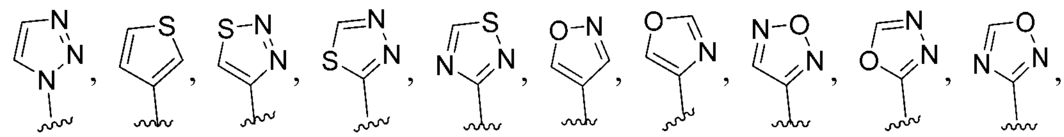

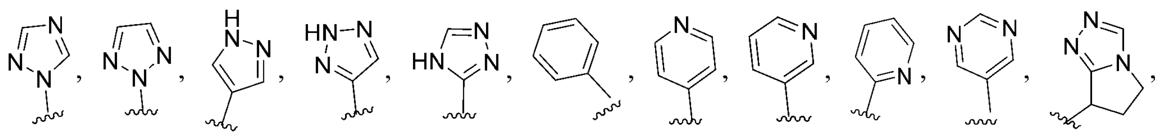

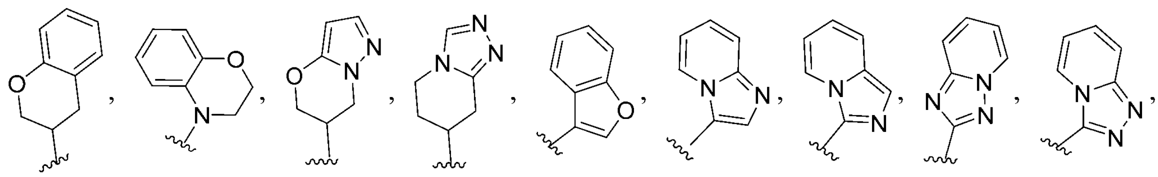

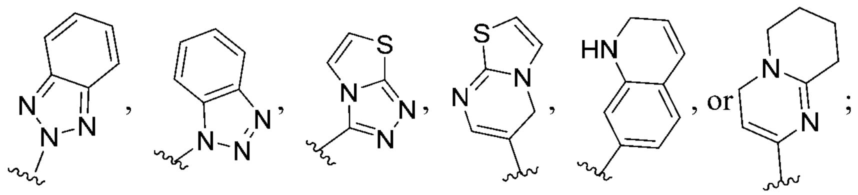

preferably, ring B is

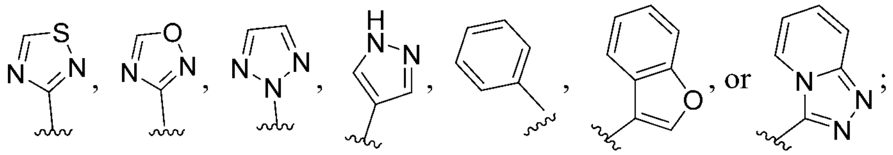

preferably, ring B is

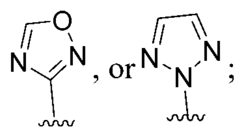

wherein, each $R_B$ is independently selected from deuterium, halogen, -OH, -CN, $-NH_2$, oxo, and the following groups that are optionally substituted: $C_{1-4}$alkyl, $C_{1-4}$oxaalkyl, $C_{1-4}$thiaalkyl, and $C_{3-6}$cycloalkyl, wherein optionally substituted means being unsubstituted or being substituted by one or more substituents selected from deuterium, halogen, oxo, -CN, -OH, $-NH_2$, $C_{1-4}$alkyl, and $C_{1-4}$haloalkyl; preferably, each $R_B$ is independently selected from F, CN, oxo, methyl, cyclopropyl, cyclobutyl, methoxy, trifluoromethyl,

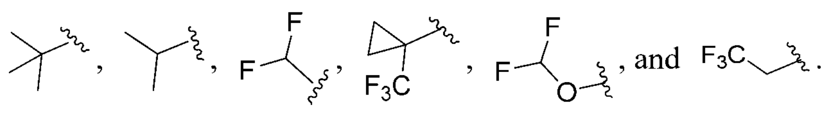

**8.** The compound according to any one of claims 1-7, or a stereoisomer thereof, a pharmaceutically acceptable salt thereof, or a mixture thereof, wherein

b is 0, 1, 2, or 3; preferably, b is 0, 1, or 2; and/or
a is 0, 1, or 2; preferably, a is 0 or 1; more preferably, a is selected from 0.

**9.** The compound according to any one of claims 1-8, or a stereoisomer thereof, a pharmaceutically acceptable salt thereof, or a mixture thereof, wherein said compound is a compound represented by the following formula (II):

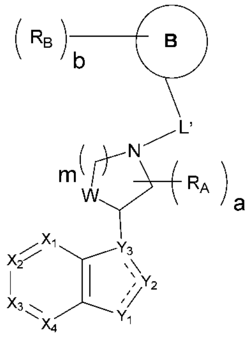
(II)

wherein, $X_1$, $X_2$, $X_3$, $X_4$, $Y_1$, $Y_2$, $Y_3$, L', ring B, $R_B$, $R_A$, a, and b are as defined for the compound of formula (I) in any one of claims 1-8;
W is $-CH_2-$, -O-, -S-, -NH-;
each $R_A$ is independently selected from deuterium, halogen, -OH, oxo, -CN, $-NH_2$, $C_{1-3}$alkyl, $C_{1-3}$alkoxy, halo$C_{1-3}$alkyl, halo$C_{1-3}$alkoxy;
m is 0, 1, 2, or 3.

**10.** The compound according to any one of claims 1-9, or a stereoisomer thereof, a pharmaceutically acceptable salt thereof, or a mixture thereof, wherein said compound is a compound represented by any one of the following formulae (III-1) to (III-8):

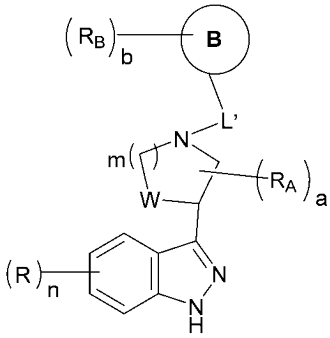
(III-1)

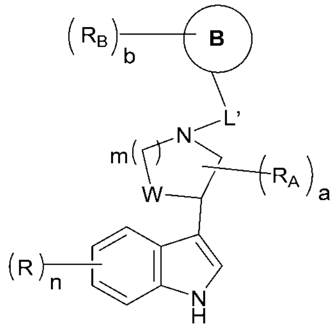
(III-2)

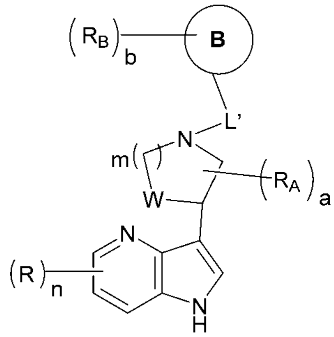
(III-3)

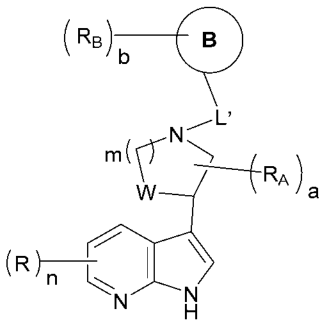
(III-4)

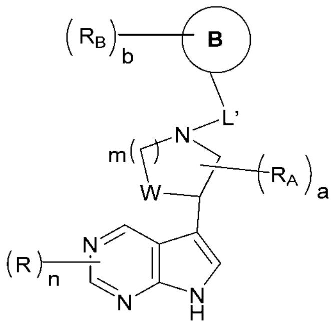
(III-5)

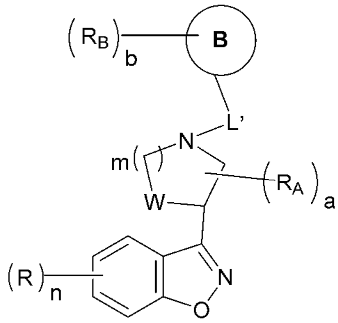
(III-6)

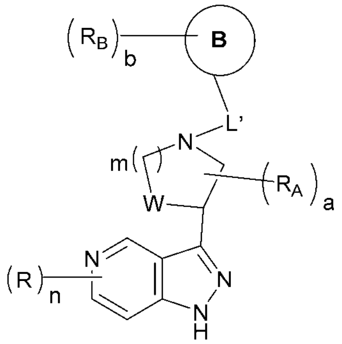
(III-7)

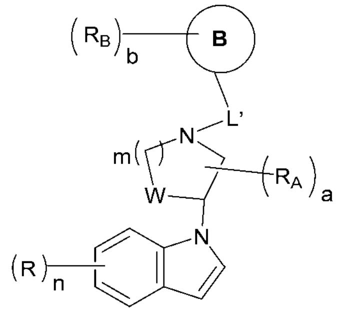
(III-8)

wherein, L', ring B, $R_B$, b, W, $R_A$, a, and m are as defined for the compound of formula (I) or (II) in any one of claims 1-9;
R is selected from deuterium, halogen, -OH, -CN, $-NH_2$, and the following groups that are optionally substituted: $C_{1-4}$alkyl, $C_{1-4}$oxaalkyl, and $C_{1-4}$thiaalkyl; wherein optionally substituted means being unsubstituted or being substituted by one or more substituents selected from deuterium, halogen, oxo, - CN, -OH, and $-NH_2$;
n is 0, 1, 2, or 3.

**11.** The compound according to any one of claims 1-10, or a stereoisomer thereof, a pharmaceutically acceptable salt thereof, or a mixture thereof, wherein said compound is a compound represented by any one of the following formulae (A1) to (A16):

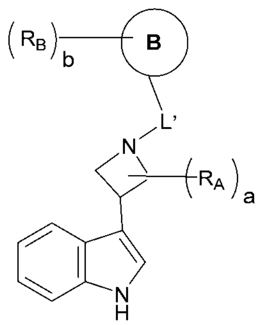
(A1)

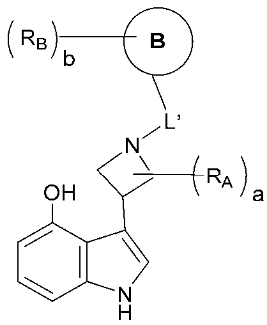
(A2)

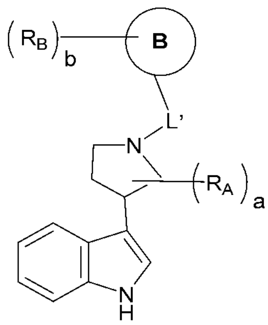
(A3)

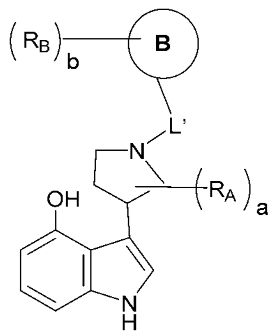
(A4)

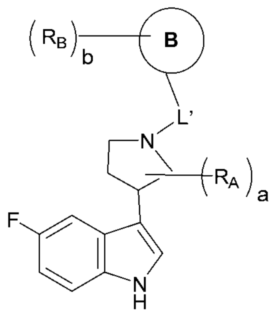
(A5)

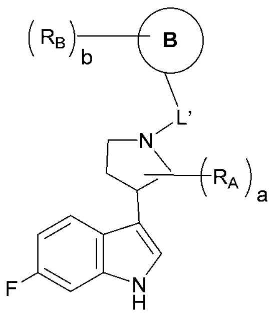
(A6)

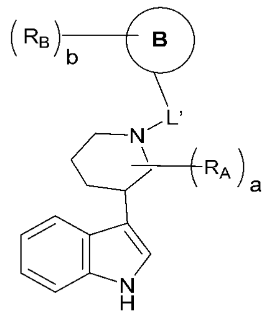
(A7)

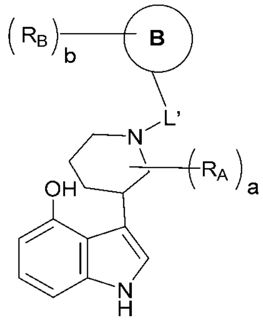
(A8)

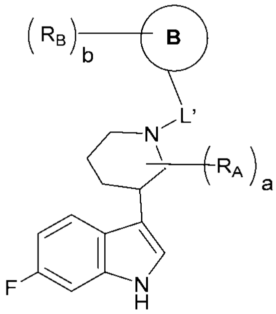
(A9)

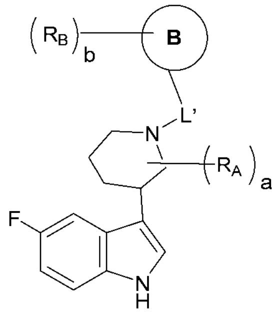
(A10)

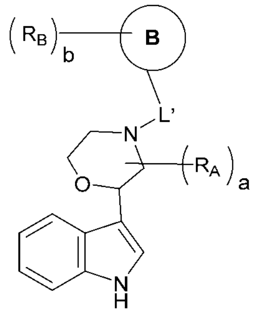
(A11)

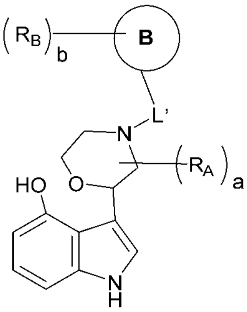
(A12)

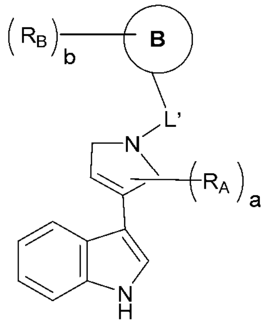
(A13)

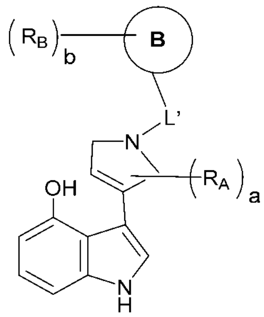
(A14)

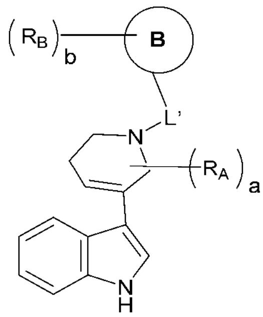
(A15)

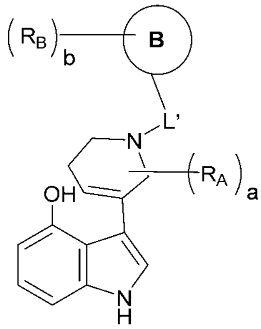
(A16)

wherein, L', ring B, $R_B$, b, $R_A$, and a are as defined for the compound of formulae (I), (II), or (III-1) to (III-8) in any one of claims 1-10;

preferably, ring B is phenyl, or 5-membered monocyclic heteroaryl; or, ring B is

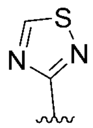,
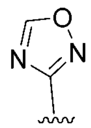,

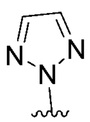,
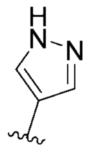,
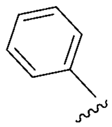,
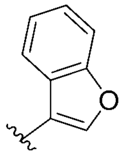, or
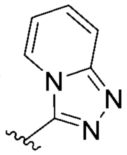.

**12.** The compound according to any one of claims 1-10, or a stereoisomer thereof, a pharmaceutically acceptable salt thereof, or a mixture thereof, wherein said compound is a compound represented by any one of the following formulae (a)-(b):

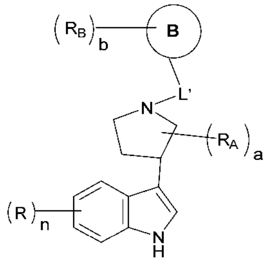
(a)

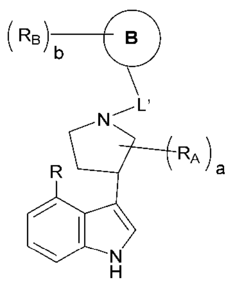
(b)

wherein, R, L', ring B, $R_A$, $R_B$, n, a, and b are as defined for the compound of formula (I), (II), or (III-2) in any one of

claims 1-10;
preferably, R is -OH or methoxy; preferably, L' is an optionally substituted -propylene-; preferably, ring B is phenyl,

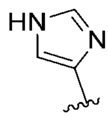, 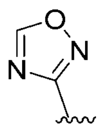, 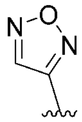, 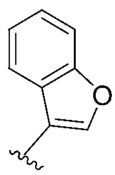, 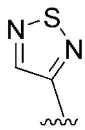, or 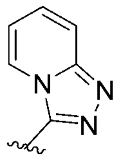;

or preferably, ring B is phenyl,

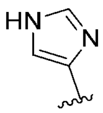, 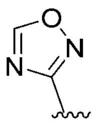, 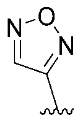, 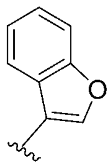, 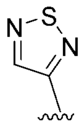, 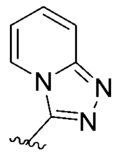, or 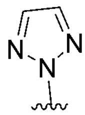, e.g. 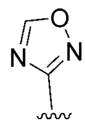, or 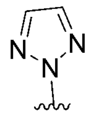.

**13.** The compound according to any one of claims 1-10, or a stereoisomer thereof, a pharmaceutically acceptable salt thereof, or a mixture thereof, wherein said compound is a compound represented by any one of the following formulae (c)-(d):

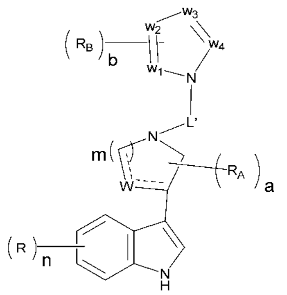

(c)

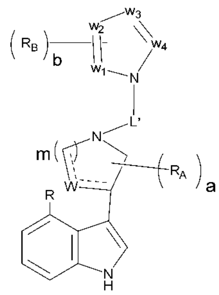

(d)

wherein, R, L', ring B, $R_A$, $R_B$, n, a, and b are as defined for the compound of formula (I), (II), or (III-2) in any one of claims 1-10;
$w_1$, $w_2$, $w_3$, and $w_4$ are each independently selected from CH and N;
W is -CH, $-CH_2-$, -O-, or -S-;
two - - -s represent a single bond or a double bond, but the two are not a double bond at the same time; when any - - - is a double bond, W is -CH-.

**14.** A compound, or a stereoisomer thereof, a pharmaceutically acceptable salt thereof, or a mixture thereof, wherein said compound is selected from

| No. | Structure | No. | Structure | No. | Structure |
|---|---|---|---|---|---|
| 1 | 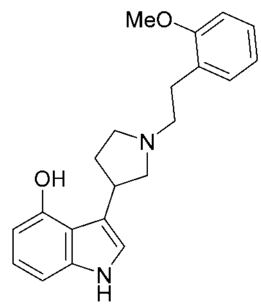 | 2 | 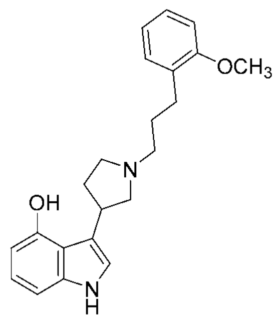 | 3 | 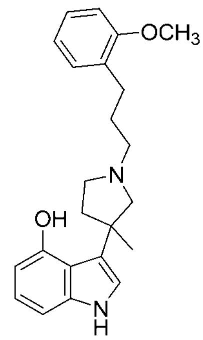 |

(continued)

| No. | Structure | No. | Structure | No. | Structure |
|---|---|---|---|---|---|
| 4 | 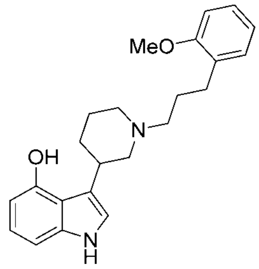 | 5 | 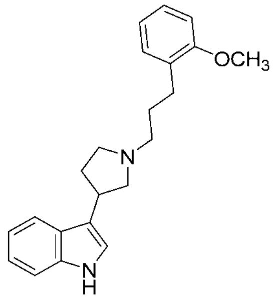 | 6 | 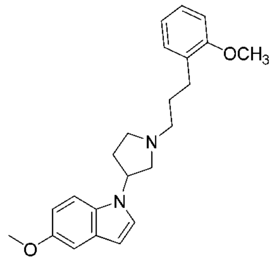 |
| 7 | 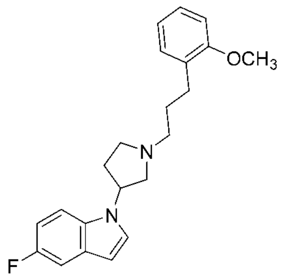 | 8 | 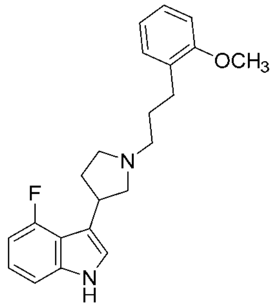 | 9 | 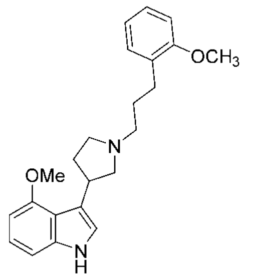 |
| 10 | 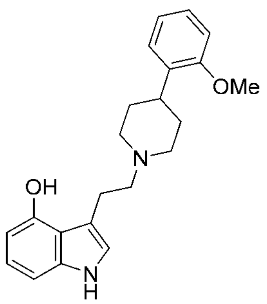 | 11 | 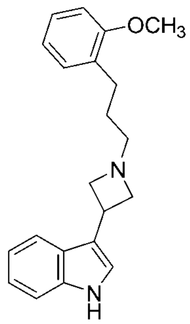 | 12 | 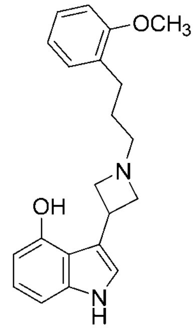 |
| 13 | 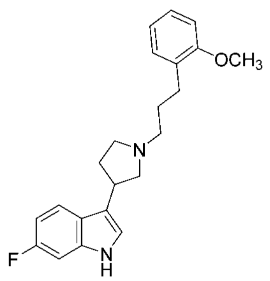 | 14 | 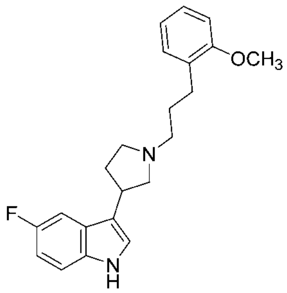 | 15 | 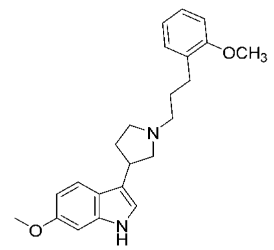 |
| 16 | 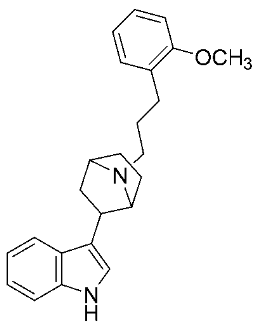 | 17 | 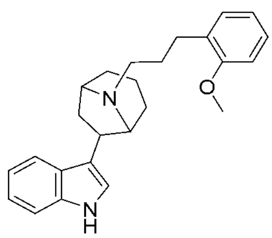 | 18 | 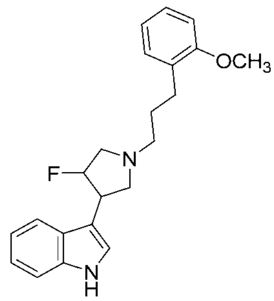 |

(continued)

| No. | Structure | No. | Structure | No. | Structure |
|---|---|---|---|---|---|
| 19 | 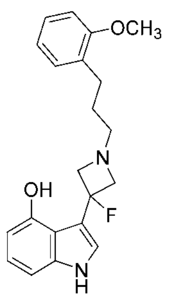 | 20 | 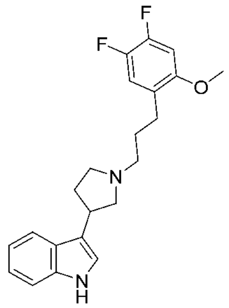 | 21 | 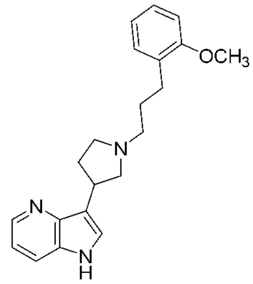 |
| 22 | 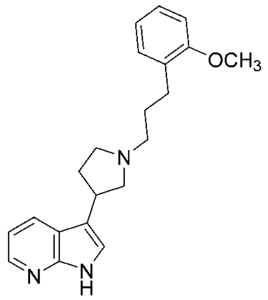 | 23 | 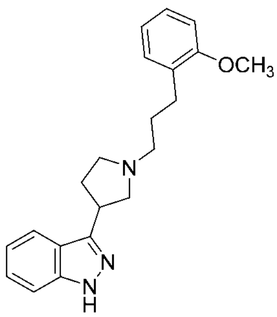 | 24 | 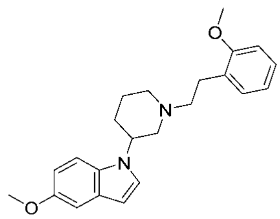 |
| 25 | 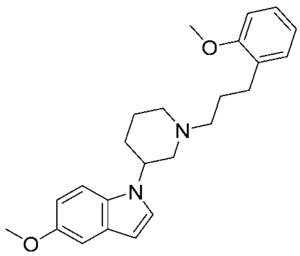 | 26 | 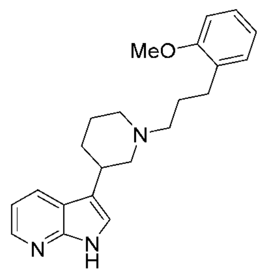 | 27 | 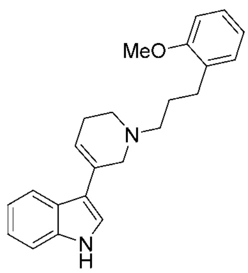 |
| 28 | 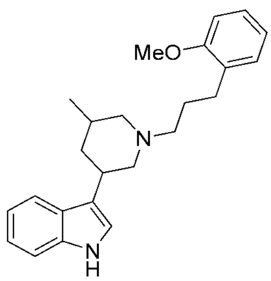 | 29 | 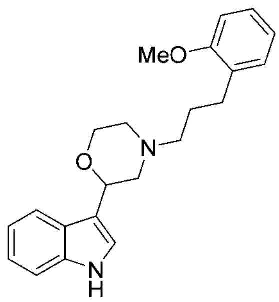 | 30 | 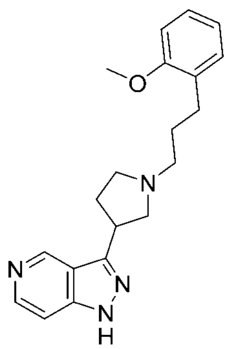 |
| 31 | 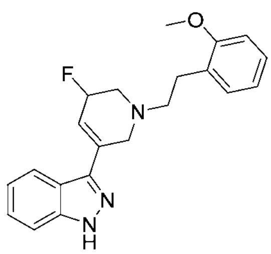 | 32 | 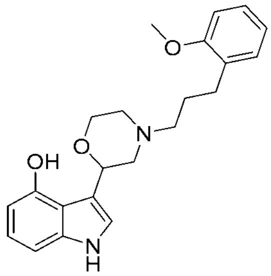 | 33 | 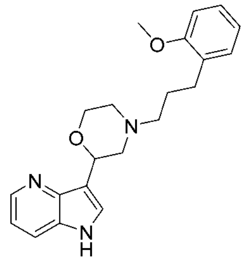 |

(continued)

| No. | Structure | No. | Structure | No. | Structure |
|---|---|---|---|---|---|
| 34 | 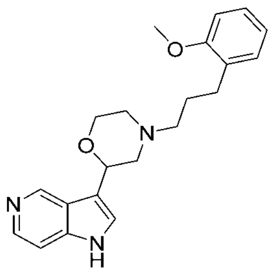 | 35 | 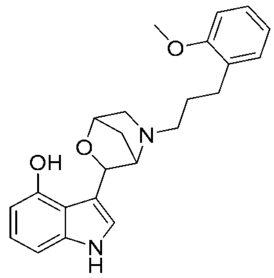 | 36 | 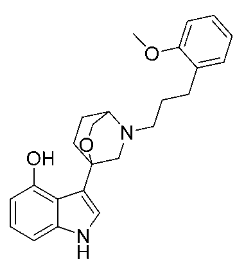 |
| 37 | 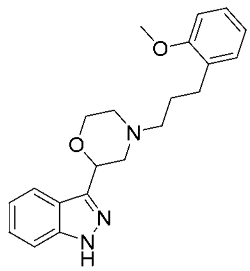 | 38 | 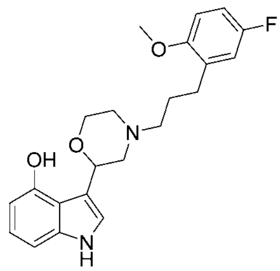 | 39 | 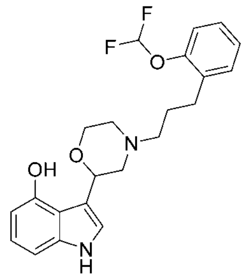 |
| 40 | 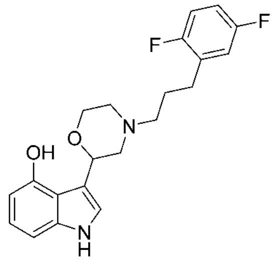 | 41 | 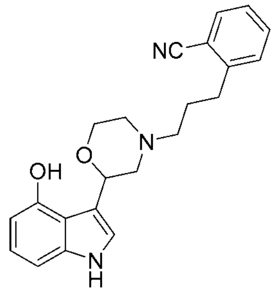 | 42 | 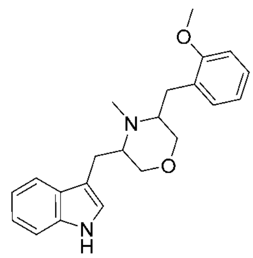 |
| 43 | 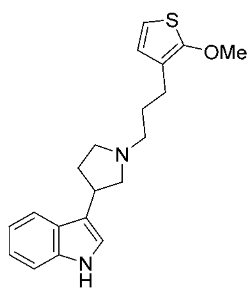 | 44 | 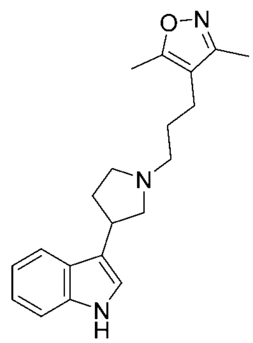 | 45 | 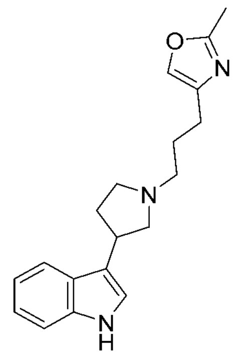 |
| 46 | 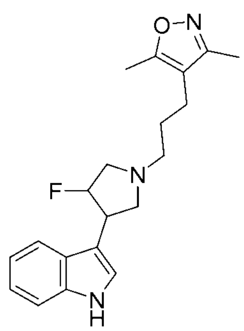 | 47 | 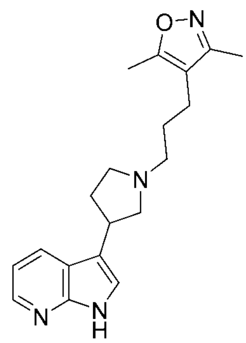 | 48 | 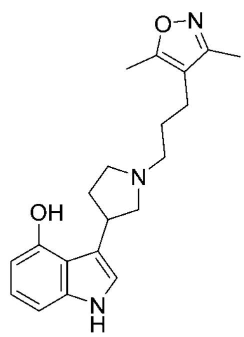 |

(continued)

| No. | Structure | No. | Structure | No. | Structure |
|---|---|---|---|---|---|
| 49 | 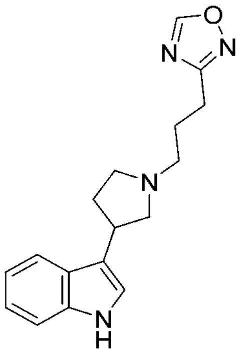 | 50 | 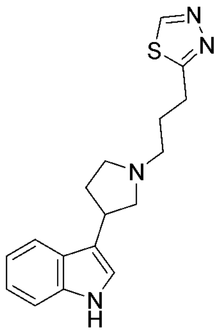 | 51 | 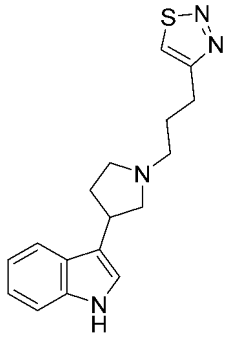 |
| 52 | 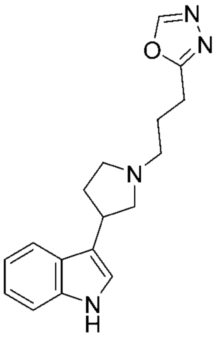 | 53 | 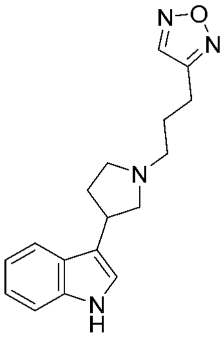 | 54 | 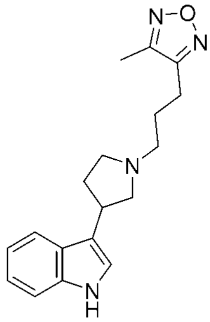 |
| 55 | 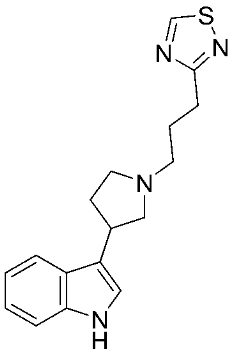 | 56 | 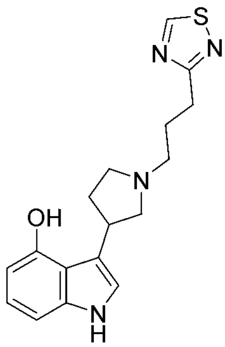 | 57 | 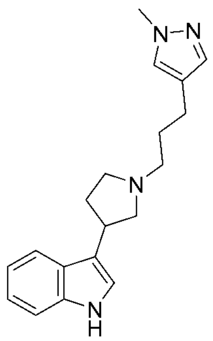 |
| 58 | 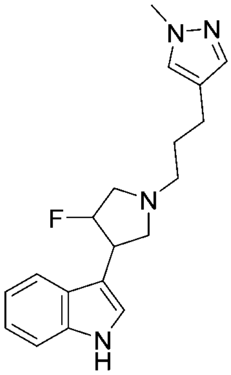 | 59 | 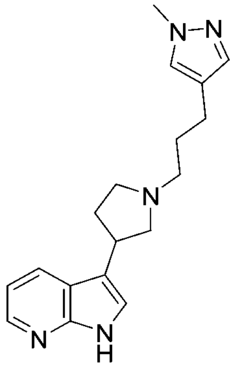 | 60 | 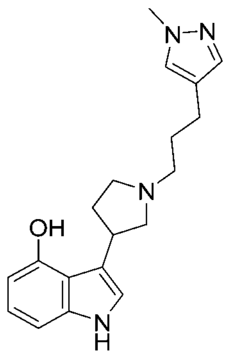 |

(continued)

| No. | Structure | No. | Structure | No. | Structure |
|---|---|---|---|---|---|
| 61 | 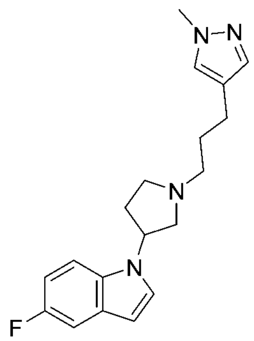 | 62 | 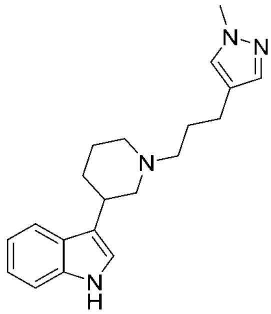 | 63 | 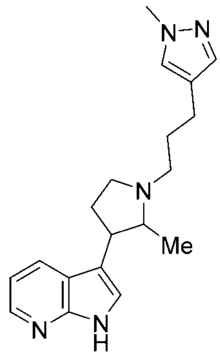 |
| 64 | 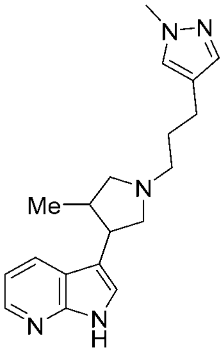 | 65 | 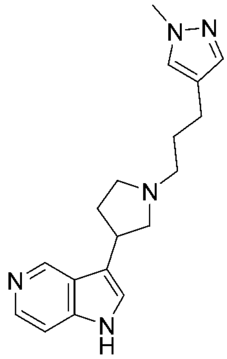 | 66 | 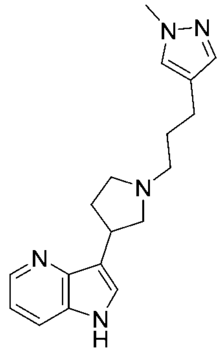 |
| 67 | 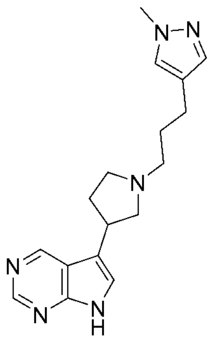 | 68 | 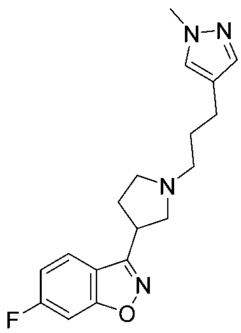 | 69 | 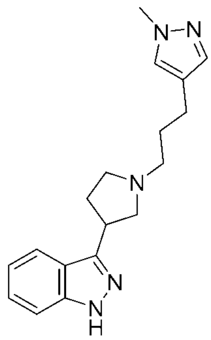 |
| 70 | 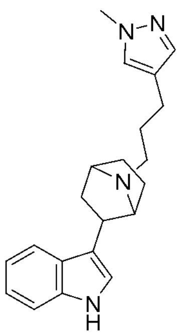 | 71 | 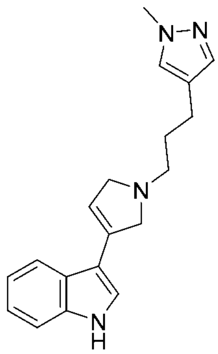 | 72 | 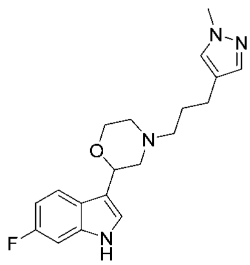 |

(continued)

| No. | Structure | No. | Structure | No. | Structure |
|---|---|---|---|---|---|
| 73 | 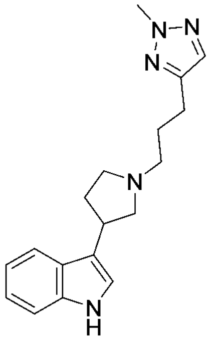 | 74 | 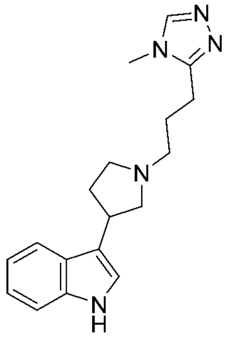 | 75 | 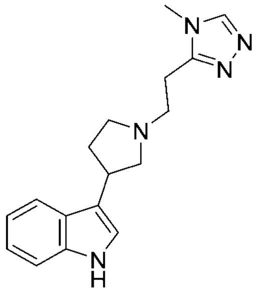 |
| 76 | 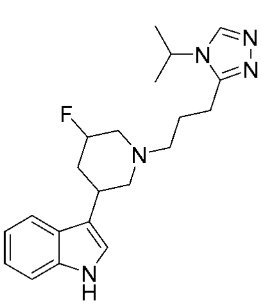 | 77 | 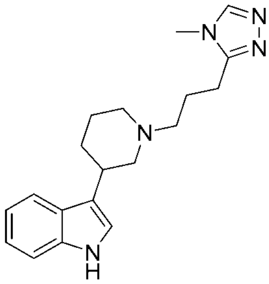 | 78 | 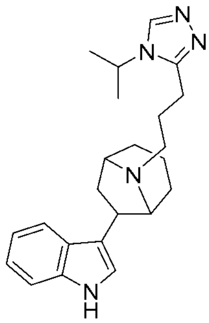 |
| 79 | 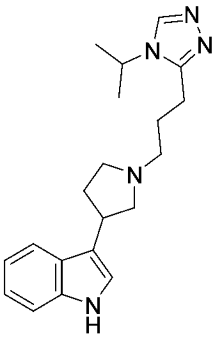 | 80 | 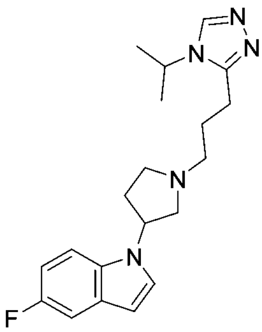 | 81 | 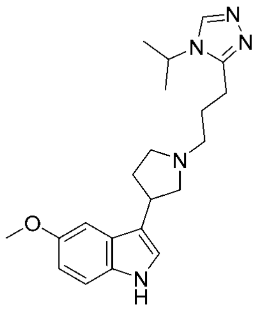 |
| 82 | 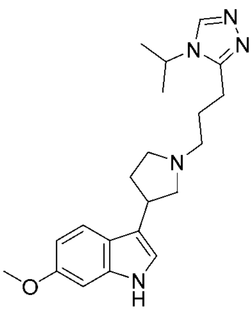 | 83 | 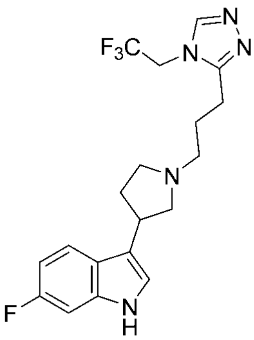 | 84 | 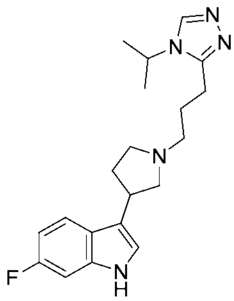 |

(continued)

| No. | Structure | No. | Structure | No. | Structure |
|---|---|---|---|---|---|
| 85 | 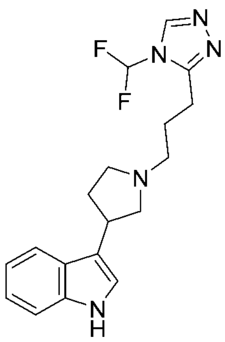 | 86 | 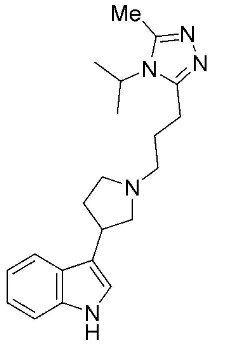 | 87 | 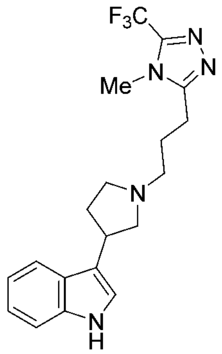 |
| 88 | 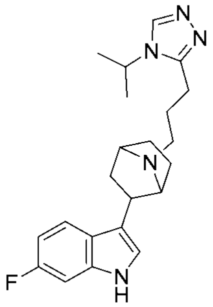 | 89 | 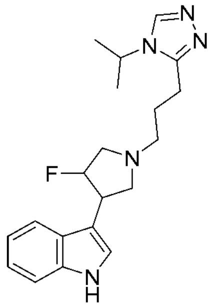 | 90 | 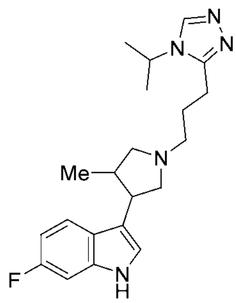 |
| 91 | 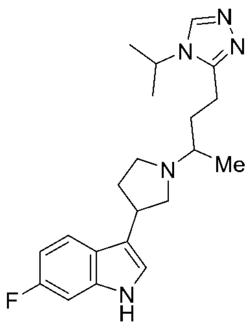 | 92 | 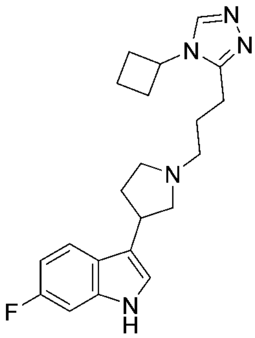 | 93 | 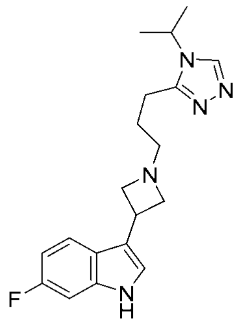 |
| 94 | 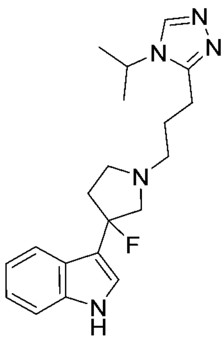 | 95 | 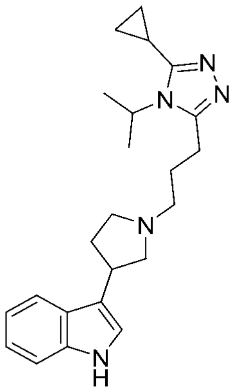 | 96 | 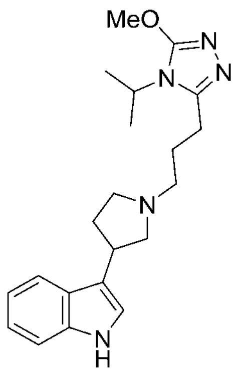 |

(continued)

| No. | Structure | No. | Structure | No. | Structure |
|---|---|---|---|---|---|
| 97 | 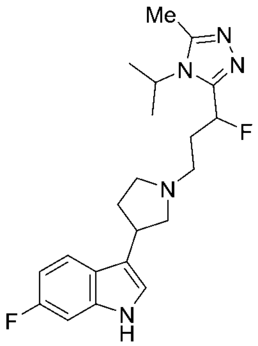 | 98 | 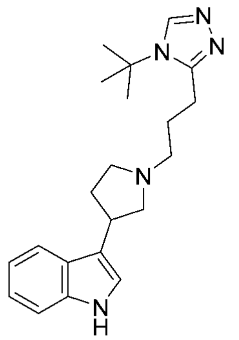 | 99 | 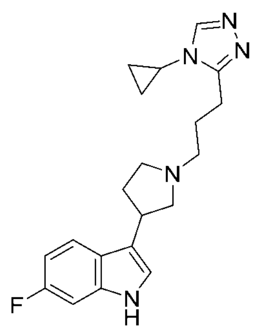 |
| 100 | 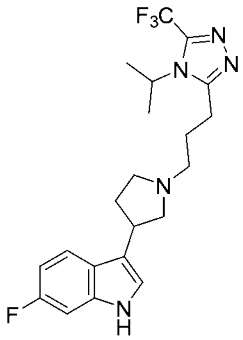 | 101 | 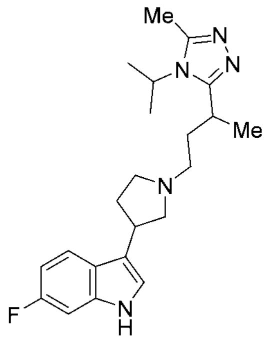 | 102 | 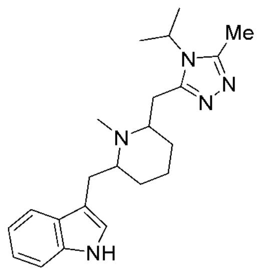 |
| 103 | 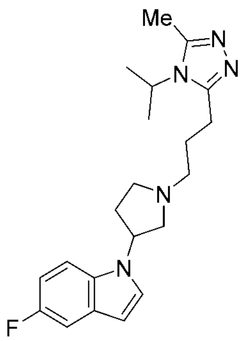 | 104 | 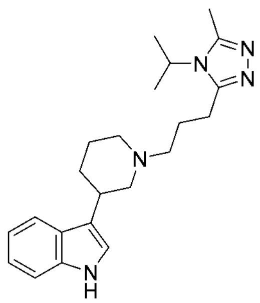 | 105 | 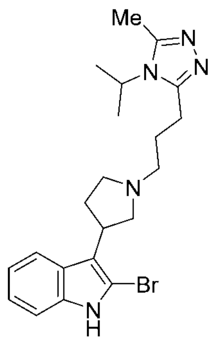 |
| 106 | 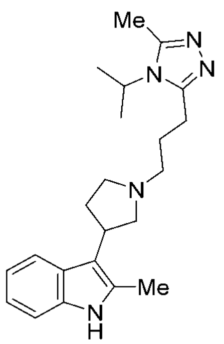 | 107 | 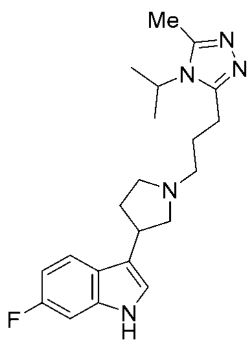 | 108 | 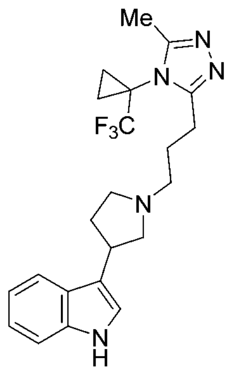 |

(continued)

| No. | Structure | No. | Structure | No. | Structure |
|---|---|---|---|---|---|
| 109 | 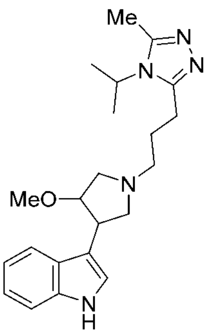 | 110 | 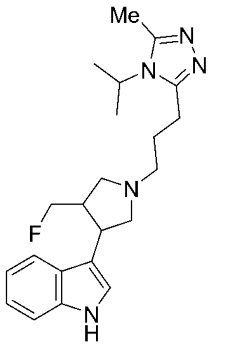 | 111 | 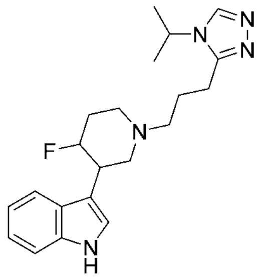 |
| 112 | 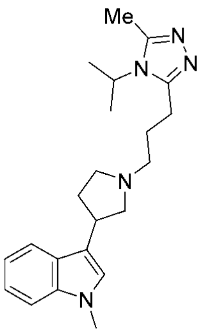 | 113 | 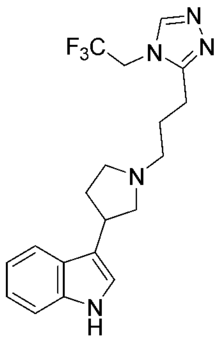 | 114 | 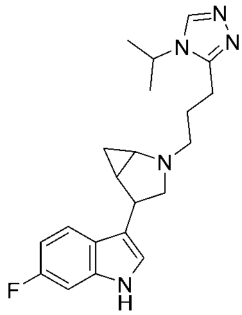 |
| 115 | 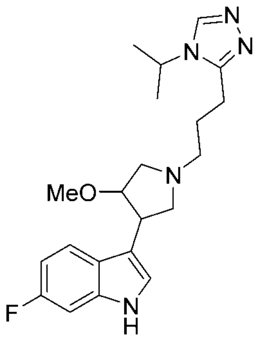 | 116 | 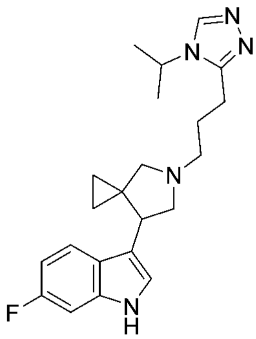 | 117 | 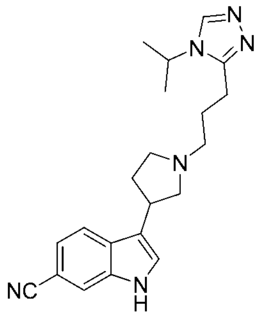 |
| 118 | 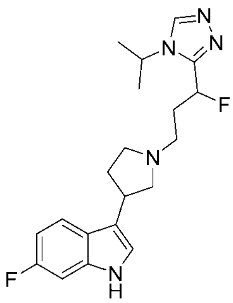 | 119 | 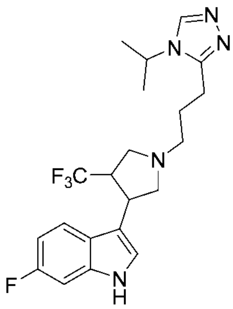 | 120 | 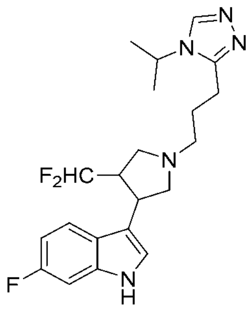 |

(continued)

| No. | Structure | No. | Structure | No. | Structure |
|---|---|---|---|---|---|
| 121 | 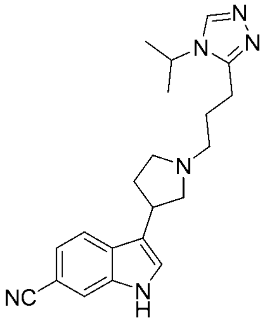 | 122 | 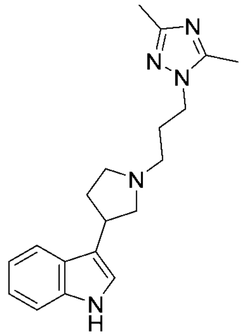 | 123 | 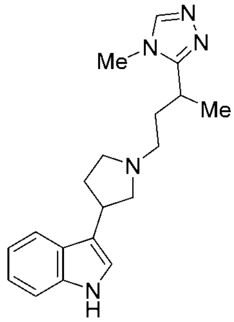 |
| 124 | 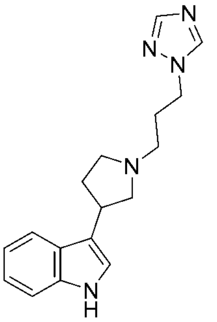 | 125 | 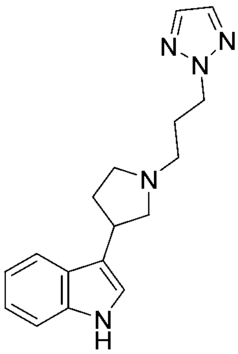 | 126 | 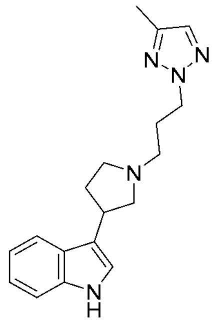 |
| 127 | 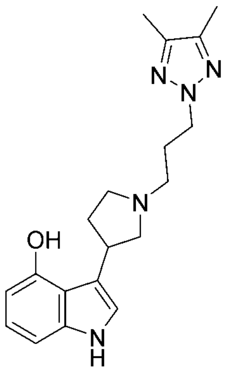 | 128 | 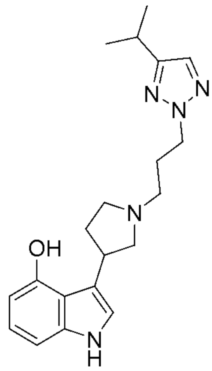 | 129 | 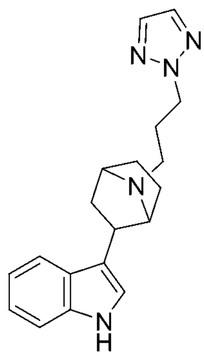 |
| 130 | 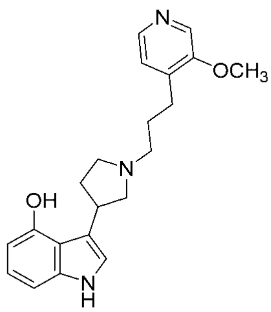 | 131 | 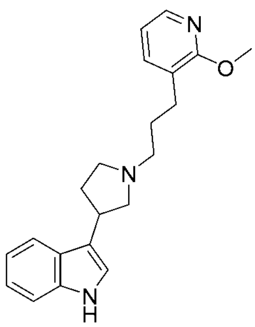 | 132 | 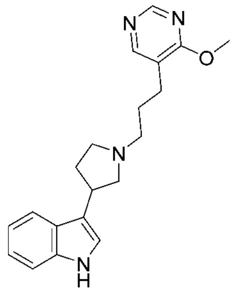 |

(continued)

| No. | Structure | No. | Structure | No. | Structure |
|---|---|---|---|---|---|
| 133 | 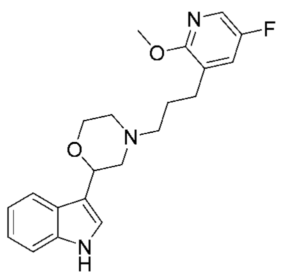 | 134 | 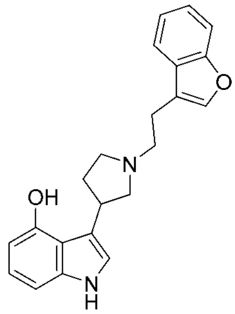 | 135 | 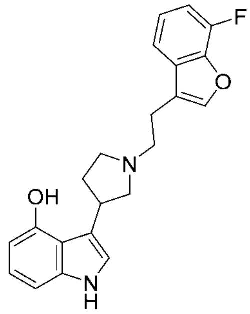 |
| 136 | 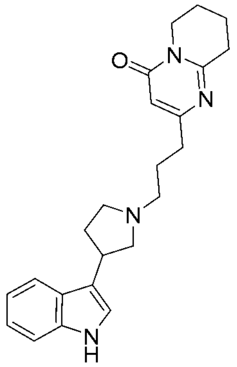 | 137 | 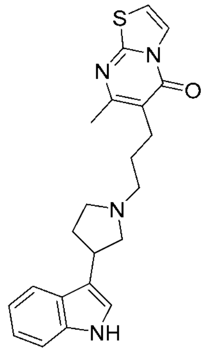 | 138 | 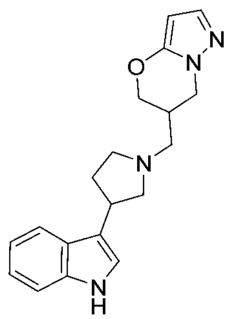 |
| 139 | 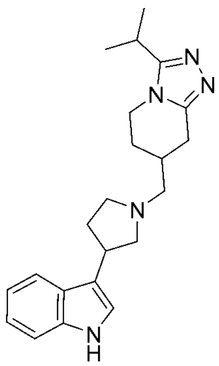 | 140 | 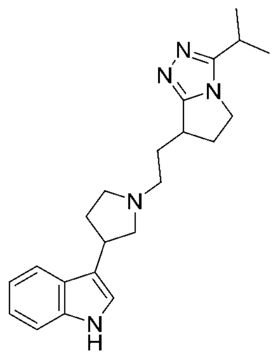 | 141 | 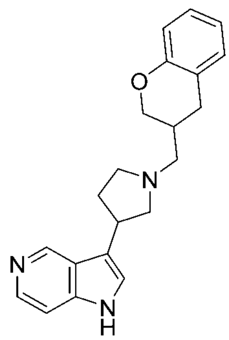 |
| 142 | 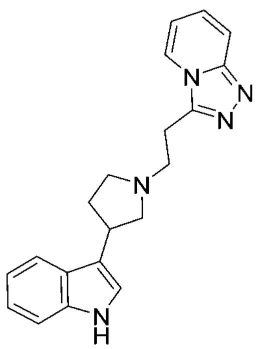 | 143 | 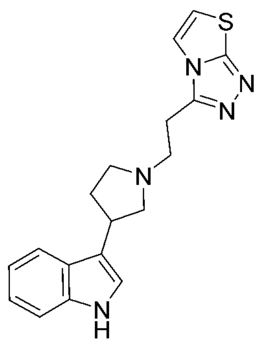 | 144 | 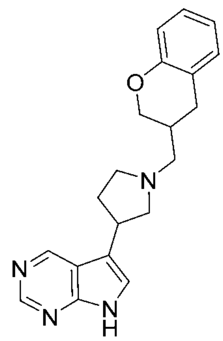 |

(continued)

| No. | Structure | No. | Structure | No. | Structure |
|---|---|---|---|---|---|
| 145 | 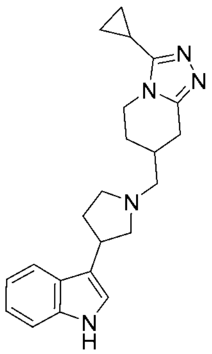 | 146 | 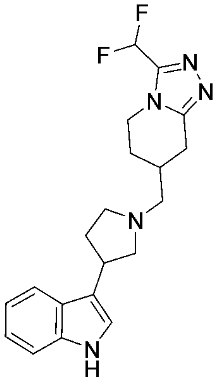 | 147 | 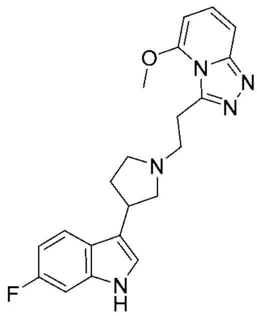 |
| 148 | 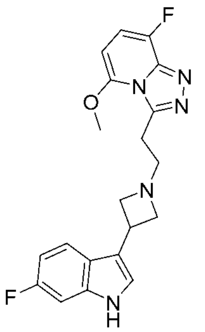 | 149 | 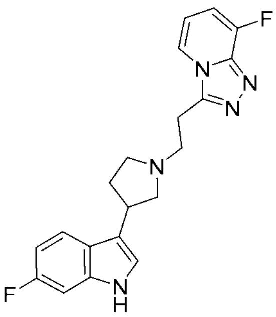 | 150 | 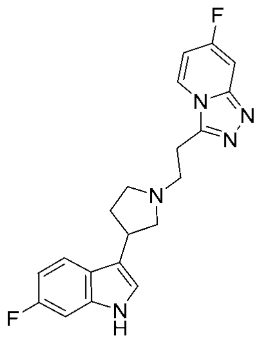 |
| 151 | 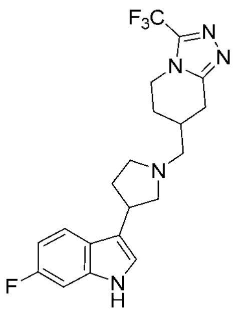 | 152 | 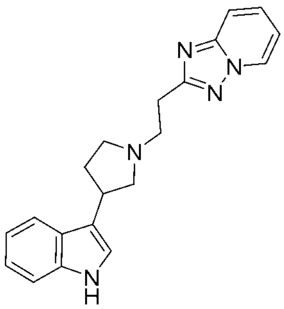 | 153 | 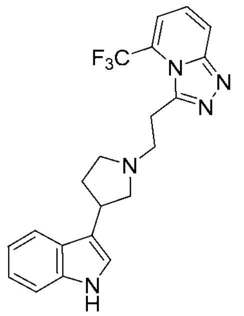 |
| 154 | 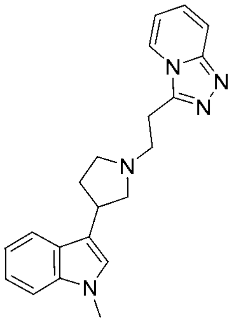 | 155 | 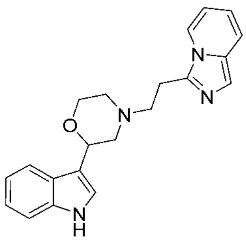 | 156 | 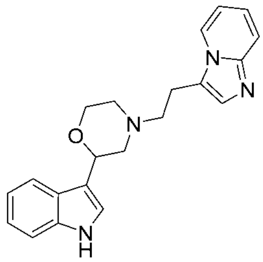 |
| 157 | 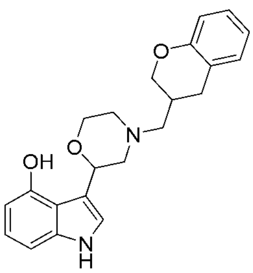 | 158 | 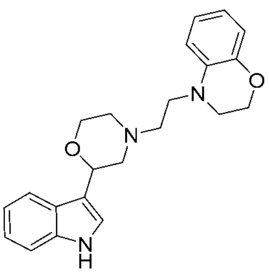 | 159 | 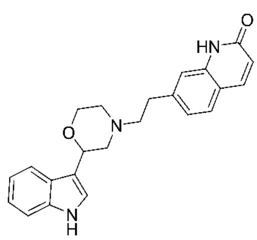 |

(continued)

| No. | Structure | No. | Structure | No. | Structure |
|---|---|---|---|---|---|
| 160 | 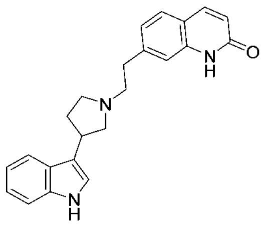 | 161 | 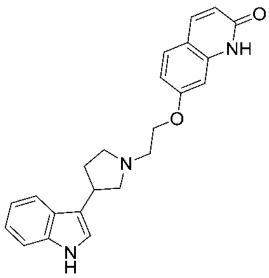 | 162 | 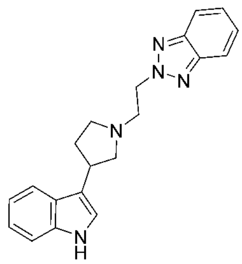 |
| 163 | 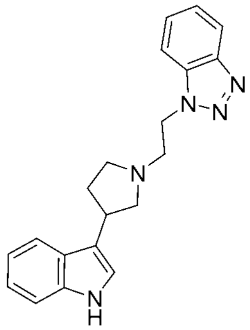 | 164 | 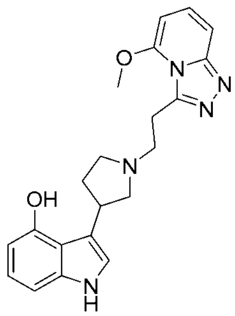 | 165 | 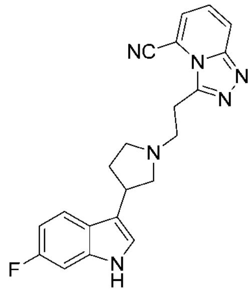 |
| 166 | 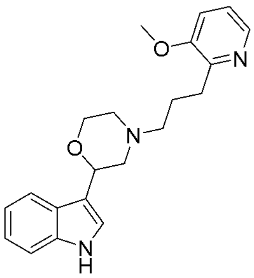 | 167 | 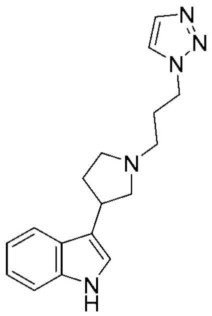 | 168 | 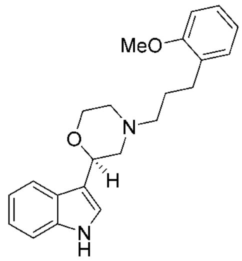 |
| 169 | 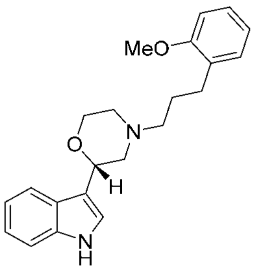 | 170 | 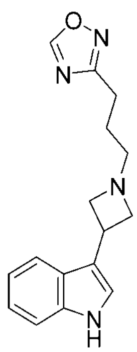 | 171 | 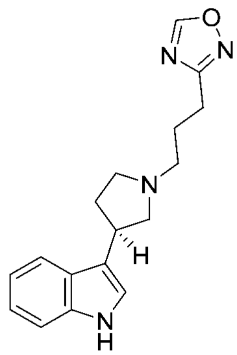 |
| 172 | 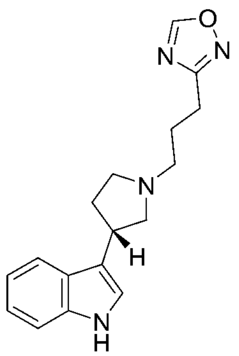 | 173 | 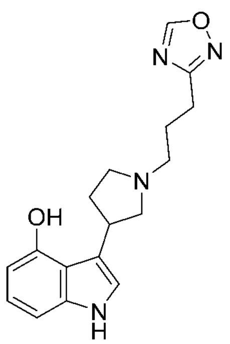 | 174 | 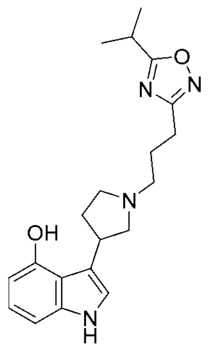 |

(continued)

| No. | Structure | No. | Structure | No. | Structure |
|---|---|---|---|---|---|
| 175 | 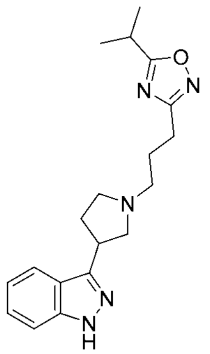 | 176 | 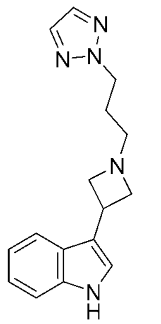 | 177 | 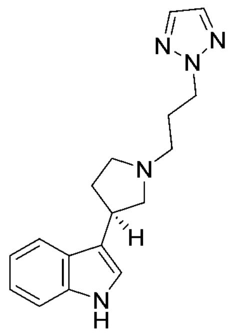 |
| 178 | 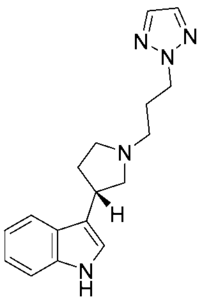 | 179 | 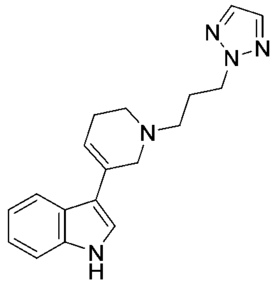 | 180 | 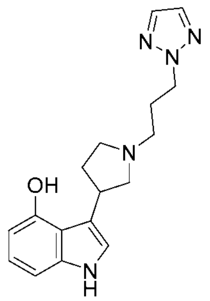 |
| 181 | 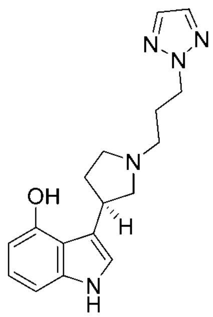 | 182 | 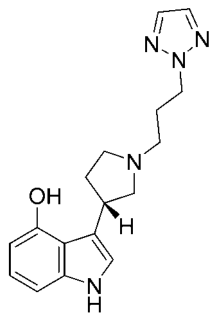 | 183 | 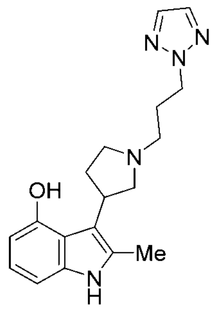 |
| 184 | 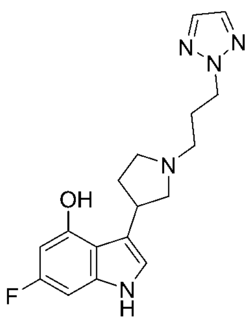 | 185 | 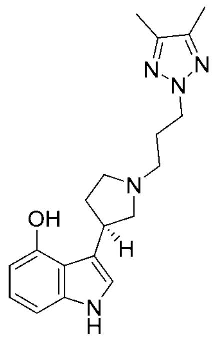 | 186 | 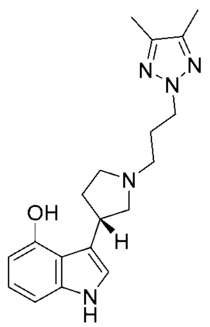 |

(continued)

| No. | Structure | No. | Structure | No. | Structure |
|---|---|---|---|---|---|
| 187 | 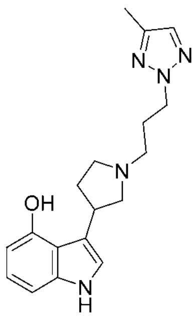 | 188 | 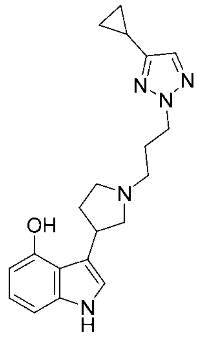 | 189 | 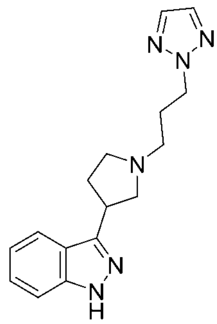 . |

**15.** A pharmaceutical composition, which contains the compound according to any one of claims 1-14, or a stereoisomer thereof, a pharmaceutically acceptable salt thereof, or a mixture thereof.

**16.** Use of the compound according to any one of claims 1-14, or a stereoisomer thereof, a pharmaceutically acceptable salt thereof, or a mixture thereof, or the pharmaceutical composition according to claim 15 in manufacture of a medicament as 5-$HT_{2A}$ receptor agonist for treating central nervous system disorder, preferably said central nervous system disorder is depression.

**17.** The compound according to any one of claims 1-14, or a stereoisomer thereof, a pharmaceutically acceptable salt thereof, or a mixture thereof, or the pharmaceutical composition according to claim 15, for use in being as 5-$HT_{2A}$ receptor agonist to treat central nervous system disorder, preferably said central nervous system disorder is depression.

**18.** A method of treating central nervous system disorder, which comprises administrating a therapeutically effective amount of the compound according to any one of claims 1-14, or a stereoisomer thereof, a pharmaceutically acceptable salt thereof, or a mixture thereof, or the pharmaceutical composition according to claim 15, to a subject in need thereof, preferably said central nervous system disorder is depression.

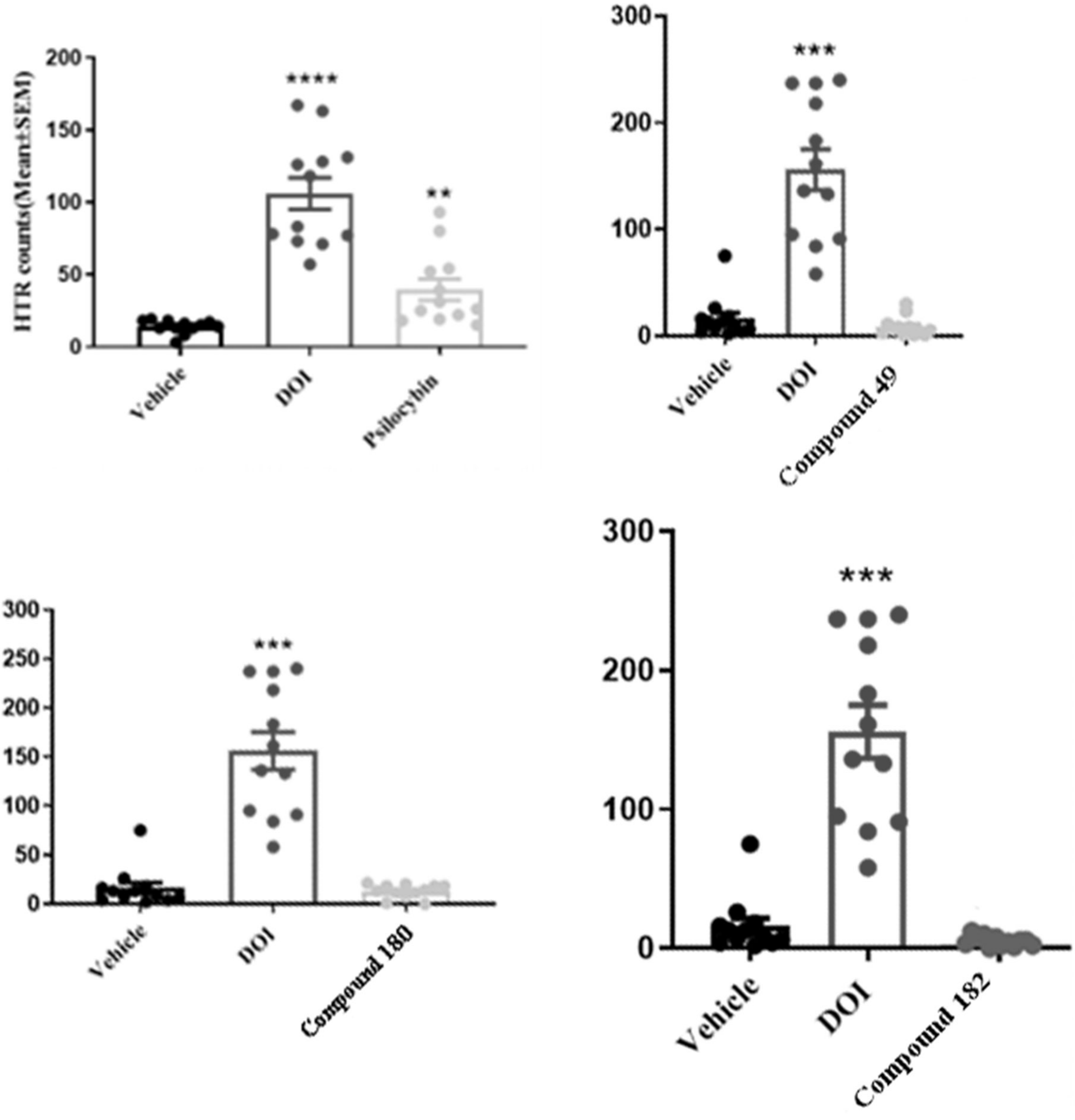


*p<0.05,**p<0.01,***p<0.005, ****p<0.001

Figure 1

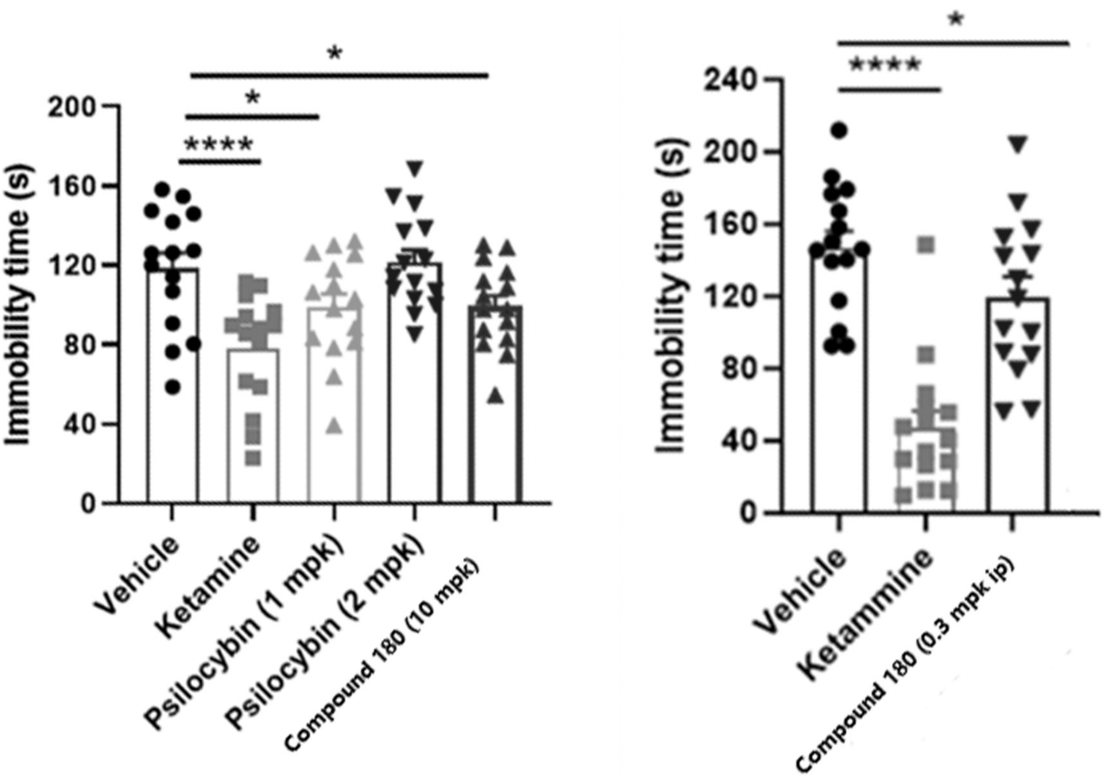


*p＜0.05,**p＜0.01,***p＜0.005, ****p＜0.001

Figure 2

**TRANSLATION**

## INTERNATIONAL SEARCH REPORT

International application No.
**PCT/CN2024/107334**

**A. CLASSIFICATION OF SUBJECT MATTER**

C07D 403/04(2006.01)i； C07D 403/14(2006.01)i； A61K 31/41(2006.01)i； A61P 25/00(2006.01)i

According to International Patent Classification (IPC) or to both national classification and IPC

**B. FIELDS SEARCHED**

Minimum documentation searched (classification system followed by classification symbols)

IPC：C07D；A61K；A61P

Documentation searched other than minimum documentation to the extent that such documents are included in the fields searched

Electronic data base consulted during the international search (name of data base and, where practicable, search terms used)

CNTXT, ENTXTC, DWPI, CNKI, STN(CAPLUS, REGISTRY): 吲哚, 5-HT2A受体激动剂, Indole, 5-HT2A receptor agonist, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | Registry. "RN 2921532-23-0 etc." *STN,* 21 April 2023 (2023-04-21), 1-44<br>pages 1-44, entire document | 1-12 |
| X | US 2004122001 A1 (ELI LILLY AND COMPANY et al.) 24 June 2004 (2004-06-24)<br>claims 1-28, and description, paragraphs [0203-0205], and embodiment 33 | 1-12, 15, 18 |
| X | US 5929072 A (ELI LILLY AND COMPANY LIMITED) 27 July 1999 (1999-07-27)<br>claims 1-8, and description, embodiment 15 | 1-12, 15-18 |
| X | CN 1121348 A (PFIZER RESEARCH AND DEVELOPMENT COMPANY) 24 April 1996 (1996-04-24)<br>description, embodiment 108 | 1-10, 12 |
| X | US 3109844 A (BRISTOL MYERS COMPANY) 05 November 1963 (1963-11-05)<br>description, page 1, lines 60-65, and embodiments 24-25 | 1-12, 15, 18 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 October 2024** | **23 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

TRANSLATION

INTERNATIONAL SEARCH REPORT

International application No.
**PCT/CN2024/107334**

**C. DOCUMENTS CONSIDERED TO BE RELEVANT**

| Category* | Citation of document, with indication, where appropriate, of the relevant passages | Relevant to claim No. |
|---|---|---|
| X | TIMMS, Graham H. et al. "SAR Development of a Selective 5-HT1D Antagonist/Serotonin Reuptake Inhibitor Lead Using Rapid Parallel Synthesis" *Bioorganic & Medicinal Chemistry Letters,* Vol. 14, No. (10), 17 May 2004 (2004-05-17), 2469-2472<br>pages 2469-2470, abstract, and table 1 | 1-12, 18 |
| X | WO 2023114472 A1 (ICAHN SCHOOL OF MEDICINE AT MOUNT SINAI et al.) 22 June 2023 (2023-06-22)<br>claims 1-30, and description, pages 186-242, and tables 1-4 | 1-18 |
| A | WO 2022067165 A1 (YALE UNIVERSITY et al.) 31 March 2022 (2022-03-31)<br>claims 1-31 | 1-18 |
| A | US 6166040 A (ELI LILLY AND COMPANY LIMITED) 26 December 2000 (2000-12-26)<br>claims 1-8 | 1-18 |
| A | WO 9727186 A1 (MERCK PATENT GESELLSCHAFT MIT BESCHRANKTER HAFTUNG et al.) 31 July 1997 (1997-07-31)<br>claims 1-10 | 1-18 |

Form PCT/ISA/210 (second sheet) (July 2022)

**TRANSLATION**

**INTERNATIONAL SEARCH REPORT**

International application No.
**PCT/CN2024/107334**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **18**
because they relate to subject matter not required to be searched by this Authority, namely:

Claim 18 relates to a method for treating a disease, and the search is performed on the basis of the use of the compound for preparing a drug for treating a corresponding disease.

2. ☑ Claims Nos.: **1-14**
because they relate to parts of the international application that do not comply with the prescribed requirements to such an extent that no meaningful international search can be carried out, specifically:

The scope of the structure defined in claims 1-14 is very broad, and related compounds cover a large number of known compounds in the prior art, which make it difficult for the examiner to carry out an exhaustive search of the prior art with regard to the current scope of the claims. The present search report is based on the technical solution of the following structure of fomula (I) involved in most of the compounds in the embodiments: "X $_1$ is selected from CR $_{X1}$; X $_2$ is selected from CR $_{X2}$; X $_3$ is selected from CR $_{X3}$; X $_4$ is selected from CR $_{X4}$; Y $_1$ is selected from NR $_{Y11}$; Y $_2$ is selected from CR $_{Y22}$; Y $_3$ is selected from C; there is a single bond between Y $_1$ and Y $_2$, and there is a double bond between Y $_2$ and Y $_3$; L is -Z-L'-; L' is

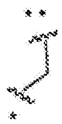, 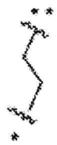, 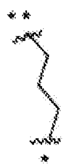, 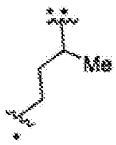, 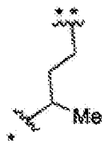, 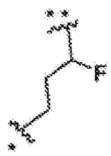

; ** is a B-ring linkage site; * is a Z-ring linkage site; Z is optionally:

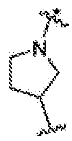,

which is substituted by a $R_A$; and * is connected to an L'-terminus".

3. ☐ Claims Nos.:
because they are dependent claims and are not drafted in accordance with the second and third sentences of Rule 6.4(a).

TRANSLATION

**INTERNATIONAL SEARCH REPORT**
**Information on patent family members**

International application No.
**PCT/CN2024/107334**

| Patent document cited in search report | | | Publication date (day/month/year) | Patent family member(s) | | | Publication date (day/month/year) |
|---|---|---|---|---|---|---|---|
| US | 2004122001 | A1 | 24 June 2004 | WO | 0250067 | A2 | 27 June 2002 |
| | | | | WO | 0250067 | A3 | 10 October 2002 |
| | | | | GB | 0031084 | D0 | 31 January 2001 |
| | | | | GB | 2370270 | A | 26 June 2002 |
| | | | | AU | 3246802 | A | 01 July 2002 |
| | | | | EP | 1345930 | A2 | 24 September 2003 |
| US | 5929072 | A | 27 July 1999 | WO | 9910346 | A1 | 04 March 1999 |
| | | | | CA | 2245331 | A1 | 22 February 1999 |
| | | | | EP | 0897921 | A1 | 24 February 1999 |
| | | | | AU | 8743098 | A | 16 March 1999 |
| | | | | JPH | 11116572 | A | 27 April 1999 |
| CN | 1121348 | A | 24 April 1996 | WO | 9424127 | A1 | 27 October 1994 |
| | | | | DK | 0695301 | T3 | 09 December 1996 |
| | | | | FI | 954944 | A | 17 October 1995 |
| | | | | IL | 109337 | A0 | 31 July 1994 |
| | | | | CZ | 274595 | A3 | 13 March 1996 |
| | | | | ATE | 144773 | T1 | 15 November 1996 |
| | | | | PL | 311204 | A1 | 05 February 1996 |
| | | | | BR | 9406481 | A | 09 January 1996 |
| | | | | NZ | 265269 | A | 25 September 1996 |
| | | | | KR | 960701865 | A | 28 March 1996 |
| | | | | GR | 3021804 | T3 | 28 February 1997 |
| | | | | JPH | 08507083 | A | 30 July 1996 |
| | | | | JP | 2802169 | B2 | 24 September 1998 |
| | | | | CA | 2157397 | A1 | 27 October 1994 |
| | | | | CA | 2157397 | C | 06 July 1999 |
| | | | | ES | 2094653 | T3 | 16 January 1997 |
| | | | | DE | 69400824 | D1 | 05 December 1996 |
| | | | | DE | 69400824 | T2 | 13 March 1997 |
| | | | | EP | 0695301 | A1 | 07 February 1996 |
| | | | | EP | 0695301 | B1 | 30 October 1996 |
| | | | | HU | 9501920 | D0 | 28 September 1995 |
| | | | | HUT | 73807 | A | 30 September 1996 |
| | | | | NO | 954168 | L | 19 October 1995 |
| | | | | AU | 6567094 | A | 08 November 1994 |
| | | | | US | 5607960 | A | 04 March 1997 |
| US | 3109844 | A | 05 November 1963 | FR | 910 | M | 06 November 1961 |
| | | | | ES | 260933 | A1 | 01 March 1961 |
| WO | 2023114472 | A1 | 22 June 2023 | AU | 2022409512 | A1 | 01 August 2024 |
| | | | | CA | 3241217 | A1 | 22 June 2023 |
| WO | 2022067165 | A1 | 31 March 2022 | EP | 4216949 | A1 | 02 August 2023 |
| | | | | CN | 116546984 | A | 04 August 2023 |
| | | | | US | 2023365542 | A1 | 16 November 2023 |
| US | 6166040 | A | 26 December 2000 | PE | 20000467 | A1 | 05 June 2000 |
| | | | | AR | 018859 | A1 | 12 December 2001 |
| | | | | JP | 2002514642 | A | 21 May 2002 |
| | | | | GB | 9810886 | D0 | 22 July 1998 |
| | | | | EP | 0963983 | A1 | 15 December 1999 |
| | | | | AU | 3838499 | A | 29 November 1999 |

Form PCT/ISA/210 (patent family annex) (July 2022)

TRANSLATION

**INTERNATIONAL SEARCH REPORT**
**Information on patent family members**

International application No.
**PCT/CN2024/107334**

| Patent document cited in search report | Publication date (day/month/year) | Patent family member(s) | Publication date (day/month/year) |
|---|---|---|---|
| | | CA 2330796 A1 | 18 November 1999 |
| | | WO 9958525 A1 | 18 November 1999 |
| | | SV 1999000059 A | 04 July 2000 |
| | | CO 5031250 A1 | 27 April 2001 |
| WO 9727186 A1 | 31 July 1997 | HUP 9900980 A2 | 28 July 1999 |
| | | BR 9707467 A | 20 July 1999 |
| | | AR 005549 A1 | 23 June 1999 |
| | | JP 2000503972 A | 04 April 2000 |
| | | ZA 97572 B | 04 August 1997 |
| | | DE 19602505 A1 | 31 July 1997 |
| | | CA 2244136 A1 | 31 July 1997 |
| | | KR 19990081970 A | 15 November 1999 |
| | | NO 983439 D0 | 24 July 1998 |
| | | NO 983439 L | 25 September 1998 |
| | | CZ 231898 A3 | 14 October 1998 |
| | | AU 1443297 A | 20 August 1997 |
| | | AU 715785 B2 | 10 February 2000 |
| | | SK 99298 A3 | 11 January 1999 |
| | | EP 0879234 A1 | 25 November 1998 |
| | | PL 328228 A1 | 18 January 1999 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Pharmaceutical Salts and Co-crystals. RSC Publishing, 2012